(19) Europäisches Patentamt
European Patent Office
Office européen des brevets

(11) EP 3 328 375 B1

(12) EUROPEAN PATENT SPECIFICATION

(45) Date of publication and mention of the grant of the patent:
**31.08.2022 Bulletin 2022/35**

(21) Application number: **16833638.6**

(22) Date of filing: **29.07.2016**

(51) International Patent Classification (IPC):
***A61K 31/22*** *(2006.01)* ***A61K 31/225*** *(2006.01)*
***A61K 31/4045*** *(2006.01)* ***A61K 31/421*** *(2006.01)*
***A61K 31/66*** *(2006.01)* ***A61K 31/7076*** *(2006.01)*
***A61K 39/395*** *(2006.01)* ***A61K 45/06*** *(2006.01)*
***A61P 35/00*** *(2006.01)*

(52) Cooperative Patent Classification (CPC):
(C-Sets available)
**A61K 31/4045; A61K 31/22; A61K 31/225; A61K 31/42; A61K 31/655; A61K 31/664; A61K 31/683; A61K 31/685; A61K 31/7034; A61K 31/7076; A61K 35/17; A61K 39/39558; A61K 45/06; A61P 35/00;** Y02A 50/30 (Cont.)

(86) International application number:
**PCT/US2016/044829**

(87) International publication number:
**WO 2017/023793 (09.02.2017 Gazette 2017/06)**

(54) **METHODS FOR CANCER AND IMMUNOTHERAPY USING GLUTAMINE ANALOGUES, INCLUDING DON**

VERFAHREN FÜR KREBS UND IMMUNTHERAPIE MIT GLUTAMINANALOGA, EINSCHLIESSLICH DON

PROCÉDÉS POUR LE CANCER ET L'IMMUNOTHÉRAPIE AU MOYEN DES ANALOGUES DE GLUTAMINE, COMPRENANT LE DÉSOXYNIVALÉNOL (DON)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2015 US 201562199381 P**

(43) Date of publication of application:
**06.06.2018 Bulletin 2018/23**

(73) Proprietor: **The Johns Hopkins University**
**Baltimore, MD 21218 (US)**

(72) Inventors:
- **SLUSHER, Barbara**
  **Kingsville, Maryland 21087 (US)**
- **POWELL, Jonathan**
  **Baltimore, Maryland 21215 (US)**
- **ENGLERT, Judson**
  **Baltimore, Maryland 21209 (US)**
- **LEONE, Robert**
  **Lutherville, Maryland 21093 (US)**
- **TENORA, Lukas**
  **Praha 166 10 (CZ)**
- **MAJER, Pavel**
  **Sykesville, MD 21784 (US)**
- **JANCARIK, Andrej**
  **Koprivnice 74221 (CZ)**

(74) Representative: **Schlich, George et al**
**Schlich**
**9 St Catherine's Road**
**Littlehampton, West Sussex BN17 5HS (GB)**

(56) References cited:

| | |
|---|---|
| **EP-A2- 0 123 170** | **WO-A1-2014/138391** |
| **WO-A1-2014/160071** | **WO-A2-2004/113363** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ALAN G. RAMSAY: "Immune checkpoint blockade immunotherapy to activate anti-tumour T-cell immunity", BRITISH JOURNAL OF HAEMATOLOGY, vol. 162, no. 3, 21 May 2013 (2013-05-21), pages 313-325, XP055336790, GB ISSN: 0007-1048, DOI: 10.1111/bjh.12380**
- **THIBAUD T. RENAULT ET AL: "Getting away with murder: how does the BCL-2 family of proteins kill with immunity? : The BCL-2 family as regulators of immunity", ANNALS OF THE NEW YORK ACADEMY OF SCIENCES, vol. 1285, no. 1, 25 March 2013 (2013-03-25), pages 59-79, XP055547396, US ISSN: 0077-8923, DOI: 10.1111/nyas.12045**
- **ANDREJ JANCARIK: "Novel lymphoid targeted prodrugs of the glutamine antagonist DON for the treatment of hematological malignancies", FASEB, 2 April 2016 (2016-04-02), pages 1-2, XP055545018, DOI: 10.1096/fasebj.30**
- **JUDSON ENGLERT ET AL: "Abstract 1035: Targeting glutamine metabolism with the novel inhibitor JHU-083 inhibits tumor growth and alters the tumor immune microenvironment", PROCEEDINGS: AACR 107TH ANNUAL MEETING, 16 April 2016 (2016-04-16), pages 1-2, XP055545012,**


(52) Cooperative Patent Classification (CPC): (Cont.)

C-Sets
**A61K 31/42, A61K 2300/00;**
**A61K 31/655, A61K 2300/00;**
**A61K 31/664, A61K 2300/00;**
**A61K 31/683, A61K 2300/00;**
**A61K 31/685, A61K 2300/00;**
**A61K 31/7034, A61K 2300/00;**
**A61K 31/7076, A61K 2300/00;**
**A61K 39/39558, A61K 2300/00**

## Description

BACKGROUND

Cells under certain conditions may undergo a metabolic switch from a metabolic profile that requires less activity of certain metabolic pathways to meet the cell's energy demands to a metabolic profile that requires greater activity of those metabolic pathways or increased activity of other metabolic pathways to meet its energy demands. For example, cells under certain conditions may undergo a switch toward increased glycolysis and away from oxidative phosphorylation (OXPHOS). While glycolysis provides less adenosine triphosphate (ATP) than oxidative phosphorylation, it has been proposed that aerobic glycolysis permits the generation of the substrates necessary for the generation of amino acids, nucleic acids and lipids, all of which are crucial for proliferation (Vander Heiden et al. (2009) Science 324(5930):1029-1033). This use of glycolysis in the presence of oxygen was first described by Otto Warburg in cancer cells (Warburg (1956) Science 124 (3215):269-270) and was subsequently found to be important in activated T cells (Warburg et al. (1958) [Metabolism of leukocytes]. Zeitschriftfur Naturforschung. Teil B: Chemie, Biochemie, Biophysik, Biologie 13B (8):515-516). These metabolically reprogrammed cells depend on the increased activity of certain metabolic pathways, such as pathways involved in glutamine metabolism, glycolysis, and fatty acid synthesis. However, specific inhibitors of individual enzymes in these metabolic pathways alone have not proven effective because multiple points within each metabolic pathway are modulated as a cell's metabolism is reprogrammed to meet the extraordinarily large energy demands of the abnormal, harmful, or unhealthy state.

WO 2014/160071, in the name of St. Jude Children's Research Hospital describes glutaminase inhibitors such as DON, azaserine, or acivicin in combination with at least one pro-apoptotic Bcl-2 inhibitor in the treatment of cancer. WO 2014/148391, in the name of The Johns Hopkins University, describes glutamine metabolism inhibitors such as DON, acivicin or azaserine for use in the treatment of cancer. EP 0123170, in the name of Amiercan Cyanamid Co, describes a compound having a similar sructre to that of DON for use n the treatment of leukaemia and tunours. WO 2004/113363 desribes the derivatives of transglutaminase inhibitor DON obtained by reacting a N-alpha-protected amino acid with 6-diazo-5-oxo-L-norleucine. Such derivatives do not include a diazo group. Alan Ramsay, in "Immune checkpoint blockade immunotherapy to activate anti-tumour T-cell immunity", British Journal of Haematology, vol. 162, no. 3, 21 May 2013, pages 313 - 325, discloses the use of ummunotherapy in the treatment of cancer and proposed its use in combination with additional targeted agents. This document does not suggest a combination with a glutaminase inhibitor.

SUMMARY

The practice of the present invention will typically employ, unless otherwise indicated, conventional techniques of cell biology, cell culture, molecular biology, transgenic biology, microbiology, recombinant nucleic acid (e.g., DNA) technology, immunology, and RNA interference (RNAi) which are within the skill of the art. Non-limiting descriptions of certain of these techniques are found in the following publications: Ausubel, F., et al., (eds.), Current Protocols in Molecular Biology, Current Protocols in Immunology, Current Protocols in Protein Science, and Current Protocols in Cell Biology, all John Wiley & Sons, N.Y., edition as of December 2008; Sambrook, Russell, and Sambrook, Molecular Cloning. A Laboratory Manual, 3rd ed., Cold Spring Harbor Laboratory Press, Cold Spring Harbor, 2001; Harlow, E. and Lane, D., Antibodies-A Laboratory Manual, Cold Spring Harbor Laboratory Press, Cold Spring Harbor, 1988; Freshney, R. I., "Culture of Animal Cells, A Manual of Basic Technique", 5th ed., John Wiley & Sons, Hoboken, N.J., 2005. Non-limiting information regarding therapeutic agents and human diseases is found in Goodman and Gilman's The Pharmacological Basis of Therapeutics, 11th Ed., McGraw Hill, 2005, Katzung, B. (ed.) Basic and Clinical Pharmacology, McGraw-Hill/Appleton & Lange 10th ed. (2006) or 11th edition (July 2009). Non-limiting information regarding genes and genetic disorders is found in McKusick, V. A.: Mendelian Inheritance in Man. A Catalog of Human Genes and Genetic Disorders. Baltimore: Johns Hopkins University Press, 1998 (12th edition) or the more recent online database: Online Mendelian Inheritance in Man, OMIM™. McKusick-Nathans Institute of Genetic Medicine, Johns Hopkins University (Baltimore, Md.) and National Center for Biotechnology Information, National Library of Medicine (Bethesda, Md.), as of May 1, 2010, World Wide Web URL: http://www.ncbi.nlm.nih.gov/omim/ and in Online Mendelian Inheritance in Animals (OMIA), a database of genes, inherited disorders and traits in animal species (other than human and mouse), at

http://omia.angis.org.au/contact.shtml.

According to the invention there is provided a compound having formula (I):

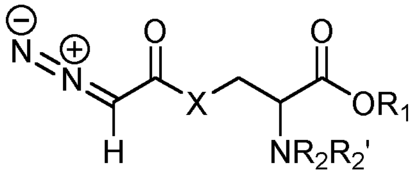

(I);

or a pharmaceutically acceptable salt thereof, wherein:

X is-$(CH_2)_n$-;
n is 1;
$R_1$ is selected from the group consisting of $C_{1-6}$ alkyl and substituted $C_{1-6}$ alkyl;
$R_2$ is selected from the group consisting of an amino acid, an N-substituted amino acid, $-C(=O)-Y-(CR_3R_4)_m-NR_5R_6$ and $-C(=O)-O-(CR_3R_4)_m-O-C(=O)-R_{10}$;
Y is -O- or a bond;
$R_2'$ is selected from the group consisting of H, $C_1$-$C_6$ alkyl, and substituted $C_1$-$C_6$ alkyl;
each $R_3$ and $R_4$ are independently H, $C_1$-$C_6$ alkyl, substituted $C_1$-$C_6$ alkyl, aryl, substituted aryl, $-(CR_3R_4)_m-NR_5R_6$, or

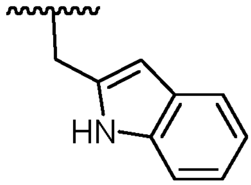

;

m is an integer selected from the group consisting of 1, 2, 3, 4, 5, 6, 7, and 8;
$R_5$ and $R_6$ are independently H or alkyl; and
$R_{10}$ is selected from the group consisting of alkyl, substituted alkyl, cycloalkyl, substituted cycloalkyl, monosaccharide, acylated monosaccharide, aryl, substituted aryl, heteroaryl, and substituted heteroaryl,
for use in treating cancer, wherein the compound having formula (I) is to be administered with immune checkpoint blockade therapy, adoptive cellular therapy, or an adenosine A2aR inhibitor.

In particular embodiments, the immune checkpoint blockade therapy is selected from the group consisting of PD-1 antagonists, PD-L1 antagonists, CTLA-4 antagonists, LAG3 antagonists, B7-H3 antagonists, and combinations thereof.

In particular embodiments, the first immunotherapy, and the second immunotherapy, is administered to the subject in the absence of a cancer therapy selected from the group consisting of: (i) chemotherapy; (ii) photodynamic therapy; (iii) proton therapy; (iv) radiotherapy; (v) surgery; and combinations thereof.

In particular embodiments, the cancer is a newly diagnosed, recurrent, and/or refractory cancer selected from the group consisting of celnasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

In unclaimed aspects, the presently disclosed subject matter provides a method of preventing a relapse in a cancer subject in remission, the method comprising administering to the subject a therapeutically effective amount of a metabolic reprogramming agent, wherein the metabolic reprogramming agent is selected from the group consisting of

acivicin (L-(alpha S, 5S)-alpha-amino-3-chloro-4,5-dihydro-5-isoxazoleacetic acid), azaserine, and 6-diazo-5-oxo-norleucine (DON), and 5-diazo-4-oxo-L-norvaline (L-DONV), and prodrugs thereof.

Certain aspects of the presently disclosed subject matter having been stated hereinabove, which are addressed in whole or in part by the presently disclosed subject matter, other aspects will become evident as the description proceeds when taken in connection with the accompanying Examples and Drawings as best described herein below.

BRIEF DESCRIPTION OF THE DRAWINGS

Having thus described the presently disclosed subject matter in general terms, reference will now be made to the accompanying Figures, which are not necessarily drawn to scale, and wherein:

FIG. 1 illustrates that metabolic reprogramming therapy with at least one metabolic reprogramming agent (e.g., DON) markedly inhibits lymphoma growth in a EL4 mouse lymphoma model, suggesting that bone marrow derived tumors may be exquisitely susceptible to metabolic reprogramming therapy with at least one metabolic reprogramming agent (e.g., DON);

FIG. 2 illustrates that metabolic reprogramming therapy with at least one metabolic reprogramming agent (e.g., DON) has a modest effect on inhibiting melanoma growth, which is a not a bone marrow derived tumor;

FIG. 3 illustrates that metabolic reprogramming therapy with at least one metabolic reprogramming agent (e.g., DON) conditions B 16 melanoma to be killed by immunotherapy by inhibiting tumor infiltrating Regulatory T cells ($Foxp3^+$);

FIG. 4 illustrates structures of DON and DON-based prodrugs;

FIG. 5A and FIG. 5B illustrate that DON (**1**) inhibits glutamine metabolism and GBM tumor growth *in vivo.* FIG. 5A shows compound **1** (0.8 mg/kg, i.p.) inhibited glutamine metabolism as evidenced by increased endogenous glutamine concentrations in flank GBM tumors 2 hours post-administration relative to vehicle-treated controls; *$p < 0.05$. FIG. 5B shows in efficacy studies, compared to Day 0 baseline, vehicle-treated mice exhibited significant growth of flank GBM tumors during the course of the experiment. By contrast, systemic administration of 1 (0.8 mg/kg, i.p, q.d. days 1-6) caused a dramatic reduction in tumor size; ***$p < 0.001$, ****$p < 0.0001$. Note the bold numbers following the terms "DON", "DON prodrugs", "DON-based prodrugs", refer to particular compounds disclosed in Table 1 below.

FIG. 6 illustrates *in vivo* brain and plasma pharmacokinetics of compound DON (1) following oral administration of DON (1) and 5c in mice. 1 and 5c were dosed in mice at 0.8 mg/kg equivalent, via oral gavage and plasma and brain concentrations of compound 1 were evaluated via LC/MS. Oral administration of compound 1 and 5c exhibited similar plasma and brain pharmacokinetic profiles due to complete and rapid metabolism of 5c to 1 in mouse plasma;

FIG. 7 illustrates *in vivo* pharmacokinetics of DON following i.v. administration of DON (1) and 5c in monkey plasma and CSF. 1 and 5c were dosed in two pigtail macaques at 1.6 mg/kg equivalent of 1 via i.v. administration and plasma (0.25-6h) and CSF (30 min) concentrations of DON were evaluated via LC/MS. Relative to 1, 5c delivered substantially lower DON plasma concentration. The reverse was observed in CSF, where 5c delivered significantly higher DON CSF concentrations, achieving a 10-fold enhanced CSF to plasma ratio at 30 minute post dose;

FIG. 8 illustrates that 25 (5 day dosing starting on day 7) is superior to CB-839 (30 day dosing starting day 1) in CT26 tumor model;

FIG. 9 illustrates that **25** (4 days starting on day 6) is superior to CB-839 (continuous twice daily dosing starting on day 1 prior to engraftment) in a CT26 tumor model. FIG. 9 shows mice received daily **25** (1.9 mg/kg) on days 6-9 vs BID glutaminase inhibitor on days 1-15;

FIG. 10 illustrates that **25** (daily days 7-22) is superior to CB-839 (continuous twice daily dosing days 1-29) in a 4T1 breast cancer model. Mice received daily **25** (1.0 mg.kg/d) for days 7-22 as compared to BID glutaminase inhibitor for days 1-29;

FIG. 11 illustrates that **25** dosing of 1 mg/kg following by 0.3 mg/kg leads to a complete and durable response in the MC38 tumor;

FIG. 12 illustrates that **25** gives a robust response and improved overall survival in multiple tumor models including, for example, *CT26 Colon Cancer;*

FIG. 13 illustrates that **25** provides a robust response and improved overall survival in multiple tumor models including, for example, *4T1 breast cancer;*

FIG. 14 illustrates that mice cured with **25** alone immunologically reject tumors upon re-challenge, demonstrating that **25** monotherapy is immunotherapy;

FIG. 15 further illustrates that **25** monotherapy is immunotherapy;

FIG. 16 illustrates that glutamine inhibition (e.g., DON) reduces the oxygen consumption and lactate production of tumor cells;

FIG. 17 illustrates that glutamine inhibition (e.g., DON) improved the CD8/Treg ratio in the tumor and reduces hypoxia

in the TILs;

FIG. 18 illustrates that **25** conditions the tumor to be eliminated by anti-PDl therapy in the MC38 Model, and in particular that **25** rescues anti-PDl failures;

FIG. 19 illustrates that even in the more difficult CT26 model, **25** enhances the response to anti-PDl therapy;

FIG. 20 illustrates that inhibiting glutamine metabolism also potentiates the anti-tumor response to adenosine A2a receptor (A2aR) blockade;

FIG. 21 illustrates that inhibiting glutamine metabolism enhances the efficacy of adoptive cellular therapy (ACT) in a B16-OVA model;

FIG. 22A, FIG. 22B, and FIG. 23C illustrate the in vivo pharmacokinetics of DON following i.v. administration of DON **(1)** and **14** in monkey plasma and cerebrospinal fluid (CSF). **1** and **14** were dosed in two pigtail macaques at 1.6 mg/kg equivalent of 1 via i.v. administration and plasma (0.25-6h) and CSF (30 min) concentrations of DON were evaluated via LC/MS. Relative to **1, 14** delivered substantially lower DON plasma concentration. The reverse was observed in CSF, where **14** delivered significantly higher DON CSF concentrations, achieving a 10-fold enhanced CSF to plasma ratio at 30 minute post dose;

FIG. 23 illustrates species specific plasma stability of **(14); 14** is stable in plasma of human, pig, dog and monkeys, but rapidly metabolized in mice;

FIG. 24 illustrates exemplary structures of DON and DON-based prodrugs **25, 9, 38** and **60;** different N-amino acid promoeities (e,g, leucine, tryptophan) provide differential plasmas and microsomal stability;

FIG. 25A, FIG. 25B, FIG. 25C, and FIG. 25D illustrate the in vitro plasma stability of DON prodrugs **9, 25, 38** and **60.** Metabolism occurs via removal of N-protecting group; both ethyl and isopropyl esters are stable in plasma of pigs and humans;

FIG. 26A, FIG. 26B, FIG. 26C, and FIG. 26D illustrates the in vitro liver microsomal stability of DON prodrugs **9, 25, 38** and **60;** all prodrugs showed moderate-high stability in human and pig microsomes;

FIG. 27A, FIG. 27B, FIG. 27C, FIG. 27D, FIG. 27E, FIG. 27F, FIG. 27G, FIG. 27H, FIG. 271, and FIG. 27J illustrate the results of ex-vivo studies in whole human and pig blood of **9, 25, 38** and **60**; DON prodrugs selectively deliver DON to PBMCs in both humans and pigs vs plasma; compared to DON, the PBMC/plasma ratio was enhanced 10-100+ fold;

FIG. 28A FIG. 28B, FIG. 28C, FIG. 28D and FIG. 28E illustrate the results of pig in vivo studies with DON prodrugs of **9, 25, 38** and **60;** DON prodrugs selectively deliver DON to PBMCs vs plasma; compared to DON, the PBMC/plasma ratio was enhanced 6- to 10-fold;

FIG. 29A, FIG. 29B, and FIG. 29C illustrate the plasma stability of compound Methyl-POM **14** and its derivatives;

FIG. 30 illustrate exemplary structures of N-acylalkyloxy DON-based prodrug analogs for intracellular targeting and brain penetration; the addition of steric bulk to the "bridge" might result in a slower hydrolysis;

FIG. 31 illustrates that anti-PDl monotherapy doesn't work in a 4T1 tumor model. 4T1 tumor cells (0.1 million) were injected into the mammary fat pads of 8-week-old female BALB/c mice. Anti-PDl (5mg/Kg) was administered on day 3, 5, 8, and 11 and tumor volume was measured 2-3 times weekly until tumors were evaluated for tumor infiltrating cells. Group: a-PD1 (d3, 5, 8, 11 100ug/mouse) = 5mg/kg I.P.;

FIG. 32A and FIG. 32B illustrate that glutamine analogue (6-diazo-5-oxo-L-norleucine (L-DON) derivative = **25**) inhibits tumor growth. FIG. 32A shows 4T1 (breast cancer cell lines) after 30 days inoculation (8 days drug free). FIG. 32B shows Group 1: PBS vehicle, Group 2: **25** 1mg/kg every day (from d5-d22). 4T1 tumor cells (0.1 million) were injected into the mammary fat pads of 8-week-old female BALB/c mice. Mice received vehicle (PBS) or 1mg/kg **25** daily from day 5 to day 22. Photos were taken on day 30 after tumor inoculation. Tumor volume was measured 2-3 times weekly until WT mice were sacrificed (when size reached to 20mm length or necrosis occurred). Day 0: 100K 4T1 cells s.c in 4th mammary pad. Day 5-22: Daily **25;**

FIG. 33 illustrates that glutamine analogue (6-diazo-5-oxo-L-norleucine (L-DON) derivative=**25**) inhibits tumor growth. 4T1-Luc tumor cells (0.1 million) were injected into the mammary fat pads of 8-week-old female BALB/c mice. Mice received vehicle (PBS) or 1mg/kg **25** daily from day 7. Anti-PDl (5mg/Kg) was administered on day 5, 8, and 12. Mice carrying 4T1-luc tumors are injected with Luciferin to measure luminescence from the tumor. IVIS imaging were taken on day 13;

FIG. 34A and FIG 34B illustrate that glutamine analogue (6-diazo-5-oxo-L-norleucine (L-DON) derivated=**25**) inhibits tumor growth. FIG. 34B shows tumor weight (mg) on harvest day 21. 4T1-Luc tumor cells (0.1 million) were injected into the mammary fat pads of 8-week-old female BALB/c mice. Mice received vehicle (PBS) or 1mg/kg **25** daily from day 7 to day 16. Anti-PDl (5mg/Kg) was administered on day 5, 8, 12 and 17. Tumor volume was measured 2-3 times weekly until tumors were evaluated for tumor infiltrating cells on day 21. Tumor weights were measured on day 21. On day 21, the PD1 group tumor size looks like it was reduced, however, the result was not due to a tumor size reduction, but rather because one of the large tumor mice died;

FIG. 35 illustrates reduced CDllb+ cells and G-MDSCs in **25** treated mice, demonstrating that metabolic reprogramming agents that decrease glutamine metabolic activity inhibit myeloid derived suppressor cells. 4T1-Luc tumor

cells (0.1 million) were injected into the mammary fat pads of 8-week-old female BALB/c mice. Mice received vehicle (PBS) or 1mg/kg **25** daily from day 7 to day 16. Anti-PD1 (5mg/Kg) was administered on day 5, 8, 12 and 17. Percentages of myeloidderived suppressor cell (MDSC) from circulating blood were monitored on day 6, 9 and 17 by flow cytometry with CD11b and Ly6C/Ly6G Granulocytic MDSC (G-MDSC): CDllb+ Ly6g+ Ly6c lo Monocytic MDSC (Mo-MDSC): CDllb+ Ly6G-Ly6c Hi;

FIG 36 illustrates increased *TNF alpha* in **25** treated mice, demonstrating that metabolic reprogramming agents that decrease glutamine metabolic activity increase inflammatory tumor associated macrophages (TAM). 4T1-Luc tumor cells (0.2 million) were injected into the mammary fat pads of 8-week-old female BALB/c mice. Mice received vehicle (PBS) or 1mg/kg **25** daily from day 7 to day 16. Anti-PD1 (5mg/Kg) was administered on day 5, 8, 12 and 17. Tumors were evaluated for tumor infiltrating cells on day 21. Cells were seeded on plates and golgi plug 200ul were added to inhibit cytokine secretion for over night (no additional stimulation). Tumor associated macrophages markers: Live/CD45+/CD11b+/F4-80+/CD8- for flow cytometry analysis. Macrophages derived from tumor. The cells were incubated with Golgi plug o.n. w/o stim. TAM: Live/CD45+/CD11b+/F4-80+/CD8-;

FIG. 37 shows reduced fibrocytes in **25** treated mice, demonstrating that metabolic reprogramming agents that decrease glutamine metabolic activity inhibit bone marro derived fibrocytes which are thought to play a role in inhibiting immunotherapy, as well as generating an extracellular matrix that surrounds tumors and inhibits chemotherapy. 4T1-Luc tumor cells (0.1 million) were injected into the mammary fat pads of 8-week-old female BALB/c mice. Mice received vehicle (PBS) or 1mg/kg **25** daily from day 7 to day 16. Anti-PDI (5mg/Kg) was administered on day 5, 8, 12 and 17. Tumors were evaluated for tumor infiltrating cells on day 21. Fibrocytes markers: CollagenI+CD11b+CD45+live were used for flow cytometry analysis.

DETAILED DESCRIPTION

The presently disclosed subject matter now will be described more fully hereinafter with reference to the accompanying Figures, in which some, but not all embodiments of the presently disclosed subject matter are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Figures. Therefore, it is to be understood that the presently disclosed subject matter is not to be limited to the specific embodiments disclosed and that modifications and other embodiments are intended to be included within the scope of the appended claims.

The presently disclosed subject matter demonstrates that certain conditions, diseases, and/or disorders involve metabolically reprogrammed cells whose activation, function, growth, proliferation, and/or survival in an abnormal, harmful, and/or unhealthy state depend on increased activity of at least one, at least two, or at least three metabolic pathways selected from the group consisting of glutamine metabolism, glycolysis, and fatty acid synthesis. It should be appreciated that the abnormal, harmful, and/or unhealthy state of the cell refers to its effect on or relative to the subject whose cells are affected by the condition, disease, or disorder rather than on or relative to the cell itself which exhibits an increased ability to thrive in the abnormal, harmful, and/or unhealthy state in a manner that is believed to be proportionate to the increase in the activity of at least one, at least two, or at least three metabolic pathways (e.g., glutamine metabolism, glycolysis, and/or fatty acid synthesis).

The presently disclosed subject matter have demonstrated that certain of such conditions, diseases, and/or disorders, referred to herein as "metabolic reprogramming disorders," are amenable to treatment using at least one, at least two, or at least three metabolic reprogramming agents that decrease activity of at least one, at least two, or at least three metabolic pathways selected from the group consisting of glutamine metabolism, glycolysis, and fatty acid synthesis. In some instances, the metabolic reprogramming disorders comprise conditions, diseases, or disorders that involve aberrant and/or excessive glutamine metabolism, aberrant and/or excessive glycolysis, or aberrant and/or excessive fatty acid synthesis.

As used herein, the term "excessive glutamine metabolism" means an increase in the amount of glutamine metabolic activity in a subject with a condition, disease, or disorder (e.g., a metabolic reprogramming disorder) as compared to the amount of glutamine metabolic activity in a subject without a similar disease or condition, such as an increase of approximately 100%, 100%, 200%, 300%, 400%, 500%, 600%, 700%, 800%, 900%, 1000%, or more. As used herein, the term "aberrant glutamine metabolism" means a change in the biological activity of glutamine in a subject with a condition, disease or disorder (e.g., a metabolic reprogramming disorder) as compared to the glutamine activity in a subject without a similar condition, disease, or disorder, such as increased utilization of glutamine in the growth and/or proliferation of malignant, neoplastic, or other pathologic cellular processes (e.g., immune disorders, neurodegenerative disorders, inflammatory disorders, etc.).

As used herein, the term "excessive glycolysis metabolism" means an increase in the amount of glycolytic metabolic activity in a subject with a condition, disease, or disorder (e.g., a metabolic reprogramming disorder) as compared to the amount of glycolytic metabolic activity in a subject without a similar disease or condition, such as an increase of approximately 100%, 100%, 200%, 300%, 400%, 500%, 600%, 700%, 800%, 900%, 1000%, or more. As used herein, the term "aberrant glycolytic metabolism" means a change in the biological activity of glycolysis in a subject with a condition, disease or disorder (e.g., a metabolic reprogramming disorder) as compared to the glycolytic activity in a subject without a similar condition, disease, or disorder, such as increased utilization of glucose in the growth and/or proliferation of malignant, neoplastic, or other pathologic cellular processes (e.g., immune disorders, neurodegenerative disorders, inflammatory disorders, etc.).

As used herein, the term "excessive fatty acid synthesis" means an increase in the amount of fatty acid synthesis in a subject with a condition, disease, or disorder (e.g., a metabolic reprogramming disorder) as compared to the amount of fatty acid synthesis in a subject without a similar condition, disease, or disorder, such as an increase of approximately 100%, 100%, 200%, 300%, 400%, 500%, 600%, 700%, 800%, 900%, 1000%, or more. As used herein, the term "aberrant fatty acid synthesis" means a change in the biological activity of fatty acid synthesis in a subject with a condition, disease or disorder (e.g., a metabolic reprogramming disorder) as compared to the fatty acid synthesis in a subject without a similar condition, disease, or disorder, such as increased utilization of fatty acids in the growth and/or proliferation of malignant, neoplastic, or other pathologic cellular processes (e.g., immune disorders, neurodegenerative disorders, inflammatory disorders, etc.).

As used herein, a "metabolically reprogrammed" cell refers to a cell in which the activity of at least one, at least two, or at least three metabolic pathways (e.g., glutamine metabolism, glycolysis, and fatty acid synthesis) has increased in response to the cells energetic and biosynthetic demands placed on the cell in order for the cell to become activated, function, grow, proliferate, and/or survive in the abnormal, harmful, and/or unhealthy state. As used herein, a "metabolic reprogramming agent" refers to an agent that is capable of reversing the metabolic reprogramming of a cell from a cell whose activation, function, growth, proliferation, and/or survival in an abnormal, harmful, and/or unhealthy state depends on increased activity of at least one, at least two, or at least three metabolic pathways (e.g., glutamine metabolism, glycolysis, and fatty acid synthesis) to a cell that has a decreased capacity or has lost its ability to thrive (e.g., activate, function, grow, proliferate, and/or survive) in the abnormal, harmful and/or unhealthy state. In some contexts, a "metabolic reprogramming agent" inhibits at least one of, at least two of, or all of aberrant and/or excessive glutamine metabolism, aberrant and/or excessive glycolysis, and aberrant and/or excessive fatty acid synthesis.

The presently disclosed subject matter now will be described more fully hereinafter with reference to the accompanying Figures, in which some, but not all embodiments of the inventions are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Figures. Therefore, it is to be understood that the presently disclosed subject matter is not to be limited to the specific embodiments disclosed and that modifications and other embodiments are intended to be included within the scope of the appended claims.

## I. METHODS OF TREATMENT USING METABOLIC REPROGRAMMING AGENTS

The presently disclosed subject matter describes a method for treating a subject having a condition, disease, or disorder that involves metabolically reprogrammed cells whose activation, function, growth, proliferation, and/or survival depends on increased activity of at least one metabolic pathway selected from the group consisting of glutamine metabolism, glycolysis, and fatty acid synthesis, the method comprising administering to the subject at least one metabolic reprogramming agent that decreases activity of at least one metabolic pathway selected from the group consisting of glutamine metabolism, glycolysis, and fatty acid synthesis in an amount effective to treat the condition, disease, or disorder.

The presently disclosed subject matter describes a method for treating a subject having a condition, disease, or disorder that involves at least one of aberrant and/or excessive glutamine metabolism, aberrant and/or excessive glycolysis, or aberrant and/or excessive fatty acid synthesis, the method comprising administering to the subject at least one metabolic reprogramming agent that decreases activity of at least one metabolic pathway selected from the group consisting of glutamine metabolism, glycolysis, and fatty acid synthesis in an amount effective to treat the condition, disease, or disorder.

In general, the presently disclosed methods result in a decrease in the severity of a condition, disease, or disorder (e.g., a metabolic reprogramming disorder) in a subject. The term "decrease" is meant to inhibit, suppress, attenuate, diminish, arrest, or stabilize a symptom of the condition, disease, or disorder. As used herein, the terms "treat,"

"treating," "treatment," refer to reducing or ameliorating a disease or condition, and/or symptoms associated therewith. It will be appreciated that, although not precluded, treating a disease or condition does not require that the disorder, condition or symptoms associated therewith be completely eliminated.

The method comprises administering to the subject at least two metabolic reprogramming agents that decrease the activity of at least two metabolic pathways selected from the group consisting of glutamine metabolism, glycolysis, and fatty acid synthesis in an amount effective to treat the condition, disease, or disorder. The method comprises administering to the subject at least three metabolic reprogramming agents that each decrease the activity of a different metabolic pathway selected from the group consisting of glutamine metabolism, glycolysis, and fatty acid synthesis in an amount effective to treat the condition, disease, or disorder.

The terms "subject" and "patient" are used interchangeably herein. The subject treated by the presently disclosed methods, uses, metabolic reprogramming agents and compositions comprising those agents in their many embodiments is desirably a human subject, although it is to be understood that the methods described herein are effective with respect to all vertebrate species, which are intended to be included in the term "subject." Accordingly, a "subject" can include a human subject for medical purposes, such as for the treatment of an existing condition or disease or the prophylactic treatment for preventing the onset of a condition or disease, or an animal subject for medical, veterinary purposes, or developmental purposes. Suitable animal subjects include mammals including, but not limited to, primates, e.g., humans, monkeys, apes,; bovines, e.g., cattle, oxen,; ovines, e.g., sheep; caprines, e.g., goats; porcines, e.g., pigs, hogs; equines, e.g., horses, donkeys, zebras; felines, including wild and domestic cats; canines, including dogs; lagomorphs, including rabbits, hares; and rodents, including mice, rats. An animal may be a transgenic animal. T the subject is a human including, but not limited to, fetal, neonatal, infant, juvenile, and adult subjects. Further, a "subject" can include a patient afflicted with or suspected of being afflicted with a condition or disease.

A. CANCER

Examples of malignant or cancer cells whose activation, function, growth, proliferation, and/or survival in an abnormal, harmful, or unhealthy state depends on increased metabolic activity of at least one, at least two, or at least three metabolic pathways selected from the group consisting of glutamine metabolism, glycolysis and fatty acid synthesis include, but are not limited to, cMyc-dependent cancer cells, glutamine-dependent cancer cells, and combinations thereof. As used herein, a "glutamine-dependent cancer cell" is a cancer cell in which glutamine is an important fuel source for cellular energy in the cancer cell (e.g., hematopoietic tumors, hepatomas, Ehrilich carcinoma (see Huber et al., "Uptake of glutamine antimetabolites 6-diazo-5-oxo-L-norleucine (DON) and acivicin in sensitive and resistant tumor cell lines," Int. J. Cancer. 1988; 41:752-755)). As used herein, cMyc-dependent cancer cells" refers to cancer cells exhibiting activation, overexpression and/or amplification of c-Myc. In some contexts, a "Myc-dependent cancer" is a cancer in which c-Myc plays a role in increased glutamine metabolism in the cancer cells, i.e., cMyc-dependent glutamine addicted cancer cells. Examples of Myc-dependent cancers include, without limitation, lymphoma, neuroblastoma, and small cell lung cancer.

The presently disclosed subject matter further involve the use of at least one metabolic reprogramming agent (e.g., a metabolic reprogramming agent that decreases glutamine metabolism) as a cancer maintenance thereapy. As used herein, "cancer maintenance therapy" refers to a therapy administered to a cancer patient who is in cancer remission.

T the presently disclosed subject matter provides a method of preventing a relapse in a cancer subject in remission, the method comprising administering to the subject a therapeutically effective amount of a metabolic reprogramming agent that decreasese glutamine metabolism. As used herein, "remission" includes partial and complete remission and refers to a decrease in or disappearance of signs and symptoms of cancer. "Partial remission" means that the cancer responded to treatment with the primary therapy, but at least a portion of the tumor and/or at least a portion of the cancerous cells are still present in the subject, for example, at least 1%, 5%, 10%, 15%, 20%, 25%, 30%, 35%, 40%, 45%, or 49% of a measurable tumor and/or measurable cancerous cells are still present in the subject post-therapy. "Complete remission" means that the subject shows no signs or symptoms of cancer, for example, after a healthcare provider has used the most accurate and up-to-date tests available to detect the cancer and is unable to detect any signs or symptoms of cancer. It is to be understood that cancerous cells might still exist in a subject in complete remission at levels that are undetectable.

The metabolic reprogramming agent is administered to the subject post transplant. As used herein, "post transplant" refers to a subject that has recently received a cell, tissue, or organ transplantation, including for example, subjects receiving immunosuppressive agents to prevent, or reduce the risk and/or severity of, a transplant rejection. The metabolic reprogramming agent is administered to the subject post chemotherapy. The metabolic reprogramming agent is administered to the subject post immunotherapy. The metabolic reprogramming agent is administered to the subject post photodynamic therapy. Tthe metabolic reprogramming agent is administered to the subject post proton therapy. The metabolic reprogramming agent is administered to the subject post radiotherapy. The metabolic reprogramming agent is administered to the subject post surgery; and combinations thereof. The metabolic reprogramming

agent is administered to the subject at two or more of post transplant, post chemotherapy, post immunotherapy, post photodynamic therapy, post proton therapy, post radiotherapy, post surgery, and combinations thereof.

As used herein, a "cancer" in a subject refers to the presence of cells possessing characteristics typical of cancer-causing cells, for example, uncontrolled proliferation, loss of specialized functions, immortality, significant metastatic potential, significant increase in anti-apoptotic activity, rapid growth and proliferation rate, and certain characteristic morphology and cellular markers. In some circumstances, cancer cells will be in the form of a tumor; such cells may exist locally within an animal, or circulate in the blood stream as independent cells, for example, leukemic cells. A "tumor," as used herein, refers to all neoplastic cell growth and proliferation, whether malignant or benign, and all precancerous and cancerous cells and tissues. A "solid tumor", as used herein, is an abnormal mass of tissue that generally does not contain cysts or liquid areas. A solid tumor may be in the brain, colon, breasts, prostate, liver, kidneys, lungs, esophagus, head and neck, ovaries, cervix, stomach, colon, rectum, bladder, uterus, testes, and pancreas, as non-limiting examples. In some embodiments, the solid tumor regresses or its growth is slowed or arrested after the solid tumor is treated with the presently disclosed methods. In other embodiments, the solid tumor is malignant. In some embodiments, the cancer comprises Stage 0 cancer. In some embodiments, the cancer comprises Stage I cancer. In some embodiments, the cancer comprises Stage II cancer. In some embodiments, the cancer comprises Stage III cancer. In some embodiments, the cancer comprises Stage IV cancer. In some embodiments, the cancer is refractory and/or metastatic. For example, the cancer may be refractory to treatment with radiotherapy, chemotherapy or monotreatment with immunotherapy.

In particular embodiments, the cancer is a cancer of the central nervous system (CNS cancer). It is believed that certain of the presently disclosed metabolic reprogramming agents and compositions are particularly useful in the treatment of CNS cancers and cancers of CNS origin. In particular, the data described in FIG. 22A, FIG. 22B, FIG. 22C, FIG. 29A, FIG. 29B, FIG. 29C and FIG. 30 demonstrate that certain metabolic reprogramming agents (e.g., prodrugs of glutamine analogs, e.g., DON prodrugs) effectively target and deliver DON to the brain, for example, achieving as much as a 10-fold enhanced CSF to plasma ratio at 30 minute post dosing. Accordingly, certain of the presently disclosed metabolic reprogramming agents are contemplated for use as cancer therapy (e.g., maintenance therapy), immunotherapy, and an enhancement to immunotherapy, for the treatment of CNS cancers and cancers of CNS origin.

Exemplary CNS cancers treatable with the presently disclosed methods, compositions and agents include, without limitation, gliomas, astrocytomas, oligodendrogliomas, ependymoas, mixed gliomas (e.g., oligoastrocytomas), meningiomas (e.g., atypical, invasive, anaplastic, etc.), medulloblastomas, gangliogliomas, schwannomas (neuroliemmomas), craniopharyngiomas, chordomas, non-Hodgkin lymphoma of CNS origin, and, pituitary tumors. In particular embodiments, the CNS cancer comprises glioblastoma multiform (GBM).

In particular embodiments, the cancer is a cancer that is associated with transplant and/or immunosuppression. It is well known that organ transplants (e.g., kidney, liver, heart, lung etc.) in the United States are at high risk of developing various types of cancer (see, e.g., Engels et al. 2011). In some instances, the cancer risk is elevated for infection-relalted cancer due to immunosuppression, for example, because of medications administered to suppress the immune system and prevent transplant rejection (e.g., organ). In some embodiments, the cancer associated with transplant and/or immunosuppression is related to an infectious agent. Examples of cancers associated with transplant and/or immunosuppression include, without limitation, anal cancer, Kaposi sarcoma, kidney cancer, liver cancer, lung cancer, melanoma, non-Hodgkin's lymphoma, and thyroid cancer. In particular embodiments, the subject is a child or elderly adult transplant recipient (e.g., liver, heart, kidney, etc.) who may or may not be infected with Epstein-Barr virus. In particular embodiments, the cancer is a cancer that is refractory to chemotherapy. In particular embodiments, the cancer is a cancer that is refractory to photodynamic therapy. In particular embodiments, the cancer is a cancer that is refractory to proton therapy.

In particular embodiments, the cancer is a cancer that is refractory to radiotherapy.

In particular embodiments, the cancer is a cancer that is refractory to surgery.

Cancer as used herein includes newly diagnosed or recurrent and/or refractory cancers, including without limitation, acute lymphoblastic leukemia, acute myelogenous leukemia, advanced soft tissue sarcoma, brain cancer, metastatic or aggressive breast cancer, breast carcinoma, bronchogenic carcinoma, choriocarcinoma, chronic myelocytic leukemia, colon carcinoma, colorectal carcinoma, Ewing's sarcoma, gastrointestinal tract carcinoma, glioma, glioblastoma multiforme, head and neck squamous cell carcinoma, hepatocellular carcinoma, Hodgkin's disease, intracranial ependymoblastoma, large bowel cancer, leukemia, liver cancer, lung carcinoma, Lewis lung carcinoma, lymphoma, malignant fibrous histiocytoma, a mammary tumor, melanoma, mesothelioma, neuroblastoma, osteosarcoma, ovarian cancer, pancreatic cancer, a pontine tumor, premenopausal breast cancer, prostate cancer, rhabdomyosarcoma, reticulum cell sarcoma, sarcoma, small cell lung cancer, a solid tumor, stomach cancer, testicular cancer, and uterine carcinoma.

In particular embodiments, the cancer treated is a newly diagnosed, or recurrent, and/or refractory cancer selected from the group consisting of celnasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison

syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

In some embodiments, the condition, disease, or disorder is lymphoma. Accordingly, in an aspect the presently disclosed subject matter provides a method for the treatment of lymphoma in a subject in need thereof, the method comprising administering to the subject at least one metabolic reprogramming agent that decreases glutamine metabolism in an amount effective to treat lymphoma in the subj ect.

In some embodiments, the condition, disease, or disorder is melanoma. Accordingly, in an aspect the presently disclosed subject matter provides a method for the treatment of melanoma in a subject in need thereof, the method comprising administering to the subject at least one metabolic reprogramming agent that decreases glutamine metabolism in an amount effective to treat melanoma in the subj ect.

In some embodiments, the methods include administering to the subject an effective amount of radiotherapy. In some embodiments, the methods include administering of the subject an effective amount of immunotherapy (e.g., a second immunotherapy). In some embodiments, the methods include administering to the subject an effective amount of photodynamic therapy. In some embodiments, the methods include administering to the subject an effective amount of proton therapy. In some embodiments, the methods include surgically resecting at least a portion of a tumor before, during, or after treatment with the at least one, at least two, or at least three metabolic reprogramming agents, and optionally at least one chemotherapeutic agent, immunotherapeutic agent, and/or radiotherapeutic agent.

In some embodiments, the condition, disease, or disorder is a newly diagnosed, or recurrent and/or refractory cancer selected from the group consisting of acute lymphoblastic leukemia, acute myelogenous leukemia, advanced soft tissue sarcoma, brain cancer, metastatic or aggressive breast cancer, breast carcinoma, bronchogenic carcinoma, choriocarcinoma, chronic myelocytic leukemia, colon carcinoma, colorectal carcinoma, Ewing's sarcoma, gastrointestinal tract carcinoma, glioma, glioblastoma multiforme, head and neck squamous cell carcinoma, hepatocellular carcinoma, Hodgkin's disease, intracranial ependymoblastoma, large bowel cancer, leukemia, liver cancer, lung carcinoma, Lewis lung carcinoma, lymphoma, malignant fibrous histiocytoma, a mammary tumor, melanoma, mesothelioma, neuroblastoma, osteosarcoma, ovarian cancer, pancreatic cancer, a pontine tumor, premenopausal breast cancer, prostate cancer, rhabdomyosarcoma, reticulum cell sarcoma, sarcoma, small cell lung cancer, a solid tumor, stomach cancer, testicular cancer, and uterine carcinoma.

In some embodiments, the cancer is not acute lymphoblastic leukemia. In some embodiments, the cancer is not acute myelogenous leukemia. In some embodiments, the cancer is not advanced soft tissue sarcoma. In some embodiments, the cancer is not brain cancer. In some embodiments, the cancer is not metastatic or aggressive breast cancer. In some embodiments, the cancer is not breast carcinoma. In some embodiments, the cancer is not bronchogenic carcinoma. In some embodiments, the cancer is not choriocarcinoma. In some embodiments, the cancer is not chronic myelocytic leukemia. In some embodiments, the cancer is not colon carcinoma. In some embodiments, the cancer is not colorectal carcinoma. In some embodiments, the cancer is not Ewing's sarcoma. In some embodiments, the cancer is not gastrointestinal tract carcinoma. In some embodiments, the cancer is not glioma. In some embodiments, the cancer is not glioblastoma multiforme. In some embodiments, the cancer is not head and neck squamous cell carcinoma. In some embodiments, the cancer is not hepatocellular carcinoma. In some embodiments, the cancer is not Hodgkin's disease. In some embodiments, the cancer is not intracranial ependymoblastoma. In some embodiments, the cancer is not large bowel cancer. In some embodiments, the cancer is not leukemia. In some embodiments, the cancer is not liver cancer. In some embodiments, the cancer is not lung carcinoma. In some embodiments, the cancer is not Lewis lung carcinoma. In some embodiments, the cancer is not lymphoma. In some embodiments, the cancer is not malignant fibrous histiocytoma. In some embodiments, the cancer is not a mammary tumor. In some embodiments, the cancer is not melanoma. In some embodiments, the cancer is not mesothelioma. In some embodiments, the cancer is not neuroblastoma. In some embodiments, the cancer is not osteosarcoma. In some embodiments, the cancer is not ovarian cancer. In some embodiments, the cancer is not pancreatic cancer. In some embodiments, the cancer is not a pontine tumor. In some embodiments, the cancer is not premenopausal breast cancer. In some embodiments, the cancer is not prostate cancer. In some embodiments, the cancer is not rhabdomyosarcoma. In some embodiments, the cancer is not reticulum cell sarcoma. In some embodiments, the cancer is not sarcoma. In some embodiments, the cancer is not small

cell lung cancer. In some embodiments, the cancer is not a solid tumor. In some embodiments, the cancer is not stomach cancer. In some embodiments, the cancer is not testicular cancer. In some embodiments, the cancer is not uterine carcinoma.

B. IMMUNOTHERAPY

Unclaimed aspects of the presently disclosed subject matter involve the use of at least one, at least two, or at least three metabolic reprogramming agents, alone, or optionally together in combination with an additional immunotherapy (e.g., checkpoint blockade, adoptive cellular therapy (ACT), vaccines (e.g., tumor vaccines), passive immunotherapy antibodies, for the treatment of cancer.

Accordingly, in one unclaimed aspect, the presently disclosed subject matter provides a method for treating a cancer in a subject in need thereof, the method comprising: (a) administering a therapeutically effective amount of a first immunotherapy to the subject, wherein the first immunotherapy is a metabolic reprogramming agent; and (b) optionally administering a therapeutically effective amount of a second immunotherapy to the subject. In particular embodiments described herein, the metabolic reprogramming agent is a glutamine antagonist. In particular embodiments described herein, the metabolic reprogramming agent is a glutamine analog that interferes with a glutamine metabolic pathway. In particular embodiments described herein, the metabolic reprogramming agent is selected from the group consisting of acivicin (L-(alpha S, 5S)-alpha-amino-3-chloro-4,5-dihydro-5-isoxazoleacetic acid), azaserine, and 6-diazo-5-oxo-norleucine (DON), and 5-diazo-4-oxo-L-norvaline (L-DONV). In particular embodiments, the metabolic reprogramming agent is a prodrug of a glutamine analog that interferes with a glutamine metabolic pathway. In particular embodiments, at least one metabolic reprogramming agent is a prodrug of acivicin, azaserine, DON, and L-DONV.

In some aspects described herein, a prodrug of a glutamine antagonist, or a pharmaceutically acceptable salt or ester thereof has a structure of formula (I):

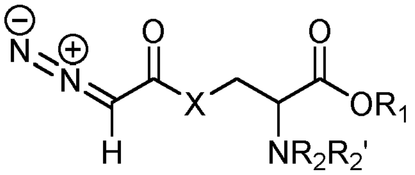

(I);

wherein: X is selected from the group consisting of a bond, -O-, and $-(CH_2)_n-$, wherein n is an integer selected from the group consisting of 1, 2, 3, 4, 5, 6, 7, and 8; $R_1$ is selected from the group consisting of H and a first prodrug-forming moiety capable of forming a salt or an ester; and $R_2$ is H or a second prodrug-forming moiety capable of forming an amide linkage, a carbamate linkage, a phosphoramidate linkage or a phosphorodiamidate linkage with the nitrogen adjacent to $R_2$; $R_2'$ is selected from the group consisting of H, $C_1$-$C_6$ alkyl, substituted $C_1$-$C_6$ alkyl, or $R_2$ and $R_2'$ together form a ring structure comprising -C(=O)-G-C(=O)-, wherein G is selected from the group consisting of $C_1$-$C_8$ alkylene, $C_1$-$C_8$ heteroalkylene, $C_5$-$C_8$ cycloalkylene, $C_6$-$C_{12}$ arylene, $C_5$-$C_{14}$ heteroarylene, bivalent $C_4$-$C_{10}$ heterocycle, each of which can be optionally substituted; or $R_1$ and $R_2'$ together form a 4-to 6-membered heterocylic ring comprising the oxygen atom adjacent to $R_1$ and the nitrogen atom adjacent to $R_2'$; provided that the compound has at least one prodrug-forming moiety selected from the group consisting of the first and the second prodrug-forming moieties.

As used herein, the term "amide linkage" comprises a structure represented by the formula:

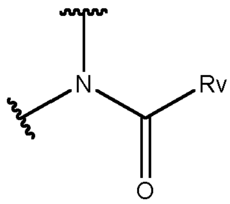

,

wherein $R_v$ is selected from the group consisting of alkyl, substituted alkyl, cycloalkyl, substituted cycloalkyl, aryl, substituted aryl, aralkyl, substituted aralkyl, heterocyclyl, substituted heterocyclyl, alkenyl, substituted alkenyl, cycloalkenyl, substituted cycloalkenyl, alkylamine, substituted alkylamine, heteroaryl, and substituted heteroaryl.

As used herein, the term "carbamate linkage" comprises a structure represented by the formula:

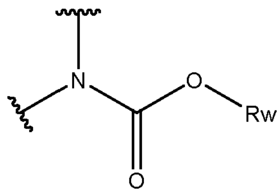

,

wherein $R_w$ is selected from the group consisting of alkyl, substituted alkyl, cycloalkyl, substituted cycloalkyl, aryl, substituted aryl, aralkyl, substituted aralkyl, heterocyclyl, substituted heterocyclyl, alkenyl, substituted alkenyl, cycloalkenyl, substituted cycloalkenyl, alkylamine, substituted alkylamine, heteroaryl, and substituted heteroaryl.

As used herein, the term "phosphoramidate linkage" comprises a structure represented by the formula:

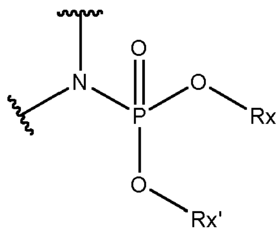

,

wherein $R_x$ and $R_x$' are each independently selected from the group consisting of alkyl, substituted alkyl, cycloalkyl, substituted cycloalkyl, aryl, substituted aryl, aralkyl, substituted aralkyl, heterocyclyl, substituted heterocyclyl, alkenyl, substituted alkenyl, cycloalkenyl, substituted cycloalkenyl, alkylamine, substituted alkylamine, heteroaryl, and substituted heteroaryl.

As used herein, the term "phosphorodiamidate linkage" comprises a structure represented by the formula:

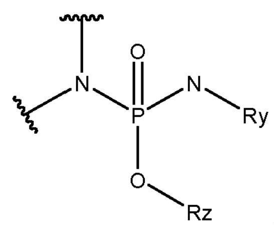

,

wherein $R_y$ and $R_z$ are each independently selected from the group consisting of H, alkyl, substituted alkyl, cycloalkyl, substituted cycloalkyl, heterocyclyl, substituted heterocyclyl, alkenyl, substituted alkenyl, cycloalkenyl, substituted cycloalkenyl, $-(CR_3R_4)_m-Z$, $-(CR_3R_4)_m-Q-Z$, aryl, substituted aryl, alkylamine, substituted alkylamine, heteroaryl, substituted heteroaryl, and

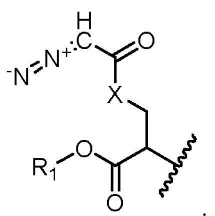

.

In some embodiments described herein, X is $-CH_2-$, and n is 1.

In other embodiments described herein, X is -O-. In some embodiments described herein, the prodrug compound has both the first prodrug-forming moiety and the second prodrug-forming moiety. In some embodiments described herein, the glutamine analog is a glutamine antagonist, i.e., the prodrug is a prodrug of a glutamine analog that antagonizes a glutamine pathway. Exemplary glutamine antagonists include, without limitation, 6-diazo-5-oxo-norleucine (DON), and azaserine, and 5-diazo-4-oxo-L-norvaline (L-DONV).

In some embodiments described herein, the presently disclosed subject matter provides a prodrug of DON. In some embodiments, the prodrug of DON has a structure of formula (I). In some embodiments described herein, the

presently disclosed subject matter provides a prodrug of L-DONV. In some embodiments, the prodrug of L-DONV has a structure of formula (I). In some embodiments described herein, the presently disclosed subject matter provides a prodrug of azaserine. In some embodiments, the prodrug of azaserine has a structure of formula (I).

In some embodiments described herein, $R_1$ of formula (I) comprises a residue $PRO_1$ of the prodrug-forming moiety, which, together with a basic moiety and the terminal hydroxyl group forms a salt.

In some embodiments described herein, $R_1$ of formula (I) comprises a residue $PRO_1$ of the prodrug-forming moiety, which, together with an alkyl group and the oxygen of an adjoining hydroxyl group forms an ester.

In some embodiments described herein, $R_1$ of formula (I) comprises a residue $PRO_1$ of the prodrug-forming moiety, which, together with an alkyl group and the nitrogen adjoining the $R_2$' group, forms an azlactone or an oxazolidone.

In some embodiments described herein, $R_1$ of formula (I) is selected from the group consisting of H, alkyl, substituted alkyl, cycloalkyl, substituted cycloalkyl, alkenyl, substituted alkenyl, alkynyl, substituted alkynyl, cycloalkenyl, substituted cycloalkenyl, tri(hydrocarbyl)ammonium, and tetra(hydrocarbyl)ammonium. Preferred alkyl group, cycloalkyl group, alkenyl group, alkynyl group, and cycloalkenyl group subsituents include alkyl, substituted alkyl, halo, arylamino, acyl, hydroxyl, aryloxyl, alkoxyl, alkylthio, arylthio, aralkyloxyl, aralkylthio, carboxyl, alkoxycarbonyl, oxo, and cycloalkyl.

In some embodiments described herein, $R_1$ of formula (I) is not H. In some embodiments, $R_1$ of formula (I) is not H when $R_2$ and $R_2$' are H. In some embodiments, $R_2$ and $R_2$' of formula (I) are each H when and $R_1$ is not H.

In some embodiments described herein, $R_1$ of formula (I) is selected from the group consisting of a $C_{1-6}$ straight-chain alkyl, a substituted $C_{1-6}$ straight-chain alkyl, a $C_{1-6}$ branched alkyl, a substituted $C_{1-6}$ branched alkyl, tri($C_1$-$C_8$-alkyl)ammonium, tetra($C_1$-$C_8$-alkyl)ammonium, triphenylammonium, tri(hydroxy-$C_1$-$C_8$alkyl)ammonium, and tetra(hydroxy-$C_1$-$C_8$-alkyl)ammonium.

In some embodiments described herein, $R_1$ of formula (I) is selected from the group consisting of methyl, ethyl, isopropyl, cyclopentyl, cyclohexyl, trimethylammonium, triethylammonium, tri(hydroxyethyl)ammonium, tripropylammonium, and tri(hydroxypropyl)ammonium. In some embodiments described herein, $R_1$ of formula (I) is methyl. In some embodiments, $R_1$ of formula (I) is ethyl. In some embodiments, $R_1$ of formula (I) is isopropyl.

In some embodiments described herein, $R_2$ of formula (I) comprises a residue $PRO_2$ of the second prodrug-forming moiety, which, together with a carbonyl, oxy carbonyl, or phosphonyl group and the nitrogen of the adjoining NH, forms an amide, a carbamate, phosphoramidate, or phosphorodiamidate linkage.

In some embodiments described herein, $R_2$ of formula (I) comprises a moiety selected from the group consisting of an amino acid, an N-substituted amino acid, a peptide, a substituted peptide, a monocyclic ring, a substituted monocyclic ring, a bicyclic ring, a substituted bicyclic ring, a purine nucleoside, a substituted purine nucleoside, a pyrimidine nucleoside, and a substituted pyrimidine nucleoside.

In some aspects described herein, a prodrug of a glutamine antagonist, or a pharmaceutically acceptable salt or ester thereof has a structure of formula (I):

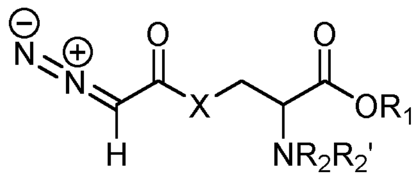

(I);

wherein: X is selected from the group consisting of a bond, -O-, and $-(CH_2)_n-$, wherein n is an integer selected from the group consisting of 1, 2, 3, 4, 5, 6, 7, and 8; $R_1$ is selected from the group consisting of H and a first prodrug-forming moiety capable of forming a salt or an ester; and $R_2$ is H or a second prodrug-forming moiety capable of forming an amide linkage, a carbamate linkage, a phosphoramidate linkage or a phosphorodiamidate linkage with the nitrogen adjacent to $R_2$; $R_2$' is selected from the group consisting of H, $C_1$-$C_6$ alkyl, substituted $C_1$-$C_6$ alkyl, or $R_2$ and $R_2$' together form a ring structure comprising -C(=O)-G-C(=O)-, wherein G is selected from the group consisting of $C_1$-$C_8$ alkylene, $C_1$-$C_8$ heteroalkylene, $C_5$-$C_8$ cycloalkylene, $C_6$-$C_{12}$ arylene, $C_5$-$C_{14}$ heteroarylene, bivalent $C_4$-$C_{10}$ heterocycle, each of which can be optionally substituted; or $R_1$ and $R_2$' together form a 4-to 6-membered heterocylic ring comprising the oxygen atom adjacent to $R_1$ and the nitrogen atom adjacent to $R_2$'; provided that the compound has at least one prodrug-forming moiety selected from the group consisting of the first and the second prodrug-forming moieties.

As used herein, the term "amide linkage" comprises a structure represented by the formula:

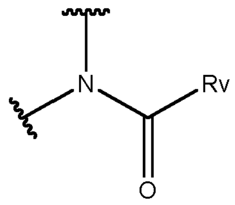

,

wherein $R_v$ is selected from the group consisting of alkyl, substituted alkyl, cycloalkyl, substituted cycloalkyl, aryl, substituted aryl, aralkyl, substituted aralkyl, heterocyclyl, substituted heterocyclyl, alkenyl, substituted alkenyl, cycloalkenyl, substituted cycloalkenyl, alkylamine, substituted alkylamine, heteroaryl, and substituted heteroaryl.

As used herein, the term "carbamate linkage" comprises a structure represented by the formula:

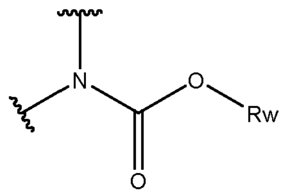

,

wherein $R_w$ is selected from the group consisting of alkyl, substituted alkyl, cycloalkyl, substituted cycloalkyl, aryl, substituted aryl, aralkyl, substituted aralkyl, heterocyclyl, substituted heterocyclyl, alkenyl, substituted alkenyl, cycloalkenyl, substituted cycloalkenyl, alkylamine, substituted alkylamine, heteroaryl, and substituted heteroaryl.

As used herein, the term "phosphoramidate linkage" comprises a structure represented by the formula:

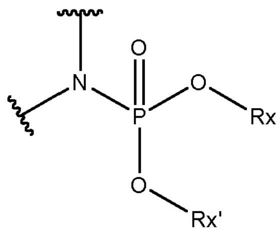

,

wherein $R_x$ and $R_x$' are each independently selected from the group consisting of alkyl, substituted alkyl, cycloalkyl, substituted cycloalkyl, aryl, substituted aryl, aralkyl, substituted aralkyl, heterocyclyl, substituted heterocyclyl, alkenyl, substituted alkenyl, cycloalkenyl, substituted cycloalkenyl, alkylamine, substituted alkylamine, heteroaryl, and substituted heteroaryl.

As used herein, the term "phosphorodiamidate linkage" comprises a structure represented by the formula:

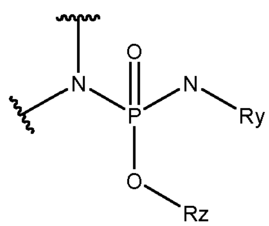

,

wherein $R_y$ and $R_z$ are each independently selected from the group consisting of H, alkyl, substituted alkyl, cycloalkyl, substituted cycloalkyl, heterocyclyl, substituted heterocyclyl, alkenyl, substituted alkenyl, cycloalkenyl, substituted cycloalkenyl, $-(CR_3R_4)_m-Z$, $-(CR_3R_4)_m-Q-Z$, aryl, substituted aryl, alkylamine, substituted alkylamine, heteroaryl, substituted heteroaryl, and

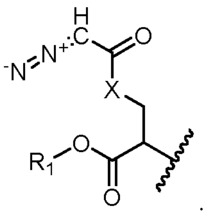

In some embodiments described herein, X is $-CH_2-$, and n is 1.

In other embodiments described herein, X is -O-. In some embodiments, the prodrug compound has both the first prodrug-forming moiety and the second prodrug-forming moiety. In some embodiments described herein, the glutamine analog is a glutamine antagonist, i.e., the prodrug is a prodrug of a glutamine analog that antagonizes a glutamine pathway. Exemplary glutamine antagonists include, without limitation, 6-diazo-5-oxo-norleucine (DON), and aza-serine, and 5-diazo-4-oxo-L-norvaline (L-DONV).

In some embodiments described herein, the presently disclosed subject matter provides a prodrug of DON. In some embodiments described herein, the prodrug of DON has a structure of formula (I). In some embodiments described herein, the presently disclosed subject matter provides a prodrug of L-DONV. In some embodiments described herein, the prodrug of L-DONV has a structure of formula (I). In some embodiments, the presently disclosed subject matter provides a prodrug of azaserine. In some embodiments described herein, the prodrug of azaserine has a structure of formula (I).

In some embodiments described herein, $R_1$ of formula (I) comprises a residue $PRO_1$ of the prodrug-forming moiety, which, together with a basic moiety and the terminal hydroxyl group forms a salt.

In some embodiments described herein, $R_1$ of formula (I) comprises a residue $PRO_1$ of the prodrug-forming moiety, which, together with an alkyl group and the oxygen of an adjoining hydroxyl group forms an ester.

In some embodiments described herein, $R_1$ of formula (I) comprises a residue $PRO_1$ of the prodrug-forming moiety, which, together with an alkyl group and the nitrogen adjoining the $R_2$' group, forms an azlactone or an oxazolidone.

In some embodiments described herein, $R_1$ of formula (I) is selected from the group consisting of H, alkyl, substituted alkyl, cycloalkyl, substituted cycloalkyl, alkenyl, substituted alkenyl, alkynyl, substituted alkynyl, cycloalkenyl, substituted cycloalkenyl, tri(hydrocarbyl)ammonium, and tetra(hydrocarbyl)ammonium. Preferred alkyl group, cycloalkyl group, alkenyl group, alkynyl group, and cycloalkenyl group subsituents include alkyl, substituted alkyl, halo, arylamino, acyl, hydroxyl, aryloxyl, alkoxyl, alkylthio, arylthio, aralkyloxyl, aralkylthio, carboxyl, alkoxycarbonyl, oxo, and cycloalkyl.

In some embodiments described herein, $R_1$ of formula (I) is not H. In some embodiments, $R_1$ of formula (I) is not H when $R_2$ and $R_2$' are H. In some embodiments, $R_2$ and $R_2$' of formula (I) are each H when and $R_1$ is not H.

In some embodiments described herein, $R_1$ of formula (I) is selected from the group consisting of a $C_{1-6}$ straight-chain alkyl, a substituted $C_{1-6}$ straight-chain alkyl, a $C_{1-6}$ branched alkyl, a substituted $C_{1-6}$ branched alkyl, tri($C_1$-$C_8$-alkyl)ammonium, tetra($C_1$-$C_8$-alkyl)ammonium, triphenylammonium, tri(hydroxy-$C_1$-$C_8$alkyl)ammonium, and tetra(hydroxy-$C_1$-$C_8$-alkyl)ammonium.

In some embodiments described herein, $R_1$ of formula (I) is selected from the group consisting of methyl, ethyl, isopropyl, cyclopentyl, cyclohexyl, trimethylammonium, triethylammonium, tri(hydroxyethyl)ammonium, tripropylammonium, and tri(hydroxypropyl)ammonium. In some embodiments described herein, $R_1$ of formula (I) is methyl. In some embodiments, $R_1$ of formula (I) is ethyl. In some embodiments, $R_1$ of formula (I) is isopropyl.

In some embodiments described herein, $R_2$ of formula (I) comprises a residue $PRO_2$ of the second prodrug-forming moiety, which, together with a carbonyl, oxy carbonyl, or phosphonyl group and the nitrogen of the adjoining NH, forms an amide, a carbamate, phosphoramidate, or phosphorodiamidate linkage.

In some embodiments described herein, $R_2$ of formula (I) comprises a moiety selected from the group consisting of an amino acid, an N-substituted amino acid, a peptide, a substituted peptide, a monocyclic ring, a substituted monocyclic ring, a bicyclic ring, a substituted bicyclic ring, a purine nucleoside, a substituted purine nucleoside, a pyrimidine nucleoside, and a substituted pyrimidine nucleoside.

In some embodiments described herein, $R_2$ of formula (I) is selected from the group consisting of H, alkyl, -C(=O)-Ar, $-C(=O)-Y-(CR_3R_4)_m-Ar$, $-C(=O)-Y-(CR_3R_4)_m-NR_5R_6$, $-P(=O)(OR_7)_n(NHR_9)_o$, $-C(=O)-Y-(CR_3R_4)_m-Ar-O-C(=O)-R_8$, $-C(=O)-Y-(CR_3R_4)_m-Ar-O-R_8$, $-C(=O)-O-(CR_3R_4)_m-O-C(=O)-R_{10}$, $-C(=O)-O-R_9$, $-C(=O)-Y-(CR_3R_4)_m-Ar-O-C(=O)-Ar$, and $-C(=O)-Y-(CR_3R_4)_m-Ar-NR_5R_6$; wherein: Y is -O- or a bond; m is an integer selected from the group consisting of 0, 1, 2, 3, 4, 5, 6, 7, and 8; each n and o is an integer from 0 to 2 provided that the sum of n and o is 2; $R_3$ and $R_4$ is independently H, $C_1$-$C_6$ alkyl or substituted $C_1$-$C_6$ alkyl, aryl or substituted aryl, $-(CR_3R_4)_m-NR_5R_6$, or

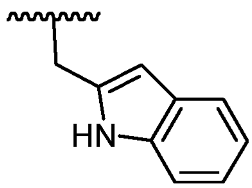

; each $R_5$ and $R_6$ is independently H, alkyl, -C(=O)-$(CR_3R_4)_m$, -C(=O)-$(NR_5R_6)$, or -C(=O)-$(CR_3R_4)_m$-$NR_5R_6$; each $R_7$ is independently selected from the group consisting of H, alkyl, substituted alkyl, cycloalkyl, substituted cycloalkyl, heterocyclyl, substituted heterocyclyl, alkenyl, substituted alkenyl, cycloalkenyl, substituted cycloalkenyl, - $(CR_3R_4)_m$-Z, -$(CR_3R_4)_m$-Q-Z, wherein Q is a monosaccharide, aryl, substituted aryl, heteroaryl, substituted heteroaryl, and wherein Z is

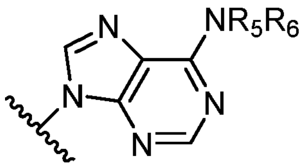

or wherein $R_7$ together with the oxygen atom to which it is attached forms a purine or pyrimidine nucleoside; each $R_9$ is independently selected from the group consisting of H, alkyl, substituted alkyl, cycloalkyl, substituted cycloalkyl, heterocyclyl, substituted heterocyclyl, alkenyl, substituted alkenyl, cycloalkenyl, substituted cycloalkenyl, - $(CR_3R_4)_m$-Z, aryl, substituted aryl, heteroaryl, substituted heteroaryl, and

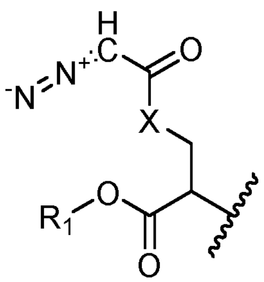

, wherein $R_1$ and X are as defined above, provided that $R_1$ is not H;
each $R_8$ is independently alkyl, substituted alkyl, cycloalkyl, substituted cycloalkyl, monosaccharide, acylated monosaccharide, aryl, substituted aryl, heteroaryl, substituted heteroaryl; each $R_{10}$ is independently alkyl, substituted alkyl, cycloalkyl, substituted cycloalkyl, monosaccharide, acylated monosaccharide, aryl, substituted aryl, heteroaryl, substituted heteroaryl; and Ar is aryl, substituted aryl, heteroaryl, or substituted heteroaryl. It should be appreciated that in addition to substitutions on the amino group of Z, one or more substitutions $R_3$, $R_4$, $R_5$, and/or $R_6$ can be made to the 5 or 6 membered rings of Z.

Structures of representative DON prodrugs described herein are provided in Table 1.

**Table 1. Structures of Representative DON Prodrugs**

| IOCB No./ Compound No. | Structure | MW |
|---|---|---|
| Compound **1** (DON) | 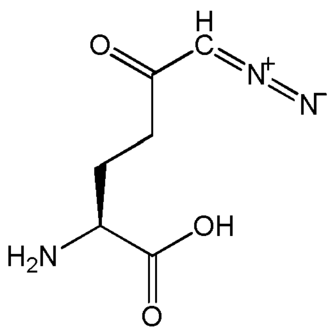 | 171.15 |

(continued)

| Table 1. Structures of Representative DON Prodrugs | | |
|---|---|---|
| IOCB No./ Compound No. | Structure | MW |
| Compound **3** | 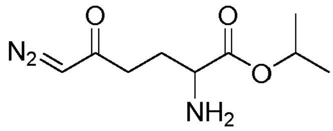 | 213.24 |
| Compound **4** | 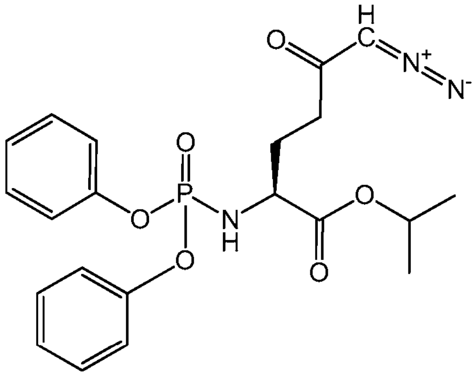 | 445.41 |
| Compound **6** | 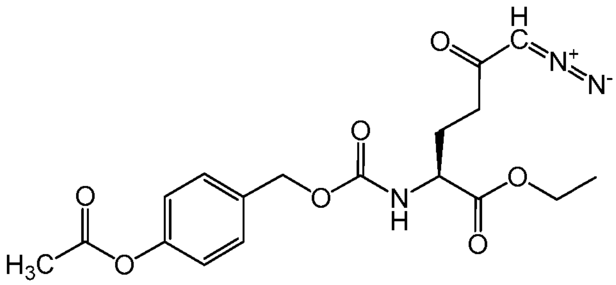 | 391.38 |
| Compound **7** | 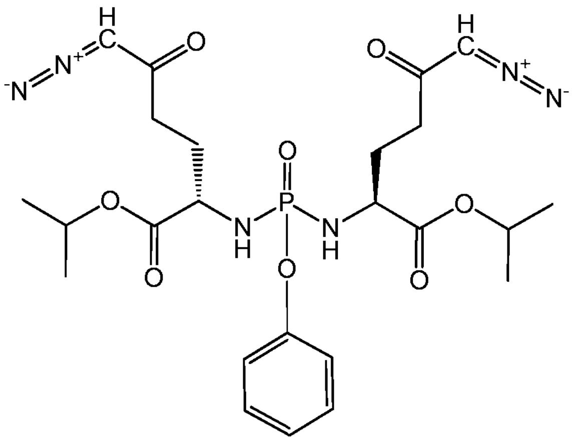 | 564.53 |

(continued)

| Table 1. Structures of Representative DON Prodrugs | | |
|---|---|---|
| IOCB No./ Compound No. | Structure | MW |
| Compound **9** | 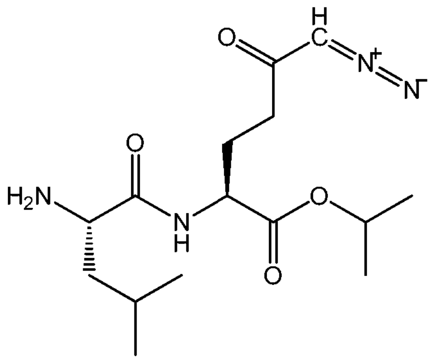 | 326.39 |
| Compound **11** | 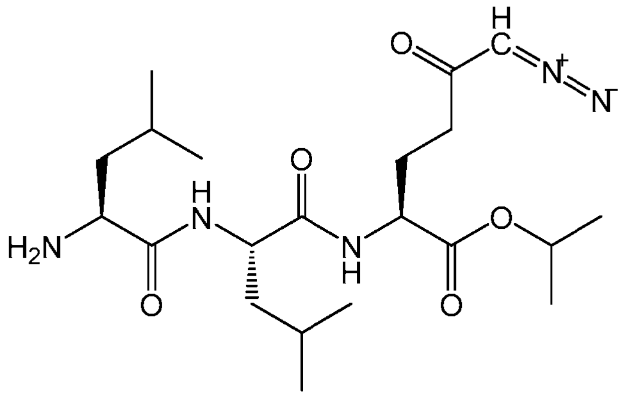 | 439.55 |
| Compound **13** | 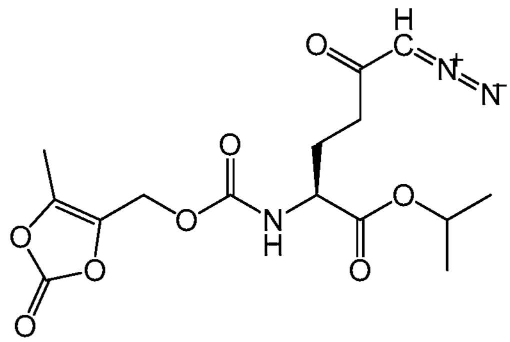 | 369.18 |
| Compound **14a** | 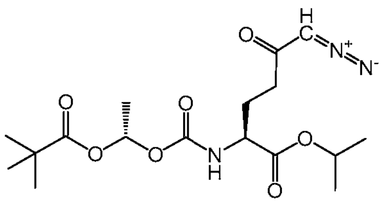 | 385.41 |
| Compound **14b** | 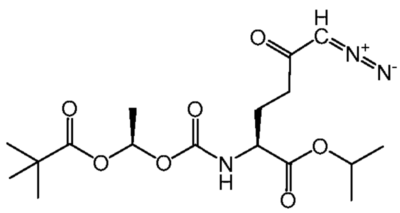 | |

(continued)

| Table 1. Structures of Representative DON Prodrugs | | |
|---|---|---|
| IOCB No./ Compound No. | Structure | MW |
| Compound **15** | 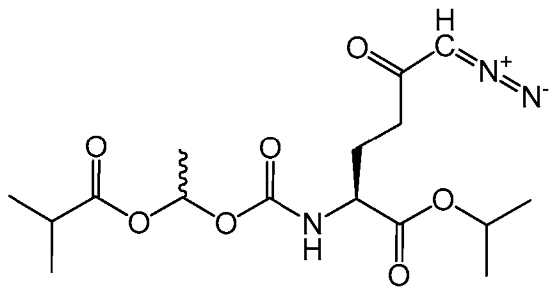 | 371.39 |
| Compound **17** | 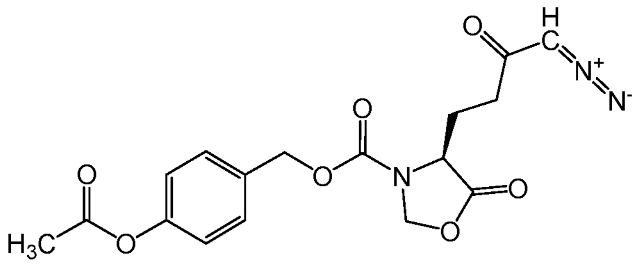 | 375.33 |
| Compound **20** | 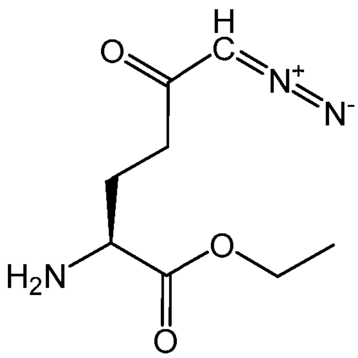 | 199.21 |
| Compound **22** | 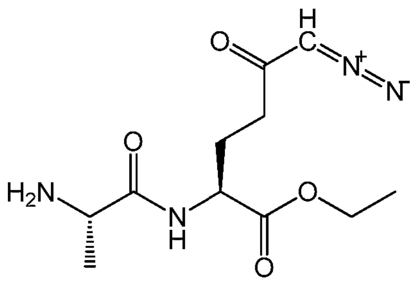 | 270.28 |
| Compound **23** | 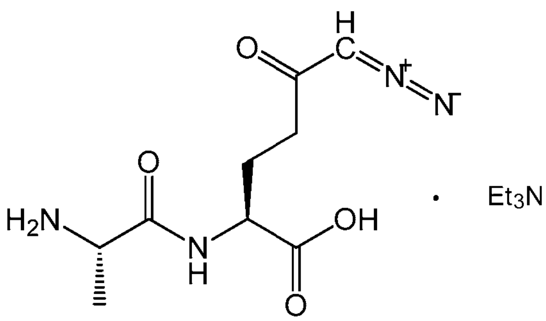 | 343.42 |

(continued)

| Table 1. Structures of Representative DON Prodrugs | | |
|---|---|---|
| IOCB No./ Compound No. | Structure | MW |
| Compound **25** | 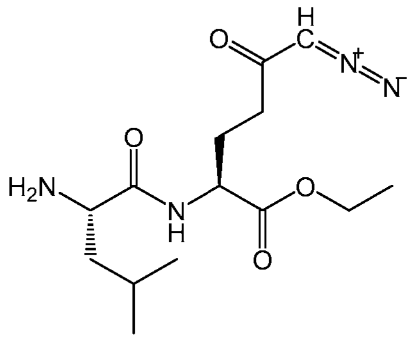 | 312.36 |
| Compound **26** | 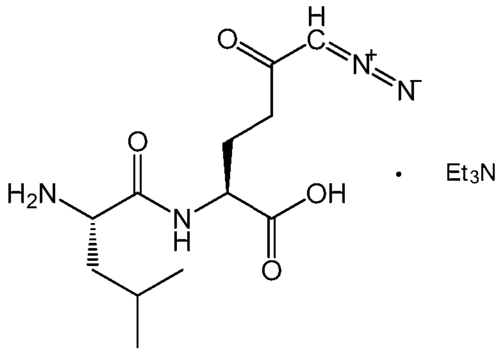 | 385.50 |
| Compound **28** | 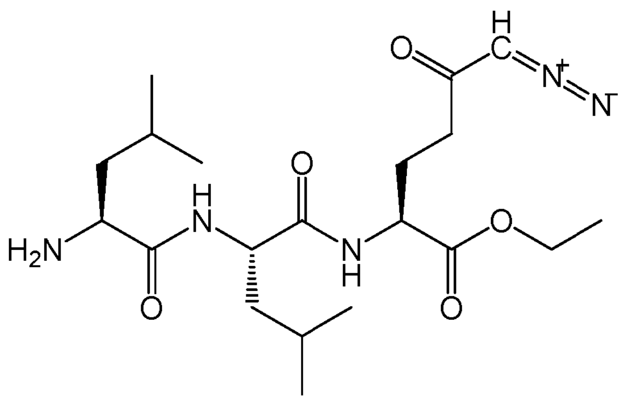 | 425.52 |
| Compound **29** | 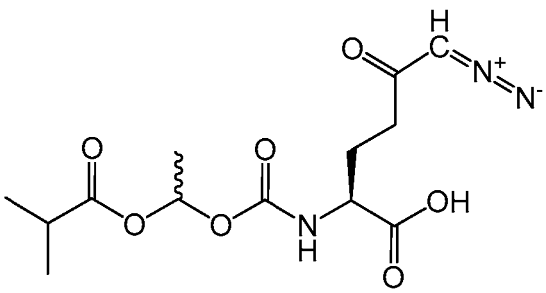 | 329.31 |

(continued)

| Table 1. Structures of Representative DON Prodrugs | | |
|---|---|---|
| IOCB No./ Compound No. | Structure | MW |
| Compound **30** | 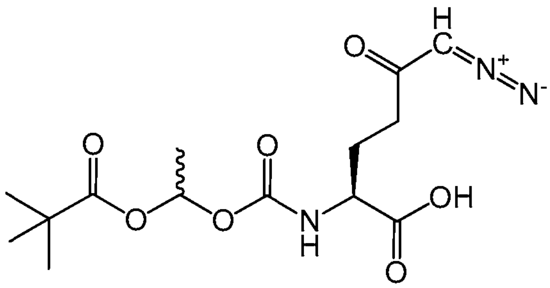 | 343.33 |
| Compound **31** | 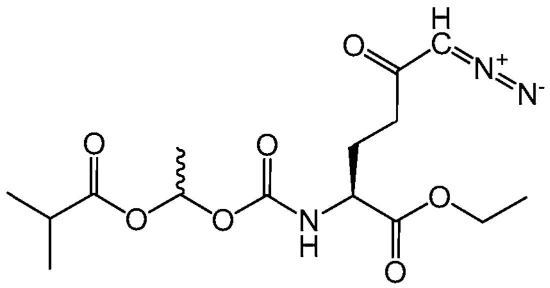 | 357.37 |
| Compound **32** | 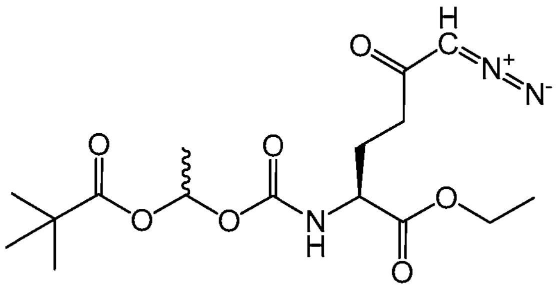 | 371.39 |
| Compound **34** | 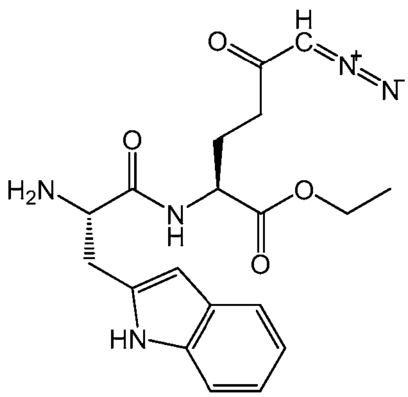 | 385.42 |

(continued)

| Table 1. Structures of Representative DON Prodrugs | | |
|---|---|---|
| IOCB No./ Compound No. | Structure | MW |
| Compound **35** | 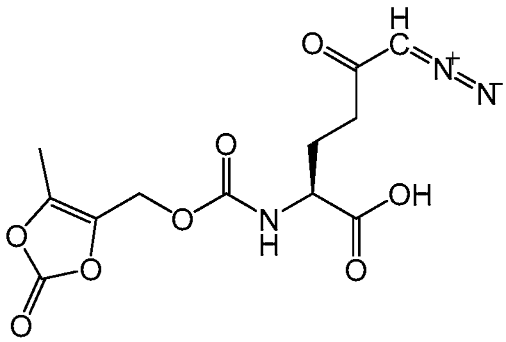 | 327.25 |
| Compound **36** | 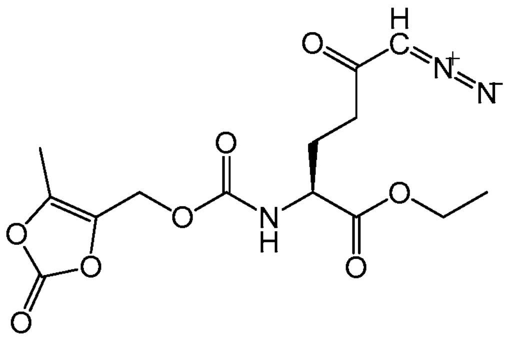 | 355.30 |
| Compound **38** | 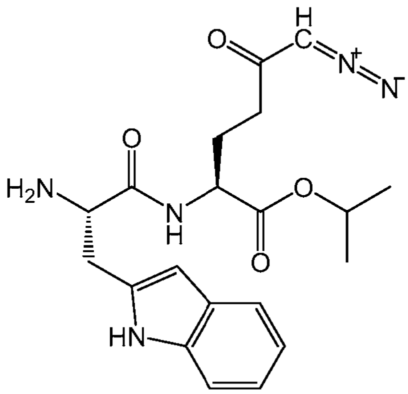 | 399.45 |
| Compound **40** | 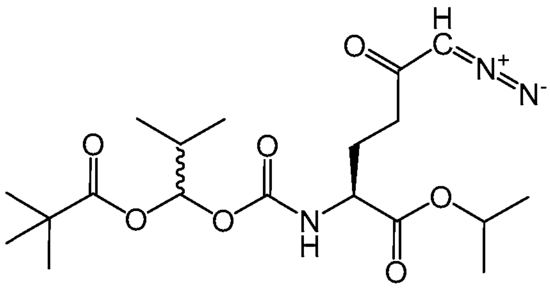 | 413.47 |

(continued)

| Table 1. Structures of Representative DON Prodrugs | | |
|---|---|---|
| IOCB No./ Compound No. | Structure | MW |
| Compound **42** | 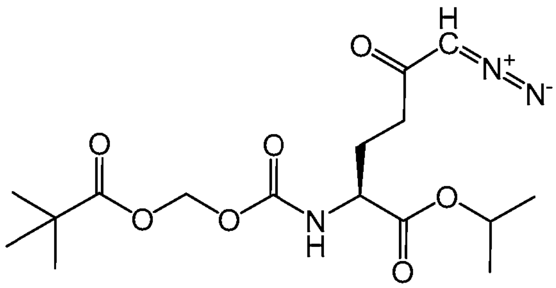 | 371.39 |
| Compound **44** | 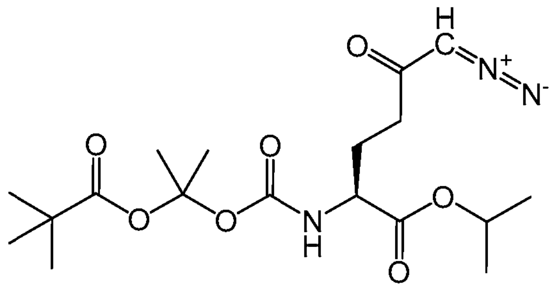 | 2.44 |
| Compound **47** | 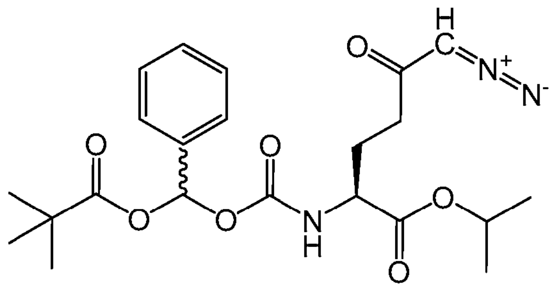 | 447.49 |
| Compound **49** | 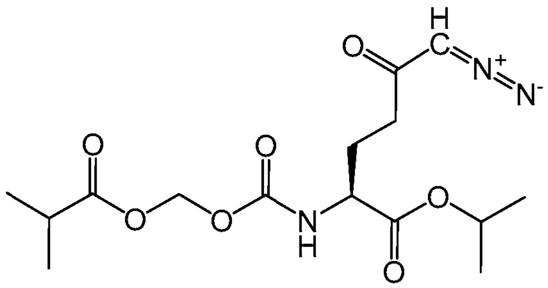 | 357.36 |
| Compound **51** | 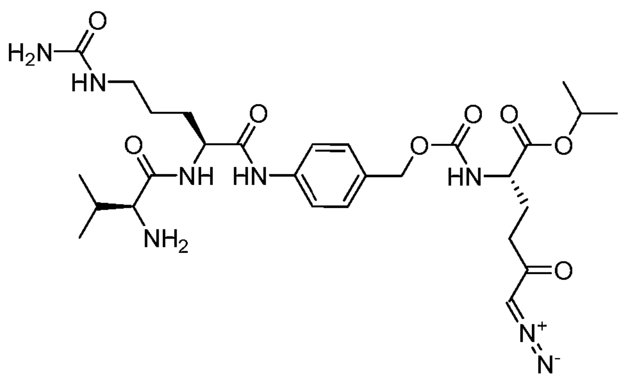 | 618.69 |

(continued)

| Table 1. Structures of Representative DON Prodrugs | | |
|---|---|---|
| IOCB No./ Compound No. | Structure | MW |
| Compound **52** | 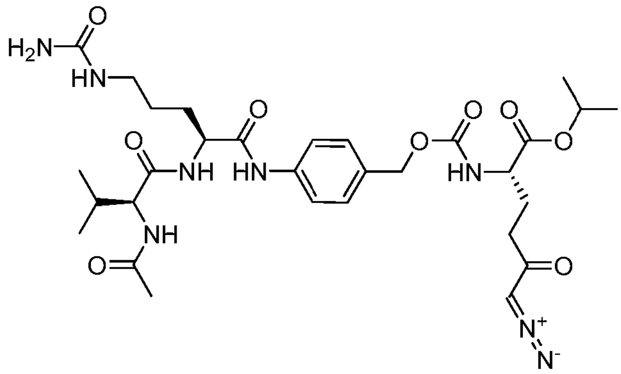 | 660.73 |
| Compound **56** | 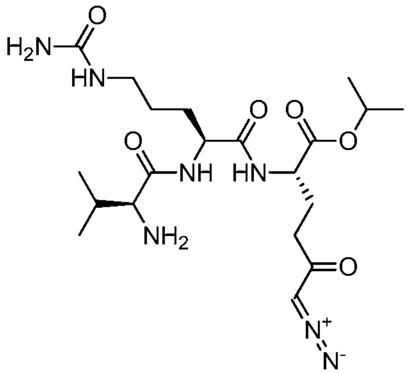 | 469.54 |
| Compound **57** | 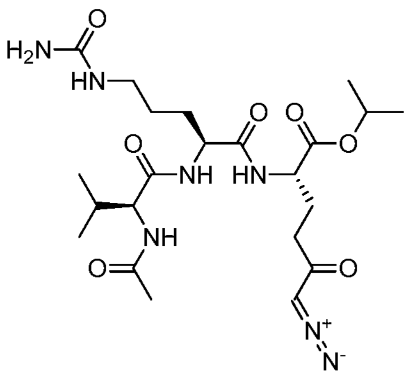 | 511.58 |
| Compound **59** | 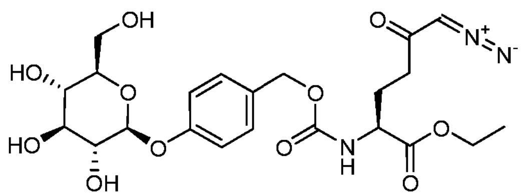 | 511.48 |

(continued)

| Table 1. Structures of Representative DON Prodrugs | | |
|---|---|---|
| IOCB No./ Compound No. | Structure | MW |
| Compound **60** | 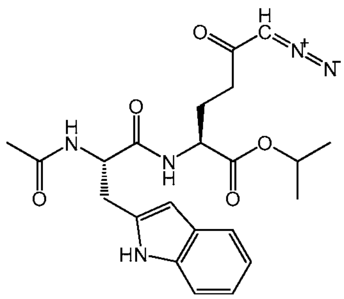 | 464.19 |
| A | 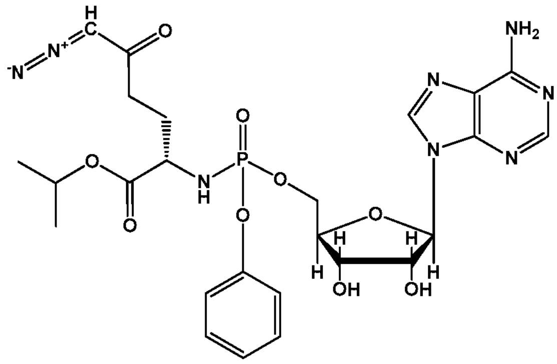 | 618.54 |
| B | 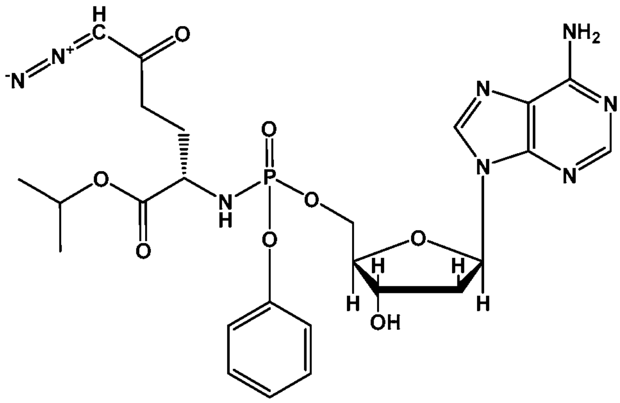 | 602.54 |
| C | 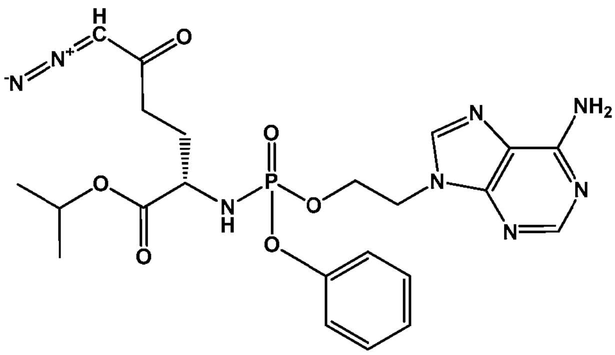 | 530.47 |

(continued)

| Table 1. Structures of Representative DON Prodrugs | | |
|---|---|---|
| IOCB No./ Compound No. | Structure | MW |
| D | 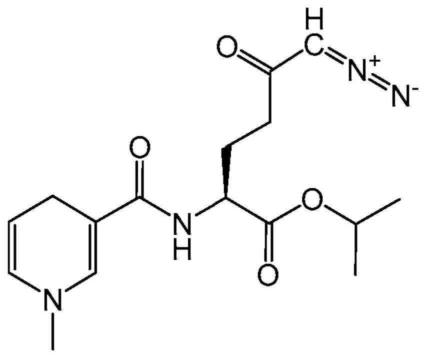 | 334.38 |
| E | 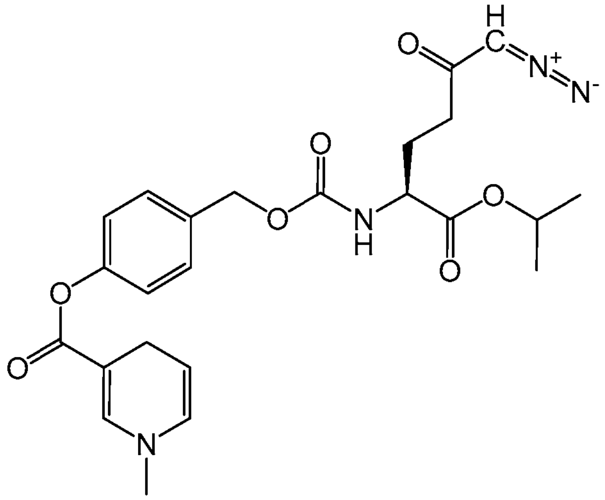 | 484.51 |
| F | 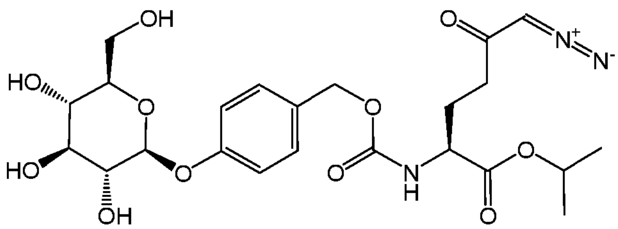 | 525.51 |
| G | 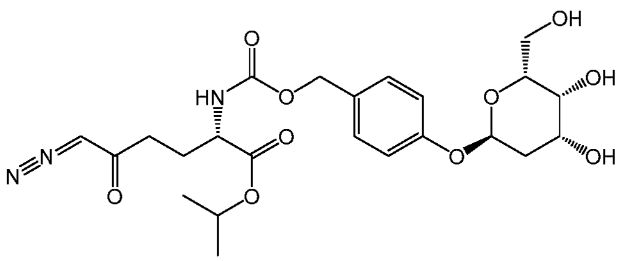 | 509.51 |

(continued)

| Table 1. Structures of Representative DON Prodrugs | | |
|---|---|---|
| IOCB No./ Compound No. | Structure | MW |
| **LTP 073** | 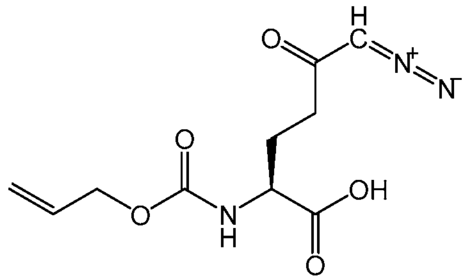 | 255.23 |
| **JAM0351** | 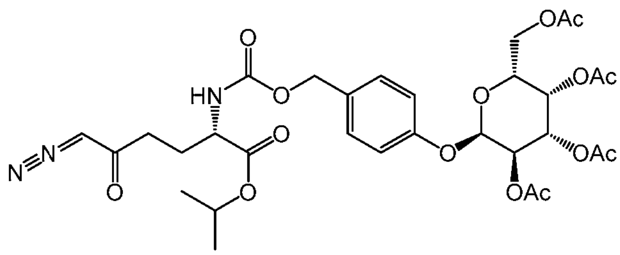 | 693.66 |
| **JAM0359** | 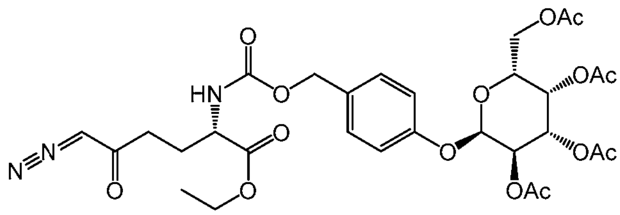 | 679.63 |

1. CHECKPOINT BLOCKADE

Aspects of the presently disclosed subject matter involve the use of metabolic reprogramming agents (e.g., DON, DON prodrugs, etc.) in a combination immunotherapy together with checkpoint blockade modulators, for example, to enhance checkpoint blockade therapies for the treatment of cancer. In some aspects, the presently disclosed subject matter involves the use of metabolic reprogramming agents (e.g., DON, DON prodrugs, etc.) in combination immunotherapy together with A2aR blockade,.

Examples of immune checkpoint modulators of use herein include, but are not limited to, small organic molecules (e.g., haptens) or small inorganic molecules; saccharides; oligosaccharides; polysaccharides; a biological macromolecule selected from the group consisting of peptides (e.g., aptides), proteins, peptide analogs and derivatives; peptidomimetics; nucleic acids selected from the group consisting of miRNAs, siRNAs, shRNAs, antisense nucleic acids, such as antisense RNAs, ribozymes, and aptamers; an extract made from biological materials selected from the group consisting of bacteria, plants, fungi, animal cells, and animal tissues; naturally occurring or synthetic compositions; and any combination thereof. Other examples of immune checkpoint modulators include orthosteric inhibitors, allosteric regulators, interfacial binders, and molecular analogues of substrates that act as competitive inhibitors.

Specific examples of immune checkpoint modulators include, without limitation, PD-1 antagonists, PD-L1 antagonists, CTLA-4 antagonists, Lag-3 antagonists, CD137 antagonists, KIR antagonists, Tim3 antagonists, Ox40 agonists, B7-H3 antagonists, and combinations thereof.

Exemplary CTLA-4 antagonists include, without limitation, ipilimumab, tremelimumab and combinations thereof. Anti-CTLA-4 antibodies are currently undergoing clinical trials for the treatment of melanoma.

Examplary Lag-3 antagonists include, without limitation, BMS-986016 and IMP321.

Exemplary CD137 antagonists include, without limitation, CD137-specific antibody, peptide, organic small molecule, antisense oligonuclotide, siRNA, antisense expression vector or recombinant virus. In some embodiments, the CD137-specific antibody is clone BBK-2 or clone 4B4-1, as described in WIPO International Application Publication No. WO200405513A2.

T-cell immunoglobulin and mucin domain 3 (TIM3) antagonists (e.g., anti-TIM3 antibodies) have been described

for use as immunotherapy (see, e.g., Ngiow et al. 2011). Exemplary Tim3 antagonists include, without limitation, anti-TIM3 monoclonal antibodies, for example, as described in the poster presentation by Jun et al. "Generation of antagonistic anti-TIM-3 and anti-LAG-3 monoclonal antibodies for potential novel immunotherapy combinations", available on the world wide web at http://www.tesarobio.com/documents/2014AACRposterLB266.pdf.

Ox40 agonists are described by Linch et al., "OX40 Agonists and Combination Immunotherapy: Putting the Pedal to the Metal" Front Oncol 5:34; 2015. Exemplary Ox40 agonists include, without limitation, anti-Ox40 agonists antibodies. Other exemplary Ox40 agonists include, without limitation, OX86 and Fc-OX40L.

Exemplary B7-H3 antagonists include, without limitation, MGA271.

Exemplary PD-L1 antagonists include, without limitation, BMS-936559/MDX-1105, MEDI4736, MPDL3280A, MPDL3280A, MSB0010718C, and combinations thereof. PD-L1 antagonists are currently undergoing clinical trials, for example, for the treatment of melanoma, non-small cell lung cancer, renal cell carcinoma, and ovarian cancer.

PD-1 antagonists have been reviewed (see, e.g., Dolan and Gupta 2014). Exemplary PD-1 antagonists of use herein include, without limitation, AMP-224, AMP-554, nivolumab, pembrolizumab, pidilizumab, and combinations thereof.

In some embodiments described herein, the PD-1 antagonists comprise anti-PD-1 antibodies. Exemplary anti-PD-1 antibodies include, without limitation, atezolizumab, nivolumab, pembrolizumab, pidilizumab, and combinations thereof. PD-1 antagonists are currently undergoing clinical trials, for example, for the treatment of colorectal cancer, gastric cancer, melanoma, non-small cell lung cancer, ovarian cancer, pancreatic cancer, and renal cell carcinoma.

In particular embodiments described herein, the presently disclosed subject matter provides a method of treating an advanced solid tumor, the method comprising: (a) administering to the subject a therapeutically effective amount of BMS-936559; and (b) sequentially or simultaneously administering to the subject a therapeutically effective amount of a metabolic reprogramming agent that decreases glutamine metabolism.

In particular embodiments described herein, the presently disclosed subject matter provides a method of treating an advanced solid tumor, the method comprising: (a) administering to the subject a therapeutically effective amount of MEDI4736; and (b) sequentially or simultaneously administering to the subject a therapeutically effective amount of a metabolic reprogramming agent that decreases glutamine metabolism.

In particular embodiments described herein, the presently disclosed subject matter provides a method of treating melanoma, the method comprising: (a) administering to the subject a therapeutically effective amount of MPDL3280A in combination with vemurafenib; and (b) sequentially or simultaneously administering to the subject a therapeutically effective amount of a metabolic reprogramming agent that decreases glutamine metabolism.

In particular embodiments described herein, the presently disclosed subject matter provides a method of treating melanoma, the method comprising: (a) administering to the subject a therapeutically effective amount of MEDI4736 in combination with dabrafenib and trametinib; and (b) sequentially or simultaneously administering to the subject a therapeutically effective amount of a metabolic reprogramming agent that decreases glutamine metabolism. In particular embodiments, the presently disclosed subject matter provides a method of treating melanoma, the method comprising: (a) administering to the subject a therapeutically effective amount of MEDI4736 in combination with trametinib; and (b) sequentially or simultaneously administering to the subject a therapeutically effective amount of a metabolic reprogramming agent that decreases glutamine metabolism.

In particular embodiments described herein, the presently disclosed subject matter provides a method of treating non-small cell lung cancer, the method comprising: (a) administering to the subject a therapeutically effective amount of MPDL3280A; and (b) sequentially or simultaneously administering to the subject a therapeutically effective amount of a metabolic reprogramming agent that decreases glutamine metabolism. In some embodiments described herein, MPDL3280A is administered in combination with erlotinib. In particular embodiments described herein, the presently disclosed subject matter provides a method of treating non-small cell lung cancer, the method comprising: (a) administering to the subject a therapeutically effective amount of MEDI4736; and (b) sequentially or simultaneously administering to the subject a therapeutically effective amount of a metabolic reprogramming agent that decreases glutamine metabolism. In some embodiments, MEDI4736 is administered in combination with tremelimumab.

In particular embodiments described herein, the presently disclosed subject matter provides a method of treating renal cell carcinoma, the method comprising: (a) administering to the subject a therapeutically effective amount of MPDL3280A; and (b) sequentially or simultaneously administering to the subject a therapeutically effective amount of a metabolic reprogramming agent that decreases glutamine metabolism. In some embodiments described herein, MPDL3280A is administered in combination with bevacizumab.

In particular embodiments described herein, the presently disclosed subject matter provides a method of treating a solid or hematological malignancy, the method comprising: (a) administering to the subject a therapeutically effective amount of MPDL3280A; and (b) sequentially or simultaneously administering to the subject a therapeutically effective amount of a metabolic reprogramming agent that decreases glutamine metabolism.

In particular embodiments described herein, the presently disclosed subject matter provides a method of treating a solid tumor, the method comprising: (a) administering to the subject a therapeutically effective amount of MPDL3280A;

and (b) sequentially or simultaneously administering to the subject a therapeutically effective amount of a metabolic reprogramming agent that decreases glutamine metabolism. In some embodiments described herein, MPDL3280A is administered in combination with bevacizumab and/or chemotherapy. In some embodiments, MPDL3280A is administered in combination with cobimetinib.

In particular embodiment described herein s, the presently disclosed subject matter provides a method of treating a solid tumor, the method comprising: (a) administering to the subject a therapeutically effective amount of MEDI4736; and (b) sequentially or simultaneously administering to the subject a therapeutically effective amount of a metabolic reprogramming agent that decreases glutamine metabolism. In some embodiment described herein s, MEDI4736 is administered in combination with tremelimumab.

In particular embodiments described herein, the presently disclosed subject matter provides a method of treating a solid tumor, the method comprising: (a) administering to the subject a therapeutically effective amount of MSB0010718C; and (b) sequentially or simultaneously administering to the subject a therapeutically effective amount of a metabolic reprogramming agent that decreases glutamine metabolism.

In particular embodiments described herein, the presently disclosed subject matter provides a method of treating advanced cancer, the method comprising: (a) administering to the subject a therapeutically effective amount of AMP-224; and (b) sequentially or simultaneously administering to the subject a therapeutically effective amount of a metabolic reprogramming agent that decreases glutamine metabolism.

In particular embodiments described herein, the presently disclosed subject matter provides a method of treating an advanced solid tumor, the method comprising: (a) administering to the subject a therapeutically effective amount nivolumab in combination with iliolumbar (anti-KIR); and (b) sequentially or simultaneously administering to the subject a therapeutically effective amount of a metabolic reprogramming agent that decreases glutamine metabolism.

In particular embodiments described herein, the presently disclosed subject matter provides a method of treating a castration-resistant prostate cancer, hepatocellular carcinoma, melanoma, non-small cell lung cancer, or renal cell carcinoma, the method comprising: (a) administering to the subject a therapeutically effective amount of nivolumab; and (b) sequentially or simultaneously administering to the subject a therapeutically effective amount of a metabolic reprogramming agent that decreases glutamine metabolism.

In particular embodiments described herein, the presently disclosed subject matter provides a method of treating colon cancer, gastric cancer, head and neck cancer, Hodgkin lymphoma, melanoma, myeloma, myelodysplastic syndrome, non-Hodkin lymphoma, non-small cell lung cancer, solid tumors, or triple-negative breast cancer, the method comprising: (a) administering to the subject a therapeutically effective amount of pembrolizumab; and (b) sequentially or simultaneously administering to the subject a therapeutically effective amount of a metabolic reprogramming agent that decreases glutamine metabolism.

In particular embodiments described herein, the presently disclosed subject matter provides a method of treating gastric cancer, pancreatic cancer, small-cell lung cancer, glioblastoma, or triple-negative breast cancer, the method comprising: (a) administering to the subject a therapeutically effective amount of nivolumab in combination with ipilimumab; and (b) sequentially or simultaneously administering to the subject a therapeutically effective amount of a metabolic reprogramming agent that decreases glutamine metabolism.

In particular embodiments described herein, the presently disclosed subject matter provides a method of treating a malignant glioma, the method comprising: (a) administering to the subject a therapeutically effective amount of pidilizumab; and (b) sequentially or simultaneously administering to the subject a therapeutically effective amount of a metabolic reprogramming agent that decreases glutamine metabolism.

In particular embodiments described herein, the presently disclosed subject matter provides a method of treating pancreatic cancer, the method comprising: (a) administering to the subject a therapeutically effective amount of pidilizumab in combination with gemcitabine; and (b) sequentially or simultaneously administering to the subject a therapeutically effective amount of a metabolic reprogramming agent that decreases glutamine metabolism.

In particular embodiments described herein, the presently disclosed subject matter provides a method of treating renal cell carcinoma, the method comprising: (a) administering to the subject a therapeutically effective amount of pidilizumab in combination with dendritic cell/RCC fusion cell vaccine; and (b) sequentially or simultaneously administering to the subject a therapeutically effective amount of a metabolic reprogramming agent that decreases glutamine metabolism.

1. ADOPTIVE CELLULAR THERAPY

Adoptive cell therapies (ACT) are a useful approach for treating cancer. Adoptive cell transfer refers to the passive transfer of ex vivo grown cells, often immune-derived cells, into a host with the aim of transferring the immunologic functionality and characteristics of the transplant. Adoptive cell transfer can be autologous, as is common in adoptive T-cell therapies, or allogeneic. The adoptive transfer of autologous tumor infiltrating lymphocytes (TILs) or genetically re-directed peripheral blood mononuclear cells has been used to successfully treat patients with advanced solid tumors

such as melanoma as well as patients with CD19-expressing hematologic malignancies. Exemplary cell types for use in ACT include, without limitation, T-cells (e.g., CD8+ cells, CD4+ cells, etc.), NK-cells, delta-gamma T-cells, regulatory T-cells and peripheral blood mononuclear cells. Such cells can be unmodified such as in TIL therapy or genetically modified. One way to achieve genetic targeting of T-cells to tumor specific targets is the transfer of a T-cell receptor with known specificity (TCR therapy) and with matched human leukocyte antigen (HLA, known as major histocompatibility complex in rodents) type. Another way is the modification of cells with artificial molecules such as chimeric antigen receptors (CAR), commonly known as CAR-T cell therapy. For example, single chain antibodies can be used and CARs can also incorporate co-stimulatory domains.

Aspects of the presently disclosed subject matter involve the use of metabolic reprogramming agents (e.g., DON, DON prodrugs, etc.) in combination immunotherapy together with adoptive cellular therapy, for example, to enhance adoptive cellular therapy for the treatment of cancer.

Accordingly, in some embodiments described herein, the method of treating cancer further includes simultaneously or sequentially administering a therapeutically effective amount of the second immunotherapy to the subj ect, wherein the second immunotherapy is an adoptive cellular therapy. In some embodiment described herein s, the adoptive cellular therapy is selected from the group consisting of T-cells, CD8+ cells, CD4+ cells, NK-cells, delta-gamma T-cells, marrow infiltrating lymphocytes (MILs), regulatory T-cells, and peripheral blood mononuclear cells. In some embodiments described herein, the adoptive cellular therapy comprises a tumor infiltrating lymphocyte (TIL). In some embodiments described herein, the adoptive cellular therapy comprises a T-cell receptor modified lymphocyte. In some embodiments described herein, the adoptive cellular therapy comprises a chimeric antigen receptor modified lymphocyte. In some embodiments described herein, the adoptive cellular therapy comprises a chimeric antigen receptor T (CAR-T) cell. In some embodiments described herein, the adoptive cellular therapy comprises marrow infiltrating lymphocytes (MILs). In some embodiments described herein, the method of treating cancer further includes simultaneously or sequentially administering a therapeutically effective amount of the second immunotherapy to the subject, wherein the second immunotherapy is marrow-infiltrating lymphocytes (MILs).

2. ADENOSINE A2AR BLOCKADE

Adenosine A2a receptor (A2aR) blockade has been reviewed and is reported to enhance tumor vaccines, checkpoint blockade and adoptive T cell therapy (see, e.g., Powell et al. 2015). Accordingly, aspects of the presently disclosed subject matter involves the use of metabolic reprogramming agents (e.g., DON, DON prodrugs, etc.) in combination immunotherapy together with adenosine A2a receptor (A2aR) blockade, for example, to enhance A2aR blockade for the treatment of cancer. In some aspects, the presently disclosed subject matter involves the use of metabolic reprogramming agents (e.g., DON, DON prodrugs, etc.) in combination immunotherapy together with A2aR blockade, and optionally in combination with a third immunotherapy, a fourth immunotherapy, or a fifth immunotherapy, such as tumor vaccines, checkpoint blockade and/or adoptive cell therapy.

In some embodiments described herein, the method of treating cancer further includes simultaneously or sequentially administering a therapeutically effective amount of the second immunotherapy to the subject, wherein the second immunotherapy is an adenosine A2aR blockade. Exemplary A2aR inhibitors of use in the A2aR blockade as an immunotherapy include, without limitation, SCH58261, SYN115, ZM241365 and FSPTP.

In particular embodiments described herein, the presently disclosed subject matter provides a method for treatment of a subject with a CD73-expressing tumor, the method comprising: (a) administering a therapeutically effective amount of SCH58261 to the subject; and (b) sequentially or simultaneously administering to the subject a therapeutically effective amount of a metabolic reprogramming agent that decreases glutamine metabolism. Exemplary CD73-expressing tumors include, without limitation, breast tumors (e.g., breast adenocarcinoma, metastatic breast cancer) and melanoma (e.g., metastatic). In some embodiments, the CD73-expressing tumor is a metastatic tumor and administration of SCH58261 suppresses metastases in the CD73-expressing tumor. In some embodiments described herein, the CD73-expressing tumor is melanoma and SCH58261 and the metabolic reprogramming agent are administered in combination with an anti-PDl antibody to prolong survival and reduce the metastatic melanoma burden. In some embodiments described herein, the CD73-expressing tumor is breast cancer and SCH58261 and the metabolic reprogramming agent are administered in combination with an anti-PDl antibody to prolong survival and reduce the metastatic breast cancer burden. In some embodiments described herein, the CD73-expressing tumor is a breast cancer tumor and SCH58261 and the metabolic reprogramming agent are administered in combination with a chemotherapeutic agent (e.g., doxorubicin) to increase the sensitivity of the breast cancer tumor to the chemotherapeutic agent.

In particular embodiments described herein, the presently disclosed subject matter provides a method for treatment of a subject with a CD73-expressing tumor, the method comprising: (a) administering a therapeutically effective amount of SYN115 to the subject; and (b) sequentially or simultaneously administering to the subject a therapeutically effective amount of a metabolic reprogramming agent that decreases glutamine metabolism. In some embodiments described herein, an anti-PD-1 antibody is administered in combination with SYN115 and the metabolic reprogramming

agent.

In particular embodiments described herein, the presently disclosed subject matter provides a method for treatment of melanoma, the method comprising: (a) administering a therapeutically effective amount of ZM241365 to the subject; and (b) sequentially or simultaneously administering to the subject a therapeutically effective amount of a metabolic reprogramming agent that decreases glutamine metabolism. In some embodiments, an anti-CTLA4 antibody is administered in combination with ZM241365 and the metabolic reprogramming agent.

In particular embodiments described herein, the presently disclosed subject matter provides a method for treatment of bladder cancer, the method comprising: (a) administering a therapeutically effective amount of FSPTP to the subject; and (b) sequentially or simultaneously administering to the subject a therapeutically effective amount of a metabolic reprogramming agent that decreases glutamine metabolism. In some embodiments, FSPTP is administered via intratumoral injection.

3. KILLER-CELL IMMUNOGLOBULIN-LIKE RECEPTOR (KIR) BLOCKADE

Unclaimed aspects of the presently disclosed subject matter involve the use of metabolic reprogramming agents (e.g., DON, DON prodrugs, etc.) in combination immunotherapy together with killer-cell immunoglobulin-like receptor (KIR) blockade, for example, to enhance KIR blockade for the treatment of cancer. In some aspects, the presently disclosed subject matter involves the use of metabolic reprogramming agents (e.g., DON, DON prodrugs, etc.) in combination immunotherapy together with KIR blockade, and optionally in combination with a third immunotherapy, a fourth immunotherapy, and/or a fifth immunotherapy, such as tumor vaccines, adoptive cell therapy, A2aR blockade, and/or checkpoint blockade.

In some embodiments described herein, the method of treating cancer further includes simultaneously or sequentially administering a therapeutically effective amount of the second immunotherapy to the subject, wherein the second immunotherapy is a KIR blockade.

Exemplary KIR inhibitors of use in the KIR blockade as an immunotherapy include, without limitation, IPH2102/BMS-986015 (lirilumab).

In particular embodiments described herein, the presently disclosed subject matter provides a method of treating acute myeloid leukemia, the method comprising: (a) administering a therapeutically effective amount of lirilumab to the subject; and (b) sequentially or simultaneously administering to the subject a therapeutically effective amount of a metabolic reprogramming agent that decreases glutamine metabolism.

In particular embodiments described herein, the presently disclosed subject matter provides a method of treating a solid tumor, the method comprising: (a) administering a therapeutically effective amount of lirilumab to the subject; and (b) sequentially or simultaneously administering to the subject a therapeutically effective amount of a metabolic reprogramming agent that decreases glutamine metabolism. In some embodiments described herein, the solid tumor is a melanoma tumor, and lirilumab is administered in combination with nivolumab. In some embodiments described herein, the solid tumor is a non-small cell lung cancer tumor, and lirilumab is administered in combination with nivolumab. In some embodiments described herein, the solid tumor is a gastrointestinal tumor and lirilumab is administered in combination with nivolumab. In some embodiments described herein, the solid tumor is a squamous cell carcinoma of the head and neck tumor and lirilumab is administered in combination with nivolumab. In some embodiments described herein, the solid tumor is a hepatocellular carcinoma tumor and lirilumab is administered in combination with nivolumab.

In particular embodiment described herein s, the presently disclosed subject matter provides a method of treating a hematological tumor, the method comprising: (a) administering a therapeutically effective amount of lirilumab to the subject; and (b) sequentially or simultaneously administering to the subject a therapeutically effective amount of a metabolic reprogramming agent that decreases glutamine metabolism. In some embodiments described herein, the hematological tumor is relapsed and/or refractory non-Hodgkin's lympohoma and lirilumab is administered in combination with nivolumab. In some embodiments described herein, the hematological tumor is relapsed and/or refractory Hodgkin's lympohoma and lirilumab is administered in combination with nivolumab. In some embodiments described herein, the hematological tumor is relapsed and/or refractory multiple myeloma and lirilumab is administered in combination with nivolumab. In some embodiments, the hematological tumor is relapsed and/or refractory chromic myelogenous leukemia and lirilumab is administered in combination with nivolumab.

In particular embodiments described herein, the presently disclosed subject matter provides a method of treating relapsed and/or refractory multiple myeloma post autologous transplant, the method comprising: (a) administering a therapeutically effective amount of lirilumab to the subject optionally in combination with elotuzumab; and (b) sequentially or simultaneously administering to the subject a therapeutically effective amount of a metabolic reprogramming agent that decreases glutamine metabolism.

In particular embodiments described herein, the presently disclosed subject matter provides a method of treating relapsed and/or refractory acute myeloid leukemia, the method comprising: (a) administering a therapeutically effective amount of lirilumab to the subject optionally in combination with 5-azacytidine; and (b) sequentially or simultaneously

administering to the subject a therapeutically effective amount of a metabolic reprogramming agent that decreases glutamine metabolism.

4. VACCINES

Vaccines stimulate the body's immune system to attack abnormal or malignant ells, such as cancer, resulting in a reduction of the those cells. Cancer or tumor vaccines typically contain a tumor antigen in an immunogeniuc formulation that stimulates tumor antigen-specific helper cells, CTLs and/or B cells. Exemplary formulations of vaccines include, without limitations, dendritic cells (e.g., autologous dendritic cells pulsed with tumor cells or antigens), heterologous tumor cells transfected with immunostimulant agents, such as GM-CSF, recombinant virus, and/or peptides or proteins administered with adjuvants, such as CpG.

Unclaimed aspects of the presently disclosed subject matter involve combination immunotherapy using metabolic reprogramming agents (e.g., that decrease glutamine metabolism (e.g., DON or a DON progrog) sequentially or simultaneously with vaccines, for example, for the treatment of cancer. It is believed that when used as a combination immunotherapy with vaccines the metabolic reprogramming agents can help delay or stop cancer cell growth, cause tumor shrinkage, prevent cancer from recurring, and/or eliminate cancer cells that have not been eradicated by other treatments.

Accordingly, in some embodiments described herein, the method of treating cancer further includes simultaneously or sequentially administering a therapeutically effective amount of the second immunotherapy to the subj ect, wherein the second immunotherapy is a vaccine (e.g., tumor vaccine).

Exemplary such vaccines include, without limitation, peptide vaccines, dendritic cell (DC) vaccines, EGFRvIII vaccines, mesothilin vaccine, G-VAX, listeria vaccines, and a dentritic cell/RCC fusion cell vaccine.

In particular embodiments described herein, the presently disclosed subject matter provides a method for the treatment or prevention of a human papillomavirus (HPV)-associated cancer in a subject in need thereof, the method comprising: (a) administering to the subject a therapeutically effective amount of a recombinant HPV vaccine; and (b) simultaneously or sequentially administering a therapeutically effective amount of a metabolic reprogramming agent that decreases glutamine metabolism.

In some embodiments described herein, the subject has an oncogenic HPV-type 6 infection and the rHPV vaccine comprises a rHPV type 6 vaccine. In some embodiments, the subject has an oncogenic HPV-type 11 infection and the rHPV vaccine comprises a rHPV type 11 vaccine. In some embodiments described herein, the subject has an oncogenic HPV-type 16 infection and the rHPV vaccine comprises a rHPV type 16 vaccine. In some embodiments described herein, the subject has an oncogenic HPV-type 18 infection and the rHPV vaccine comprises a rHPV type 18 vaccine. In some embodiments described herein, the subject is a female and the cancer is selected from the group consisting of cervical, vaginal and vulvar cancer. In some embodiments described herein, the subject is a female between the 9 and 26 years old. In some embodiments, the subject is a female between 9 and 26 years old and the HPV-associated cancer is cervical cancer. In some embodiments described herein, the subject is a female between 9 and 26 years old and the HPV-associated cancer is vaginal cancer. In some embodiments described herein, the subject is a female between 9 and 26 years old and the HPV-associated cancer is vulvar cancer. In some embodiments, the subject is male or female and the HPV-associated cancer comprises anal cancer. In some embodiments described herein, the subject is a male or female between 9 and 26 years old and the the HPV-associated cancer is anal cancer. In some embodiments described herein, the HPV vaccine comprises GARDASIL. In some embodiments described herein, the subject is a female between 10 and 25 years old and the HPV-associated cancer is cervical cancer. In some embodiments described herein, the HPV vaccine comprises CERVARIX. In some embodiments described herein, the HPV vaccine is administered intramuscularly via injection as a suspension.

In particular embodiments described herein, the presently disclosed subject matter provides a method for the treatment or prevention of a hepatitis B-associated cancer in a subject in need thereof, the method comprising: (a) administering to the subject a therapeutically effective amount of a hepatitis B vaccine; and (b) simultaneously or sequentially administering a therapeutically effective amount of a metabolic reprogramming agent that decreases glutamine metabolism. In some embodiments described herein, the subject has a hepatitis B virus infection and the cancer is ilver cancer.

6. PASSIVE IMMUNOTHERAPY

Unclaimed aspects of the presently disclosed subject matter involve combination immunotherapy using metabolic reprogramming agents (e.g., that decrease glutamine metabolism (e.g., DON or a DON prodrug) sequentially or simultaneously with passive immunotherapy antibodies for the treatment of cancer.

Accordingly, in some embodiments described herein, the method of treating cancer further includes simultaneously or sequentially administering a therapeutically effective amount of the second immunotherapy to the subj ect,

wherein the second immunotherapy is a passive immunotherapy antibody. As used herein, "passive immunotherapy antibody" refers to monoclonal antibodies targeted to cancer cell-surface specific antigens to provide cancer immunity without actively stimulating a patient's immune system.

Exemplary passive immunotherapy antibodies of use herein include, without limitation, bevacizumab (e.g., AVASTIN), cetuximab (e.g., ERBITUX), rituximab (e.g., RITUXAN), trastuzumab (e.g., HERCEPTIN), alemtuzumab (e.g., CAMPATH), ibritumomab tiuxetan (e.g., ZEVALIN), panitumumab (e.g., VECTIBIX).

In particular embodiments described herein, the presently disclosed subject matter provides a method for treatment of a subject with metastatic colorectal cancer, the method comprising: (a) administering a therapeutically effective amount of bevacizumab to the subject; and (b) sequentially or simultaneously administering to the subject a therapeutically effective amount of a metabolic reprogramming agent that decreases glutamine metabolism. In some embodiments described herein, bevacizumab is administered as a first- or second-line treatment in combination with intravenous 5-fluorouracil-based chemotherapy. In some embodiments described herein, bevacizumab is administered as a second-line treatment in combination with fluoropyrimidine-based (combined with irinotecan or oxaliplatin) chemotherapy after cancer progression following a first-line treatment that includes bevacizumab.

In particular embodiments described herein, the presently disclosed subject matter provides a method for treatment of a subject with non-small cell lung cancer, the method comprising: (a) administering a therapeutically effective amount of bevacizumab to the subject; and (b) sequentially or simultaneously administering to the subject a therapeutically effective amount of a metabolic reprogramming agent that decreases glutamine metabolism. In some embodiments described herein, the non-small cell lung cancer comprises advanced nonsquamous non-small cell lung cancer and bevacizumab is administered to subjects who have not received chemotherapy for their advanced disease in combination with carboplatin and paclitaxel.

In particular embodiments described herein, the presently disclosed subject matter provides a method for treatment of a subject with plantinum-resistant ovarian cancer, the method comprising: (a) administering a therapeutically effective amount of bevacizumab to the subject; and (b) sequentially or simultaneously administering to the subject a therapeutically effective amount of a metabolic reprogramming agent that decreases glutamine metabolism. In some embodiments described herein, the subject has had no more than two prior chemotherapy treatments and bevacizumab is used to treat plantinum-resistant recurrent epithelial ovarian, fallopian tube or primary peritoneal cancer in the subject in combination with paclitaxel, pegylated liposomal doxorubicin or topotecan.

In particular embodiments described herein, the presently disclosed subject matter provides a method for treatment of a subject with advanced cervical cancer, the method comprising: (a) administering a therapeutically effective amount of bevacizumab to the subject; and (b) sequentially or simultaneously administering to the subject a therapeutically effective amount of a metabolic reprogramming agent that decreases glutamine metabolism. In some embodiments described herein, the subject has persistent, recurrent, or metastatic cervical cancer and bevacizumab is administered in combination with paclitaxel and cisplatin. In some embodiments described herein, the subject has persistent, recurrent, or metastatic cervical cancer and bevacizumab is administered in combination with paclitaxel and topotecan.

In particular embodiments described herein, the presently disclosed subject matter provides a method for treatment of a subject with metastatic renal cell carcinoma, the method comprising: (a) administering a therapeutically effective amount of bevacizumab to the subject; and (b) sequentially or simultaneously administering to the subject a therapeutically effective amount of a metabolic reprogramming agent that decreases glutamine metabolism. In some embodiments described herein, the subject has metastatic kidney cancer and bevacizumab is administered in combination with interferon alfa.

In particular embodiments described herein, the presently disclosed subject matter provides a method for treatment of a subject with recurrent glioblastoma, the method comprising: (a) administering a therapeutically effective amount of bevacizumab to the subject; and (b) sequentially or simultaneously administering to the subject a therapeutically effective amount of a metabolic reprogramming agent that decreases glutamine metabolism. In some embodiments described herein, the subject with recurrent glioblastoma is an adult.

In particular embodiments described herein, the presently disclosed subject matter provides a method for treatment of a subject with head and neck cancer, the method comprising: (a) administering a therapeutically effective amount of cetuximab to the subject; and (b) sequentially or simultaneously administering to the subject a therapeutically effective amount of a metabolic reprogramming agent that decreases glutamine metabolism. In some embodiments described herein, the subject has locally or regionally advanced squamous cell carcinoma of the head and neck and cetuximab is administered in combination with radiotherapy. In some embodiments described herein, the subject has recurrent locoregional disease or metastatic squamous cell carcinoma of the head and neck and cetuximab is administered in combination with platinum-based therapy with 5-FU. In some embodiments described herein, the subject has recurrent or metastatic squamous cell carcinoma of the head and neck and has failed to respond to prior platinum-based therapy.

In particular embodiments described herein, the presently disclosed subject matter provides a method for treatment of a subject with metastatic colorectal cancer, the method comprising: (a) administering a therapeutically

effective amount of cetuximab to the subject; and (b) sequentially or simultaneously administering to the subject a therapeutically effective amount of a metabolic reprogramming agent that decreases glutamine metabolism. In some embodiments described herein, the metastatic colorectal cancer comprises *KRAS* wild-type, epidermal growth factor rector (EGFR)-expressing, metastatic colorectal cancer, and cetuximab is administered in combination with FOLFIRI (irinotecan, 5-fluorouracil, and lucovorin). In some embodiments described herein, the metastatic colorectal cancer comprises *KRAS* wild-type, epidermal growth factor rector (EGFR)-expressing, metastatic colorectal cancer, the subject is refractory to irinotecan-based chemotherapy, and cetuximab is administered in combination with irinotecan. In some embodiments described herein, the metastatic colorectal cancer comprises *KRAS* wild-type, epidermal growth factor rector (EGFR)-expressing, metastatic colorectal cancer, and the subject has failed to respond to oxaliplatin and irinotecan-based chemotherapy. In some embodiments described herein, the metastatic colorectal cancer comprises *KRAS* wild-type, epidermal growth factor rector (EGFR)-expressing, metastatic colorectal cancer, and the subject is inteolerant to irinotecan.

In particular embodiments described herein, the presently disclosed subject matter provides a method for treatment of a subject with non-Hodkin's lymphoma, the method comprising: (a) administering a therapeutically effective amount of rituximab to the subject; and (b) sequentially or simultaneously administering to the subject a therapeutically effective amount of a metabolic reprogramming agent that decreases glutamine metabolism.

In some embodiments described herein, the subject has recurrent or refractory low-grade or follicular CD20-positive non-Hodgkin's lymphoma. In some embodiment, the subject has newly diagnosed CD20-positive non-Hodgkin's lymphoma. In some embodiments described herein, the subject has low-grade or follicular CD20-positive non-Hodgkin's lymphoma and responded to initial treatment with CVP chemotherapy (cyclophosphamide, vincristine and prednisone). In some embodiments described herein, the subject has CD20-positive diffuse large B-cell non-Hodgkin's lymphoma and the rituximab is administered in combination with CHOP chemotherapy (cyclophosphamide, doxorubicin hydrochloride, vincristine and prednisolone). In some embodiments described herein, the subject has newly disagnosed or recurrent CD20-positive chronic lymphocytic leukemia and the rituximab is administered in combination with FC chemotherapy (fludarabine and cyclophosphamide).

In particular embodiments described herein, the presently disclosed subject matter provides a method for treatment of a subject with early-stage breast cancer that is human epidermal growth factor receptor 2-positive (HER2+), the method comprising: (a) administering a therapeutically effective amount of alemtuzumab to the subject; and (b) sequentially or simultaneously administering to the subject a therapeutically effective amount of a metabolic reprogramming agent that decreases glutamine metabolism.

In some embodiments described herein, alemtuzumab is administered to the subject as part of a treatment course comprising doxorubicin, cyclophosphamide and either paclitaxel or docetaxel. In some embodiments described herein, alemtuzumab is administered to the subject with docetaxel and carboplatin. In some embodiments described herein, alemtuxumab is administered to the subject after treatment with anhtracylcine-based thereapy. In some embodiments described herein, the HER2+ breast cancer has spread into the subject's lymph nodes. In some embodiments described herein, the HER2+ breast cancer has not spread into the subject's lymph nodes and the cancer is estrogen receptor/progesterone receptor (ER/PR)-negative or has one high risk feature selected from the group consisting of a tumor size > 2 cm, age <35 years, or tumor grade of 2 or 3.

In particular embodiments described herein, the presently disclosed subject matter provides a method for treatment of a subject with B-cell chronic lymphocytic leukemia (B-CLL), the method comprising: (a) administering a therapeutically effective amount of alemtuzumab to the subject; and (b) sequentially or simultaneously administering to the subject a therapeutically effective amount of a metabolic reprogramming agent that decreases glutamine metabolism.

In particular embodiments described herein, the presently disclosed subject matter provides a method for treatment of a subject with low-grade or follicular B-cell non-Hodgkin's lymphoma (NHL) that has relapsed during or after treatment with other anticancer drugs or newly diagnosed follicular NHL following a response to initial anticancer therapy, the method comprising: (a) administering a therapeutically effective amount of ibritumomab tiuxetan to the subject; and (b) sequentially or simultaneously administering to the subject a therapeutically effective amount of a metabolic reprogramming agent that decreases glutamine metabolism. In some embodiments described herein, administering ibritumomab tiuxetan comprises intravenous injection comprising two infusions of rituximab (e.g., to reduce the number of B-cells in the subject's blood) and one injection of Yttrium-90 ibritumomab tiuxetan.

In particular embodiments described herein, the presently disclosed subject matter provides a method for treatment of a subject with wild-type *KRAS* (exon 2 in codons 12 or 13) metastatic colorectal cancer, the method comprising: (a) administering a therapeutically effective amount of panitumumab to the subject as a first-line therapy in combination with folinic acid, fluorouracil and oxaliplatin; and (b) sequentially or simultaneously administering to the subject a therapeutically effective amount of a metabolic reprogramming agent that decreases glutamine metabolism.

In particular embodiments described herein, the presently disclosed subject matter provides a method for treatment of a subject with wild-type *KRAS* (exon 2 in codons 12 or 13) metastatic colorectal cancer, the method comprising: (a) administering a therapeutically effective amount of panitumumab following disease progression after

prior treatment with fluoropyrimidine, oxalipltin, and irinotecan-containing chemotherapy; and (b) sequentially or simultaneously administering a therapeutically effective amount of a metabolic reprogramming agent that decreases glutamine metabolism.

7. COMBINATION IMMUNOTHERAPY

Unclaimed aspects of the presently disclosed subject matter involve the use of metabolic reprogramming agents (e.g., DON, DON-prodrugs, etc.) in combination immunotherapy together with a second, third, fourth, and/or fifth immunotherapy, for example, to enhance the immunotherapy for the treatment of cancer.

In some embodiments described herein, the method of treating cancer further includes simultaneously or sequentially administering a therapeutically effective amount of a third immunotherapy to the subject, wherein the third immunotherapy is selected from the group consisting of checkpoint blockade, adoptive cell therapy, CAR-T cell therapy, marrow-infiltrating lymphocytes, A2aR blockade, KIR blockade, vaccines (e.g., tumor vaccines), passive immunotherapy antibodies, and combinations thereof.

In some embodiments described herein, the method of treating cancer further includes simultaneously or sequentially administering a therapeutically effective amount of a third and/or fourth immunotherapy to the subject, wherein the third and/or fourth immunotherapy is selected from the group consisting of checkpoint blockade, adoptive cell therapy, CAR-T cell therapy, marrow-infiltrating lymphocytes, A2aR blockade, KIR blockade, vaccines (e.g., tumor vaccines), passive immunotherapy antibodies, and combinations thereof.

In some embodiments described herein, the method of treating cancer further includes simultaneously or sequentially administering a therapeutically effective amount of a third, fourth, and/or fifth immunotherapy to the subject, wherein the third, fourth, and/or fifth immunotherapy is selected from the group consisting of checkpoint blockade, adoptive cell therapy, CAR-T cell therapy, marrow-infiltrating lymphocytes, A2aR blockade, KIR blockade, vaccines (e.g., tumor vaccines), passive immunotherapy antibodies, and combinations thereof.

8. ADJUVANT TO CANCER THERAPY

Unclaimed aspects of the presently disclosed subject matter involve the use of metabolic reprogramming agents (e.g., DON or a DON prodrug) as an adjuvant to cancer therapy, for example, for the treatment or prevention of cancer.

Accordingly, in some embodiments described herein, the method of treating cancer further includes simultaneously or sequentially administering to the subject a therapeutically effective amount of a cancer therapy selected from the group consisting of: (i) chemotherapy; (ii) photodynamic therapy; (iii) proton therapy; (iv) radiotherapy; (v) surgery; and combinations thereof.

In some embodiments described herein, the first immunotherapy, and the second immunotherapy if administered, is/are administered to the subject in the absence of chemotherapy. In some embodiments described herein, the first immunotherapy, and the second immunotherapy if administered, is/are administered to the subject in the absence of photodynamic therapy. In some embodiments described herein, the first immunotherapy, and the second immunotherapy if administered, is/are administered to the subject in the absence of proton therapy. In some embodiments described herein, the first immunotherapy, and the second immunotherapy if administered, is/are administered to the subject in the absence of radiotherapy. In some embodiments described herein, the first immunotherapy, and the second immunotherapy if administered, is/are administered to the subject in the absence of surgery.

II. METABOLIC REPROGRAMMING AGENTS

The presently disclosed subject matter describes the use of various agents in connection with the methods, uses, and compositions described herein. Certain of the methods and compositions described herein relate to the metabolic reprogramming of cells using at least one metabolic reprogramming agent described herein to treat conditions, diseases, or disorders that involve metabolically reprogrammed cells whose activation, function, growth, proliferation, and/or survival depends on increased activity of at least one, at least two, or at least three metabolic pathways selected from the group consisting of glutamine metabolism, glycolysis, and fatty acid synthesis. Aspects of the methods and compositions described herein relate to the use of least one metabolic reprogramming agent described herein to treat conditions, diseases, or disorders that involve aberrant and/or excessive amounts of at least one, at least two, or at least three metabolic pathways selected from the group consisting of aberrant and/or excessive glutamine metabolism, aberrant and/or excessive glycolysis, or aberrant and/or excessive fatty acid synthesis.

As used herein, "metabolic reprogramming agent" generally refers to an agent that modulates the metabolic activity of at least one metabolic pathway in a cell, for example, to alter activation, function, growth, proliferation, and/or survival of the cell. As used herein, "modulate" broadly means to cause or facilitate a qualitative or quantitative change, alteration, or modification in a molecule, a process, pathway, or phenomenon of interest. Without limitation, such change

may be an increase, decrease, a change in binding characteristics, or change in relative strength or activity of different components or branches of the process, pathway, or phenomenon. The term "modulator" broadly refers to any molecule or agent that causes or facilitates a qualitative or quantitative change, alteration, or modification in a process, pathway, or phenomenon of interest. As used herein, the term "modulator" comprises both inhibitors and activators of a metabolic pathway or target. For example, "modulator" comprises both inhibitors and activators of expression and/or activity of a component involved glutamine metabolism, a component involved in glycolysis, and/or a component involved in fatty acid metabolism (e.g., fatty acid synthesis or fatty acid oxidation).

As used herein, the phrase "modulation of a metabolic pathway" refers to modulation of activity of at least one component of the metabolic pathway. It is contemplated herein that modulator of the metabolic pathway can be, for example, a receptor ligand (e.g., a small molecule, an antibody, a siRNA), a ligand sequestrant (e.g., an antibody, a binding protein), a modulator of phosphorylation of a pathway component or a combination of such modulators. One of skill in the art can easily test an agent to determine if it modulates a metabolic pathway by assessing, for example, phosphorylation status of a receptor or expression or synthesis of downstream proteins or enzymes controlled by the pathway in cultured cells and comparing the results to cells not treated with a modulator. A modulator is determined to be a metabolic pathway modulator if the level of phosphorylation of the receptor or expression of or synthesis of downstream proteins or enzymes in a culture of cells is reduced by at least 20% compared to the level of phosphorylation of the receptor or expression or synthesis of downstream proteins or enzymes in cells that are cultured in the absence of the modulator; preferably the level of phosphorylation or expression or synthesis of downstream proteins or enzymes is altered by at least 30%, at least 40%, at least 50%, at least 60%, at least 70%, at least 80%, at least 90%, at least 95%, or at least 99% in the presence of a metabolic pathway modulator.

The terms "decrease" , "reduced", "reduction", "decrease" or "inhibit" are all used herein generally to mean a decrease by a statistically significant amount. However, for avoidance of doubt, ""reduced", "reduction", "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90%, where the decrease is less than 100%. In one embodiment, the decrease includes a 100% decrease (e.g. absent level as compared to a reference sample), or any decrease between 10-100% as compared to a reference level.

The terms "increased", ’increase", "enhance" or "activate" are all used herein to generally mean an increase by a statically significant amount; for the avoidance of any doubt, the terms "increased", "increase", "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4- fold, or at least about a 5-fold or at least about a 10-fold increase, or any increase between 2-fold and 10-fold or greater as compared to a reference level.

Certain methods, compositions, and agents contemplated herein modulate an immune response. In the contexts of decreasing an immune response (e.g., inhibiting an immunosuppressive pathway, e.g., via checkpoint blockade, A2aR blockade, KIR blockade, etc.), the methods, compositions, and agents contemplated herein can decrease the immune response by at least about 10%, 20%, 30%, 40%, 50%, 60%, 70%, 80, 90%, or as much as 100% as compared to a reference level (e.g., an objective measure of the immune response before employing the method, composition, and/or agent). In the contexts of increasing an immune response (e.g., activating a T-cell co-stimulatory signal), the methods, compositions, and agents contemplated herein can increase the immune response by at least about 10%, 20%, 30%, 40%, 50%, 60%, 70%, 80, 90%, or as much as 100%, at least about a 2-fold, or at least about a 3-fold, or at least about a 4-fold, or at least about a 5-fold or at least about a 10- fold increase, or any increase between 2-fold and 10-fold or greater as compared to a reference level (e.g., an objective measure of the immune response before employing the method, composition, and/or agent).

Certain methods, compositions, and agents contemplated herein modulate growth, proliferation, metastasis, and invasion of malignant cells (e.g., cancer cells). In the contexts of inhibiting growth, proliferation, metastasis, invasion, and/or survival of malignant cells (e.g., cancer cells), the methods, compositions, and agents contemplated herein can decrease the growth, proliferation, metastasis, invasion, and/or survival of malignant cells (e.g., cancer cells) by at least about 10%, 20%, 30%, 40%, 50%, 60%, 70%, 80, 90%, or as much as 100% as compared to a reference level (e.g., an objective measure of the growth, proliferation, metastasis, invasion, and/or survival of malignant cells before employing the method, composition, and/or agent).

The term "statistically significant" or "significantly" refers to statistical significance and generally means a two standard deviation (2SD) below normal, or lower, concentration of the marker. The term refers to statistical evidence that there is a difference. It is defined as the probability of making a decision to reject the null hypothesis when the null hypothesis is actually true. The decision is often made using the p-value.

As used more particularly herein in some contexts, "modulates", "modulating", and "modulation" are used

interchangeably and refer to any one or a combination of a decrease in glutamine metabolism, a decrease in glycolysis, and a decrease in fatty acid synthesis. In other contexts, "modulates", "modulating", and "modulation" are used interchangeably and refer to any one or a combination of an increase in glutamine metabolism, an increase in glycolysis, and an increase in fatty acid synthesis. In certain contexts, "modulates", "modulating", and "modulation" are used interchangeably and refer to any one or a combination of an increase in oxidative phosphorylation.

Glutamine (2-amino-4-carbamoylbutanoic acid), is used by the cell for both bioenergetic and biosynthetic needs. Glutamine can be used as an amino acid for protein synthesis, as a carbon source, or as the primary nitrogen donor for multiple essential biosynthetic reactions in the cell. Once taken up by the cell, much of the glutamine is converted to glutamate by mitochondrial glutaminase. Both glutamine and glutamate contribute to anabolic metabolism; glutamine supplies nitrogen for nucleotide and hexosamine synthesis while glutamate is the nitrogen donor for the synthesis of many nonessential amino acids. Glutamate can be used to support the production of NADPH or converted to the metabolic intermediates pyruvate and $\alpha$-ketoglutarate. As used herein, the term "glutamine metabolism" or "glutamine metabolic activity" refers to the chemical reactions, enzymes, and pathways involving glutamine. As used herein, the term "glutamine metabolic pathway" is a biochemical pathway that involves glutamine.

As can be envisioned by a person with skill in the art, a metabolic reprogramming agent can modulate any of the chemical reactions, enzymes and/or pathways involving glutamine. In some embodiments, at least one metabolic reprogramming agent can modulate chemical reactions, enzymes and/or pathways that do not directly involve glutamine, such as the conversion of pyruvate to acetyl CoA or the citric acid cycle, but indirectly affect any of the chemical reactions, enzymes and/or pathways involving glutamine. Certain methods, compositions, and metabolic reprogramming agents contemplated herein decrease glutamine metabolism in cells. In the context of decreasing glutamine metabolism in cells, the methods, compositions, and agents contemplated herein can decrease glutamine metabolism in cells by at least about 10%, 20%, 30%, 40%, 50%, 60%, 70%, 80, 90%, or as much as 100% as compared to a reference level (e.g., an objective measure of the glutamine metabolic activity before employing the method, composition, and/or agent).

In some embodiments described herein, at least one metabolic reprogramming agent is a glutamine antagonist (i.e., an agent that decrease glutamine metabolism). As used herein, the term "glutamine antagonist" refers to an agent that blocks or interferes with the synthesis or use of glutamine in a cell, and preferably in a cell that is part of a living organism. When it is said that the glutamine antagonist interferes with the synthesis of glutamine, it is meant that the antagonist acts to reduce the amount or rate of glutamine synthesis to less than the amount or rate that would be experienced in the absence of the glutamine antagonist. When it is said that the glutamine antagonist interferes with the use of glutamine, it is meant that the antagonist acts to inhibit or block a metabolic pathway downstream of glutamine, that is, a pathway in which glutamine acts as a precursor of one or more non-glutamine compounds, or that the antagonist acts to deplete glutamine in a cell or an organism by reacting the glutamine to form a non-glutamine product, or by reversibly or irreversibly binding with glutamine to reduce its availability.

In some embodiments described herein, at least one metabolic reprogramming agent of the presently disclosed subject matter can be a glutamine analog that interferes with a glutamine metabolic pathway, an antagonist that inhibits the synthesis of glutamine, a glutamine depleting enzyme, a compound that reacts with glutamine under intracellular conditions to form a non-glutamine product, an antagonist that inhibits glutamine uptake by cells, an agent that inhibits glutamine oxidation, an agent that inhibits a glutamine transporter, an agent that inhibits glutaminolysis (a series of biochemical reactions by which glutamine is lysed to glutamate, aspartate, carbon dioxide, pyruvate, lactate, alanine and/or citrate), or a glutamine binding compound that reduces the biological availability of glutamine. It should be recognized that a compound that is a useful metabolic reprogramming agent may have two or more of these characteristics. For example, a compound that is a glutamine analog that interferes with a glutamine metabolic pathway might also act as an antagonist that inhibits the synthesis of glutamine.

In some embodiments described herein, at least one metabolic reprogramming agent can be an antagonist that inhibits the synthesis of glutamine. Examples of compounds having this activity include inhibitors of glutamine synthase (EC 6.3.1.2), such as L-methionine-DL-sulfoximine, and phosphinothricin; inhibitors of glutamate synthase (EC 1.4.1.13); inhibitors of amidophosphoribosyltransferase (EC 2.4.2.14); and inhibitors of glutamate dehydrogenase; and mixtures of any two or more of these.

In some embodiments described herein, at least one metabolic reprogramming agent can be a glutamine depleting enzyme. Examples of such enzymes include carbamoyl-phosphate synthase (EC 6.3.5.5), glutamine-pyruvate transaminase (EC 2.6.1.15), glutamine-tRNA ligase (EC 6.1.1.18), glutaminase (EC 3.5.1.2), D-glutaminase (EC 3.5.1.35), glutamine N-acyltransferase (EC2.3.1.68), glutaminase-asparaginase (in particular glutaminase-asparaginase of *Pseudomonas* 7a and *Acinatobacter* sp.), and mixtures of any two or more of these.

In some embodiments described herein, at least one metabolic reprogramming agent can be a compound that reacts with glutamine under intracellular conditions to form a non-glutamine product. An example of a compound having this property is phenylbutyrate (See Darmaun et al., Phenylbutyrate-induce glutamine depletion in humans: effect on leucine metabolism, pp. E801-E807, in Glutamine Depletion and Protein Catabolism, Am. Physiol. Soc. (1998)). Another example of a glutamine antagonist having this characteristic is phenylacetate (See, U.S. Pat. No. 6,362,226).

In some embodiments described herein, at least one metabolic reprogramming agent can be an antagonist that inhibits glutamine uptake by cells. Examples of compounds having this property include alpha-methylaminoisobutyric acid (inhibits GynT plasma membrane glutamine transporter; See, Varoqui et al., J. Biol. Chem., 275(6):4049-4054 (2000), wortmannin, and LY-294002 (inhibits hepatic glutamine transporter; See, Pawlik et al., Am. J. Physiol. Gastrointest. Liver Physiol., 278:G532-G541 (2000)).

In some embodiments described herein, at least one metabolic reprogramming agent can be a glutamine binding compound that reduces the biological availability of glutamine.

In some embodiments described herein, at least one metabolic reprogramming agent can be a glutamine analog that interferes with a glutamine metabolic pathway (e.g., decreases glutamine metabolism/metabolic activity). Examples of compounds that can act in this manner include acivicin (L-(alpha S,5S)-alpha-amino-3-chloro-4,5-dihydro-5-isoxazoleacetic acid), DON (6-d iazo-5-oxo-L-norleucine), azaserine, azotomycin, chloroketone (L-2-amino-4-oxo-5-chloropentanoic acid), $N^3$-(4-methoxyfumaroyl)-L-2,3-diaminopropanoic acid (FMDP) (inactivates glucosamine-6-phosphate synthase (EC 2.6.1.16), See, Zgòdka et al., Microbiology, 147:1955-1959 (2001)), (3 S,4R)-3,4-dimethyl-L-glutamine, (3 S,4R)-3,4-dimethyl-L-pyroglutamic acid (See, Acevedo et al., Tetrahedron., 57:6353-6359 (2001)), 1,5-N,N'-disubstituted-2-(substituted benzenesulphonyl) glutamamides (See, Srikanth et al., Bioorganic and Medicinal Chemistry, (2002)), or a mixture of any two or more of these. In some embodiments, at least one metabolic reprogramming agent is selected from the group consisting of acivicin (L-(alpha S, 5S)-alpha-amino-3-chloro-4,5-dihydro-5-isoxazoleacetic acid), azaserine, 6-diazo-5-oxo-norleucine (DON), and 5-diazo-4-oxo-L-norvaline (L-DONV).

In some embodiments described herein, at least one metabolic reprogramming agent is a prodrug of a glutamine analog that interferes with a glutamine metabolic pathway (e.g., decreases glutamine metabolism/metabolic activity). In some embodiments, at least one metabolic reprogramming agent is a prodrug of acivicin (L-(alpha S, 5S)-alpha-amino-3-chloro-4,5-dihydro-5-isoxazoleacetic acid), azaserine, 6-diazo-5-oxo-norleucine (DON), and 5-diazo-4-oxo-L-norvaline (L-DONV). Suitable exemplary prodrugs of acivicin, azaserine, DON, and L-DONV can be found in "Prodrugs of Glutamine Analogs" (Attorney Docket No. 111232-00403, filed concurrently herewith, now published as WO 2017/023774 A1.).

Glycolysis is the metabolic pathway that converts glucose into pyruvate with the concurrent production of ATP. Pyruvate is a metabolic intermediate that can then enter the tricarboxylic acid (TCA) cycle within mitochondria to produce NADH and $FADH_2$. The first step in glycolysis is the phosphorylation of glucose by hexokinase to form glucose 6-phosphate.

In some embodiments described herein, at least one metabolic reprogramming agent can modulate any of the chemical reactions and/or enzymes involved in glycolysis. In some embodiments described herein, at least one metabolic reprogramming agent can modulate chemical reactions, enzymes and/or pathways that do not directly involve glycolysis, but indirectly affect any of the chemical reactions, enzymes and/or pathways involving glycolysis. Certain methods, compositions, and metabolic reprogramming agents contemplated herein decrease glycolysis in cells. In the context of decreasing glycolysis in cells, the methods, compositions, and agents contemplated herein can decrease glycolysis in cells by at least about 10%, 20%, 30%, 40%, 50%, 60%, 70%, 80, 90%, or as much as 100% as compared to a reference level (e.g., an objective measure of the glycolytic metabolic activity before employing the method, composition, and/or agent). As used herein, the term "glycolytic metabolic activity" refers to the chemical reactions and enzymes involving the glycolysis pathway.

In some embodiments described herein, at least one metabolic reprogramming agent of the presently disclosed subject matter can be an agent that interferes with glycolysis or a related pathway that affects glycolysis; an agent that inhibits the synthesis of pyruvate and/or one of the intermediate products of glycolysis; an agent that inhibits one or more of the enzymes involved in glycolysis, such as hexokinase, phosphoglucose isomerase, phosphofructokinase, fructose-bisphosphate aldolase, triophosphate isomerase, glyceraldehyde phosphate dehydrogenase, phosphoglycerate kinase, phosphoglycerate mutase, enolase, and/or pyruvate kinase; an agent that depletes glucose-6-phosphate, one of the rate-limiting products in glycolysis; an agent that inhibits glucose uptake and/or transport across the plasma membrane by cells; or a glucose binding compound that reduces the biological availability of glucose. It should be recognized that a compound that is a useful metabolic reprogramming agent may have two or more of these characteristics.

In some embodiments described herein, at least one metabolic reprogramming agent interferes or inhibits the expression and/or activity of hexokinase. Examples of inhibitors of hexokinase include, but are not limited to, 2-deoxyglucose (2-DG), 3-bromopyruvate (3-BrPA), lonidamine (LND), sodium fluoride, and potassium fluoride. In some embodiments, at least one metabolic reprogramming agent is 2-deoxy-D-glucose (2-DG).

Fatty acid synthesis is the process in the cell that creates fatty acids from acetyl-CoA and malonyl-CoA precursors. Fatty acid oxidation is the process by which fatty acid molecules are broken down in the mitochondria to generate acetyl-CoA, which enters the citric acid cycle, and NADH and $FADH_2$, which are used in the electron transport chain. The enzyme AMP-activated protein kinase (AMPK) plays a role in cellular energy homeostasis and is a stimulator of fatty acid oxidation.

In some embodiments described herein, at least one metabolic reprogramming agent can modulate any of the

chemical reactions and/or enzymes involved in fatty acid synthesis and/or fatty acid oxidation. In some embodiments described herein, at least one metabolic reprogramming agent can modulate chemical reactions, enzymes and/or pathways that do not directly involve fatty acid synthesis and/or fatty acid oxidation, but indirectly affect any of the chemical reactions, enzymes and/or pathways involving fatty acid synthesis and/or fatty acid oxidation.

Certain methods, compositions, and metabolic reprogramming agents contemplated herein decrease fatty acid synthesis and/or increase fatty acid oxidation in cells. In the context of decreasing fatty acid synthesis and/or increasing fatty acid oxidation in cells, the methods, compositions, and agents contemplated herein can decrease fatty acid synthesis and/or increase fatty acid oxidation in cells by at least about 10%, 20%, 30%, 40%, 50%, 60%, 70%, 80, 90%, or as much as 100% as compared to a reference level (e.g., an objective measure of the synthesis of fatty before employing the method, composition, and/or agent).

In some embodiments described herein, at least one metabolic reprogramming agent of the presently disclosed subject matter can be an agent that interferes with fatty acid synthesis and/or fatty acid oxidation or a related pathway that affects fatty acid synthesis and/or fatty acid oxidation; an agent that increases fatty acid oxidation; an agent that increases one or more of the products of fatty acid oxidation; an agent that increases the expression and/or activity of one or more of the enzymes involved in fatty acid oxidation, such as acyl CoA dehydrogenase, enoyl CoA hydratase, 3-hydroxyacyl-CoA dehydrogenase, and β-ketothiolase; an agent that increases expression and/or activity of AMP-activated protein kinase (AMPK); an agent that increases uptake and/or transfer of activated fatty acids across the mitochondrial membrane; and an agent that increases the expression and/or activity of enzymes involved in the uptake and/or transfer of activated fatty acids across the mitochondrial membrane. It should be recognized that a compound that is a useful metabolic reprogramming agent may have two or more of these characteristics. In some embodiments, at least one metabolic reprogramming agent is an activator of 5' AMP-activated protein kinase (AMPK) activity.

At least one metabolic reprogramming agent that is an activator of AMPK activity can be an agent that increases concentrations of AMP in the cell; an AMP analogue, such as 5-amino-4-imidazolecarboxamide ribotide (ZMP); an agent that increases phosphorylation of AMPK, such as an agent that increases the expression and/or activity of a kinase that can phosphorylate AMPK; and an agent that is an allosteric modulator of AMPK, such as one that can modify AMPK to make it a better substrate for a kinase that can phosphorylate AMPK.

In some embodiments described herein, at least one metabolic reprogramming agent is metformin.

It should be appreciated that modulation of glutamine metabolism, glycolysis, and fatty acid metabolism may result in modulation of one or more genes or expression products of genes or biosynthesis or degradation of one or more enzymes.

The term "expression" means the process by which information from a gene or nucleic acid (e.g., DNA) is used in the synthesis of gene products (e.g., mRNA, RNA and/or proteins) and includes, but is not limited to, one or more of the steps of replication, transcription and translation. The steps of expression which may be modulated by the agents contemplated herein may include, for example, transcription, splicing, translation and post-translational modification of a protein. Those skilled in the art will appreciate that the method of modulating any particular protein may depend on the type of protein (e.g., protein kinase, transcriptional regulator, enzyme, etc.), its function (e.g., transcriptional regulation, catalysis, phosphorylation, signal transduction, etc.), and its subcellular localization (e.g., extracellular space, cytoplasm, nucleus, membrane, etc.). Those skilled in the art will readily appreciate appropriate agents to be used for modulation depending on the particular context (e.g., type of protein, biological function, subcellular localization, composition, method of use, mode of inhibition, etc.). For example, an agent can be used to inhibit enzymatic activity of an enzyme (e.g., at least one metabolic reprogramming agent that inhibits glutaminolysis catalyzed by glutaminase (e.g., a glutamine antagonist), at least one metabolic reprogramming agent that inhibits glycolysis catalyzed in part by hexokinase (e.g., 2-DG), etc.), inhibits the level or activity of phosphorylation of a protein kinase, inhibit activation of transcription or a signaling pathway.

The metabolic reprogramming agents, chemotherapeutic agents, cytotoxic agents, immunotherapeutic agents, immunosuppressant agents, radiotherapeutic agents, anti-inflammatory agents, and neuroprotective agents described herein can be any type of agent. Exemplary types of agents that can be used as such agents in the methods, compositions, and uses described herein include small organic or inorganic molecules; saccharides; oligosaccharides; polysaccharides; a biological macromolecule selected from the group consisting of peptides, proteins, peptide analogs and derivatives; peptidomimetics; nucleic acids selected from the group consisting of siRNAs, shRNAs, antisense RNAs, ribozymes, dendrimers and aptamers; an extract made from biological materials selected from the group consisting of bacteria, plants, fungi, animal cells, and animal tissues; naturally occurring or synthetic compositions; microcarrier or nanocarrier consisting of one or more polymers, proteins, nucleic acids, lips, or metals; and any combination thereof.

As used herein, the term "small molecule" can refer to agents that are "natural product-like," however, the term "small molecule" is not limited to "natural product-like" agents. Rather, a small molecule is typically characterized in that it contains several carbon- carbon bonds, and has a molecular weight of less than 5000 Daltons (5 kD), preferably less than 3 kD, still more preferably less than 2 kD, and most preferably less than 1 kD. In some cases it is preferred that a small molecule have a molecular weight equal to or less than 700 Daltons.

As used herein, an "RNA interference molecule" refers to an agent which interferes with or inhibits expression of a target gene or genomic sequence by RNA interference (RNAi). Such RNA interfering agents include, but are not limited to, nucleic acid molecules including RNA molecules which are homologous to the target gene or genomic sequence, or a fragment thereof, short interfering RNA (siRNA), short hairpin or small hairpin RNA (shRNA), microRNA (miRNA) and small molecules which interfere with or inhibit expression of a target gene by RNA interference (RNAi).

The term "polynucleotide" is used herein interchangeably with "nucleic acid" to indicate a polymer of nucleosides. Typically a polynucleotide is composed of nucleosides that are naturally found in DNA or RNA (e.g., adenosine, thymidine, guanosine, cytidine, uridine, deoxyadenosine, deoxythymidine, deoxyguanosine, and deoxycytidine) joined by phosphodiester bonds. However the term encompasses molecules comprising nucleosides or nucleoside analogs containing chemically or biologically modified bases, modified backbones, etc., whether or not found in naturally occurring nucleic acids, and such molecules may be preferred for certain applications. Where this application refers to a polynucleotide it is understood that both DNA, RNA, and in each case both single- and double-stranded forms (and complements of each single-stranded molecule) are provided. Polynucleotide sequence" as used herein can refer to the polynucleotide material itself and/or to the sequence information (e.g. the succession of letters used as abbreviations for bases) that biochemically characterizes a specific nucleic acid. A polynucleotide sequence presented herein is presented in a 5' to 3' direction unless otherwise indicated.

The nucleic acid molecules that modulate the metabolic pathways or targets described herein can, in some embodiments, be inserted into vectors and used as gene therapy vectors. Gene therapy vectors can be delivered to a subject by, for example, intravenous injection, local administration (see US Pat. No. 5,328,470) or by stereotactic injection (see e.g., Chen et al. Proc. Natl. Acad. Sci. USA 91 :3054-3057, 1994). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The terms "polypeptide" as used herein refers to a polymer of amino acids. The terms "protein" and "polypeptide" are used interchangeably herein. A peptide is a relatively short polypeptide, typically between about 2 and 60 amino acids in length. Polypeptides used herein typically contain amino acids, such as the 20 L-amino acids that are most commonly found in proteins. However, other amino acids and/or amino acid analogs known in the art can be used. One or more of the amino acids in a polypeptide may be modified, for example, by the addition of a chemical entity, such as a carbohydrate group, a phosphate group, a fatty acid group, a linker for conjugation, functionalization, etc. A polypeptide that has a non-polypeptide moiety covalently or non-covalently associated therewith is still considered a "polypeptide". Exemplary modifications include glycosylation and palmitoylation. Polypeptides may be purified from natural sources, produced using recombinant DNA technology, synthesized through chemical means, such as conventional solid phase peptide synthesis, etc. The term "polypeptide sequence" or "amino acid sequence" as used herein can refer to the polypeptide material itself and/or to the sequence information (e.g., the succession of letters or three letter codes used as abbreviations for amino acid names) that biochemically characterizes a polypeptide. A polypeptide sequence presented herein is presented in an N-terminal to C-terminal direction unless otherwise indicated.

The term "identity" as used herein refers to the extent to which the sequence of two or more nucleic acids or polypeptides is the same. The percent identity between a sequence of interest and a second sequence over a window of evaluation, e.g. , over the length of the sequence of interest, may be computed by aligning the sequences, determining the number of residues (nucleotides or amino acids) within the window of evaluation that are opposite an identical residue allowing the introduction of gaps to maximize identity, dividing by the total number of residues of the sequence of interest or the second sequence (whichever is greater) that fall within the window, and multiplying by 100. When computing the number of identical residues needed to achieve a particular percent identity, fractions are to be rounded to the nearest whole number. Percent identity can be calculated with the use of a variety of computer programs known in the art. For example, computer programs, such as BLAST2, BLASTN, BLASTP, Gapped BLAST, etc., generate alignments and provide percent identity between sequences of interest. The algorithm of Karlin and Altschul (Karlin and Altschul, Proc. Natl. Acad. Sci. USA 87:22264-2268, 1990) modified as in Karlin and Altschul, Proc. Natl. Acad. Sci. USA 90:5873-5877, 1993 is incorporated into the NBLAST and XBLAST programs of Altschul et al. (Altschul, et al., J. MoT Biol. 215:403-410, 1990). To obtain gapped alignments for comparison purposes, Gapped BLAST is utilized as described in Altschul et al. (Altschul, et al. Nucleic Acids Res. 25: 3389-3402, 1997). When utilizing BLAST and Gapped BLAST programs, the default parameters of the respective programs may be used. A PAM250 or BLOSUM62 matrix may be used. Software for performing BLAST analyses is publicly available through the National Center for Biotechnology Information (NCBI). See the Web site having URL www.ncbi.nlm.nih.gov for these programs. In a specific embodiment, percent identity is calculated using BLAST2 with default parameters as provided by the NCBI.

Generally, at least one metabolic reprogramming agent described herein can be used in combination with an additional therapeutic agent (e.g., a pharmaceutically active agent, e.g., a drug approved by a regulatory agency). The therapeutic agent may act synergistically with the agent described herein, or they may independently exert their intended

effects. The disclosure contemplates any therapeutic agent which a skilled artisan would use in connection with a method, use, or composition described herein. Examples of therapeutic agents contemplated for use in the presently disclosed methods, uses and compositions in combination with the metabolic reprogramming agents include, but are not limited to, chemotherapeutic agents/chemotherapy, immunotherapeutic agents/immunotherapy, immunosuppressant agents, anti-inflammatory agents, neuroprotective agents, neuroregenerative agents, neurotrophic factors, radiotherapeutic agents/radiotherapy, proton therapy, photodynamic therapy, and stem and progenitor cells used to replace and/or repair endogenous populations of abnormal, harmful, or unhealthy cells.

Chemotherapy and chemotherapeutic agnet are used synonymously herein. A "chemotherapeutic agent" is used to connote a compound or composition that is administered in the treatment of cancer. Chemotherapeutic agents contemplated for use in combination with at least one metabolic reprogramming agent, at least two metabolic reprogramming agents, or at least three metabolic reprogramming agents described herein include, but are not limited to, alkylating agents, such as thiotepa and cyclophosphamide; alkyl sulfonates, such as busulfan, improsulfan and piposulfan; aziridines, such as benzodopa, carboquone, meturedopa, and uredopa; ethylenimines and methylamelamines including altretamine, triethylenemelamine, trietylenephosphoramide, triethylenethiophosphaoramide and trimethylolomelamime; nitrogen mustards, such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas, such as carmustine, chlorozotocin, fotemustine, lomustine, nimustine, ranimustine; antibiotics, such as aclacinomysins, actinomycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites, such as methotrexate and 5-fluorouracil (5-FU); folic acid analogues, such as denopterin, methotrexate, pteropterin, trimetrexate; purine analogs, such as fludarabine, 6-mercaptopurine, thiamiprine, thioguanine; pyrimidine analogs, such as ancitabine, azacitidine, 6-azauridine, carmofur, cytosine arabinoside, dideoxyuridine, doxifluridine, enocitabine, floxuridine, 5-FU; androgens, such as calusterone, dromostanolone propionate, epitiostanol, mepitiostane, testolactone; anti-adrenals, such as aminoglutethimide, mitotane, trilostane; folic acid replenishers, such as folinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK; razoxane; sizofuran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside (Ara-C); taxoids, e.g., paclitaxel and docetaxel; chlorambucil; gemcitabine; 6-thioguanine; mercaptopurine; platinum analogs, such as cisplatin and carboplatin; vinblastine; platinum; etoposide; ifosfamide; mitomycin C; mitoxantrone; vincristine; vinorelbine; navelbine; novantrone; teniposide; daunomycin; aminopterin; xeloda; ibandronate; CPT11; topoisomerase inhibitor RFS 2000; difluoromethylornithine; retinoic acid; esperamicins; capecitabine; and pharmaceutically acceptable salts, acids or derivatives of any of the above. Chemotherapeutic agents also include anti-hormonal agents that act to regulate or inhibit hormone action on tumors, such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens, such as flutamide, nilutamide, bicalutamide, leuprolide, and goserelin; and pharmaceutically acceptable salts, acids or derivatives of any of the above.

In some embodiments described herein, the chemotherapeutic agent is a topoisomerase inhibitor. Topoisomerase inhibitors are chemotherapy agents that interfere with the action of a topoisomerase enzyme (e.g., topoisomerase I or II). Topoisomerase inhibitors include, but are not limited to, doxorubicin HCl, daunorubicin citrate, mitoxantrone HCl, actinomycin D, etoposide, topotecan HCl, teniposide, and irinotecan, as well as pharmaceutically acceptable salts, acids, or derivatives of any of these.

In some embodiments described herein, the chemotherapeutic agent is an anti-metabolite. An anti-metabolite is a chemical with a structure that is similar to a metabolite required for normal biochemical reactions, yet different enough to interfere with one or more normal functions of cells, such as cell division. Anti-metabolites include, but are not limited to, gemcitabine, fluorouracil, capecitabine, methotrexate sodium, ralitrexed, pemetrexed, tegafur, cytosine arabinoside, thioguanine, 5-azacytidine, 6-mercaptopurine, azathioprine, 6-thioguanine, pentostatin, fludarabine phosphate, and cladribine, as well as pharmaceutically acceptable salts, acids, or derivatives of any of these.

In certain embodiments described herein, the chemotherapeutic agent is an antimitotic agent, including, but not limited to, agents that bind tubulin. In some embodiments, the agent is a taxane. In certain embodiments described herein, the agent is paclitaxel or docetaxel, or a pharmaceutically acceptable salt, acid, or derivative of paclitaxel or docetaxel. In certain alternative embodiments, the antimitotic agent comprises a vinca alkaloid, such as vincristine, binblastine, vinorelbine, or vindesine, or pharmaceutically acceptable salts, acids, or derivatives thereof.

As used herein, the term "immunotherapeutic agent" refers to a molecule that can aid in the treatment of a disease by inducing, enhancing, or suppressing an immune response in a cell, tissue, organ or subject. Examples of

immunotherapeutic agents contemplated for use in combination with at least one metabolic reprogramming agent, at least two metabolic reprogramming agents, or at least three metabolic reprogramming agents described herein include, but are not limited to, immune checkpoint molecules (e.g., antibodies to immune checkpoint proteins), interleukins (e.g., IL-2, IL-7, IL-12, IL-15), cytokines (e.g., interferons, G-CSF, imiquimod), chemokines (e.g., CCL3, CCL26, CXCL7), vaccines (e.g., peptide vaccines, dendritic cell (DC) vaccines, EGFRvIII vaccines, mesothilin vaccine, G-VAX, listeria vaccines), and adoptive T cell therapy including chimeric antigen receptor T cells (CAR T cells).

As used herein, "immunosuppressant agent" means an agent which may be used in immunotherapy to reduce or prevent an immune response in a cell, organ, tissue, or subject. Examples of immunosuppressant agents contemplated for use in combination with at least one metabolic reprogramming agent, at least two metabolic reprogramming agents, or at least three metabolic reprogramming agents include, without limitation, corticosteriods, calcineurin inhibitors, antiproliferative agents, SIP receptor agonists, kinase inhibitors, monoclonal antilymphocyte antibodies and polyclonal antilymphocyte antibodies. Non-limiting examples of corticosteroids include Prednisone (Deltasone® and Orasone®) and Methylprednisolone (SoluMedrol®). Non-limiting examples of calcineurin inhibitors include Cyclosporine (Cyclosporin A, SangCya, Sandimmune®, Neoral®, Gengraf®), ISA, Tx247, ABT-281, ASM 981 and Tacrolimus (Prograf®, FK506). Non-limiting examples of antiproliferative agents include Mycophenolate Mofetil (CellCept®), Azathioprene (Imuran®), and Sirolimus (Rapamune®). Non-limiting examples of SIP receptor agonists include FTY 720 or analogues thereof. Non-limiting examples of kinase inhibitors include mTOR kinase inhibitors, which are compounds, proteins or antibodies that target, decrease or inhibit the activity and/or function of members of the serine/threonine mTOR family. These include, without limitation, CCI-779, ABT578, SAR543, rapamycin and derivatives or analogs thereof, including 40-O-(2-hydroxyethyl)-rapamycin, rapalogs, including AP23573, AP23464, AP23675 and AP23841 from Ariad, Everolimus (CERTICAN, RAD001), biolimus 7, biolimus 9 and sirolimus (RAPAMUNE). Kinase inhibitors also include protein kinase C inhibitors, which include the compounds described the PCT publications WO 2005/097108 and WO 2005/068455. Non-limiting examples of monoclonal antilymphocyte antibodies include Muromonab-CD3 (Orthoclone OKT3®), Interleukin-2 Receptor Antagonist (Basiliximab, Simulect®), and Daclizumab (Zenapax®). Non-limiting examples of polyclonal antilymphocyte antibodies include Antithymocyte globulin-equine (Atgam®) and Antithymocyte globulin-rabbit (RATG, Thymoglobulin®). Other immunosuppressants include, without limitation, SERP-1, a serine protease inhibitor produced by malignant rabbit fibroma virus (MRV) and myxoma virus (MYX), described in US Patent Publication No. 2004/0029801.

Immunosuppressant agents can be classified according to their specific molecular mode of action. The four main categories of immunosuppressant drugs currently used in treating patients with transplanted organs are the following. Calcineurin inhibitors inhibit T-cell activation, thus preventing T-cells from attacking the transplanted organ. Azathioprines disrupt the synthesis of DNA and RNA as well as the process of cell division. Monoclonal antibodies inhibit the binding of interleukin-2, which in turn slows down the production of T-cells in the patient's immune system. Corticosteroids suppress inflammation associated with transplant rejection.

Immunosuppressants can also be classified according to the specific organ that is transplanted. Basiliximab (Simulect) is also used in combination with such other drugs as cyclosporine and corticosteroids in kidney transplants. IL-2 blockers, including Simulect from Novartis, FK506 or CyA, MMF, prednisone or Rapamycin are also used in kidney transplants. Daclizumab (Zenapax) is also used in combination with such other drugs as cyclosporin and corticosteroids in kidney transplants. Similar drugs are used in heart transplants, but anti-lymphocyte globulin (ALG) is often used instead of Simulect. Muromonab CD3 (Orthoclone OKT3) is used along with cyclosporine in kidney, liver and heart transplants. Tacrolimus (Prograf) is used in liver and kidney transplants. It is under study for bone marrow, heart, pancreas, pancreatic island cell and small bowel transplantation.

As used herein, "photodynamic therapy", also known as photoradiation therapy, phototherapy, and photochemotherapy, refers to a treatment that uses photosensitizing agents in combination with light to kill cancer cells. The photosensitizing agents kill cancer cells upon light activation.

As used herein, "proton therapy", also known as proton beam therapy, refers to a treatment that uses a beam of protons to irradiate and kill cancer cells.

As used herein, "anti-inflammatory agent" refers to an agent that may be used to prevent or reduce an inflammatory response or inflammation in a cell, tissue, organ, or subject. Exemplary anti-inflammatory agents contemplated for use in combination with at least one metabolic reprogramming agent, at least two metabolic reprogramming agents, or at least three metabolic reprogramming agents include, without limitation, steroidal anti-inflammatory agents, a nonsteroidal anti-inflammatory agent, or a combination thereof. In some embodiments, anti-inflammatory agents include clobetasol, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cormethasone acetate, cortodoxone, deflazacort, desonide, desoximetasone, dexamethasone, dexamethasone acetate, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate,

felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus, prodrugs thereof, co-drugs thereof, and combinations thereof. The anti-inflammatory agent may also be a biological inhibitor of proinflammatory signaling molecules including antibodies to such biological inflammatory signaling molecules.

Exemplary neuroprotective agents include, without limitation, L-dopa, dopamine agonists (e.g., apomorphine, bromocriptine, pergolide, ropinirole, pramipexole, or cabergoline), adenosine A2a antagonists (Shah et al., Curr. Opin. Drug Discov. Devel. 13:466-80 (2010)); serotonin receptor agonists; continuous-release levodopa (Sinemet CR.RTM., MSD, Israel); continuous duodenal levodopa administration (Duodopa.RTM., Abbott, UK); catechol-O-methyltransferase (COMT) inhibitors (e.g., Stalevo.RTM., Novartis Pharma, USA; entacapone (Comtan.RTM., Novartis Pharma, USA)); tolcapone; coenzyme Q10, and/or MAO-B inhibitors (e.g., Selegiline or Rasagiline). Additional neuroprotective agents are described in, e.g., Hart et al., Mov. Disord. 24: 647-54 (2009).

As used herein, a "radiotherapeutic agent" refers to those agents conventionally adopted in the therapeutic field of cancer treatment and includes photons having enough energy for chemical bond ionization, such as, for instance, alpha ($\alpha$), beta ($\beta$), and gamma ($\gamma$) rays from radioactive nuclei as well as x-rays. The radiation may be high-LET (linear energy transfer) or low-LET. LET is the energy transferred per unit length of the distance. High LET is said to be densely ionizing radiation and Low LET is said to be sparsely ionizing radiation. Representative examples of high-LET are neutrons and alpha particles. Representative examples of low-LET are x-ray and gamma rays. Low LET radiation including both x-rays and $\gamma$rays is most commonly used for radiotherapy of cancer patients. The radiation may be used for external radiation therapy that is usually given on an outpatient basis or for internal radiation therapy that uses radiation that is placed very close to or inside the tumor. In case of internal radiation therapy, the radiation source is usually sealed in a small holder called an implant. Implants may be in the form of thin wires, plastic tubes called catheters, ribbons, capsules, or seeds. The implant is put directly into the body. Internal radiation therapy may require a hospital stay. The ionizing radiation source is provided as a unit dose of radiation and is preferably an x-ray tube since it provides many advantages, such as convenient adjustable dosing where the source may be easily turned on and off, minimal disposal problems. A unit dose of radiation is generally measured in gray (Gy). The ionizing radiation source may also comprise a radioisotope, such as a solid radioisotopic source (e.g., wire, strip, pellet, seed, bead), or a liquid radioisotopic filled balloon. In the latter case, the balloon has been specially configured to prevent leakage of the radioisotopic material from the balloon into the body lumen or blood stream. Still further, the ionizing radiation source may comprise a receptacle in the catheter body for receiving radioisotopic materials like pellets or liquids. The radioisotopic material may be selected to emit $\alpha$, $\beta$ and $\gamma$. Usually, $\alpha$ and $\beta$ radiations are preferred since they may be quickly absorbed by the surrounding tissue and will not penetrate substantially beyond the wall of the body lumen being treated. Accordingly, incidental irradiation of the heart and other organs adjacent to the treatment region can be substantially eliminated. The total number of units provided will be an amount determined to be therapeutically effective by one skilled in treatment using ionizing radiation. This amount will vary with the subject and the type of malignancy or neoplasm being treated. The amount may vary but a patient may receive a dosage of about 30-75 Gy over several weeks.

Exemplary radiotherapeutic agents contemplated for use in combination with at least one metabolic reprogramming agent, at least two metabolic reprogramming agents, or at least three metabolic reprogramming agents include, factors that cause DNA damage, such as $\gamma$-rays, X-rays, and/or the directed delivery of radioisotopes to tumor cells. Other forms of DNA damaging factors are also contemplated, such as microwaves and UV-irradiation. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the target cell. In some embodiments, the radiotherapeutic agent is selected from the group consisting of $^{47}Sc$, $^{67}Cu$, $^{90}Y$, $^{109}Pd$, $^{123}I$, $^{125}I$, $^{131}I$, $^{186}Re$, $^{188}Re$, $^{199}Au$, $^{211}At$, $^{212}Pb$, $^{212}B$, $^{32}P$ and $^{33}P$, $^{71}Ge$, $^{77}As$, $^{103}Pb$, $^{105}Rh$, $^{111}Ag$, $^{119}Sb$, $^{121}Sn$, $^{131}Cs$, $^{143}Pr$, $^{161}Tb$, $^{177}Lu$, $^{191}Os$, $^{193}NPt$, $^{197}H$, $^{43}K$, $^{43}K$, $^{52}Fe$, $^{57}Co$, $^{67}Cu$, $^{67}Ga$, $^{68}Ga$, $^{77}Br$, $^{81}Rb$/$^{81}MKr$, $^{87}MSr$, $^{99}MTc$, $^{111}In$, $^{113}MIn$, $^{127}Cs$, $^{129}Cs$, $^{132}I$, $^{197}Hg$, $^{203}Pb$ and $^{206}Bi$, as described in U.S. Pat. No. 8,946,168.

In some contexts, an agent described herein can be administered with an antigen (e.g., to induce an immune response). In some embodiments, an adjuvant can be used in combination with the antigen.

An agent described herein can also be used in combination with an imaging agent. An agent (e.g., a metabolic reprogramming agent) can be attached to imaging agents for imaging and diagnosis of various diseased organs, tissues or cell types. The agent can be labeled or conjugated a fluorophore or radiotracer for use as an imaging agent. Many appropriate imaging agents are known in the art, as are methods for their attachment to agents (e.g., attaching an imaging agent to a proteins or peptides using metal chelate complexes, radioisotopes, fluorescent markers, or enzymes whose presence can be detected using a colorimetric markers (such as, but not limited to, urease, alkaline phosphatase, (horseradish) hydrogen peroxidase and glucose oxidase)). An agent may also be dual labeled with a radioisotope in order to combine imaging through nuclear approaches and be made into a unique cyclic structure and optimized for binding affinity and pharmacokinetics. Such agents can be administered by any number of methods known to those of ordinary skill in the art including, but not limited to, oral administration, inhalation, subcutaneous (sub-q), intravenous (I.V.), intraperitoneal (LP.), intramuscular (I.M.), intrathecal injection, or intratumoral injection. The methods, compositions, and uses described herein can be used alone or in combination with other techniques, to diagnose access and monitor and direct therapy of metabolic reprogramming disorders. In some contexts, the imaging agent can be used for detecting and/or monitoring tumors or sites of metastasis in a subject. For example, an agent (e.g., a metabolic reprogramming agent) can be administered in vivo and monitored using an appropriate label. Exemplary methods for detecting and/or monitoring an agent labeled with an imaging agent in vivo include Gamma Scintigraphy, Positron Emission Tomography (PET), Single Photon Emission Computer Tomography (SPECT), Magnetic Resonance Imaging (MRI), X-ray, Computer Assisted X-ray Tomography (CT), Near Infrared Spectroscopy, and Ultrasound. These techniques provide information regarding detection of neoplastic involvement, particularly of inaccessible nodes in subjects with malignant diseases. Knowledge on the size of the node and the filling of nodes can also be instructive. For example, agents or compositions targeted to the lymph nodes in detection applications will contain suitable contrast or imaging agents, such as ferromagnetic materials like iron oxide, perfluorochemicals such as perfluorooctylbromide, or gamma emitting radiolabels such as Technetium-99m, Indium-Ill, Gallium-67, Thallium-201, Iodine-131, 125, or 123, positron emitting radiolabels, such as Fluorine-18, or those produced by neutron activation, such as Samarium-153.

Imaging agents of use in the present disclosure include radioisotopes and dyes. Any conventional method according to radiolabeling which is suitable for labeling isotopes for in vivo use will be generally suitable for labeling detection agents according to the disclosure. Internal detection procedures include intraoperative, intravascular or endoscopic, including laparoscopic, techniques, both surgically invasive and noninvasive. For example, when detecting a lymph node, a high signal-to-background ratio should to be achieved. Therapy also requires a high absolute accretion of the therapeutic agent in the lymph node, as well as a reasonably long duration of uptake and binding.

Suitable radioisotopes for the methods of the disclosure include:

Actinium- 225, Astatine-211, Iodine-123, Iodine-125, Iodine-126, Iodine-131, Iodine-133, Bismuth-212, Bromine-77, Indium-111, Indium-113m, Gallium-67, Gallium-68, Ruthenium-95, Ruthenium-97, Ruthenium- 103, Ruthenium- 105, Mercury- 107, Mercury- 203, Rhenium-186, Rhenium-188, Tellurium- 121m, Tellurium- 122m, Tellurium- 125m, Thulium-165, Thulium- 167, Thulium- 168, Technetium-99m, Fluorine- 18, Silver-111, Platinum-197, Palladium- 109, Copper-67, Phosphorus-32, Phosphorus-33, Yttrium-90, Scandium-47, Samarium-153, Lutetium-177, Rhodium-105, Praseodymium- 142, Praseodymium- 143, Terbium-161, Holmium-166, Gold-199, Cobalt-57, Cobalt-58, Chromium-51, Iron-59, Selenium-75, Thallium-201, and Ytterbium- 169. The most preferred radioisotope for use in the presently disclosed subject matter is Technetium-99m. Preferably the radioisotope will emit a particle or ray in the 10-7,000 keV range, more preferably in the 50-1,500 keV range, and most preferably in the 80-250 keV range.

Isotopes preferred for external imaging include: Iodine-123, Iodine-131, Indium-111, Gallium-67, Ruthenium-97, Technetium-99m, Cobalt-57, Cobalt-58, Chromium-51, Iron-59, Selenium-75, Thallium-201, and Ytterbium- 169. Technetium- 99m is the most preferred radioisotope for external imaging in the disclosure.

Isotopes most preferred for internal detection include: Iodine-125, Iodine- 123, Iodine-131, Indium-111, Technetium-99m and Gallium-67. Technetium-99m is the most preferred isotope for internal detection.

## III. USES OF METABOLIC REPROGRAMMING AGENTS

The presently disclosed subject matter contemplates the use of at least one, at least two, or at least three metabolic reprogramming agents that decrease activity of at least one metabolic pathway selected from the group consisting of glutamine metabolism, glycolysis, and fatty acid synthesis, alone, or optionally together with one or more additional therapeutic agents described herein. Accordingly, in an unclaimed aspect of the presently disclosed subject matter involves the use of at least one metabolic reprogramming agent that decreases activity of at least one metabolic pathway selected from the group consisting of glutamine metabolism, glycolysis, and fatty acid synthesis for treating a condition, disease, or disorder that involves (i) metabolically reprogrammed cells whose activation, function, growth, proliferation, and/or survival depends on increased activity of at least one metabolic pathway selected from the group

consisting of glutamine metabolism, glycolysis, and fatty acid synthesis or (ii) at least one of aberrant and/or excessive glutamine metabolism, aberrant and/or excessive glycolysis, or aberrant and/or excessive fatty acid synthesis.

In some embodiments described herein, the presently disclosed subject matter involves the use of at least two metabolic reprogramming agents. In some embodiments, the presently disclosed subject matter involves the use of at least three metabolic reprogramming agents.

In some aspects described herein, the presently disclosed subject matter involves the use of at least one metabolic reprogramming agent that decreases glutamine metabolism as an immunotherapy to treat a cancer. In other aspects described herein, the presently disclosed subject matter involves the use of at least one metabolic reprogramming agent that decreases glutamine metabolism as an immunotherapy in combination with an additional immunotherapy to treat a cancer. Exemples of additional immunotherapy contemplated for use in combination with the at least one metabolic reprogramming agent include, without limitation, checkpoint blockade, adoptive cellular therapy, CAR-T cell therapy, marrow-infiltrating lymphocytes, A2aR blockade, KIR blockade, vaccines (e.g., tumor vaccines), passive immunotherapy antibodies, and combinations thereof.

In an aspect described herein, the presently disclosed subject matter involves the use of an effective amount of at least one metabolic reprogramming agent that decreases glutamine metabolism to treat lymphoma in a subject in need thereof.

In an aspect described herein, the presently disclosed subject matter involves the use of an effective amount of at least one metabolic reprogramming agent that decreases glutamine metabolism to treat melanoma in a subject in need thereof.

In some embodiments described herein, a use described herein further comprises using an effective amount of at least one metabolic reprogramming agent that decreases glycolysis. In some embodiments, a use described herein further comprises uses an effective amount of at least one metabolic reprogramming agent that increases fatty acid oxidation.

## IV. PHARMACEUTICAL COMPOSITIONS COMPRISING METABOLIC REPROGRAMMING AGENTS

The presently described subject matter also contemplates pharmaceutical compositions comprising one or more metabolic reprogramming agents for the treatment of certain conditions, diseases, and/or disorders involving metabolically reprogrammed cells. In some embodiments described herein, the presently disclosed methods comprise the use of the presently disclosed metabolic reprogramming agents for the manufacture of a medicament for the treatment of certain conditions, diseases, and/or disorders involve metabolically reprogrammed cells. The disclosure contemplates various pharmaceutical compositions comprising at least one, at least two, and or at least three metabolic reprogramming agents.

Accordingly, in an unclaimed aspect the presently disclosed subject matter provides a pharmaceutical composition comprising an effective amount of at least one, at least two, or at least three metabolic reprogramming agents that decrease the activity of at least one metabolic pathway selected from the group consisting of glutamine metabolism, glycolysis, and fatty acid synthesis, and a pharmaceutically acceptable carrier, diluent, or excipient.

In some unclaimed aspects, the presently disclosed subject matter provides a pharmaceutical composition comprising at least one metabolic reprogramming agent that decreases glutamine metabolism as an immunotherapy to treat a cancer, and a pharmaceutically acceptable carrier, diluent, or excipient. It should be appreciated that additional forms of immunotherapy are contemplated for use in combination with the pharmaceutical composition comprising at least one metabolic reprogramming agent, such as checkpoint blockade, adoptive cellular therapy, CAR-T cell therapy, marrow-infiltrating lymphocytes, A2aR blockade, KIR blockade, vaccines (e.g., tumor vaccines), passive immunotherapy antibodies, and combinations thereof.

In some embodiments described herein, the metabolic reprogramming composition comprises one or more additional therapeutic agents described herein. Generally, the presently disclosed compositions (e.g., comprising at least one metabolic reprogramming agent) can be administered to a subject for therapy by any suitable route of administration, including orally, nasally, transmucosally, ocularly, rectally, intravaginally, parenterally, including intramuscular, subcutaneous, intramedullary injections, as well as intrathecal, direct intraventricular, intravenous, intra-articular, intrasternal, intra-synovial, intra-hepatic, intralesional, intracranial, intraperitoneal, intranasal, intraocular injections, intratumoral injections, intracisternally, topically, as by powders, ointments or drops (including eyedrops), including buccally and sublingually, transdermally, through an inhalation spray, or other modes of delivery known in the art.

The phrases "systemic administration", "administered systemically", "peripheral administration" and "administered peripherally" as used herein mean the administration of compositions comprising at least one metabolic reprogramming agent, such that it enters the patient’s system and, thus, are subject to metabolism and other like processes, for example, subcutaneous administration.

The phrases "parenteral administration" and "administered parenterally" as used herein mean modes of administration other than enteral and topical administration, usually by injection, and includes, without limitation, intravenous,

intramuscular, intarterial, intrathecal, intracapsular, intraorbital, intraocular, intracardiac, intradermal, intraperitoneal, transtracheal, subcutaneous, subcuticular, intraarticular, subcapsular, subarachnoid, intraspinal and intrasternal injection and infusion.

The presently disclosed pharmaceutical compositions can be manufactured in a manner known in the art, *e.g.* by means of conventional mixing, dissolving, granulating, dragee-making, levitating, emulsifying, encapsulating, entrapping or lyophilizing processes.

In some embodiments described herein, the presently disclosed pharmaceutical compositions can be administered by rechargeable or biodegradable devices. For example, a variety of slow-release polymeric devices have been developed and tested *in vivo* for the controlled delivery of drugs, including proteinacious biopharmaceuticals. Suitable examples of sustained release preparations include semipermeable polymer matrices in the form of shaped articles, *e.g.,* films or microcapsules. Sustained release matrices include polyesters, hydrogels, polylactides (U.S. Patent No. 3,773,919; EP 58,481), copolymers of L-glutamic acid and gamma ethyl-L-glutamate (Sidman et al., Biopolymers 22:547, 1983), poly (2-hydroxyethyl-methacrylate) (Langer et al. (1981) J. Biomed. Mater. Res. 15:167; Langer (1982), Chem. Tech. 12:98), ethylene vinyl acetate (Langer et al. (1981) J. Biomed. Mater. Res. 15:167), or poly-D-(-)-3-hydroxybutyric acid (EP 133,988A). Sustained release compositions also include liposomally entrapped compositions comprising at least one metabolic reprogramming agent which can be prepared by methods known in the art (Epstein et al. (1985) Proc. Natl. Acad. Sci. U.S.A. 82:3688; Hwang et al. (1980) Proc. Natl. Acad. Sci. U.S.A. 77:4030; U.S. Patent Nos. 4,485,045 and 4,544,545; and EP 102,324A). Ordinarily, the liposomes are of the small (about 200-800 angstroms) unilamelar type in which the lipid content is greater than about 30 mol % cholesterol, the selected proportion being adjusted for the optimal therapy. Such materials can comprise an implant, for example, for sustained release of the presently disclosed compositions, which, in some embodiments, can be implanted at a particular, pre-determined target site.

In another embodiment described herein, the presently disclosed pharmaceutical compositions may comprise PEGylated therapeutics (e.g., PEGylated antibodies). PEGylation is a well established and validated approach for the modification of a range of antibodies, proteins, and peptides and involves the attachment of polyethylene glycol (PEG) at specific sites of the antibodies, proteins, and peptides (Chapman (2002) Adv. Drug Deliv. Rev. 54:531-545). Some effects of PEGylation include: (a) markedly improved circulating half-lives *in vivo* due to either evasion of renal clearance as a result of the polymer increasing the apparent size of the molecule to above the glomerular filtration limit, and/or through evasion of cellular clearance mechanisms; (b) improved pharmacokinetics; (c) improved solubility- PEG has been found to be soluble in many different solvents, ranging from water to many organic solvents, such as toluene, methylene chloride, ethanol and acetone; (d) PEGylated antibody fragments can be concentrated to 200 mg/ml, and the ability to do so opens up formulation and dosing options, such as subcutaneous administration of a high protein dose; this is in contrast to many other therapeutic antibodies which are typically administered intravenously; (e) enhanced proteolytic resistance of the conjugated protein (Cunningham-Rundles et.al. (1992) J. Immunol. Meth. 152:177-190); (f) improved bioavailability via reduced losses at subcutaneous injection sites; (g) reduced toxicity has been observed; for agents where toxicity is related to peak plasma level, a flatter pharmacokinetic profile achieved by sub-cutaneous administration of PEGylated protein is advantageous; proteins that elicit an immune response which has toxicity consequences may also benefit as a result of PEGylation; and (h) improved thermal and mechanical stability of the PEGylated molecule.

Pharmaceutical compositions for parenteral administration include aqueous solutions of compositions comprising at least one metabolic reprogramming agent. For injection, the presently disclosed pharmaceutical compositions can be formulated in aqueous solutions, for example, in some embodiments, in physiologically compatible buffers, such as Hank's solution, Ringer's solution, or physiologically buffered saline. Aqueous injection suspensions can contain substances that increase the viscosity of the suspension, such as sodium carboxymethyl cellulose, sorbitol, or dextran. Additionally, suspensions of compositions include fatty oils, such as sesame oil, or synthetic fatty acid esters, such as ethyl oleate or triglycerides, or liposomes. Optionally, the suspension also can contain suitable stabilizers or agents that increase the solubility of the compositions comprising at least one metabolic reprogramming agent to allow for the preparation of highly concentrated solutions.

For nasal or transmucosal administration generally, penetrants appropriate to the particular barrier to be permeated are used in the formulation. Such penetrants are generally known in the art.

Additional ingredients can be added to compositions for topical administration, as long as such ingredients are pharmaceutically acceptable and not deleterious to the epithelial cells or their function. Further, such additional ingredients should not adversely affect the epithelial penetration efficiency of the composition, and should not cause deterioration in the stability of the composition. For example, fragrances, opacifiers, antioxidants, gelling agents, stabilizers, surfactants, emollients, coloring agents, preservatives, buffering agents can be present. The pH of the presently disclosed topical composition can be adjusted to a physiologically acceptable range of from about 6.0 to about 9.0 by adding buffering agents thereto such that the composition is physiologically compatible with a subject's skin.

Regardless of the route of administration selected, the presently disclosed compositions are formulated into

pharmaceutically acceptable dosage forms, such as described herein or by other conventional methods known to those of skill in the art.

In general, the "effective amount" or "therapeutically effective amount" of an active agent or drug delivery device refers to the amount necessary to elicit the desired biological response. As will be appreciated by those of ordinary skill in this art, the effective amount of an agent or device may vary depending on such factors as the desired biological endpoint, the agent to be delivered, the composition of the encapsulating matrix, the target tissue.

The term "combination" is used in its broadest sense and means that a subject is administered at least two agents. More particularly, the term "in combination" refers to the concomitant administration of two (or more) active agents for the treatment of a, e.g., single disease state. As used herein, the active agents may be combined and administered in a single dosage form, may be administered as separate dosage forms at the same time, or may be administered as separate dosage forms that are administered alternately or sequentially on the same or separate days. In one embodiment of the presently disclosed subject matter, the active agents are combined and administered in a single dosage form. In another embodiment, the active agents are administered in separate dosage forms (e.g., wherein it is desirable to vary the amount of one but not the other). The single dosage form may include additional active agents for the treatment of the disease state.

Further, the presently disclosed compositions can be administered alone or in combination with adjuvants that enhance stability of the agents, facilitate administration of pharmaceutical compositions containing them in certain embodiments, provide increased dissolution or dispersion, increase activity, provide adjuvant therapy, including other active ingredients. Advantageously, such combination therapies utilize lower dosages of the conventional therapeutics, thus avoiding possible toxicity and adverse side effects incurred when those agents are used as monotherapies.

The timing of administration of at least one metabolic reprogramming agent can be varied so long as the beneficial effects of the combination of these agents are achieved. Accordingly, the phrase "in combination with" refers to the administration of at least one metabolic reprogramming agent, at least two metabolic reprogramming agents, or at least three metabolic reprogramming agents, and optionally additional agents either simultaneously, sequentially, or a combination thereof. Therefore, a subject administered a combination of at least one, at least two, or at least three metabolic reprogramming agents, and optionally additional agents can receive at least one metabolic reprogramming agent, at least two metabolic reprogramming agents, and at least three metabolic reprogramming agents, and optionally additional agents at the same time (i.e., simultaneously) or at different times (i.e., sequentially, in either order, on the same day or on different days), so long as the effect of the combination of all agents is achieved in the subject.

When administered sequentially, the agents can be administered within 1, 5, 10, 30, 60, 120, 180, 240 minutes or longer of one another. In other embodiments, agents administered sequentially, can be administered within 1, 2, 3, 4, 5, 10, 15, 20 or more days of one another. Where the agents are administered simultaneously, they can be administered to the subject as separate pharmaceutical compositions, each comprising either at least one metabolic reprogramming agent, at least two metabolic reprogramming agents, or at least three metabolic reprogramming agents, and optionally additional agents, or they can be administered to a subject as a single pharmaceutical composition comprising all agents.

When administered in combination, the effective concentration of each of the agents to elicit a particular biological response may be less than the effective concentration of each agent when administered alone, thereby allowing a reduction in the dose of one or more of the agents relative to the dose that would be needed if the agent was administered as a single agent. The effects of multiple agents may, but need not be, additive or synergistic. The agents may be administered multiple times.

In some embodiments, when administered in combination, the two or more agents can have a synergistic effect. As used herein, the terms "synergy," "synergistic," "synergistically" and derivations thereof, such as in a "synergistic effect" or a "synergistic combination" or a "synergistic composition" refer to circumstances under which the biological activity of a combination of an agent and at least one additional therapeutic agent is greater than the sum of the biological activities of the respective agents when administered individually.

Synergy can be expressed in terms of a "Synergy Index (SI)," which generally can be determined by the method described by F. C. Kull et al. Applied Microbiology 9, 538 (1961), from the ratio determined by:

$$Q_aQ_A + Q_bQ_B = \text{Synergy Index (SI)}$$

wherein:

$Q_A$ is the concentration of a component A, acting alone, which produced an end point in relation to component A;
$Q_a$ is the concentration of component A, in a mixture, which produced an end point;
$Q_B$ is the concentration of a component B, acting alone, which produced an end point in relation to component B; and
$Q_b$ is the concentration of component B, in a mixture, which produced an end point.

Generally, when the sum of $Q_a/Q_A$ and $Q_b/Q_B$ is greater than one, antagonism is indicated. When the sum is equal to one, additivity is indicated. When the sum is less than one, synergism is demonstrated. The lower the SI, the greater the synergy shown by that particular mixture. Thus, a "synergistic combination" has an activity higher that what can be expected based on the observed activities of the individual components when used alone. Further, a "synergistically effective amount" of a component refers to the amount of the component necessary to elicit a synergistic effect in, for example, another therapeutic agent present in the composition.

In another aspect, the presently disclosed subject matter provides a pharmaceutical composition including at least one metabolic reprogramming agent, at least two metabolic reprogramming agents, at least three metabolic reprogramming agents, and optionally additional agents, alone or in combination with one or more additional therapeutic agents in admixture with a pharmaceutically acceptable excipient.

More particularly, the presently disclosed subject matter provides a pharmaceutical composition comprising at least one metabolic reprogramming agent, at least two metabolic reprogramming agents, at least three metabolic reprogramming agents, and optionally additional agents, and a pharmaceutically acceptable carrier.

In therapeutic and/or diagnostic applications, the compounds of the disclosure can be formulated for a variety of modes of administration, including systemic and topical or localized administration. Techniques and formulations generally may be found in Remington: The Science and Practice of Pharmacy (20th ed.) Lippincott, Williams and Wilkins (2000).

Use of pharmaceutically acceptable inert carriers to formulate the compounds herein disclosed for the practice of the disclosure into dosages suitable for systemic administration is within the scope of the disclosure. With proper choice of carrier and suitable manufacturing practice, the compositions of the present disclosure, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the disclosure to be formulated as tablets, pills, capsules, liquids, gels, syrups, slurries, suspensions, for oral ingestion by a subject (e.g., patient) to be treated.

For nasal or inhalation delivery, the agents of the disclosure also may be formulated by methods known to those of skill in the art, and may include, for example, but not limited to, examples of solubilizing, diluting, or dispersing substances, such as saline; preservatives, such as benzyl alcohol; absorption promoters; and fluorocarbons.

Pharmaceutical compositions suitable for use in the present disclosure include compositions wherein the active ingredients are contained in an effective amount to achieve its intended purpose. Determination of the effective amounts is well within the capability of those skilled in the art, especially in light of the detailed disclosure provided herein. Generally, the compounds according to the disclosure are effective over a wide dosage range. For example, in the treatment of adult humans, dosages from 0.01 to 1000 mg, from 0.5 to 100 mg, from 1 to 50 mg per day, and from 5 to 40 mg per day are examples of dosages that may be used. A non-limiting dosage is 10 to 30 mg per day. The exact dosage will depend upon the route of administration, the form in which the compound is administered, the subject to be treated, the body weight of the subject to be treated, and the preference and experience of the attending physician.

In addition to the active ingredients, these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions.

The term "instructing" a patient as used herein means providing directions for applicable therapy, medication, treatment, treatment regimens, by any means, but preferably in writing. Instructing can be in the form of prescribing a course of treatment, or can be in the form of package inserts or other written promotional material. Accordingly, aspects of the presently disclosed subject matter include instructing a patient to receive a method of treatment or use an agent to treat a metabolic reprogramming disorder described herein.

The term "promoting" as used herein means offering, advertising, selling, or describing a particular drug, combination of drugs, or treatment modality, by any means, including writing, such as in the form of package inserts. Promoting herein refers to promotion of a metabolic reprogramming agent for an indication, where such promoting is authorized by the Food and Drug Administration (FDA) as having been demonstrated to be associated with statistically significant therapeutic efficacy and acceptable safety in a population of subjects. In some embodiments, promoting is not authorized by the Food and Drug Administration (FDA) (or other health regulatory agency, such as the European Medicines Agency (EMA), and promoting is for an off-label use. Accordingly, aspects of the presently disclosed subject matter include promoting a method of treatment or use described herein.

V. GeneralDefinitions

Although specific terms are employed herein, they are used in a generic and descriptive sense only and not for purposes of limitation. Unless otherwise defined, all technical and scientific terms used herein have the same meaning as commonly understood by one of ordinary skill in the art to which this presently described subject matter belongs.

While the following terms in relation to compounds of Formula (I) are believed to be well understood by one of ordinary skill in the art, the following definitions are set forth to facilitate explanation of the presently disclosed subject matter. These definitions are intended to supplement and illustrate, not preclude, the definitions that would be apparent to one of ordinary skill in the art upon review of the present disclosure.

The terms substituted, whether preceded by the term "optionally" or not, and substituent, as used herein, refer to the ability, as appreciated by one skilled in this art, to change one functional group for another functional group on a molecule, provided that the valency of all atoms is maintained. When more than one position in any given structure may be substituted with more than one substituent selected from a specified group, the substituent may be either the same or different at every position. The substituents also may be further substituted (e.g., an aryl group substituent may have another substituent off it, such as another aryl group, which is further substituted at one or more positions).

Where substituent groups or linking groups are specified by their conventional chemical formulae, written from left to right, they equally encompass the chemically identical substituents that would result from writing the structure from right to left, e.g., $-CH_2O-$ is equivalent to $-OCH_2-$; -C(=O)O- is equivalent to -OC(=O)-; -OC(=O)NR- is equivalent to -NRC(=O)O-.

When the term "independently selected" is used, the substituents being referred to (e.g., R groups, such as groups $R_1$, $R_2$, or variables, such as "m" and "n"), can be identical or different. For example, both $R_1$ and $R_2$ can be substituted alkyls, or $R_1$ can be hydrogen and $R_2$ can be a substituted alkyl.

The terms "a," "an," or "a(n)," when used in reference to a group of substituents herein, mean at least one. For example, where a compound is substituted with "an" alkyl or aryl, the compound is optionally substituted with at least one alkyl and/or at least one aryl. Moreover, where a moiety is substituted with an R substituent, the group may be referred to as "R-substituted." Where a moiety is R-substituted, the moiety is substituted with at least one R substituent and each R substituent is optionally different.

A named "R" or group will generally have the structure that is recognized in the art as corresponding to a group having that name, unless specified otherwise herein. For the purposes of illustration, certain representative "R" groups as set forth above are defined below.

Descriptions of compounds of the present disclosure are limited by principles of chemical bonding known to those skilled in the art. Accordingly, where a group may be substituted by one or more of a number of substituents, such substitutions are selected so as to comply with principles of chemical bonding and to give compounds which are not inherently unstable and/or would be known to one of ordinary skill in the art as likely to be unstable under ambient conditions, such as aqueous, neutral, and several known physiological conditions. For example, a heterocycloalkyl or heteroaryl is attached to the remainder of the molecule via a ring heteroatom in compliance with principles of chemical bonding known to those skilled in the art thereby avoiding inherently unstable compounds.

Unless otherwise explicitly defined, a "substituent group," as used herein, includes a functional group selected from one or more of the following moieties, which are defined herein:

The term hydrocarbon, as used herein, refers to any chemical group comprising hydrogen and carbon. The hydrocarbon may be substituted or unsubstituted. As would be known to one skilled in this art, all valencies must be satisfied in making any substitutions. The hydrocarbon may be unsaturated, saturated, branched, unbranched, cyclic, polycyclic, or heterocyclic. Illustrative hydrocarbons are further defined herein below and include, for example, methyl, ethyl, *n*-propyl, isopropyl, cyclopropyl, allyl, vinyl, *n*-butyl, tert-butyl, ethynyl, cyclohexyl.

The term "alkyl," by itself or as part of another substituent, means, unless otherwise stated, a straight (i.e., unbranched) or branched chain, acyclic hydrocarbon group, or combination thereof, which is fully saturated, and can include di- and multivalent groups, having the number of carbon atoms designated (i.e., $C_1$-$C_{10}$ means one to ten carbons, including 1, 2, 3, 4, 5, 6, 7, 8, 9, and 10 carbons). In particular embodiments, the term "alkyl" refers to $C_{1-20}$ inclusive, including 1, 2, 3, 4, 5, 6, 7, 8, 9, 10, 11, 12, 13, 14, 15, 16, 17, 18, 19, and 20 carbons, linear (i.e., "straight-chain"), branched, saturated hydrocarbon radicals derived from a hydrocarbon moiety containing between one and twenty carbon atoms by removal of a single hydrogen atom.

Representative saturated hydrocarbon groups include, but are not limited to, methyl, ethyl, *n*-propyl, isopropyl, *n*-butyl, isobutyl, *sec*-butyl, *tert*-butyl, *n*-pentyl, *sec*-pentyl, isopentyl, neopentyl, *n*-hexyl, *sec*-hexyl, *n*-heptyl, *n*-octyl, *n*-decyl, *n*-undecyl, dodecyl, cyclohexyl, (cyclohexyl)methyl, cyclopropylmethyl, and homologs and isomers thereof.

"Branched" refers to an alkyl group in which a lower alkyl group, such as methyl, ethyl or propyl, is attached to a linear alkyl chain. "Lower alkyl" refers to an alkyl group having 1 to about 8 carbon atoms (i.e., a $C_{1-8}$ alkyl), e.g., 1, 2, 3, 4, 5, 6, 7, or 8 carbon atoms. "Higher alkyl" refers to an alkyl group having about 10 to about 20 carbon atoms, e.g., 10, 11, 12, 13, 14, 15, 16, 17, 18, 19, or 20 carbon atoms. In certain embodiments, "alkyl" refers, in particular, to $C_{1-8}$ straight-chain alkyls. In other embodiments, "alkyl" refers, in particular, to $C_{1-8}$ branched-chain alkyls.

Alkyl groups can optionally be substituted (a "substituted alkyl") with one or more alkyl group substituents, which can be the same or different. The term "alkyl group substituent" includes but is not limited to alkyl, substituted alkyl, halo, arylamino, acyl, hydroxyl, aryloxyl, alkoxyl, alkylthio, arylthio, aralkyloxyl, aralkylthio, carboxyl, alkoxycarbonyl, oxo, and cycloalkyl. There can be optionally inserted along the alkyl chain one or more oxygen, sulfur or substituted or

unsubstituted nitrogen atoms, wherein the nitrogen substituent is hydrogen, lower alkyl (also referred to herein as "alkylaminoalkyl"), or aryl.

Thus, as used herein, the term "substituted alkyl" includes alkyl groups, as defined herein, in which one or more atoms or functional groups of the alkyl group are replaced with another atom or functional group, including for example, alkyl, substituted alkyl, halogen, aryl, substituted aryl, alkoxyl, hydroxyl, nitro, amino, alkylamino, dialkylamino, sulfate, and mercapto.

The term "heteroalkyl," by itself or in combination with another term, means, unless otherwise stated, a stable straight or branched chain, hydrocarbon group, or combinations thereof, consisting of at least one carbon atoms and at least one heteroatom selected from the group consisting of O, N, P, Si and S, and wherein the nitrogen, phosphorus, and sulfur atoms may optionally be oxidized and the nitrogen heteroatom may optionally be quaternized. The heteroatom(s) O, N, P and S and Si may be placed at any interior position of the heteroalkyl group or at the position at which alkyl group is attached to the remainder of the molecule. Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_{25}-S(O)-CH_3$, $-CH_2-CH_2-S(O)_2-CH_3$, -CH=CH-O-$CH_3$, $-Si(CH_3)_3$, $-CH_2-CH=N-OCH_3$, $-CH=CH-N(CH_3)-CH_3$, $O-CH_3$, $-O-CH_2-CH_3$, and -CN. Up to two or three heteroatoms may be consecutive, such as, for example, $-CH_2-NH-OCH_3$ and $-CH_2-O-Si(CH_3)_3$.

As described above, heteroalkyl groups, as used herein, include those groups that are attached to the remainder of the molecule through a heteroatom, such as -C(O)NR', -NR'R", -OR', -SR, -S(O)R, and/or $-S(O_2)R'$. Where "heteroalkyl" is recited, followed by recitations of specific heteroalkyl groups, such as -NR'R, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R".

"Cyclic" and "cycloalkyl" refer to a non-aromatic mono- or multicyclic ring system of about 3 to about 10 carbon atoms, e.g., 3, 4, 5, 6, 7, 8, 9, or 10 carbon atoms. The cycloalkyl group can be optionally partially unsaturated. The cycloalkyl group also can be optionally substituted with an alkyl group substituent as defined herein, oxo, and/or alkylene. There can be optionally inserted along the cyclic alkyl chain one or more oxygen, sulfur or substituted or unsubstituted nitrogen atoms, wherein the nitrogen substituent is hydrogen, unsubstituted alkyl, substituted alkyl, aryl, or substituted aryl, thus providing a heterocyclic group. Representative monocyclic cycloalkyl rings include cyclopentyl, cyclohexyl, and cycloheptyl. Multicyclic cycloalkyl rings include adamantyl, octahydronaphthyl, decalin, camphor, camphane, and noradamantyl, and fused ring systems, such as dihydro- and tetrahydronaphthalene.

The term "cycloalkylalkyl," as used herein, refers to a cycloalkyl group as defined hereinabove, which is attached to the parent molecular moiety through an alkyl group, also as defined above. Examples of cycloalkylalkyl groups include cyclopropylmethyl and cyclopentylethyl.

The terms "cycloheteroalkyl" or "heterocycloalkyl" refer to a non-aromatic ring system, unsaturated or partially unsaturated ring system, such as a 3- to 10-member substituted or unsubstituted cycloalkyl ring system, including one or more heteroatoms, which can be the same or different, and are selected from the group consisting of nitrogen (N), oxygen (O), sulfur (S), phosphorus (P), and silicon (Si), and optionally can include one or more double bonds.

The cycloheteroalkyl ring can be optionally fused to or otherwise attached to other cycloheteroalkyl rings and/or non-aromatic hydrocarbon rings. Heterocyclic rings include those having from one to three heteroatoms independently selected from oxygen, sulfur, and nitrogen, in which the nitrogen and sulfur heteroatoms may optionally be oxidized and the nitrogen heteroatom may optionally be quaternized. In certain embodiments, the term heterocylic refers to a non-aromatic 5-, 6-, or 7-membered ring or a polycyclic group wherein at least one ring atom is a heteroatom selected from O, S, and N (wherein the nitrogen and sulfur heteroatoms may be optionally oxidized), including, but not limited to, a bi- or tri-cyclic group, comprising fused six-membered rings having between one and three heteroatoms independently selected from the oxygen, sulfur, and nitrogen, wherein (i) each 5-membered ring has 0 to 2 double bonds, each 6-membered ring has 0 to 2 double bonds, and each 7-membered ring has 0 to 3 double bonds, (ii) the nitrogen and sulfur heteroatoms may be optionally oxidized, (iii) the nitrogen heteroatom may optionally be quaternized, and (iv) any of the above heterocyclic rings may be fused to an aryl or heteroaryl ring. Representative cycloheteroalkyl ring systems include, but are not limited to pyrrolidinyl, pyrrolinyl, imidazolidinyl, imidazolinyl, pyrazolidinyl, pyrazolinyl, piperidyl, piperazinyl, indolinyl, quinuclidinyl, morpholinyl, thiomorpholinyl, thiadiazinanyl, tetrahydrofuranyl.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl. Examples of heterocycloalkyl include, but are not limited to, 1-(1,2,5,6-tetrahydropyridyl), 1-piperidinyl, 2-piperidinyl, 3-piperidinyl, 4- morpholinyl, 3-morpholinyl, tetrahydrofuran-2-yl, tetrahydrofuran-3-yl, tetrahydrothien-2-yl, tetrahydrothien-3-yl, 1 -piperazinyl, 2-piperazinyl. The terms "cycloalkylene" and "heterocycloalkylene" refer to the divalent derivatives of cycloalkyl and heterocycloalkyl, respectively.

An unsaturated alkyl group is one having one or more double bonds or triple bonds. Examples of unsaturated

alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. Alkyl groups which are limited to hydrocarbon groups are termed "homoalkyl."

More particularly, the term "alkenyl" as used herein refers to a monovalent group derived from a $C_{1-20}$ inclusive straight or branched hydrocarbon moiety having at least one carbon-carbon double bond by the removal of a single hydrogen molecule. Alkenyl groups include, for example, ethenyl (i.e., vinyl), propenyl, butenyl, 1-methyl-2-buten-1-yl, pentenyl, hexenyl, octenyl, allenyl, and butadienyl.

The term "cycloalkenyl" as used herein refers to a cyclic hydrocarbon containing at least one carbon-carbon double bond. Examples of cycloalkenyl groups include cyclopropenyl, cyclobutenyl, cyclopentenyl, cyclopentadiene, cyclohexenyl, 1,3-cyclohexadiene, cycloheptenyl, cycloheptatrienyl, and cyclooctenyl.

The term "alkynyl" as used herein refers to a monovalent group derived from a straight or branched $C_{1-20}$ hydrocarbon of a designed number of carbon atoms containing at least one carbon-carbon triple bond. Examples of "alkynyl" include ethynyl, 2-propynyl (propargyl), 1-propynyl, pentynyl, hexynyl, and heptynyl groups.

The term "alkylene" by itself or a part of another substituent refers to a straight or branched bivalent aliphatic hydrocarbon group derived from an alkyl group having from 1 to about 20 carbon atoms, e.g., 1, 2, 3, 4, 5, 6, 7, 8, 9, 10, 11, 12, 13, 14, 15, 16, 17, 18, 19, or 20 carbon atoms. The alkylene group can be straight, branched or cyclic. The alkylene group also can be optionally unsaturated and/or substituted with one or more "alkyl group substituents." There can be optionally inserted along the alkylene group one or more oxygen, sulfur or substituted or unsubstituted nitrogen atoms (also referred to herein as "alkylaminoalkyl"), wherein the nitrogen substituent is alkyl as previously described. Exemplary alkylene groups include methylene ($-CH_2-$); ethylene ($-CH_2-CH_2-$); propylene ($-(CH_2)_3-$); cyclohexylene ($-C_6H_{10}-$); $-CH=CH-CH=CH-$; $-CH=CH-CH_2-$; $-CH_2CH_2CH_2CH_2-$, $-CH_2CH=CHCH_2-$, $-CH_2CsCCH_2-$, $-CH_2CH_2CH(CH_2CH_2CH_3)CH_2-$, $-(CH_2)_q-N(R)-(CH_2)_r-$, wherein each of q and r is independently an integer from 0 to about 20, e.g., 0, 1, 2, 3, 4, 5, 6, 7, 8, 9, 10, 11, 12, 13, 14, 15, 16, 17, 18, 19, or 20, and R is hydrogen or lower alkyl; methylenedioxyl ($-O-CH_2-O-$); and ethylenedioxyl ($-O-(CH_2)_2-O-$). An alkylene group can have about 2 to about 3 carbon atoms and can further have 6-20 carbons. Typically, an alkyl (or alkylene) group will have from 1 to 24 carbon atoms, with those groups having 10 or fewer carbon atoms being some embodiments of the present disclosure. A "lower alkyl" or "lower alkylene" is a shorter chain alkyl or alkylene group, generally having eight or fewer carbon atoms.

The term "heteroalkylene" by itself or as part of another substituent means a divalent group derived from heteroalkyl, as exemplified, but not limited by, $-CH_2-CH_2-S-CH_2-CH_2-$ and $-CH_2-S-CH_2-CH_2-NH-CH_2-$. For heteroalkylene groups, heteroatoms also can occupy either or both of the chain termini (e.g., alkyleneoxo, alkylenedioxo, alkyleneamino, alkylenediamino). Still further, for alkylene and heteroalkylene linking groups, no orientation of the linking group is implied by the direction in which the formula of the linking group is written. For example, the formula -C(O)OR'- represents both -C(O)OR'- and -R'OC(O)-.

The term "aryl" means, unless otherwise stated, an aromatic hydrocarbon substituent that can be a single ring or multiple rings (such as from 1 to 3 rings), which are fused together or linked covalently. The term "heteroaryl" refers to aryl groups (or rings) that contain from one to four heteroatoms (in each separate ring in the case of multiple rings) selected from N, O, and S, wherein the nitrogen and sulfur atoms are optionally oxidized, and the nitrogen atom(s) are optionally quaternized. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4- oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of above noted aryl and heteroaryl ring systems are selected from the group of acceptable substituents described below. The terms "arylene" and "heteroarylene" refer to the divalent forms of aryl and heteroaryl, respectively.

For brevity, the term "aryl" when used in combination with other terms (e.g., aryloxy, arylthioxy, arylalkyl) includes both aryl and heteroaryl rings as defined above. Thus, the terms "arylalkyl" and "heteroarylalkyl" are meant to include those groups in which an aryl or heteroaryl group is attached to an alkyl group (e.g., benzyl, phenethyl, pyridylmethyl, furylmethyl) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl). However, the term "haloaryl," as used herein is meant to cover only aryls substituted with one or more halogens.

Where a heteroalkyl, heterocycloalkyl, or heteroaryl includes a specific number of members (e.g. "3 to 7 membered"), the term "member" refers to a carbon or heteroatom.

Further, a structure represented generally by the formula:

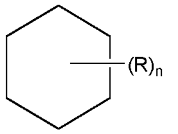
or
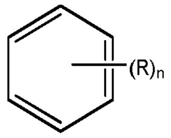

as used herein refers to a ring structure, for example, but not limited to a 3-carbon, a 4-carbon, a 5-carbon, a 6-carbon, a 7-carbon, aliphatic and/or aromatic cyclic compound, including a saturated ring structure, a partially saturated ring structure, and an unsaturated ring structure, comprising a substituent R group, wherein the R group can be present or absent, and when present, one or more R groups can each be substituted on one or more available carbon atoms of the ring structure. The presence or absence of the R group and number of R groups is determined by the value of the variable "n," which is an integer generally having a value ranging from 0 to the number of carbon atoms on the ring available for substitution. Each R group, if more than one, is substituted on an available carbon of the ring structure rather than on another R group. For example, the structure above where n is 0 to 2 would comprise compound groups including, but not limited to:

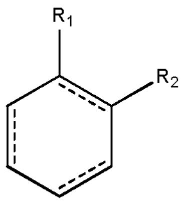
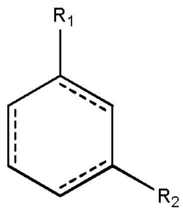
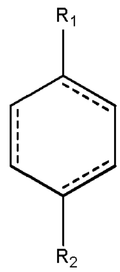
.

A dashed line representing a bond in a cyclic ring structure indicates that the bond can be either present or absent in the ring. That is, a dashed line representing a bond in a cyclic ring structure indicates that the ring structure is selected from the group consisting of a saturated ring structure, a partially saturated ring structure, and an unsaturated ring structure.

The symbol ( ~~~~ ) denotes the point of attachment of a moiety to the remainder of the molecule.

When a named atom of an aromatic ring or a heterocyclic aromatic ring is defined as being "absent," the named atom is replaced by a direct bond.

Each of above terms (e.g., "alkyl," "heteroalkyl," "cycloalkyl, and "heterocycloalkyl", "aryl," "heteroaryl," "phosphonate," and "sulfonate" as well as their divalent derivatives) are meant to include both substituted and unsubstituted forms of the indicated group. Optional substituents for each type of group are provided below.

Substituents for alkyl, heteroalkyl, cycloalkyl, heterocycloalkyl monovalent and divalent derivative groups (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to: -OR', =O, =NR', =N-OR', -NR'R", -SR', -halogen, -SiR'R"R‴, -OC(O)R', -C(O)R', $-CO_2R'$,-C(O)NR'R", -OC(O)NR'R", --S(O)R', - $S(O)_2R'$, $-S(O)_2NR'R"$, $-NRSO_2R'$, -CN and $-NO_2$ in a number ranging from zero to (2m'+1), where m' is the total number of carbon atoms in such groups. R', R", R‴ and R⁗ each may independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted alkyl, alkoxy or thioalkoxy groups, or arylalkyl groups. As used herein, an "alkoxy" group is an alkyl attached to the remainder of the molecule through a divalent oxygen. When a compound of the disclosure includes more than one R group, for example, each of the R groups is independently selected as are each R', R", R‴ and R⁗ groups when more than one of these groups is present. When R' and R" are attached to the same nitrogen atom, they can be combined with the nitrogen atom to form a 4-, 5-, 6-, or 7- membered ring. For example, -NR'R" is meant to include, but not be limited to, 1- pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., $-CF_3$ and - $CH_2CF_3$) and acyl (e.g., $-C(O)CH_3$, $-C(O)CF_3$, $-C(O)CH_2OCH_3$).

Similar to the substituents described for alkyl groups above, exemplary substituents for aryl and heteroaryl groups (as well as their divalent derivatives) are varied and are selected from, for example: halogen, -OR', -NR'R", -SR', -SiR'R"R‴, -OC(O)R', -C(O)R', $-CO_2R'$, -C(O)NR'R", -OC(O)NR'R", - NR"C(O)R', -NR'-C(O)NR"R‴, -NR"C(O)OR', -NR-C(NR'R"R‴)=NR⁗, -NR-C(NR'R")=NR‴ -S(O)R', $-S(O)_2R'$, $-S(O)_2NR'R"$, $-NRSO_2R'$, -CN and $-NO_2$, -R', $-N_3$, $-CH(Ph)_2$,

fluoro($C_1$-$C_4$)alkoxo, and fluoro($C_1$-$C_4$)alkyl, in a number ranging from zero to the total number of open valences on aromatic ring system; and where R', R", R'" and R"" may be independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. When a compound of the disclosure includes more than one R group, for example, each of the R groups is independently selected as are each R', R", R'" and R"" groups when more than one of these groups is present.

Two of the substituents on adjacent atoms of aryl or heteroaryl ring may optionally form a ring of the formula -T-C(O)-$(CRR')_q$-U-, wherein T and U are independently -NR-, -O-, -CRR'- or a single bond, and q is an integer of from 0 to 3. Alternatively, two of the substituents on adjacent atoms of aryl or heteroaryl ring may optionally be replaced with a substituent of the formula -A-$(CH_2)_r$-B-, wherein A and B are independently -CRR'-, -O-, -NR-, -S-, -S(O)-, -$S(O)_2$-, -$S(O)_2$NR'- or a single bond, and r is an integer of from 1 to 4.

One of the single bonds of the new ring so formed may optionally be replaced with a double bond. Alternatively, two of the substituents on adjacent atoms of aryl or heteroaryl ring may optionally be replaced with a substituent of the formula -$(CRR')_s$-X'- $(C"R'")_d$-, where s and d are independently integers of from 0 to 3, and X' is -O-, -NR'-, -S-, -S(O)-, -$S(O)_2$-, or -$S(O)_2$NR'-. The substituents R, R', R" and R'" may be independently selected from hydrogen, substituted or unsubstituted alkyl, substituted or unsubstituted cycloalkyl, substituted or unsubstituted heterocycloalkyl, substituted or unsubstituted aryl, and substituted or unsubstituted heteroaryl.

As used herein, the term "acyl" refers to an organic acid group wherein the -OH of the carboxyl group has been replaced with another substituent and has the general formula RC(=O)-, wherein R is an alkyl, alkenyl, alkynyl, aryl, carbocylic, heterocyclic, or aromatic heterocyclic group as defined herein). As such, the term "acyl" specifically includes arylacyl groups, such as a 2-(furan-2-yl)acetyl)- and a 2-phenylacetyl group. Specific examples of acyl groups include acetyl and benzoyl. Acyl groups also are intended to include amides, -RC(=O)NR', esters, -RC(=O)OR', ketones, -RC(=O)R', and aldehydes, -RC(=O)H.

The terms "alkoxyl" or "alkoxy" are used interchangeably herein and refer to a saturated (i.e., alkyl-O-) or unsaturated (i.e., alkenyl-O- and alkynyl-O-) group attached to the parent molecular moiety through an oxygen atom, wherein the terms "alkyl," "alkenyl," and "alkynyl" are as previously described and can include $C_{1-20}$ inclusive, linear, branched, or cyclic, saturated or unsaturated oxo-hydrocarbon chains, including, for example, methoxyl, ethoxyl, propoxyl, isopropoxyl, *n*-butoxyl, sec-butoxyl, tert-butoxyl, and *n*-pentoxyl, neopentoxyl, *n*-hexoxyl.

The term "alkoxyalkyl" as used herein refers to an alkyl-O-alkyl ether, for example, a methoxyethyl or an ethoxymethyl group.

"Aryloxyl" refers to an aryl-O- group wherein the aryl group is as previously described, including a substituted aryl. The term "aryloxyl" as used herein can refer to phenyloxyl or hexyloxyl, and alkyl, substituted alkyl, halo, or alkoxyl substituted phenyloxyl or hexyloxyl.

"Aralkyl" refers to an aryl-alkyl-group wherein aryl and alkyl are as previously described, and included substituted aryl and substituted alkyl. Exemplary aralkyl groups include benzyl, phenylethyl, and naphthylmethyl.

"Aralkyloxyl" refers to an aralkyl-O- group wherein the aralkyl group is as previously described. An exemplary aralkyloxyl group is benzyloxyl, i.e., $C_6H_5$-$CH_2$-O-. An aralkyloxyl group can optionally be substituted.

"Alkoxycarbonyl" refers to an alkyl-O-C(=O)- group. Exemplary alkoxycarbonyl groups include methoxycarbonyl, ethoxycarbonyl, butyloxycarbonyl, and *tert*-butyloxycarbonyl.

"Aryloxycarbonyl" refers to an aryl-O-C(=O)- group. Exemplary aryloxycarbonyl groups include phenoxy- and naphthoxy-carbonyl.

"Aralkoxycarbonyl" refers to an aralkyl-O-C(=O)- group. An exemplary aralkoxycarbonyl group is benzyloxycarbonyl.

"Carbamoyl" refers to an amide group of the formula -C(=O)$NH_2$. "Alkylcarbamoyl" refers to a R'RN-C(=O)- group wherein one of R and R' is hydrogen and the other of R and R' is alkyl and/or substituted alkyl as previously described. "Dialkylcarbamoyl" refers to a R'RN-C(=O)- group wherein each of R and R' is independently alkyl and/or substituted alkyl as previously described.

The term carbonyldioxyl, as used herein, refers to a carbonate group of the formula -O-C(=O)-OR.

"Acyloxyl" refers to an acyl-O- group wherein acyl is as previously described.

The term "amino" refers to the -$NH_2$ group and also refers to a nitrogen containing group as is known in the art derived from ammonia by the replacement of one or more hydrogen radicals by organic radicals. For example, the terms "acylamino" and "alkylamino" refer to specific N-substituted organic radicals with acyl and alkyl substituent groups respectively.

An "aminoalkyl" as used herein refers to an amino group covalently bound to an alkylene linker. More particularly, the terms alkylamino, dialkylamino, and trialkylamino as used herein refer to one, two, or three, respectively, alkyl groups, as previously defined, attached to the parent molecular moiety through a nitrogen atom. The term alkylamino refers to a group having the structure -NHR' wherein R' is an alkyl group, as previously defined; whereas the term dialkylamino refers to a group having the structure -NR'R", wherein R' and R" are each independently selected from the group

consisting of alkyl groups. The term trialkylamino refers to a group having the structure -NR'R"R‴, wherein R', R", and R‴ are each independently selected from the group consisting of alkyl groups. Additionally, R', R", and/or R'" taken together may optionally be $-(CH_2)_k-$ where k is an integer from 2 to 6. Examples include, but are not limited to, methylamino, dimethylamino, ethylamino, diethylamino, diethylaminocarbonyl, methylethylamino, isopropylamino, piperidino, trimethylamino, and propylamino.

The amino group is -NR'R", wherein R' and R" are typically selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

The terms alkylthioether and thioalkoxyl refer to a saturated (i.e., alkyl-S-) or unsaturated (i.e., alkenyl-S- and alkynyl-S-) group attached to the parent molecular moiety through a sulfur atom. Examples of thioalkoxyl moieties include, but are not limited to, methylthio, ethylthio, propylthio, isopropylthio, *n*-butylthio.

"Acylamino" refers to an acyl-NH- group wherein acyl is as previously described. "Aroylamino" refers to an aroyl-NH- group wherein aroyl is as previously described.

The term "carbonyl" refers to the -C(=O)- group, and can include an aldehyde group represented by the general formula R-C(=O)H.

The term "carboxyl" refers to the -COOH group. Such groups also are referred to herein as a "carboxylic acid" moiety.

The terms "halo," "halide," or "halogen" as used herein refer to fluoro, chloro, bromo, and iodo groups. Additionally, terms, such as "haloalkyl," are meant to include monohaloalkyl and polyhaloalkyl. For example, the term "halo($C_1$-$C_4$)alkyl" is mean to include, but not be limited to, trifluoromethyl, 2,2,2-trifluoroethyl, 4-chlorobutyl, 3-bromopropyl.

The term "hydroxyl" refers to the -OH group.

The term "hydroxyalkyl" refers to an alkyl group substituted with an -OH group.

The term "mercapto" refers to the -SH group.

The term "oxo" as used herein means an oxygen atom that is double bonded to a carbon atom or to another element.

The term "nitro" refers to the $-NO_2$ group.

The term "thio" refers to a compound described previously herein wherein a carbon or oxygen atom is replaced by a sulfur atom.

The term "sulfate" refers to the $-SO_4$ group.

The term thiohydroxyl or thiol, as used herein, refers to a group of the formula -SH.

More particularly, the term "sulfide" refers to compound having a group of the formula -SR.

The term "sulfone" refers to compound having a sulfonyl group $-S(O_2)R$.

The term "sulfoxide" refers to a compound having a sulfinyl group -S(O)R

The term ureido refers to a urea group of the formula $-NH-CO-NH_2$.

Throughout the specification and claims, a given chemical formula or name shall encompass all tautomers, congeners, and optical- and stereoisomers, as well as racemic mixtures where such isomers and mixtures exist.

Certain compounds of the present disclosure may possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)-or (S)- or, as D- or L- for amino acids, and individual isomers are encompassed within the scope of the present disclosure. The compounds of the present disclosure do not include those which are known in art to be too unstable to synthesize and/or isolate. The present disclosure is meant to include compounds in racemic, scalemic, and optically pure forms. Optically active (R)- and (S)-, or D- and $_L$-isomers may be prepared using chiral synthons or chiral reagents, or resolved using conventional techniques. When the compounds described herein contain olefenic bonds or other centers of geometric asymmetry, and unless specified otherwise, it is intended that the compounds include both E and Z geometric isomers.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the disclosure.

It will be apparent to one skilled in the art that certain compounds of this disclosure may exist in tautomeric forms, all such tautomeric forms of the compounds being within the scope of the disclosure. The term "tautomer," as used herein, refers to one of two or more structural isomers which exist in equilibrium and which are readily converted from one isomeric form to another.

Unless otherwise stated, structures depicted herein are also meant to include compounds which differ only in the presence of one or more isotopically enriched atoms. For example, compounds having the present structures with the replacement of a hydrogen by a deuterium or tritium, or the replacement of a carbon by $^{13}C$- or $^{14}C$-enriched carbon are within the scope of this disclosure.

The compounds of the present disclosure may also contain unnatural proportions of atomic isotopes at one or more of atoms that constitute such compounds. For example, the compounds may be radiolabeled with radioactive

isotopes, such as for example tritium ($^3H$), iodine-125 ($^{125}I$) or carbon-14 ($^{14}C$). All isotopic variations of the compounds of the present disclosure, whether radioactive or not, are encompassed within the scope of the present disclosure.

The compounds of the present disclosure may exist as salts. The present disclosure includes such salts. Examples of applicable salt forms include hydrochlorides, hydrobromides, sulfates, methanesulfonates, nitrates, maleates, acetates, citrates, fumarates, tartrates (e.g. (+)-tartrates, (-)-tartrates or mixtures thereof including racemic mixtures, succinates, benzoates and salts with amino acids, such as glutamic acid. These salts may be prepared by methods known to those skilled in art. Also included are base addition salts, such as sodium, potassium, calcium, ammonium, organic amino, or magnesium salt, or a similar salt. When compounds of the present disclosure contain relatively basic functionalities, acid addition salts can be obtained by contacting the neutral form of such compounds with a sufficient amount of the desired acid, either neat or in a suitable inert solvent or by ion exchange. Examples of acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids, as well as the salts derived organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic. Also included are salts of amino acids, such as arginate, and salts of organic acids like glucuronic or galactunoric acids. Certain specific compounds of the present disclosure contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents.

Certain compounds of the present disclosure can exist in unsolvated forms as well as solvated forms, including hydrated forms. In general, the solvated forms are equivalent to unsolvated forms and are encompassed within the scope of the present disclosure. Certain compounds of the present disclosure may exist in multiple crystalline or amorphous forms. In general, all physical forms are equivalent for the uses contemplated by the present disclosure and are intended to be within the scope of the present disclosure.

In addition to salt forms, the present disclosure provides compounds, which are in a prodrug form. Prodrugs of the compounds described herein are those compounds that readily undergo chemical changes under physiological conditions to provide the compounds of the present disclosure. Additionally, prodrugs can be converted to the compounds of the present disclosure by chemical or biochemical methods in an ex vivo environment. For example, prodrugs can be slowly converted to the compounds of the present disclosure when placed in a transdermal patch reservoir with a suitable enzyme or chemical reagent.

Following long-standing patent law convention, the terms "a," "an," and "the" refer to "one or more" when used in this application, including the claims. Thus, for example, reference to "a subject" includes a plurality of subjects, unless the context clearly is to the contrary (e.g., a plurality of subjects), and so forth.

Throughout this specification and the claims, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error, and other factors known to those of skill in the art depending on the desired properties sought to be obtained by the presently disclosed subject matter. For example, the term "about," when referring to a value can be meant to encompass variations of, in some embodiments, $\pm$ 100% in some embodiments $\pm$ 50%, in some embodiments $\pm$ 20%, in some embodiments $\pm$ 10%, in some embodiments $\pm$ 5%, in some embodiments $\pm$1%, in some embodiments $\pm$ 0.5%, and in some embodiments $\pm$ 0.1% from the specified amount, as such variations are appropriate to perform the disclosed methods or employ the disclosed compositions.

Further, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range (for example, the recitation of 1 to 5 includes 1, 2, 3, 4, and 5, as well as fractions thereof, e.g., 1.5, 2.25, 3.75, 4.1) and any range within that range.

EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter. The synthetic descriptions and specific examples that follow are only intended for the purposes of illustration, and are not to be construed as limiting in any manner to make compounds of the disclosure by other methods.

REFERENCE EXAMPLE 1

To explore the effect of DON on cancer, an EL4 mouse lymphoma model was used and it showed that DON could markedly inhibit lymphoma growth, suggesting that bone marrow derived tumors may be exquisitely susceptible to DON (FIG. 1). However, DON had a modest effect on inhibiting melanoma growth, which is a not a bone marrow derived tumor (FIG. 2).

REFERENCE EXAMPLE 2

FIG. 3 shows that DON conditioned B 16 melanoma to be killed by immunotherapy by inhibiting tumor infiltrating regulatory T cells ($Foxp3^+$).

REFERENCE EXAMPLE 3

**Summary**

The glutamine antagonist 6-diazo-5-oxo-L-norleucine (DON, 1) has shown robust anti-cancer efficacy in preclinical and clinical studies, but its development was halted due to marked systemic toxicities. Herein we demonstrate that DON inhibits glutamine metabolism and provides antitumor efficacy in a murine model of gliobastoma, although toxicity was observed. To enhance DON's therapeutic index, we utilized a prodrug strategy to increase its brain delivery and limit systemic exposure. While these dual moiety prodrugs exhibited rapid metabolism in mouse plasma, several provided excellent stability in monkey and human plasma. The most stable compound (**5c,** methyl-POM_DON-isopropyl-ester) was evaluated in monkeys, where it achieved 10-fold enhanced brain:plasma ratio versus DON. This strategy may provide a path to DON utilization in GBM patients.

**Introduction**

Glioblastoma multiforme (GBM) is the most common and lethal form of glioma (Ostrom, et al., 2015). Current therapeutic options that extend survival rates in GBM patients after tumor resection are limited to radiotherapy with concomitant administration of the DNA-alkylating agent Temodar™ and/or the carmustine releasing polymer Gliadel™ (Weller, et al., 2014). But even under this standard of care, postoperative median survival rates approximate 14.6 months, (Stupp, et al., 20015) and average five-year survival is less than 10% (Stupp, et al., 2009). There is, therefore, a significant unmet medical need for more effective GBM treatments.

The ability of GBMs and other cancers rapidly and persistently proliferate often outgrowing vascular supplies necessitates that they develop a specialized metabolism (Schulze, et al., 2012; Hensley, et al., 2013). Glutamine metabolism plays an essential role in this specialized metabolism by entering the TCA cycle and then contributing to the biosynthetic pathways (nucleotide, protein and lipid synthesis) necessary for unchecked cell growth, (Hensley, et al., 2013) rendering cancer cells dependent on glutamine. This so called "glutamine addiction" has been well characterized in GBMs (Dranoff, et al., 1985; Fogal, et al., 2015; Ru, et al., 2013; Tanaka, et al., 2015) and other cancers including leukemia/lymphomas, lung, triple-negative breast cancer and pancreatic cancer (Wise, et al., 2010; Hu, et al., 2009). To combat this glutamine addiction, several groups have explored the utility of selective glutaminase inhibitors (Erickson, et al., 2010; Lee, et al., 2014). Recently compounds that selectively target glutaminase (GLS1) have been developed (McDermott, et al., 2016; Shukla, et al., 2012) and one such compound is in clinical trials (Konopleva, et al., 2015). However, the brain penetration of this GLS1 inhibitor is poor (Gross, et al., 2014) and so far the clinical efficacy of this approach has been modest (Harding, et al., 2015).

Accumulating evidence shows that, as opposed to selective inhibition of one glutamine utilizing enzyme, broadly antagonizing glutamine utilization is a highly effective means of inhibiting tumor cell growth *in vitro* and *in vivo* (Hensley, et al., 2013; Ahluwalia, et al., 1990; Dranoff, et al., Cancer Res, 1985). 6-Diazo-5-oxo-L-norleucine (DON), non-natural amino acid with structural similarity to glutamine, was first isolated from Streptomyces bacteria in the early 1950's.

Because of its reactive diazo group, DON has demonstrated the ability to alkylate several glutamine-utilizing enzymes such as glutaminase, (Thangavelu, et al., 2014) NAD synthase, (Barclay, et al., 1966) and CTP synthetase (Hofer, et al., 2001) and FGAR aminotransferase (Grayzel, et al., 1960) in the purine and pyrimidine biosynthetic pathways, respectively. In preclinical models, DON robustly inhibited the growth of glutamine-dependent human cancer cells *in vitro,* and reduced tumor size and improved survival rates *in vivo* (Ahluwalia, et al., 1990; Cervantes-Madrid, et al., 2015). Because of the robust preclinical data, DON was evaluated in several clinical studies where it demonstrated promising results (Eagan, et al., 1982; Earhart, et al., 1982; Lynch, et al., 1982; Magill, et al., 1957; Rahman, et al., 1985; Sklaroff, et al., 1980; Sullivan, et al., 1962). For example, DON administration caused >50% regression or stable disease in late stage adult patients (Magill, et al., 1957) and in children with hematological malignancies or solid tumors (Sullivan, et al., 1988). Unfortunately, its development was hampered by dose limiting toxicities, many of which were GI-related (Magill, et al., 1957; Rahman, et al., 1985; Earhart, et al., 1990) as the GI system is highly dependent on glutamine utilization.

One strategy to improve the therapeutic index of DON for GBM therapy would be to increase its brain exposure while limiting its systemic exposure and thus toxicity (Upadhyay, et al., 2014). The prodrug approach is a well-established strategy to alter the pharmacokinetic and tissue distribution of drug molecules, however synthetically this approach is challenging with DON.

Herein we describe, for the first time, profound efficacy of DON in murine model of GBM, although overt toxicities were observed. In attempt to increase DON's therapeutic index, we systematically synthesized several DON prodrugs. We utilized three types of amine promoieties including (oxodioxolenyl)methyl carbamate esters (3a-b), dipeptides (4a-d), and pivaloyl-oxy-methyl (POM)-based esters (5a-c). The dual promoiety-containing prodrugs resulted in sufficient chemical stability permitting further evaluation in *in vitro* metabolic stability assays. While all of the prodrugs exhibited rapid metabolism in mouse plasma, some provided excellent plasma stability in monkeys and humans. When evaluated *in vivo,* the most stable DON prodrug (5c, methyl-POM-DON-isopropyl-ester) achieved 10-fold enhanced brain: plasma ratio versus DON in monkeys, thus providing a possible clinical path to DON utilization in GBM patients.

## RESULTS AND DISCUSSION

### DON showed robust inhibition of glutamine metabolism and antitumor efficacy in a murine GBM model

Despite several lines of evidence indicating the potential therapeutic efficacy of targeting glutamine metabolism in GBM, the effect of DON on GBM tumor growth has not yet been reported *in vivo.* Using the U87 flank xenograft mouse model of GBM, (Eshleman, et al., 2002) we first confirmed that systemic administration of DON (0.8 mg/kg, i.p) inhibited glutamine metabolism as reflected by an accumulation of endogenous glutamine in the tumor (FIG. 5A; $p < 0.05$) similar to other model systems. (Willis, et al., 1977; Windmueller, et al., 1974) We next evaluated its antitumor efficacy, and observed that DON not only halted tumor growth, but also effectively induced tumor regression. Specifically, vehicle-treated mice displayed significant tumor growth over the course of the experiment, while DON-treated mice (0.8 mg/kg, i.p, q.d.) exhibited >50% reduction in tumor volume (FIG. 5B; main effect of time [$F(3,48) = 6.049$, $p = 0.0014$]; treatment [$F(1,16) = 33.42$, $p < 0.0001$]; interaction [$F(3,48) = 21.70$, $p < 0.0001$]). Although DON exhibited excellent anti-tumor efficacy, all mice receiving DON displayed significant signs of toxicity including weight loss (12 $\pm$ 4.1%), hunching, ptosis, and lethargy. These findings are consistent with other reports of DON's efficacy and toxicity both *in vitro* and *in vivo.* (Fogal et al., 2015; Cervantes-Madrid, et al., 2015; Potter, et al., 2015)

### Simple DON alkyl ester prodrugs found to be unstable. Masking both DON's carboxylate and amine functionalities required to obtain stable prodrugs.

A prodrug strategy is often employed to enhance tissue penetration and change the pharmacokinetic parameters of effective drugs. Indeed, prodrug strategies are common in drug development as 5-7% of the approved worldwide drugs are prodrugs. (Rautio, et al., 2008) Our initial prodrug strategy for DON involved masking the carboxylic acid with simple alkyl esters such as ethyl **2a** and isopropyl **2b.** The synthesis of these two derivatives was straightforward affording compounds **2a** and **2b** in good yield. It is surprising to us that these simple DON alkyl esters had not previously been reported in the chemical literature, given that DON chemistry and utility has been described by numerous groups for over 60 years. (Magill, et al., 1957; Dion, et al., 1956; Magill, et al., 1956; Coffey, et al., 1956) One potential reason is that we discovered that **2a** and **2b** were unstable, slowing cyclizing to form unique diazo-imines **9a** and **9b.** These two unique derivatives were found to be chemically stable even at acidic pH, precluding their use as DON prodrugs.

Given the instability of simple ester prodrugs, we next masked both the primary amine and the carboxylate of DON with prodrug moieties. This dual promoiety strategy was rationalized to eliminate the potential for cyclization and potentially further improve the lipophilicity. We utilized three amine promoieties including (oxodioxolenyl)methyl carbamate esters (FIG. 4, **3a-b),** dipeptides **(4a-d),** and pivaloyl-oxy-methyl (POM)-based esters **(5a-c).** These promoieties

were chosen because they target distinct metabolic enzymes including paraoxonase, aminopeptidases, and carboxylesterases, respectively. To impart further metabolic stability of the POM derivative **(Table 1, 5a),** we prepared corresponding methyl-POM analogs **(5b, 5c).** All dual promoiety-containing prodrugs exhibited sufficient chemical stability to permit further evaluation.

**All DON prodrugs were rapidly metabolized in mouse plasma, however 5b and 5c found to be stable in human and monkey plasma**

**Table 2** outlines the plasma stability of DON prodrugs **3a-b, 4a-d** and **5a-c.** All prodrugs were completely metabolized in mouse plasma within the 60 min incubation time. However in monkey and human plasma, the prodrugs **5b** and **5c,** with methyl-POM on the amine and ethyl or isopropyl ester on the carboxylate respectively, demonstrated moderate/high stability with 60-75% of the prodrug remaining in monkey plasma, and 80-90% remaining in human plasma within the 60 min incubation time. Given 5c had the best stability profile in human plasma, it was selected for further evaluation in pharmacokinetic studies and compared to DON for its ability to penetrate the brain and liberate DON.

**Table 2. Plasma stability of DON prodrugs following 60 min incubation in mouse, monkey and human plasma.**

| **Compound #** | **PLASMA STABILITY** | | |
|---|---|---|---|
| | ***Mouse*** | ***Monkey*** | ***Human*** |
| **3a** | 0 | 0 | 0 |
| **3b** | 0 | 0 | 0 |
| **4a** | 0 | 1 | 1 |
| **4b** | 0 | 1 | 1 |
| **4c** | 0 | 4 | 12 |
| **4d** | 0 | 10 | 30 |
| **5a** | 0 | 0 | 9 |
| **5b** | 0 | 75 | 88 |
| **5c** | 0 | 61 | 91 |

**Lead prodrug 5c enhanced brain delivery of DON in monkeys but not in mice**

As expected from a DON prodrug which is completely metabolized in mouse plasma, we found that oral administration of DON **(1)** (0.8 mg/kg) and **5c** (0.8 mg/kg equivalent) exhibited similar DON plasma (FIG. 6) and brain (FIG. 6) concentration profiles when dosed in mice. The $AUC_{0-t}$ of DON following administration of DON and 5c in plasma were 1.25 nmol*h/mL and 1.22 nmol*h/mL respectively, suggesting rapid and complete liberation of DON from **5c** *in vivo.* Similarly in the mouse brain, the $AUC_{0-t}$ of DON following DON or **5c** administration was 0.57 nmol*h/g and 0.69 nmol*h/g, respectively, with the brain/plasma approximately 0.46 from DON vs 0.56 from prodrug **5c.** These pharmacokinetic results corroborated the *in vitro* metabolism studies suggesting **5c** was completely converted to DON in mouse plasma.

Following the mouse studies, we evaluated the pharmacokinetics of DON and **5c** (distereomer 1) in monkeys, as monkeys better mimicked the human plasma stability profile. In pigtail macaques, i.v. administration of DON and **5c** (1.6 mg/kg DON equivalent) demonstrated significantly different DON plasma profiles (FIG. 7). DON administration provided high plasma exposures with $AUC_{0-t}$ of 42.7 nmol*h/mL. In contrast, **5c** administration delivered ~ 7 fold lower plasma exposure of DON with $AUC_{0-t}$ of 5.71 nmol*h/mL. The opposite observation was seen in the CSF where enhanced DON levels were observed after **5c** administration. In the CSF at 30 min post dose, DON administration resulted in 0.33 nmol/g DON while **5c** delivered 1.43 nmol/g DON. When comparing plasma to CSF ratio at 30 min, **5c** demonstrated 10-fold enhancement of DON CSF delivery versus DON (FIG. 7).

**CONCLUSION**

We demonstrate profound efficacy of DON in a murine model of GBM, although overt toxicity was observed. To enhance DON's therapeutic index, we utilized a prodrug strategy to increase its brain delivery and limit its systemic exposure. While our dual promoeity prodrugs exhibited rapid metabolism in mice, our lead prodrug **5c** provided excellent

stability in human and monkey plasma, and achieved a 10-fold enhanced brain:plasma ratio versus DON in monkeys. This strategy may provide a path to DON utilization in GBM patients.

**Mice Efficacy Studies**

All mouse efficacy studies were conducted according to protocol #MO13M69 approved by the Animal Care and Use Committee at Johns Hopkins University. Female athymic (RH Foxnlnu mice) mice between 25 and 30g were obtained (Harlan Sprague Dawley Inc, Indianapolis, Indiana), and maintained on a 12 hour light-dark cycle with *ad libitum* access to food and water. U87 human glioma cells were injected s.c. ($5 \times 10^6$ cells in 100 ml of PBS) in four separate locations on the flanks of each mouse. When tumors grew to a mean volume of around 200 $mm^3$, mice were randomized into either vehicle (HEPES-buffered saline, i.p.) or DON (1; 0.8 mg/kg, i.p.). In one cohort, mice were administered a single dose of the appropriate solution two hours after which glutamine levels were quantified in the tumor as described previously.[49] In brief, tumors were harvested, snap frozen, and homogenized in liquid $N_2$ then subjected to metabolite extraction using methanol and DI water. Quantification was performed using Agilent 6520 Quadrupole-Time-of-Flight (Q-TOF) mass spectrometer with Agilent 1290 HPLC and using Agilent Mass Hunter and Agilent Qualitative and Quantitative Analysis Software packages. Glutamine content was averaged by group for each individual tumor (n=3-4/group), depicted as relative intensity, and analyzed by one-tailed t test. In a second cohort, efficacy experiments were conducted. Mice were injected once daily for six days; tumor volumes were measured using digital calipers and calculated according to the formula: [volume= (largest tumor dimension) x (smallest tumor dimension)$^2$ x 0.52] at 2, 4, and 6 days after the onset of treatment. Each individual tumor (n=8-10/group) was normalized to its pretreatment volume, averaged and analyzed by repeated measures two-way analysis of variance (ANOVA). If significant, a Bonferroni post hoc test was subsequently applied. Significance was defined as $p < .05$.

***In vitro* Metabolic Stability Studies**

For metabolic stability, plasma from rodent (mouse) and non-rodent species (human and monkeys) were used. For stability, prodrugs (10 μM) were spiked in each plasma matrix and incubated in an orbital shaker at 37 °C. At predetermined times (0 and 60 min), 100 μL aliquots of the mixture in duplicate were removed and the reaction quenched by addition of three times the volume of ice cold acetonitrile spiked with the internal standard (losartan 5μM). The samples were vortexed for 30 s and centrifuged at 12000 g for 10 min. 50 μL of the supernatant was diluted with 50 μL water and transferred to a 250 μL polypropylene vial sealed with a Teflon cap. Prodrug disappearance was monitored over time using a liquid chromatography and tandem mass spectrometry (LC/MS/MS) method as described below.

**Pharmacokinetic Studies in Mice**

All pharmacokinetic studies in mice were conducted according to protocol (#MO13M113) approved by the Animal Care and Use Committee at Johns Hopkins University. C57BL/6 mice between 25 and 30g were obtained from Harlan, and maintained on a 12 hour light-dark cycle with *ad libitum* access to food and water. To evaluate the brain and plasma pharmacokinetics of DON and its prodrug **5c,** 8-12 week old C57BL/6 were administered DON **(1;** 0.8 mg/kg, p.o. in phosphate-buffered saline) and its prodrug **5c** (at 0.8 mg/kg equivalent DON **(1),** p.o. in phosphate-buffered saline with 5% EtOH and 5% Tween-80). The mice were sacrificed by pentobarbital injection at 10, 30 and 90 minutes post drug administration, and blood was collected via cardiac puncture and placed into iced EDTA coated BD microtainers. Blood samples were spun at 2,000 g for 15 minutes, and plasma was removed and stored at -80°C. Brain tissues were harvested following blood collection and immediately snap frozen in liquid nitrogen and stored at -80°C until LC/MS analysis.

**Pharmacokinetic Studies in Non-human primates**

All monkey studies were conducted according to protocol (#PR15M298) approved by the Animal Care and Use Committee at Johns Hopkins University. Two female pigtail monkeys (approximately 3.5 kg, non-drug naive) were adjacently housed in stainless steel cages on a social interaction rack (contains 4 cages, each 32.5" wide x 28" deep x 32" high) maintaining temperature of 64-84°F, humidity of 30 - 70% with alternating 14-10 hour light/dark cycle as per the USDA Animal Welfare Act (9 CFR, Parts 1, 2, and 3). Food was provided daily in amounts appropriate for the size and age of the animals and RO purified water provided *ad libitum* through an in-cage lixit valve. Food enrichment was provided Monday through Friday. Prior to drug administration, macaques were sedated with ketamine given as an intramuscular injection prior to test article administration. Sedation was maintained through blood and cerebrospinal fluid (CSF) sample collections with ketamine at a starting rate of 15 mg/kg with additional doses of 20-30 mg during the first hour. At subsequent time points ketamine was given at 10-15 mg/kg. DON (50mM HEPES buffered saline) and **5c**

(Diastereoisomer 1), (50mM HEPES buffered saline containing 5% ethanol and 5% tween) were administered (1.6 mg/kg equivalent) to the animals at a dosing volume of 1mL/kg intravenously. CSF sample (target of 50 μL) was obtained by percutaneous puncture of the cisterna magna at 30 min post dose. Blood samples (1 mL) were collected at 15, 30, 1, 2 4 and 6 h post dose by percutaneous puncture of a peripheral vein. Samples were processed for plasma (centrifuged at a temperature of 4°C, at 3,000xg, for 10 minutes). All samples were maintained chilled on ice throughout processing. Samples were collected in microcentrifuge tubes, flash frozen, and placed in a freezer set to maintain -80°C until LC/MS analysis.

**Bioanalysis of DON**

We have previously published a highly sensitive method for analysis of DON in biological matrices (Alt, et al., 2015). However due to chemical lability of DON and its prodrugs, a milder derivatization method employing dabsyl chloride was developed and validated. Briefly, DON was extracted from samples (50 mg) with 250 μL methanol containing Glutamate-d5 (10 μM ISTD) by vortexing in low retention tubes. Samples were centrifuged at 16,000 X g for 5 minutes to precipitate proteins. Supernatants (200μL) were moved to new tube and dried at 45°C under vacuum for 1 hour. To each tube, 50 μL of 0.2 M sodium bicarbonate buffer (pH 9.0) and 100 μL of 10 mM dabsyl chloride in acetone was added. After vortexing, samples were incubated at 60°C for 15 minutes to derivatize. Samples (2 μL) were injected and separated on an Agilent 1290 equipped with a an Agilent Eclipse plus C18 RRHD 2.1 X100mm column over a 2.5 minute gradient from 20-95% acetonitrile + 0.1% formic acid and quantified on an Agilent 6520 QTOF mass spectrometer. Calibration curves over the range of .005-17.1 μg/mL in plasma and CSF for DON were constructed from the peak area ratio of the analyte to the internal standard using linear regression with a weighting factor of 1/(nominal concentration). Correlation coefficient of greater than 0.99 was obtained in all analytical runs. The mean predicted relative standard deviation for back calculated concentrations of the standards and QC's for all analytes were within the range of 85 to 115%, except for the lowest concentration which was within the range of 80 to 120% with an overall accuracy and precision of 6.7% and 6.6% respectively.

**Pharmacokinetic Analysis**

Mean concentration-time data was used for pharmacokinetic analysis. Non-compartmental-analysis module in WinNonlin® (version 5.3) was used to assess pharmacokinetic parameters. Peak plasma concentrations ($C_{max}$) and time to $C_{max}$ ($T_{max}$) were the observed values. Area under the curve (AUC) was calculated by log-linear trapezoidal rule to the end of sample collection ($AUC_{last}$).

REFERENCE EXAMPLE 4

In head-to-head comparisions, 25 was found to be markedly more effective than a clinical stage selective glutaminase inhibitor CB-839.

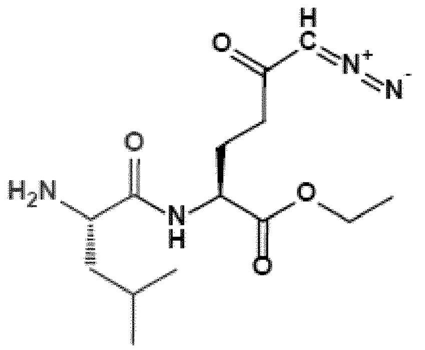

JHU-083

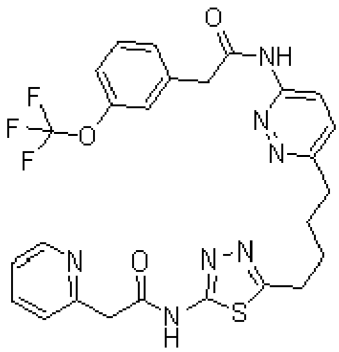

CB-839

FIG. 8 illustrates that 25 (5 day dosing starting on day 7) is superior to CB-839 (30 day dosing starting day 1) in a CT26 tumor model.

FIG. 9 illustrates that 25 (4 days starting on day 6) is superior to CB-839 (continuous twice daily dosing starting on day 1 prior to engraftment) in a CT26 tumor model. Mice received daily 25 (1.9 mg/kg) on days 6-9 as compared to BID glutaminase inhibitor on days 1-15.

FIG. 10 illustrates that **25** (daily days 7-22) is superior to CB-839 (continuous twice daily dosing days 1-29) in a 4T1 breast cancer model. Mice received daily **25** (1.0 mg.kg/d) for days 7-22 as compared to BID glutaminase inhibitor for days 1-29.

REFERENCE EXAMPLE 5

DON pro-drugs demonstrate efficacy in multiple tumor types, including efficacy in B & T cell lymphomas, colon cancers, breast cancer and melanoma. Daily dosing provides effective monotherapy. Every other day dosing leads to minimal resistance. FIG. 11 illustrates that **25** dosing of 1 mg/kg following by 0.3 mg/kg leads to a complete and durable response in the MC38 tumor.

FIG. 12 illustrates that **25** providese a robust response and improved overall survival in multiple tumor models, including *CT26 Colon Cancer.*

FIG. 13 illustrates that **25** provides a robust response and improved overall survival in multiple tumor models, including *4T1 Breast Cancer.*

FIG. 14 illustrates that mice cured with **25** alone immunologically reject tumors upon re-challenge, demonstrating that monotherapy with certain DON prodrugs, such as **25** monotherapy, is immunotherapy. FIG. 15 additionally illustrates that **25** is immunotherapy.

EXAMPLE 6

DON/DON prodrugs condition tumors to immunotherapy and significantly enhance the response to checkpoint inhibitors, adoptive cell transfer and A2aR inhibition

*Conclusions:* DON/DON prodrugs robustly enhance the immune mediated response to therapy with anti-PDl, the response to adoptive cell transfer, and the response to adenosine A2a receptor blockade. Immunotherapy fails patients with rapidly progressive disease due to the lag in response. 30-40% of patients treated with anti-PDl therapy progress rapidly in the first few months, chemotherapy showed an early survival advantage in NSCLC likely due to it's quick effect on rapidly growing disease compared to immunotherapy, and conditioning and adjuvant therapies to immunotherapy must be able to control tumor growth to be effective in these patients.

FIG. 16 illustrates that glutamine inhibition (e.g., using DON) reduces the oxygen consumption and lactate production of tumor cells. FIG. 17 illustrates that glutamine inhibition (e.g., using DON) also improved the CD8/Treg ratio in the tumor and reduces hypoxia in the TILs.

FIG. 18 illustrates that **25** conditions tumors to be eliminated by anti-PDl therapy in the MC38 Model, and that **25** rescues anti-PDl failures. In addition, FIG. 19 illustrates that even in the more difficult CT26 model, **25** enhances the response to anti-PDl. Similarly, FIG. 20 illustrates that inhibiting glutamine metabolism potentiates the anti-tumor response to A2aR inhibition. Finally, FIG. 21 illustrates that inhibiting glutamine metabolism enhances the efficacy of adoptive cellular therapy (ACT) in a B16-OVA model.

REFERENCES

Ahluwalia, G. S.; Grem, J. L.; Hao, Z.; Cooney, D. A. Metabolism and action of amino acid analog anti-cancer agents. Pharmacol Ther 1990, 46, 243-271.

Alt, J.; Potter, M. C.; Rojas, C.; Slusher, B. S. Bioanalysis of 6-diazo-5-oxo-l-norleucine in plasma and brain by ultra-performance liquid chromatography mass spectrometry. Anal Biochem 2015, 474, 28-34.

Barclay, R. K.; Phillipps, M. A. Effects of 6-diazo-5-oxol-norleucine and other tumor inhibitors on the biosynthesis of nicotinamide adenine dinucleotide in mice. Cancer Res 1966, 26, 282-286.

Cervantes-Madrid, D.; Romero, Y.; Duenas-Gonzalez, A. Reviving Lonidamine and 6-Diazo-5-oxo-L-norleucine to Be Used in Combination for Metabolic Cancer Therapy. Biomed Res Int 2015, 2015, 690492.

Coffey, G. L.; Ehrlich, J.; Fisher, M. W.; Hillegas, A. B.; Kohberger, D. L.; Machamer, H. E.; Rightsel, W. A.; Roegner, F. R. 6-Diazo-5-oxo-L-norleucine, a new tumor-inhibitory substance. I. Biologic studies. Antibiot Chemother (Northfield) 1956, 6, 487-497.

D'Andrea, S.; Zheng, Z.; Scola, P. Inhibitors of Hepatitis C Virus. In Google Patents: 2008.

Dion, H. W.; Fusari, S. A.; Jakubowski, Z. L.; Zora, J. G.; Bartz, Q. R. 6-Diazo-5-oxo-L-norleucine, A New Tumor-inhibitory Substance. II.1 Isolation and Characterization. J. Am. Chem. Soc., 1956, 78, 3075-3077.

Dolan D.E.; Gupta S. PD-1 pathway inhibitors: changing the landscape of cancer immunotherapy. Cancer Control, 2014, 21(3):231-7.

Dranoff, G.; Elion, G. B.; Friedman, H. S.; Bigner, D. D. Combination chemotherapy in vitro exploiting glutamine

metabolism of human glioma and medulloblastoma. Cancer Res 1985, 45, 4082-4086.

Dranoff, G.; Elion, G. B.; Friedman, H. S.; Campbell, G. L.; Bigner, D. D. Influence of glutamine on the growth of human glioma and medulloblastoma in culture. Cancer Res 1985, 45, 4077-4081.

Eagan, R. T.; Frytak, S.; Nichols, W. C.; Creagan, E. T.; Ingle, J. N. Phase II study on DON in patients with previously treated advanced lung cancer. Cancer Treat Rep 1982, 66, 1665-1666.

Earhart, R. H.; Amato, D. J.; Chang, A. Y.; Borden, E. C.; Shiraki, M.; Dowd, M. E.; Comis, R. L.; Davis, T. E.; Smith, T. J. Phase II trial of 6-diazo-5-oxo-L-norleucine versus aclacinomycin-A in advanced sarcomas and mesotheliomas. Invest New Drugs 1990, 8, 113-119.

Earhart, R. H.; Koeller, J. M.; Davis, H. L. Phase I trial of 6-diazo-5-oxo-L-norleucine (DON) administered by 5-day courses. Cancer Treat Rep 1982, 66, 1215-1217.

Engels, E.A., et al. Spectrum of Cancer Risk among U.S. Solid Organ Transplant Recipients: The Transplant Cancer Match Study. JAMA, 2011.

Erickson, J. W.; Cerione, R. A. Glutaminase: a hot spot for regulation of cancer cell metabolism? Oncotarget 2010, 1, 734-740.

Eshleman, J. S.; Carlson, B. L.; Mladek, A. C.; Kastner, B. D.; Shide, K. L.; Sarkaria, J. N. Inhibition of the mammalian target of rapamycin sensitizes U87 xenografts to fractionated radiation therapy. Cancer Res 2002, 62, 7291-7297.

Fogal, V.; Babic, I.; Chao, Y.; Pastorino, S.; Mukthavaram, R.; Jiang, P.; Cho, Y. J.; Pingle, S. C.; Crawford, J. R.; Piccioni, D. E.; Kesari, S. Mitochondrial p32 is upregulated in Myc expressing brain cancers and mediates glutamine addiction. Oncotarget 2015, 6, 1157-1170

Gallop, M. A.; Xu, F.; Phan, T.; Dilip, U.; Peng, G. Acyloxyalkyl carbamate prodrugs, methods of synthesis and use. In Google Patents: 2008.

Grayzel, A. I.; Seegmiller, J. E.; Love, E. Suppression of uric acid synthesis in the gouty human by the use of 6-diazo-5-oxo-L-norleucine. J Clin Invest 1960, 39, 447-454.

Gross, M. I.; Demo, S. D.; Dennison, J. B.; Chen, L.; Chernov-Rogan, T.; Goyal, B.; Janes, J. R.; Laidig, G. J.; Lewis, E. R.; Li, J.; MacKinnon, A. L.; Parlati, F.; Rodriguez, M. L. M.; Shwonek, P. J.; Sjogren, E. B.; Stanton, T. F.; Wang, T.; Yang, J.; Zhao, F. Y.; Bennett, M. K. Antitumor Activity of the Glutaminase Inhibitor CB-839 in Triple-Negative Breast Cancer. Mol Cancer Ther 2014.

Harding, J. J. T., M.L.; Munster, P.N.; Le, M.H.; Molineaux, C.; Bennett, M.K.; Mittra, E.; Burris,H.A.; Clark, A.S.; Dunphy, M.; Meric-Bernstam, F.; Patel, M.R.; DeMichele, A.; Infante, J.R. Safety and tolerability of increasing doses of CB-839, a first-in-class, orally administered small molecule inhibitor of glutaminase, in solid tumors. J Clin Oncol 2015.

Hensley, C. T.; Wasti, A. T.; DeBerardinis, R. J. Glutamine and cancer: cell biology, physiology, and clinical opportunities. J Clin Invest 2013, 123, 3678-3684.

Hofer, A.; Steverding, D.; Chabes, A.; Brun, R.; Thelander, L. Trypanosoma brucei CTP synthetase: a target for the treatment of African sleeping sickness. Proc Natl Acad Sci USA 2001, 98, 6412-6416.

Hu, X.; Stern, H. M.; Ge, L.; O'Brien, C.; Haydu, L.; Honchell, C. D.; Haverty, P. M.; Peters, B. A.; Wu, T. D.; Amler, L. C.; Chant, J.; Stokoe, D.; Lackner, M. R.; Cavet, G. Genetic alterations and oncogenic pathways associated with breast cancer subtypes. Mol Cancer Res 2009, 7, 511-522.

Keicher, J. D.; Roberts, C. D.; Rajwanshi, V. K.; Griffith, R. C.; Zheng, X.; Liehr, S. J. R.; Prhavc, M.; Kim, C. U.; Ray, A. S. Amino tricyclic-nucleoside compounds, compositions, and methods of use. In Google Patents: 2009.

Konopleva, M. Y.; Flinn, I. W.; Wang, E.; DiNardo, C. D.; Bennett, M.; Molineaux, C.; Le, M.; Maris, M.; Frankfurt, O. In Phase 1 study: Safety and tolerability of increasing doses of cb-839, an orally-administered small molecule inhibitor of glutaminase, in acute leukemia, Haematologica, 2015; Ferrata Storti Foundation Via Giuseppe Belli 4, 27100 Pavia, Italy: 2015; pp 378-379.

Le, A.; Lane, A. N.; Hamaker, M.; Bose, S.; Gouw, A.; Barbi, J.; Tsukamoto, T.; Rojas, C. J.; Slusher, B. S.; Zhang, H.; Zimmerman, L. J.; Liebler, D. C.; Slebos, R. J.; Lorkiewicz, P. K.; Higashi, R. M.; Fan, T. W.; Dang, C. V. Glucose-independent glutamine metabolism via TCA cycling for proliferation and survival in B cells. Cell Metab 2012, 15, 110-121.

Lee, Y. Z.; Yang, C. W.; Chang, H. Y.; Hsu, H. Y.; Chen, I. S.; Chang, H. S.; Lee, C. H.; Lee, J. C.; Kumar, C. R.; Qiu, Y. Q.; Chao, Y. S.; Lee, S. J. Discovery of selective inhibitors of Glutaminase-2, which inhibit mTORC1, activate autophagy and inhibit proliferation in cancer cells. Oncotarget 2014, 5, 6087-6101.

Liddy et al., Nature Med. 18:980-7, (2012); Grupp et al., New England J. Med. 368:1509-18, (2013)).

Lynch, G.; Kemeny, N.; Casper, E. Phase II evaluation of DON (6-diazo-5-oxo-L-norleucine) in patients with advanced colorectal carcinoma. Am J Clin Oncol 1982, 5, 541-543.

Magill, G. B.; Myers, W. P. Alterations in calcium metabolism in cancer patients treated with 6-diazo-5-oxo-L-norleucine. Proc Soc Exp Biol Med 1956, 93, 314-318.

Magill, G. B.; Myers, W. P.; Reilly, H. C.; Putnam, R. C.; Magill, J. W.; Sykes, M. P.; Escher, G. C.; Karnofsky, D. A.; Burchenal, J. H. Pharmacological and initial therapeutic observations on 6-diazo-5-oxo-1-norleucine (DON) in

human neoplastic disease. Cancer 1957, 10, 1138-1150.
McDermott, L. A.; Iyer, P.; Vernetti, L.; Rimer, S.; Sun, J.; Boby, M.; Yang, T.; Fioravanti, M.; O'Neill, J.; Wang, L.; Drakes, D.; Katt, W.; Huang, Q.; Cerione, R. Design and evaluation of novel glutaminase inhibitors. Bioorg Med Chem 2016, 24, 1819-1839.
Ngiow S.F.; Teng M.W., Smyth M.J. Prospects for TIM3-Targeted Antitumor Immunotherapy. Cancer Res 2011, 71(21):6567-71.
Ostrom, Q. T.; Gittleman, H.; Fulop, J.; Liu, M.; Blanda, R.; Kromer, C.; Wolinsky, Y.; Kruchko, C.; Barnholtz-Sloan, J. S. CBTRUS Statistical Report: Primary Brain and Central Nervous System Tumors Diagnosed in the United States in 2008-2012. Neuro Oncol 2015, 17 Suppl 4, iv1-iv62.
Potter, M. C.; Baxter, V. K.; Mathey, R. W.; Alt, J.; Rojas, C.; Griffin, D. E.; Slusher, B. S. Neurological sequelae induced by alphavirus infection of the CNS are attenuated by treatment with the glutamine antagonist 6-diazo-5-oxo-l-norleucine. J Neurovirol 2015, 21, 159-173.
Powell et al., "A2aR antagonists: Next generation checkpoint blockade for cancer immunotherapy," Comput Struct-Biotechnol J. 2015; 13: 265-272.
Rahman, A.; Smith, F. P.; Luc, P. T.; Woolley, P. V. Phase I study and clinical pharmacology of 6-diazo-5-oxo-L-norleucine (DON). Invest New Drugs 1985, 3, 369-374.
Rautio, J.; Kumpulainen, H.; Heimbach, T.; Oliyai, R.; Oh, D.; Jarvinen, T.; Savolainen, J. Prodrugs: design and clinical applications. Nat Rev Drug Discov 2008, 7, 255-270.
Ru, P.; Williams, T. M.; Chakravarti, A.; Guo, D. Tumor metabolism of malignant gliomas. Cancers (Basel) 2013, 5, 1469-1484.
Schulze, A.; Harris, A. L. How cancer metabolism is tuned for proliferation and vulnerable to disruption. Nature 2012, 491, 364-373.
Shukla, K.; Ferraris, D. V.; Thomas, A. G.; Stathis, M.; Duvall, B.; Delahanty, G.; Alt, J.; Rais, R.; Rojas, C.; Gao, P.; Xiang, Y.; Dang, C. V.; Slusher, B. S.; Tsukamoto, T. Design, synthesis, and pharmacological evaluation of bis-2-(5-phenylacetamido-1,2,4-thiadiazol-2-yl)ethyl sulfide 3 (BPTES) analogs as glutaminase inhibitors. J Med Chem 2012, 55, 10551-10563.
Sklaroff, R. B.; Casper, E. S.; Magill, G. B.; Young, C. W. Phase I study of 6-diazo-5-oxo-L-norleucine (DON). Cancer Treat Rep 1980, 64, 1247-1251.
Stupp, R.; Hegi, M. E.; Mason, W. P.; van den Bent, M. J.; Taphoorn, M. J.; Janzer, R. C.; Ludwin, S. K.; Allgeier, A.; Fisher, B.; Belanger, K.; Hau, P.; Brandes, A. A.; Gijtenbeek, J.; Marosi, C.; Vecht, C. J.; Mokhtari, K.; Wesseling, P.; Villa, S.; Eisenhauer, E.; Gorlia, T.; Weller, M.; Lacombe, D.; Cairncross, J. G.; Mirimanoff, R. O. Effects of radiotherapy with concomitant and adjuvant temozolomide versus radiotherapy alone on survival in glioblastoma in a randomised phase III study: 5-year analysis of the EORTC-NCIC trial. Lancet Oncol 2009, 10, 459-466.
Stupp, R.; Mason, W. P.; van den Bent, M. J.; Weller, M.; Fisher, B.; Taphoorn, M. J.; Belanger, K.; Brandes, A. A.; Marosi, C.; Bogdahn, U.; Curschmann, J.; Janzer, R. C.; Ludwin, S. K.; Gorlia, T.; Allgeier, A.; Lacombe, D.; Cairncross, J. G.; Eisenhauer, E.; Mirimanoff, R. O. Radiotherapy plus concomitant and adjuvant temozolomide for glioblastoma. N Engl J Med 2005, 352, 987-996.
Sullivan, M. P.; Beatty, E. C., Jr.; Hyman, C. B.; Murphy, M. L.; Pierce, M. I.; Severo, N. C. A comparison of the effectiveness of standard dose 6-mercaptopurine, combination 6-mercaptopurine and DON, and high-loading 6-mercaptopurine therapies in treatment of the acute leukemias of childhood: results of a coperative study. Cancer Chemother Rep 1962, 18, 83-95.
Sullivan, M. P.; Nelson, J. A.; Feldman, S.; Van Nguyen, B. Pharmacokinetic and phase I study of intravenous DON (6-diazo-5-oxo-L-norleucine) in children. Cancer Chemother Pharmacol 1988, 21, 78-84.
Tanaka, K.; Sasayama, T.; Irino, Y.; Takata, K.; Nagashima, H.; Satoh, N.; Kyotani, K.; Mizowaki, T.; Imahori, T.; Ejima, Y.; Masui, K.; Gini, B.; Yang, H.; Hosoda, K.; Sasaki, R.; Mischel, P. S.; Kohmura, E. Compensatory glutamine metabolism promotes glioblastoma resistance to mTOR inhibitor treatment. J Clin Invest 2015,125, 1591-1602.
Thangavelu, K.; Chong, Q. Y.; Low, B. C.; Sivaraman, J. Structural basis for the active site inhibition mechanism of human kidney-type glutaminase (KGA). Sci Rep 2014, 4, 3827.
Upadhyay, R. K. Drug delivery systems, CNS protection, and the blood brain barrier. Biomed Res Int 2014, 2014, 869269.
Weller, M.; van den Bent, M.; Hopkins, K.; Tonn, J. C.; Stupp, R.; Falini, A.; Cohen-Jonathan-Moyal, E.; Frappaz, D.; Henriksson, R.; Balana, C.; Chinot, O.; Ram, Z.; Reifenberger, G.; Soffietti, R.; Wick, W. EANO guideline for the diagnosis and treatment of anaplastic gliomas and glioblastoma. Lancet Oncol 2014, 15, e395-403.
Willis, R. C.; Seegmiller, J. E. The inhibition by 6-diazo-5-oxo-1-norleucine of glutamine catabolism of the cultured human lymphoblast. J Cell Physiol 1977, 93, 375-382.
Windmueller, H. G.; Spaeth, A. E. Uptake and metabolism of plasma glutamine by the small intestine. J Biol Chem 1974, 249, 5070-5079.
Wise, D. R.; Thompson, C. B. Glutamine addiction: a new therapeutic target in cancer. Trends Biochem Sci 2010,

35, 427-433.

All publications, patent applications, patents, and other references mentioned in the specification are indicative of the level of those skilled in the art to which the presently disclosed subject matter pertains. It will be understood that, although a number of patent applications, patents, and other references are referred to herein, such reference does not constitute an admission that any of these documents forms part of the common general knowledge in the art. In case of a conflict between the specification and any of the incorporated references, the specification shall control. Standard art-accepted meanings of terms are used herein unless indicated otherwise. Standard abbreviations for various terms are used herein.

## Claims

1. A compound having formula (I):

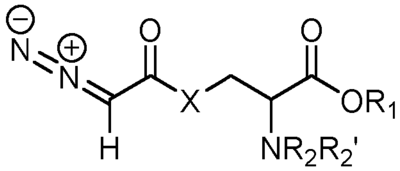

(I);

or a pharmaceutically acceptable salt thereof, wherein:

X is-$(CH_2)_n$-;
n is 1;
$R_1$ is selected from the group consisting of $C_{1-6}$ alkyl and substituted $C_{1-6}$ alkyl;
$R_2$ is selected from the group consisting of an amino acid, an N-substituted amino acid, -C(=O)-Y-$(CR_3R_4)_m$-$NR_5R_6$ and -C(=O)-O-$(CR_3R_4)_m$-O-C(=O)-$R_{10}$;
Y is -O- or a bond;
$R_2$' is selected from the group consisting of H, $C_1$-$C_6$ alkyl, and substituted $C_1$-$C_6$ alkyl;
each $R_3$ and $R_4$ are independently H, $C_1$-$C_6$ alkyl, substituted $C_1$-$C_6$ alkyl, aryl, substituted aryl, -$(CR_3R_4)_m$-$NR_5R_6$, or

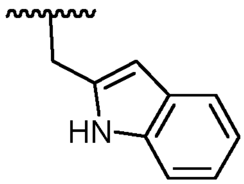

;

m is an integer selected from the group consisting of 1, 2, 3, 4, 5, 6, 7, and 8;
$R_5$ and $R_6$ are independently H or alkyl; and
Rio is selected from the group consisting of alkyl, substituted alkyl, cycloalkyl, substituted cycloalkyl, monosaccharide, acylated monosaccharide, aryl, substituted aryl, heteroaryl, and substituted heteroaryl,
for use in treating cancer, wherein the compound having formula (I) is to be administered with immune checkpoint blockade therapy, adoptive cellular therapy, or an adenosine A2aR inhibitor.

2. The compound for use according to claim 1, wherein $R_1$ is selected from the group consisting of methyl, ethyl, and isopropyl.

3. The compound for use according to claims 1 or 2, wherein:

$R_2$ is selected from the group consisting -C(=O)-Y-$(CR_3R_4)_m$-$NR_5R_6$ and -C(=O)-O-$(CR_3R_4)_m$-O-C(=O)-$R_{10}$;
Y is a bond;

m is an integer selected from the group consisting of 0, 1, 2, 3, 4, 5, 6, 7, and 8;
each $R_3$ and $R_4$ is independently H, $C_1$-$C_6$ alkyl or substituted $C_1$-$C_6$ alkyl, aryl or substituted aryl; and
$R_{10}$ is alkyl, substituted alkyl, cycloalkyl, substituted cycloalkyl, aryl, substituted aryl, heteroaryl, or substituted heteroaryl.

**4.** The compound for use according to claim 1, wherein $R_2$ is an amino acid.

**5.** The compound for use according to claim 1, wherein the compound having formula (I) is selected from the group consisting of:

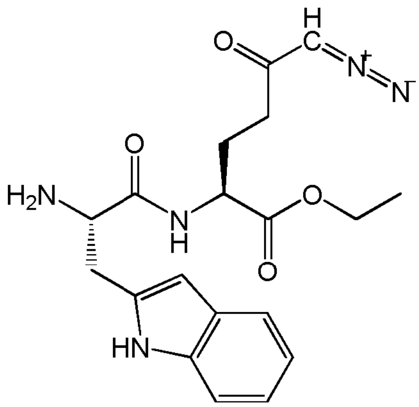
;
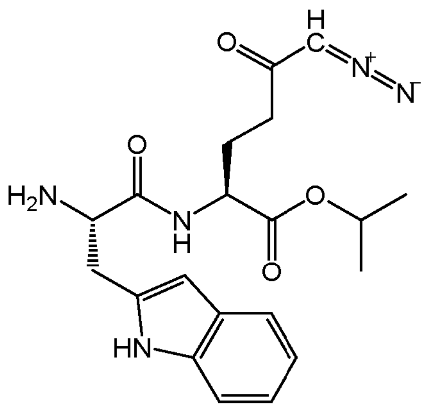
;
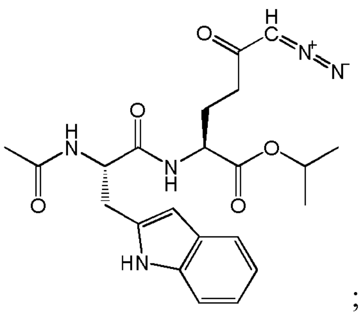
;
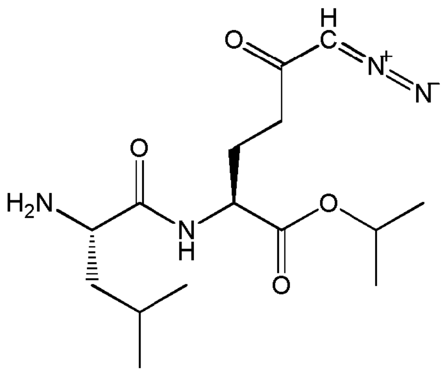
;
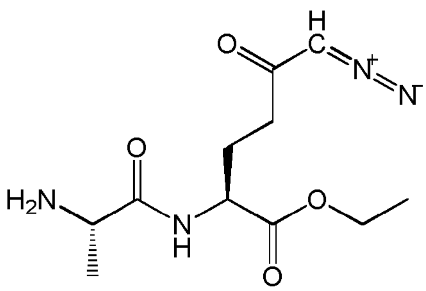
;
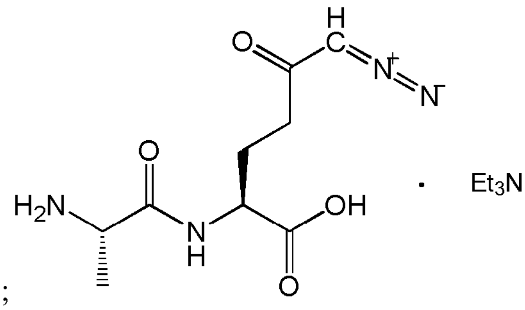
;

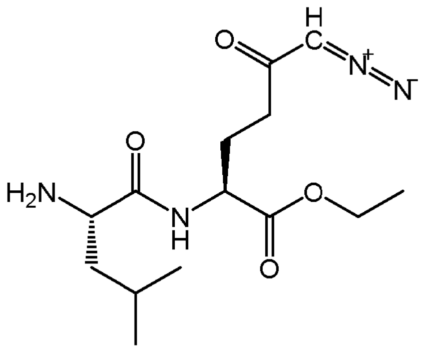
;
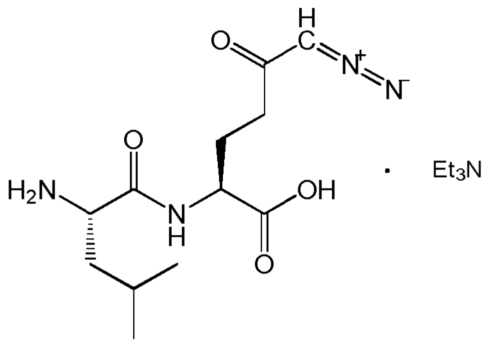
;

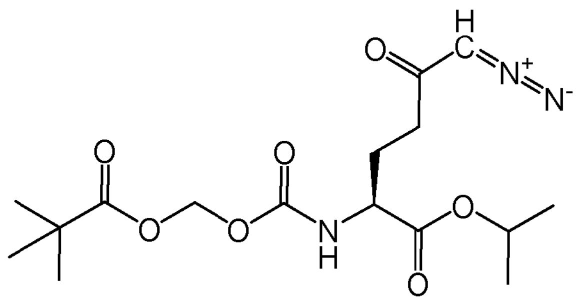
;

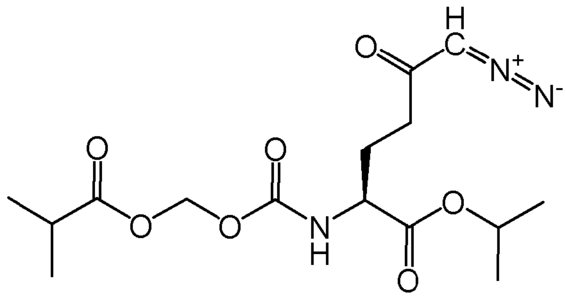
;

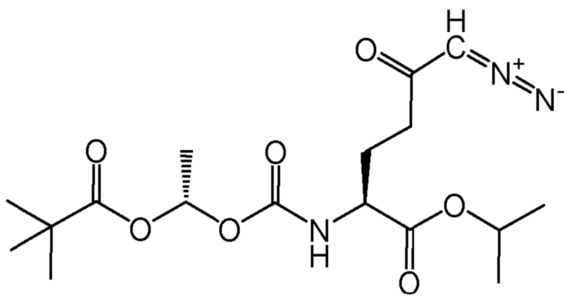
;

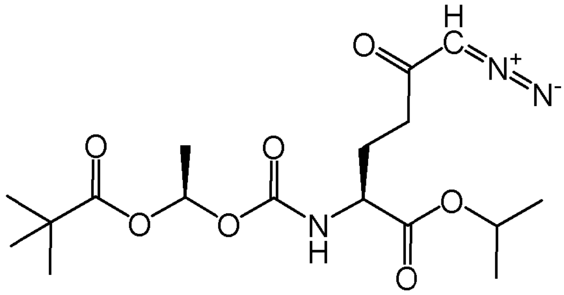
;

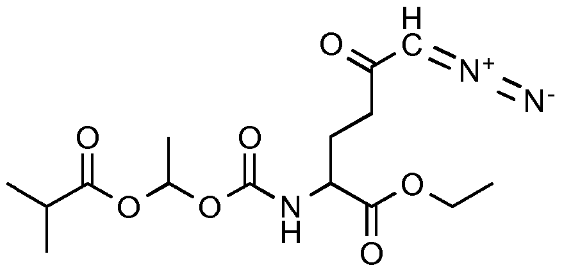

;

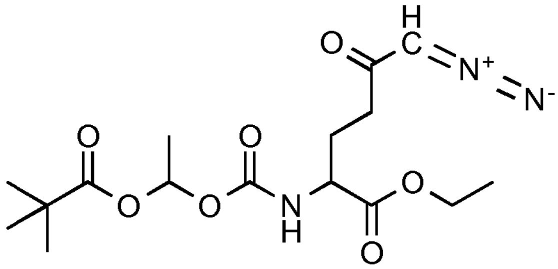

;

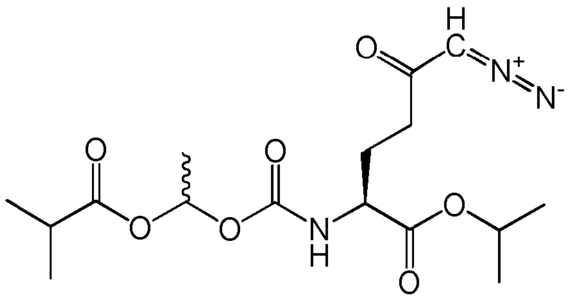

;

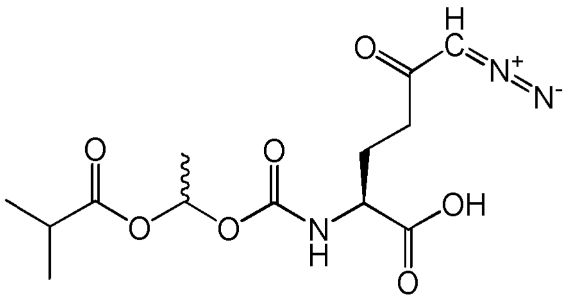

;

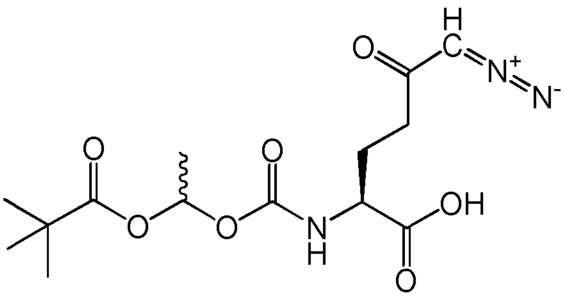

;

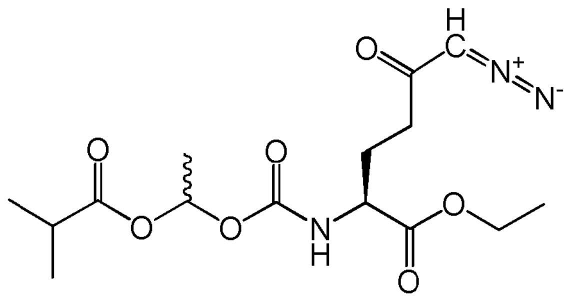

;

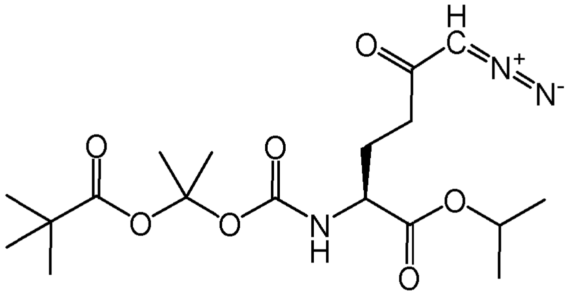

;

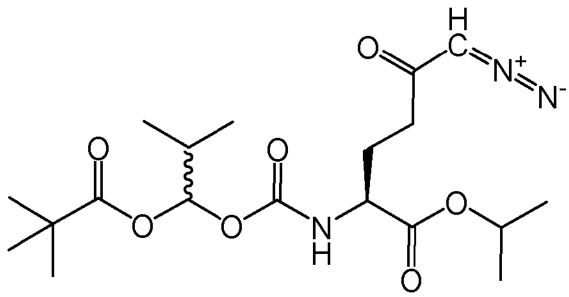

; and

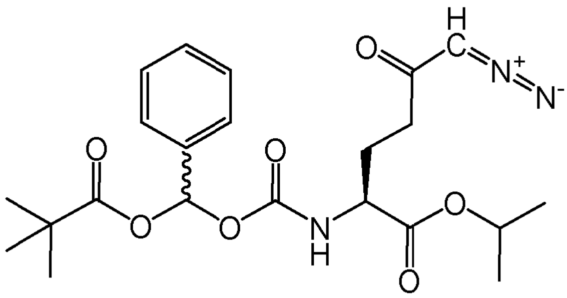

.

6. The compound for use according to any one of claims 1-5, wherein the cancer is a newly diagnosed, recurrent, and/or refractory cancer selected from the group consisting of celnasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer,

glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

**7.** The compound for use of any one of claims 1-6, wherein the compound is to be administered with checkpoint blockade therapy.

**8.** The compound for use of claim 7, wherein the checkpoint blockade therapy is selected from the group consisting of PD-1 antagonists, PD-L1 antagonists, CTLA-4 antagonists, Lag-3 antagonists, CD137 antagonists, KIR antagonists, Tim3 antagonists, Ox40 agonists, and B7-H3 antagonists.

**9.** The compound for use of claim 7, wherein the compound is to be administered with nivolumab, pembrolizumab, or pidilizumab.

**10.** The compound for use of any one of claims 1-6, wherein the compound is to be administered with an adoptive cellular therapy.

**11.** The compound for use of claim 10, wherein the compound is to be administered with T-cells, CD8+ cells, CD4+ cells, NK-cells, delta-gamma T-cells, marrow infiltrating lymphocytes (MILs), regulatory T-cells, peripheral blood mononuclear cells, or tumor infiltrating lymphocytes (TIL).

**12.** The compound for use of any one of claims 1-6, wherein the compound is to be administered with an adenosine A2aR inhibitor.

**Patentansprüche**

**1.** Verbindung, die Formel (I) aufweist:

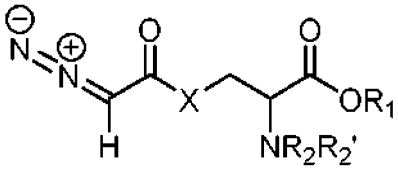

(I);

oder pharmazeutisch unbedenkliches Salz davon, wobei:

X $-(CH_2)_n-$ ist;
n 1 ist;
$R_1$ aus der Gruppe bestehend aus $C_{1-6}$-Alkyl und substituiertem $C_{1-6}$-Alkyl ausgewählt ist;
$R_2$ aus der Gruppe bestehend aus einer Aminosäure, einer N-substituierten Aminosäure, $-C(=O)-Y-(CR_3R_4)_m-NR_5R_6$ und $-C(=O)-O-(CR_3R_4)_m-O-C(=O)-R_{10}$ ausgewählt ist;
Y -O- oder eine Bindung ist;
$R_2'$ aus der Gruppe bestehend aus H, $C_1$-$C_6$-Alkyl und substituiertem $C_1$-$C_6$-Alkyl ausgewählt ist;
jedes $R_3$ und $R_4$ unabhängig H, $C_1$-$C_6$-Alkyl, substituiertes $C_1$-$C_6$-Alkyl, Aryl substituiertes Aryl, $-(CR_3R_4)_m-NR_5R_6$ ist, oder

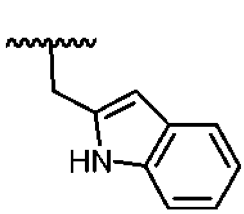

;

m eine ganze Zahl ist, die aus der Gruppe bestehend aus 1, 2, 3, 4, 5, 6, 7 und 8 ausgewählt ist;
$R_5$ und $R_6$ unabhängig H oder Alkyl sind; und
Rio aus der aus Folgendem bestehenden Gruppe ausgewählt ist: Alkyl, substituiertem Alkyl, Cycloalkyl, substituiertem Cycloalkyl, Monosaccharid, acyliertem Monosaccharid, Aryl, substituiertem Aryl, Heteroaryl und substituiertem Heteroaryl, zur Verwendung bei der Behandlung von Krebs, wobei die Verbindung, die Formel (I) aufweist, mit einer Immun-Checkpoint-Blockade-Therapie, einer adoptiven Zelltherapie oder einem Adenosin-A2aR-Inhibitor zu verabreichen ist.

**2.** Verbindung zur Verwendung nach Anspruch 1, wobei $R_1$ aus der Gruppe bestehend aus Methyl, Ethyl und Isopropyl ausgewählt ist.

**3.** Verbindung zur Verwendung nach Anspruch 1 oder 2, wobei:

$R_2$ aus der Gruppe bestehend aus $-C(=O)-Y-(CR_3R_4)_m-NR_5R_6$ und $-C(=O)-O-(CR_3R_4)_m-O-C(=O)-R_{10}$ ausgewählt ist;
Y eine Bindung ist;
m eine ganze Zahl ist, die aus der Gruppe bestehend aus 0, 1, 2, 3, 4, 5, 6, 7 und 8 ausgewählt ist;
jedes $R_3$ und R4 unabhängig H, $C_1$-$C_6$-Alkyl oder substituiertes $C_1$-$C_6$-Alkyl, Aryl oder substituiertes Aryl ist; und
$R_{10}$ Alkyl, substituiertes Alkyl, Cycloalkyl, substituiertes Cycloalkyl, Aryl, substituiertes Aryl, Heteroaryl oder substituiertes Heteroaryl ist.

**4.** Verbindung zur Verwendung nach Anspruch 1, wobei $R_2$ eine Aminosäure ist.

**5.** Verbindung zur Verwendung nach Anspruch 1, wobei die Verbindung, die Formel (I) aufweist, aus der aus Folgendem bestehenden Gruppe ausgewählt ist:

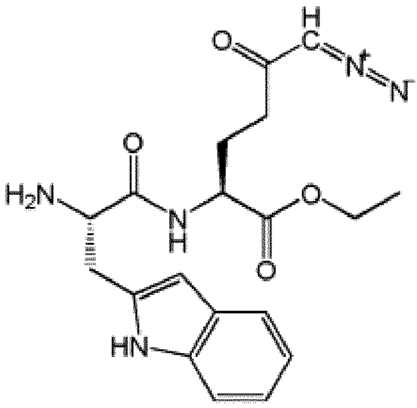
;
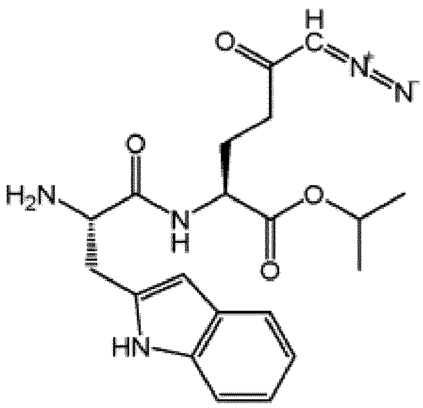
;
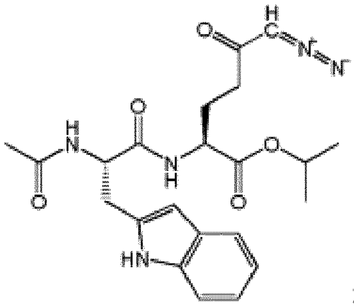
;
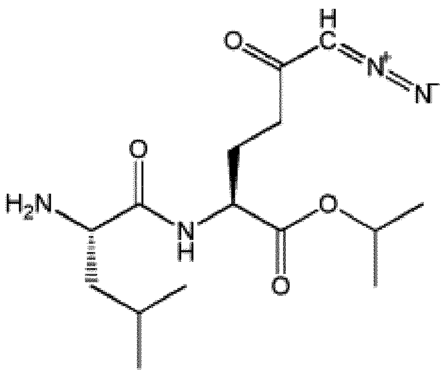
;

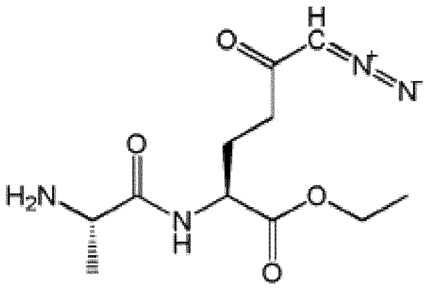;
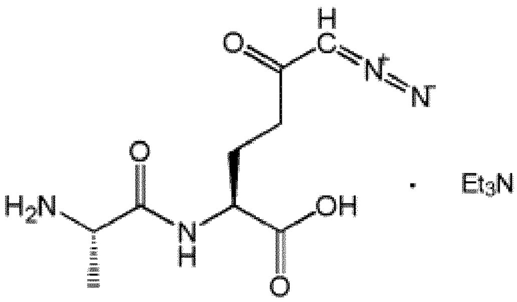;

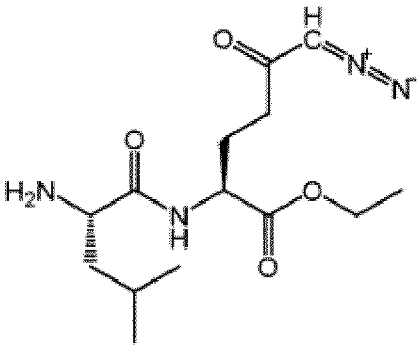;
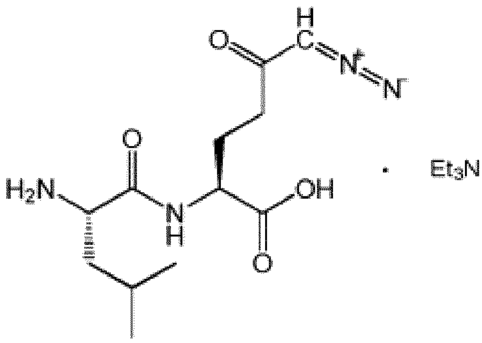;

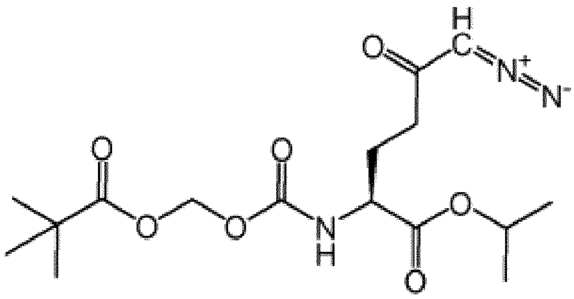;

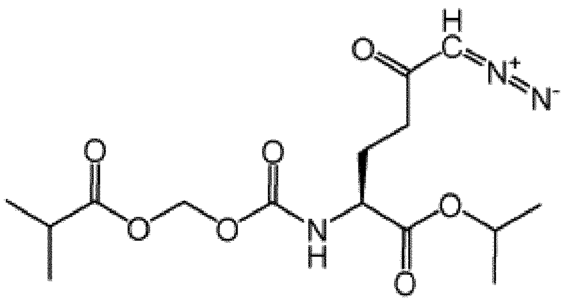;

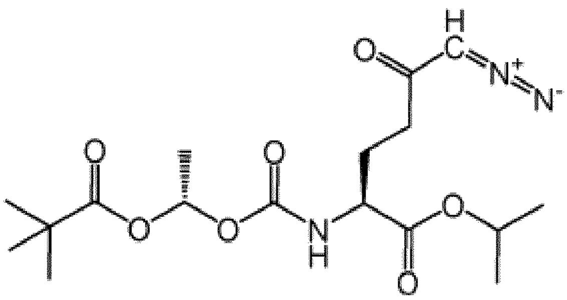;

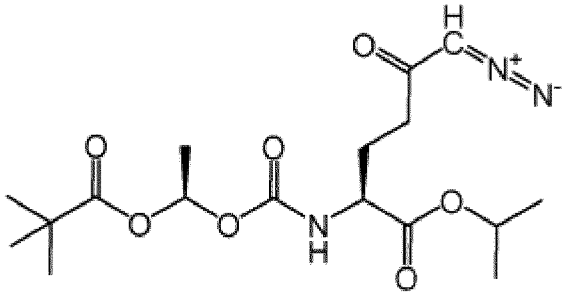

;

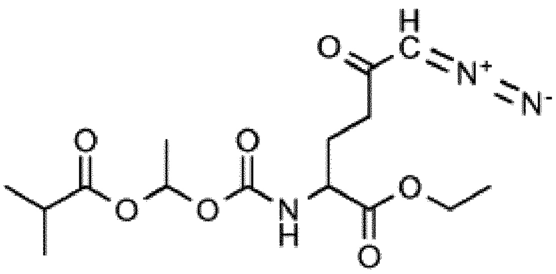

;

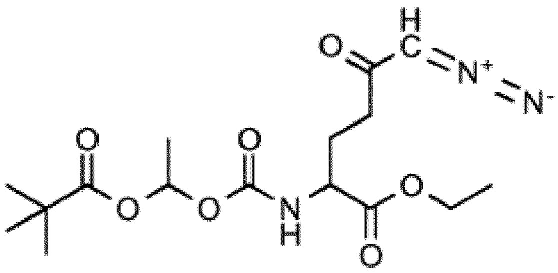

;

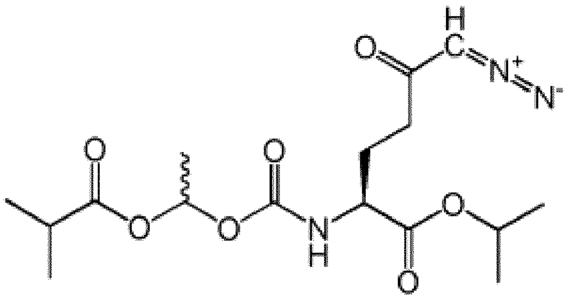

;

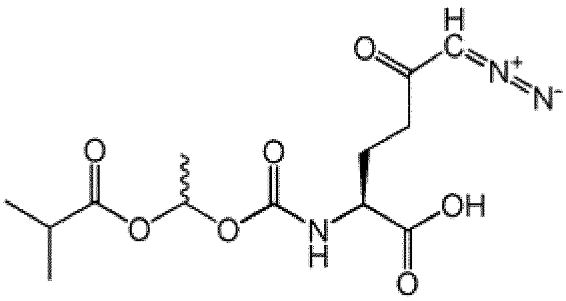

;

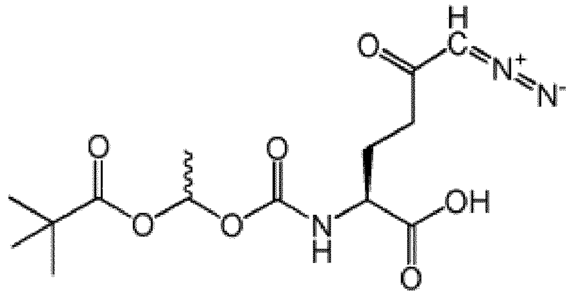

;

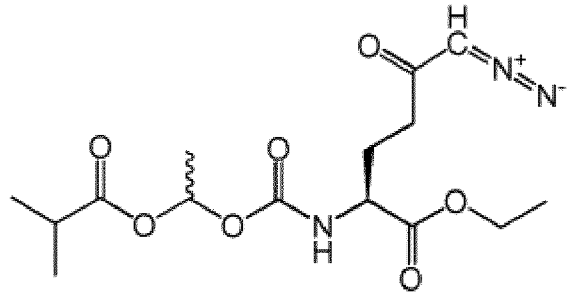

;

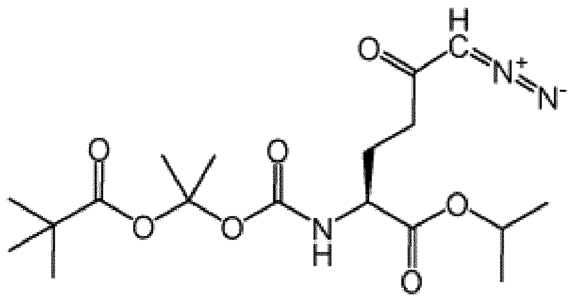

;

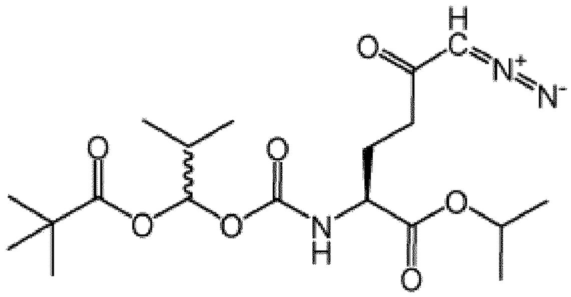

;

und

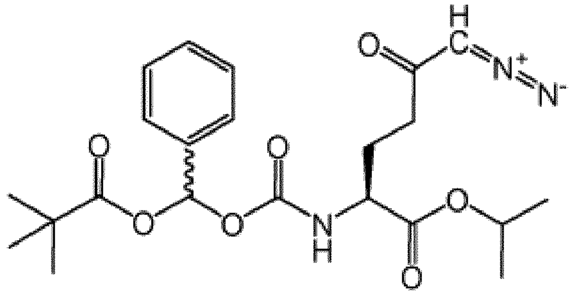

.

**6.** Verbindung zur Verwendung nach einem der Ansprüche 1-5, wobei der Krebs ein neu diagnostizierter, rezidivierender und/oder refraktärer Krebs ist, der aus der aus Folgendem bestehenden Gruppe ausgewählt ist: Celnasopharynxkrebs, Synovialkrebs, hepatozellulärem Krebs, Nierenkrebs, Bindegewebskrebs, Melanom, Lungenkrebs, Darmkrebs, Dickdarmkrebs, Mastdarmkrebs, Kolorektalkrebs, Gehirnkrebs, Kehlkopfkrebs, Mundhöhlenkrebs, Leberkrebs, Knochenkrebs, Bauchspeicheldrüsenkrebs, Chorionkarzinom, Gastrinom, Phäochromozytom, Prolaktinom, T-Zell-Leukämie/Lymphom, Neurom, von Hippel-Lindau-Krankheit, Zollinger-Ellison-Syndrom, Nebennierenkrebs, Analkrebs, Gallengangkrebs, Blasenkrebs, Harnleiterkrebs, Gehirnkrebs, Oligodendrogliom, Neuroblastom, Meningiom, Rückenmarkstumor, Knochenkrebs, Osteochondrom, Chondrosarkom, Ewing-Sarkom, Krebs unbekannter Primärlokalisation, Karzinoid, Karzinoid des Gastrointestinaltrakts, Fibrosarkom, Brustkrebs, Paget-Krankheit, Gebärmutterhalskrebs, Kolorektalkrebs, Mastdarmkrebs, Speiseröhrenkrebs, Gallenblasenkrebs, Kopfkrebs, Augenkrebs, Halskrebs, Nierenkrebs, Wilms-Tumor, Leberkrebs, Kaposi-Sarkom, Prostatakrebs, Lungenkrebs, Hodenkrebs, Morbus Hodgkin, Non-Hodgkin-Lymphom, Mundhöhlenkrebs, Hautkrebs, Mesotheliom, multiplem Myelom, Eierstockkrebs, endokrinem Bauchspeicheldrüsenkrebs, Glukagonom, Bauchspeicheldrüsenkrebs, Nebenschilddrüsenkrebs, Peniskrebs, Hypophysenkrebs, Weichteilsarkom, Retinoblastom, Dünndarmkrebs, Magenkrebs, Thymuskrebs, Schilddrüsenkrebs, Trophoblastkrebs, Blasenmole, Gebärmutterkrebs, Endometriumkrebs, Vaginakrebs, Vulvakrebs, Akustikusneurinom, Mycosis fungoides, Insulinom, Karzinoid-Syndrom, Somatostatinom, Zahnfleischkrebs, Herzkrebs, Lippenkrebs, Hirnhautkrebs, Mundkrebs, Nervenkrebs, Gaumenkrebs, Ohrspeicheldrüsenkrebs, Bauchfellkrebs, Rachenkrebs, Brustfellkrebs, Speicheldrüsenkrebs, Zungenkrebs und Tonsillenkrebs.

7. Verbindung zur Verwendung nach einem der Ansprüche 1-6, wobei die Verbindung mit einer Checkpoint-Blockade-Therapie zu verabreichen ist.

8. Verbindung zur Verwendung nach Anspruch 7, wobei die Checkpoint-Blockade-Therapie aus der Gruppe bestehend aus PD-1-Antagonisten, PD-L1-Antagonisten, CTLA-4-Antagonisten, Lag-3-Antagonisten, CD137-Antagonisten, KIR-Antagonisten, Tim3-Antagonisten, Ox40-Agonisten und B7-H3-Antagonisten ausgewählt ist.

9. Verbindung zur Verwendung nach Anspruch 7, wobei die Verbindung mit Nivolumab, Pembrolizumab oder Pidilizumab zu verabreichen ist.

10. Verbindung zur Verwendung nach einem der Ansprüche 1-6, wobei die Verbindung mit einer adoptiven Zelltherapie zu verabreichen ist.

11. Verbindung zur Verwendung nach Anspruch 10, wobei die Verbindung mit T-Zellen, CD8+-Zellen, CD4+-Zellen, NK-Zellen, Delta-Gamma-T-Zellen, markinfiltrierenden Lymphozyten (MILs), regulatorischen T-Zellen, peripheren mononukleären Blutzellen oder tumorinfiltrierenden Lymphozyten (TIL) zu verabreichen ist.

12. Verbindung zur Verwendung nach einem der Ansprüche 1-6, wobei die Verbindung mit einem Adenosin-A2aR-Inhibitor zu verabreichen ist.

## Revendications

1. Composé présentant la formule (I):

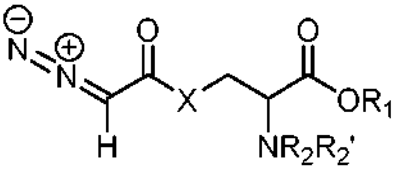

(I);

ou sel pharmaceutiquement acceptable de celui-ci, dans laquelle :

X représente un groupe $-(CH_2)_n-$;
n vaut 1 ;
$R_1$ est choisi dans le groupe constitué par un groupe $C_{1-6}$ alkyle et un groupe $C_{1-6}$ alkyle substitué ;
$R_2$ est choisi dans le groupe constitué par un groupe acide aminé, acide aminé N-substitué, $-C(=O)-Y-(CR_3R_4)_m-NR_5R_6$ et $-C(=O)-O-(CR_3R_4)_m-O-C(=O)-R_{10}$;
Y représente un groupe -O- ou une liaison ;

$R_2$' est choisi dans le groupe constitué par un atome H, un groupe $C_1$-$C_6$ alkyle et $C_1$-$C_6$ alkyle substitué ; chaque $R_3$ et $R_4$ représentent indépendamment un atome H, un groupe $C_1$-$C_6$ alkyle, $C_1$-$C_6$ alkyle substitué, aryle, aryle substitué, $-(CR_3R_4)_m-NR_5R_6$, ou

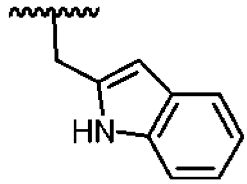
;

m est un entier choisi dans le groupe constitué par 1, 2, 3, 4, 5, 6, 7 et 8 ;
$R_5$ et $R_6$ représentent indépendamment un atome H ou un groupe alkyle ; et
$R_{10}$ est choisi dans le groupe constitué par un groupe alkyle, alkyle substitué, cycloalkyle, cycloalkyle substitué, monosaccharide, monosaccharide acylé, aryle, aryle substitué, hétéroaryle, et hétéroaryle substitué,
pour une utilisation dans le traitement d'un cancer, ledit composé présentant la formule (I) étant destiné à être administré avec une thérapie de blocage de points de contrôle immunitaire, une thérapie cellulaire adoptive, ou un inhibiteur du A2aR de l'adénosine.

**2.** Composé pour une utilisation selon la revendication 1, $R_1$ étant choisi dans le groupe constitué par les groupes méthyle, éthyle et isopropyle.

**3.** Composé pour une utilisation selon la revendication 1 ou 2 :

$R_2$ étant choisi dans le groupe constitué par les groupes $-C(=O)-Y-(CR_3R_4)_m-NR_5R_6$ et $-C(=O)-O-(CR_3R_4)_m-O-C(=O)-R_{10}$;
Y représentant une liaison ;
m étant un entier choisi dans le groupe constitué par 0, 1, 2, 3, 4, 5, 6, 7 et 8 ;
chaque $R_3$ et $R_4$ représentant indépendamment un atome H, un groupe $C_1$-$C_6$ alkyle ou Ci-$C_6$ alkyle substitué, aryle ou aryle substitué ; et
$R_{10}$ représentant un groupe alkyle, alkyle substitué, cycloalkyle, cycloalkyle substitué, aryle, aryle substitué, hétéroaryle ou hétéroaryle substitué.

**4.** Composé pour une utilisation selon la revendication 1, $R_2$ représentant un groupe acide aminé.

**5.** Composé pour une utilisation selon la revendication 1, ledit composé présentant la formule (I) étant choisi dans le groupe constitué par :

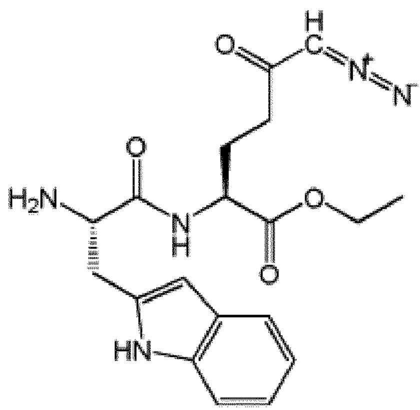
;
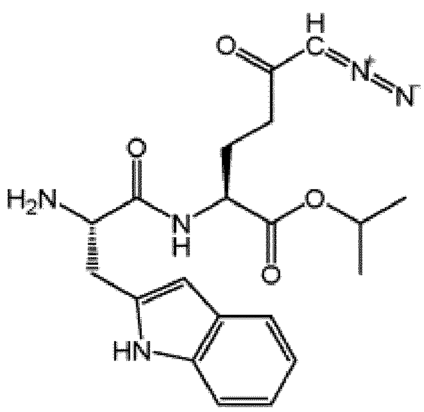
;

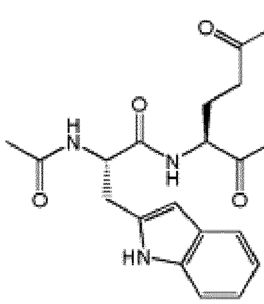 ; 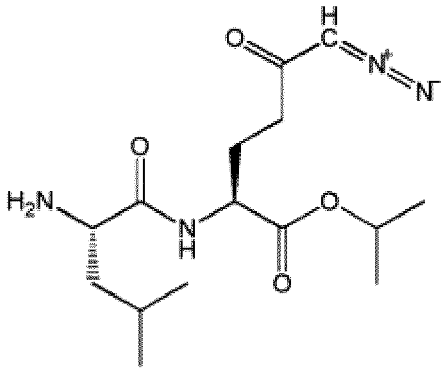 ;

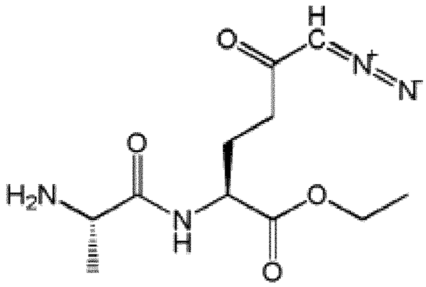 ; 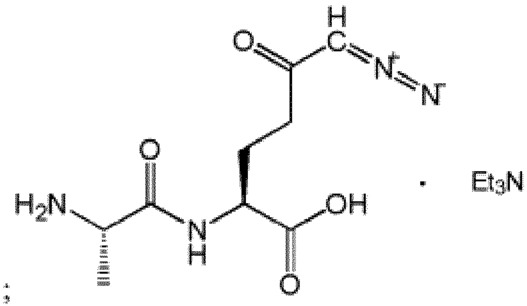 ;

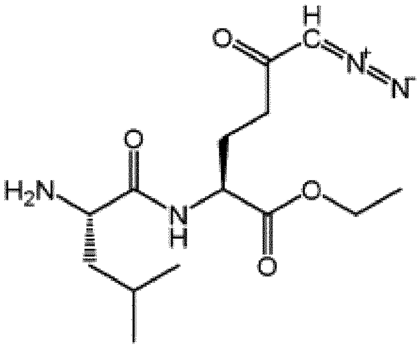 ; 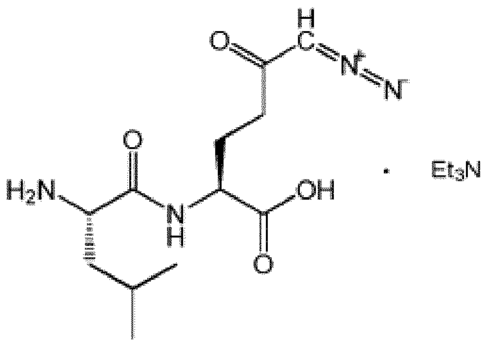 ;

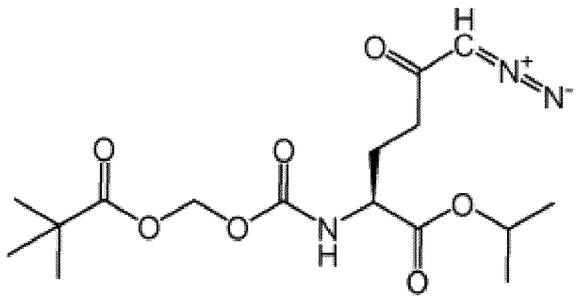 ;

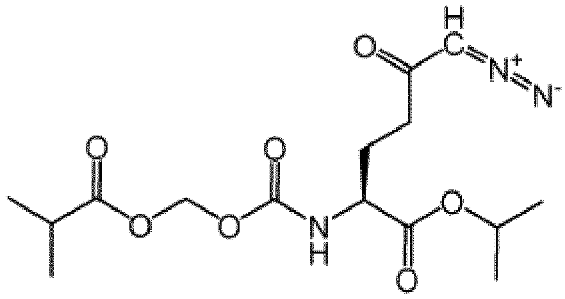
;

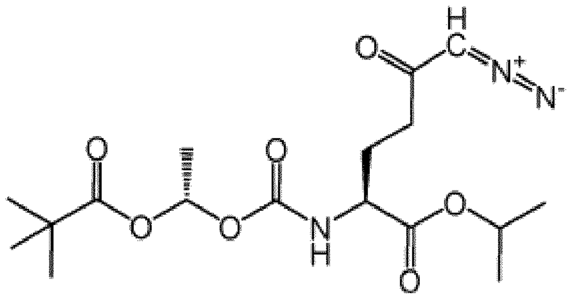
;

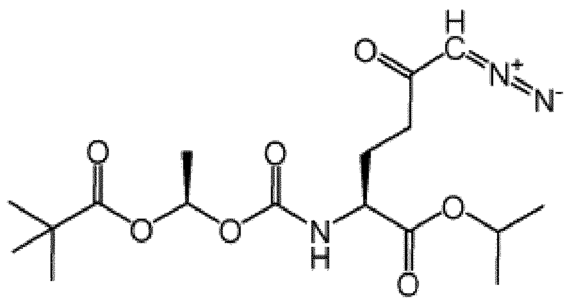
;

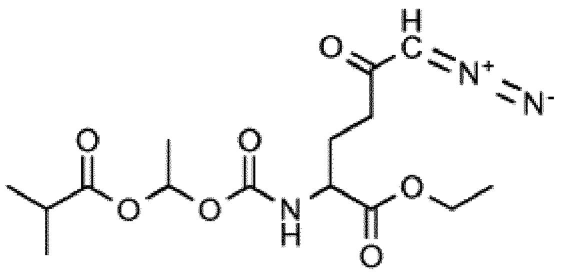
;

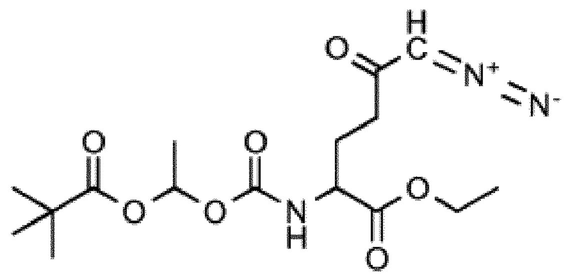
;

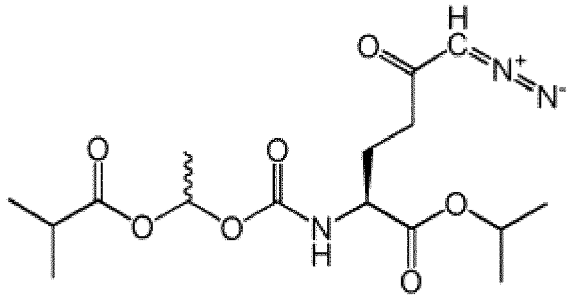

;

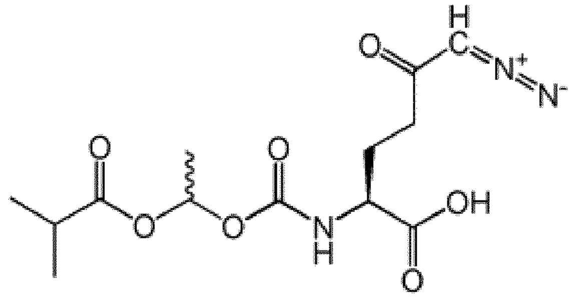

;

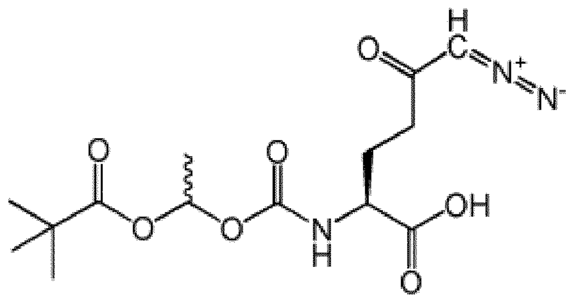

;

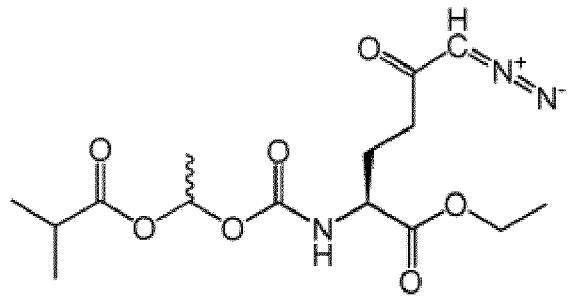

;

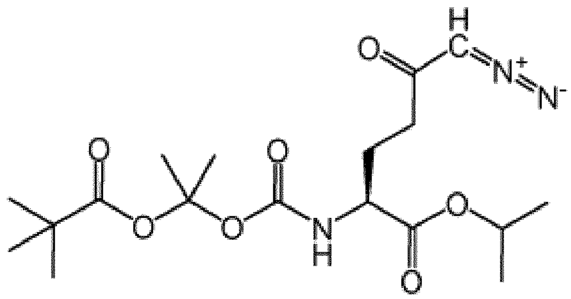

;

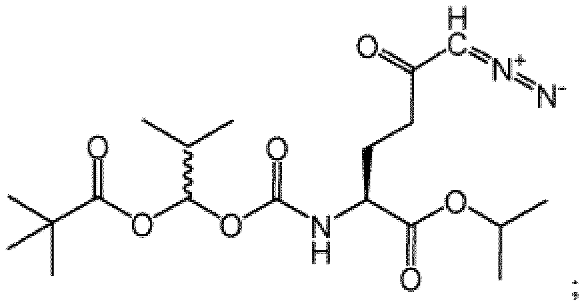

et

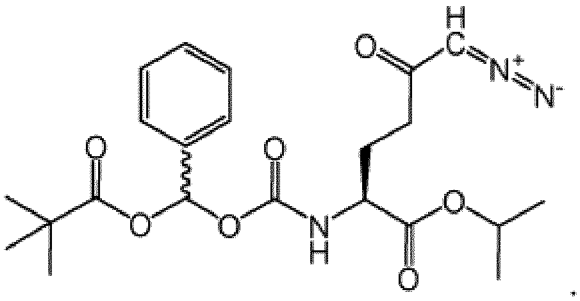

6. Composé pour une utilisation selon l'une quelconque des revendications 1 à 5, ledit cancer étant un cancer nouvellement diagnostiqué, récurrent et/ou réfractaire, choisi dans le groupe constitué par le cancer celnasopharyngé, le cancer synovial, le cancer hépatocellulaire, le cancer du rein, le cancer des tissus conjonctifs, un mélanome, le cancer du poumon, le cancer de l'intestin, le cancer du côlon, le cancer du rectum, le cancer colorectal, le cancer du cerveau, le cancer de la gorge, le cancer de la bouche, le cancer du foie, le cancer des os, le cancer du pancréas, un choriocarcinome, un gastrinome, un phéochromocytome, un prolactinome, une leucémie des lymphocytes T/un lymphome, un névrome, la maladie de von Hippel-Lindau, le syndrome de Zollinger-Ellison, le cancer des glandes surrénales, le cancer de l'anus, le cancer des voies biliaires, le cancer de la vessie, le cancer de l'uretère, le cancer du cerveau, un oligodendrogliome, un neuroblastome, un méningiome, une tumeur de la moelle épinière, le cancer des os, un ostéochondrome, un chondrosarcome, un sarcome d'Ewing, un cancer de site primaire inconnu, un carcinoïde, un carcinoïde du tractus gastro-intestinal, un fibrosarcome, le cancer du sein, la maladie de Paget, le cancer du col de l'utérus, le cancer colorectal, le cancer du rectum, le cancer de l'œsophage, le cancer de la vésicule biliaire, le cancer de la tête, le cancer de l'œil, le cancer du cou, le cancer du rein, une tumeur de Wilms, le cancer du foie, un sarcome de Kaposi, le cancer de la prostate, le cancer du poumon, le cancer du testicule, la maladie de Hodgkin, un lymphome non hodgkinien, le cancer de la bouche, le cancer de la peau, un mésothéliome, un myélome multiple, le cancer de l'ovaire, le cancer endocrinien du pancréas, un glucagonome, le cancer du pancréas, le cancer parathyroïdien, le cancer du pénis, le cancer de l'hypophyse, un sarcome des tissus mous, un rétinoblastome, le cancer de l'intestin grêle, le cancer de l'estomac, le cancer du thymus, le cancer de la thyroïde, le cancer trophoblastique, une môle hydatiforme, le cancer de l'utérus, le cancer de l'endomètre, le cancer du vagin, le cancer de la vulve, un neurinome acoustique, une mycose fongoïde, un insulinome, le syndrome carcinoïde, un somatostatinome, le cancer de la gencive, le cancer du cœur, le cancer des lèvres, le cancer des méninges, le cancer de la bouche, le cancer des nerfs, le cancer du palais, le cancer de la glande parotide, le cancer du péritoine, le cancer du pharynx, le cancer de la plèvre, le cancer des glandes salivaires, le cancer de la langue et le cancer des amygdales.

7. Composé pour une utilisation selon l'une quelconque des revendications 1 à 6, ledit composé étant destiné à être administré avec une thérapie de blocage de points de contrôle.

8. Composé pour une utilisation selon la revendication 7, ladite thérapie de blocage de points de contrôle étant choisie dans le groupe constitué par les antagonistes de PD-1, les antagonistes de PD-L1, les antagonistes de CTLA-4, les antagonistes de Lag-3, les antagonistes de CD137, les antagonistes de KIR, les antagonistes de Tim3, les agonistes de Ox40 et les antagonistes de B7-H3.

9. Composé pour une utilisation selon la revendication 7, ledit composé étant destiné à être administré avec du nivolumab, du pembrolizumab ou du pidilizumab.

**10.** Composé pour une utilisation selon l'une quelconque des revendications 1 à 6, ledit composé étant destiné à être administré avec une thérapie cellulaire adoptive.

**11.** Composé pour une utilisation selon la revendication 10, ledit composé étant destiné à être administré avec des lymphocytes T, des lymphocytes CD8+, des lymphocytes CD4+, des lymphocytes NK, des lymphocytes T delta-gamma, des lymphocytes infiltrant la moelle (MIL), des lymphocytes T régulateurs, des cellules mononucléaires du sang périphérique ou des lymphocytes infiltrant les tumeurs (TIL).

**12.** Composé pour une utilisation selon l'une quelconque des revendications 1 à 6, ledit composé étant destiné à être administré avec un inhibiteur du A2aR de l'adénosine.

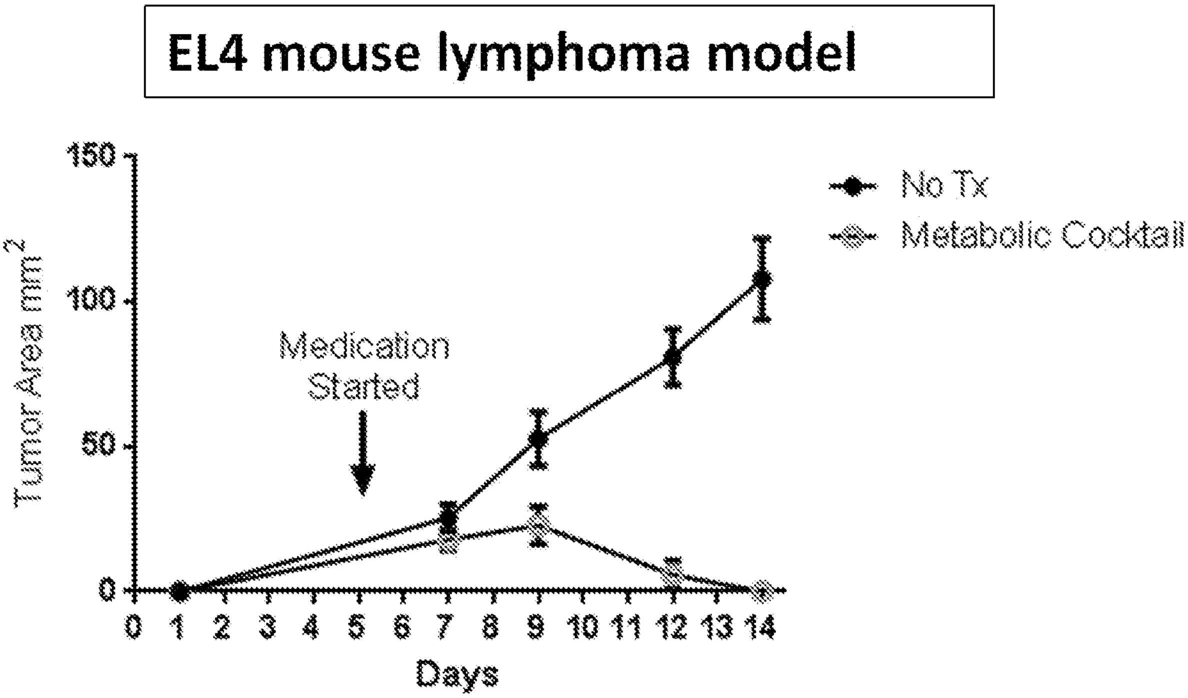
EL4 mouse lymphoma model
No Tx
Metabolic Cocktail
Tumor Area mm2
150
100
50
0
Medication
Started
0 1 2 3 4 5 6 7 8 9 10 11 12 13 14
Days

*FIG. 1*

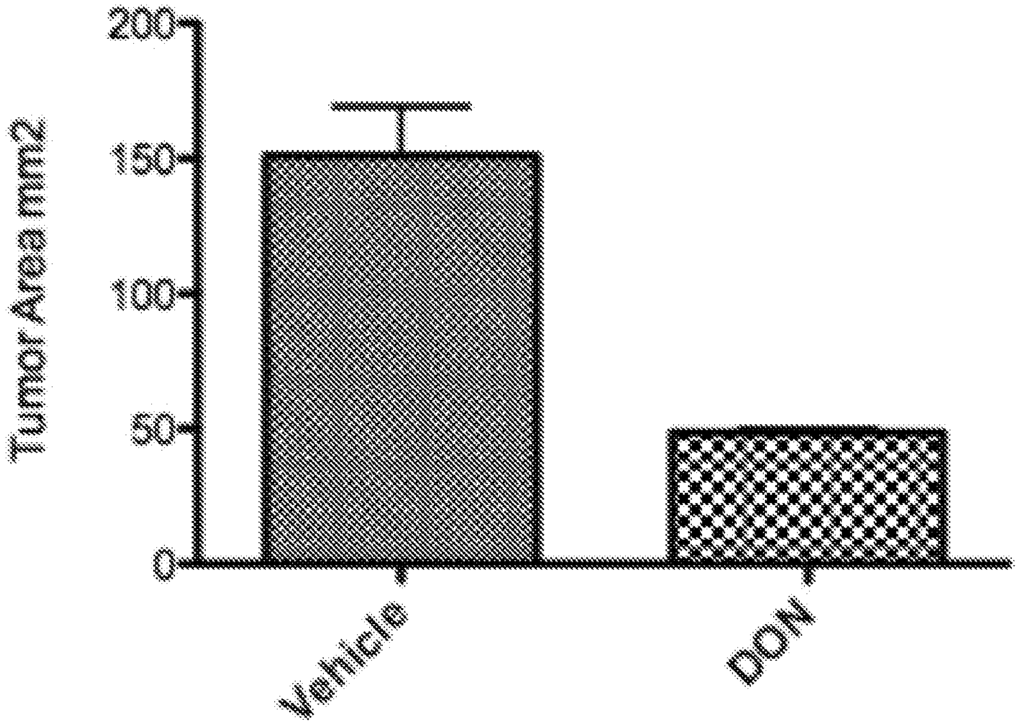


*FIG. 2*

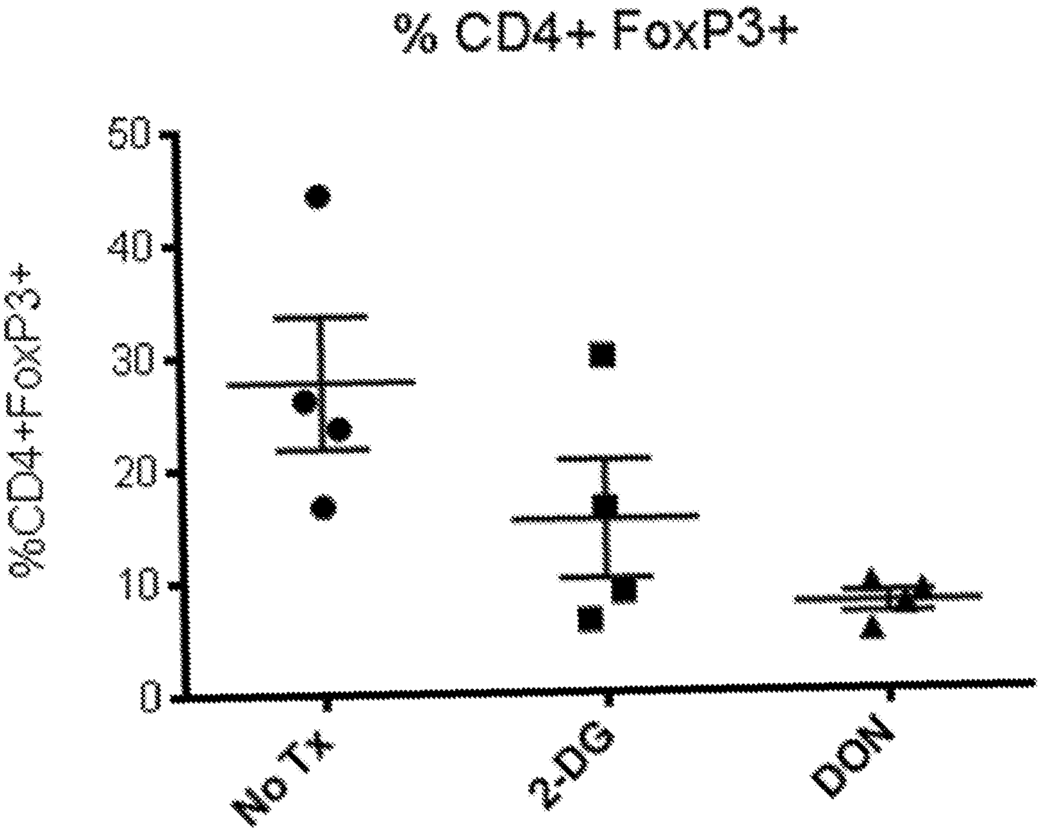


*FIG. 3*

# *FIG. 4*

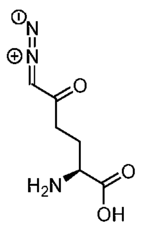

1, 6-Diazo-5-oxo-L-norleucine (DON)

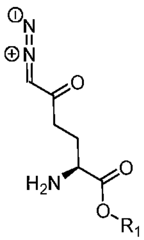

**20**($R_1$ = Et)
**3** ($R_1$ = iPr)

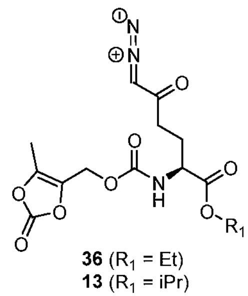

**36** ($R_1$ = Et)
**13** ($R_1$ = iPr)

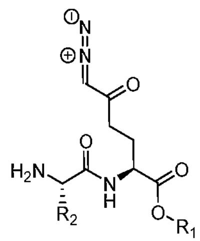

**25** ($R_1$ = Et, $R_2$ = iBu)
**9** ($R_1$ = iPr, $R_2$ = iBu)
**34** ($R_1$ = Et, $R_2$ = $-CH_2$-3-indolyl)
**38** ($R_1$ = iPr, $R_2$ = $-CH_2$-3-indolyl)

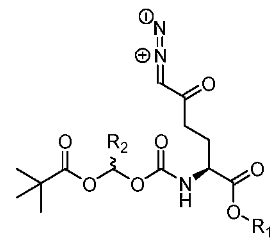

**42** ($R_1$ = iPr, $R_2$ = H)
**32** ($R_1$ = Et, $R_2$ = $CH_3$)
**14** ($R_1$ = iPr, $R_2$ = $CH_3$)

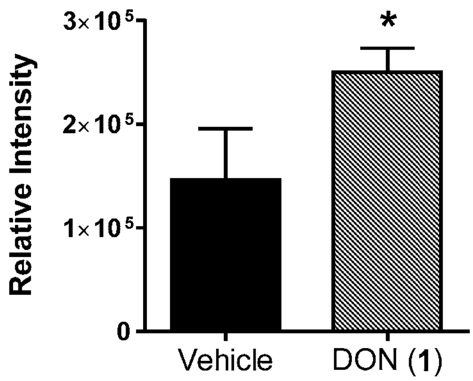
3×10⁵
2×10⁵
1×10⁵
0
Relative Intensity
*
Vehicle
DON (1)

*FIG. 5A*

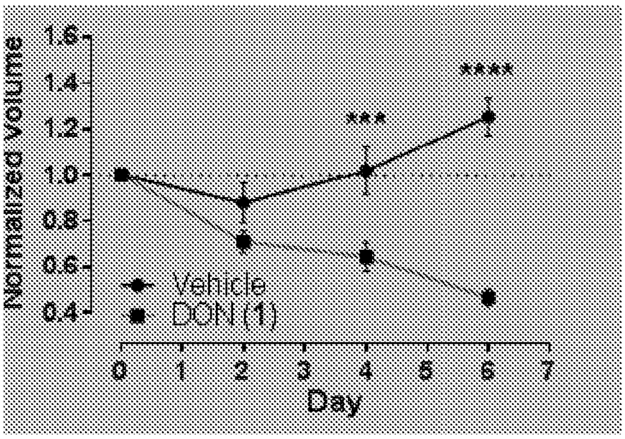
1.6
1.4
1.2
1.0
0.8
0.6
0.4
Normalized Volume
***
****
Vehicle
DON (1)
0 1 2 3 4 5 6 7
Day

*FIG. 5B*

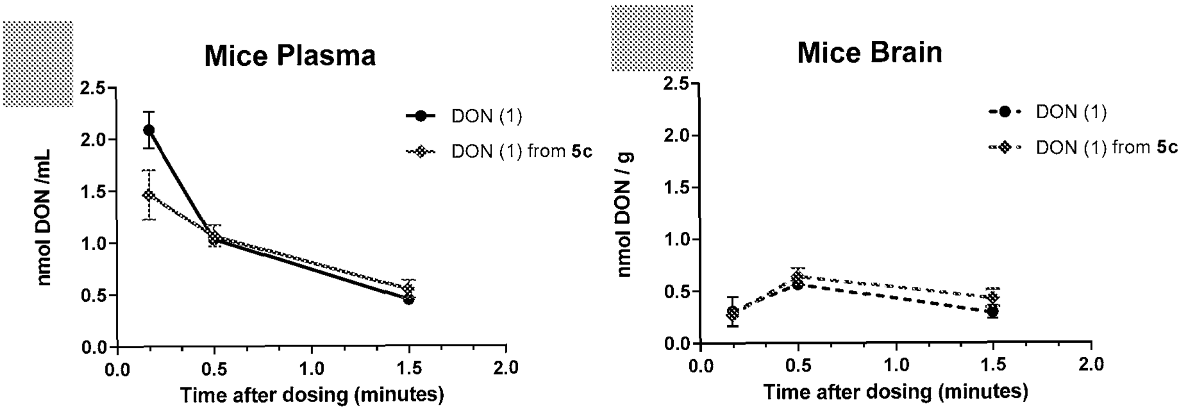


| **Compound Dosed** | **Dose** *(mg/kg equiv)* | **Tissue** | **DON $C_{max}$** *(nmol/mL or nmol/g )* | **DON $T_{max}$** *(hr)* | **DON $AUC_{0-t}$** *(hr*nmol/mL or hr*nmol/g)* | **Brain to plasma ratio** |
|---|---|---|---|---|---|---|
| DON (**1**) | 0.8 | Plasma | 2.2 | 0.17 | 1.25 | 0.46 |
| | | Brain | 0.56 | 0.50 | 0.57 | |
| **5c** | 0.8 | Plasma | 1.5 | 1.50 | 1.22 | 0.57 |
| | | Brain | 0.64 | 30 | 0.69 | |

***FIG. 6***

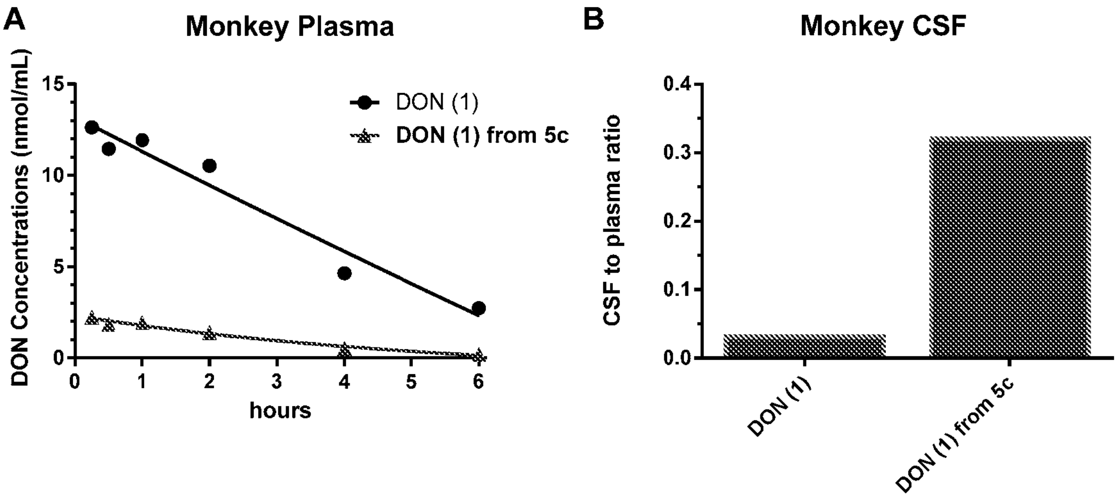


| Compound Dosed | Dose (*mg/kg equiv*) | DON $C_{max}$ (*nmol/mL*) | DON $T_{max}$ (*hr*) | DON $AUC_{0-t}$ (*hr*nmol/mL*) |
|---|---|---|---|---|
| 1 | 1.6 | 12.6 | 0.25 | 42.7 |
| 5c | 1.6 | 2.23 | 0.25 | 5.71 |

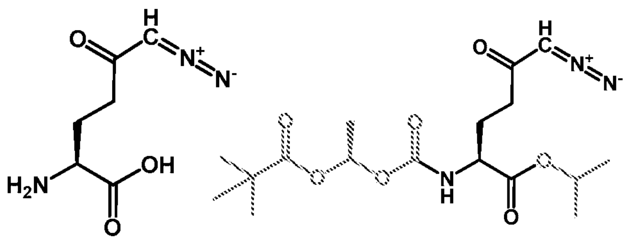

DON (1) Prodrug 5c

*FIG. 7*

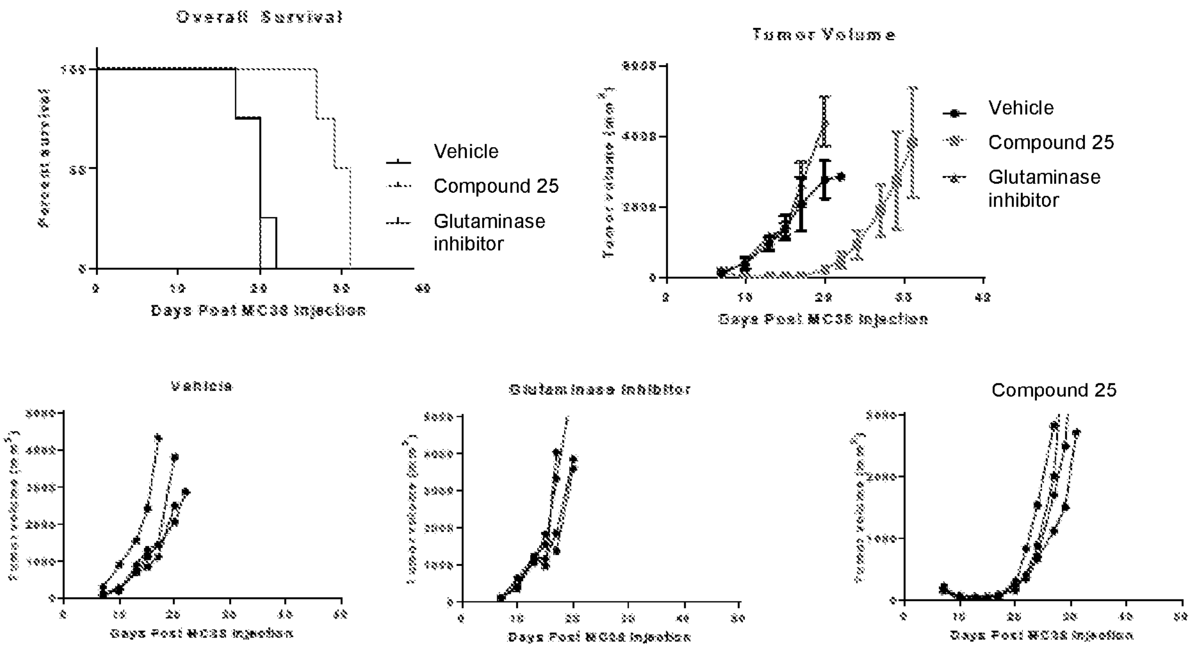

Overall Survival
Percent survival
Days Post MC38 Injection
Vehicle
Compound 25
Glutaminase inhibitor
Tumor Volume
Days Post MC38 Injection
Vehicle
Compound 25
Glutaminase inhibitor
Vehicle
Days Post MC38 Injection
Glutaminase inhibitor
Days Post MC38 Injection
Compound 25
Days Post MC38 Injection


*FIG. 8*

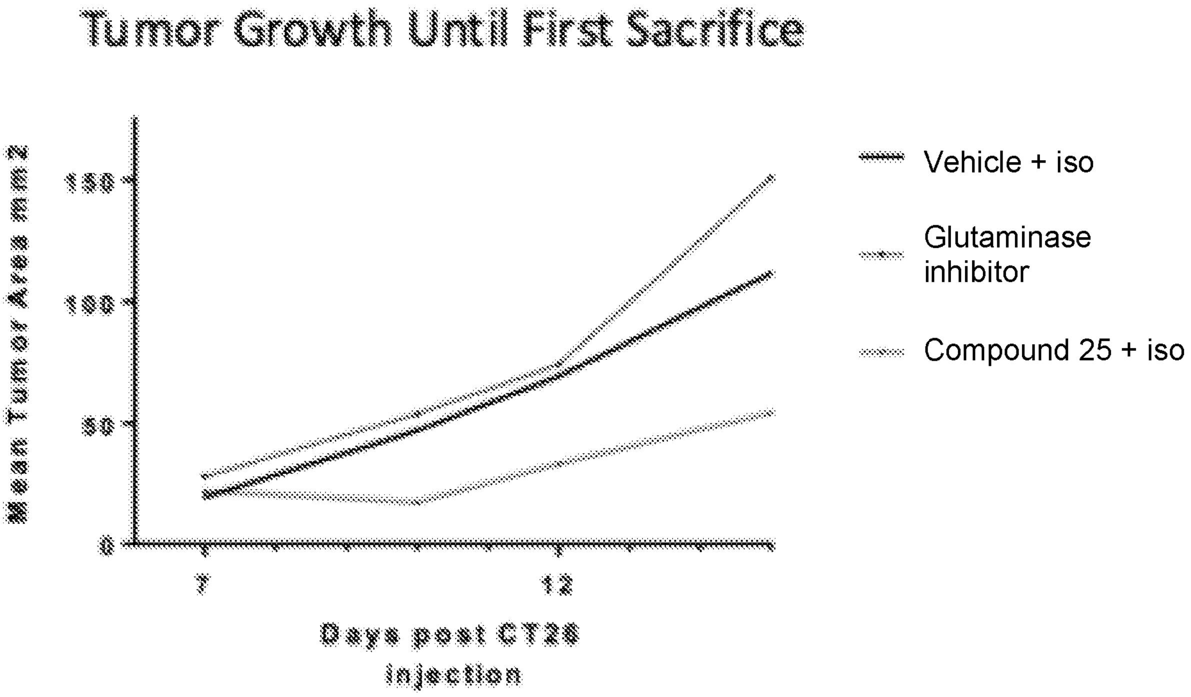
Tumor Growth Until First Sacrifice
Mean Tumor Area mm2
150
100
50
0
7
12
Days post CT26 injection
Vehicle + iso
Glutaminase inhibitor
Compound 25 + iso

*FIG. 9*

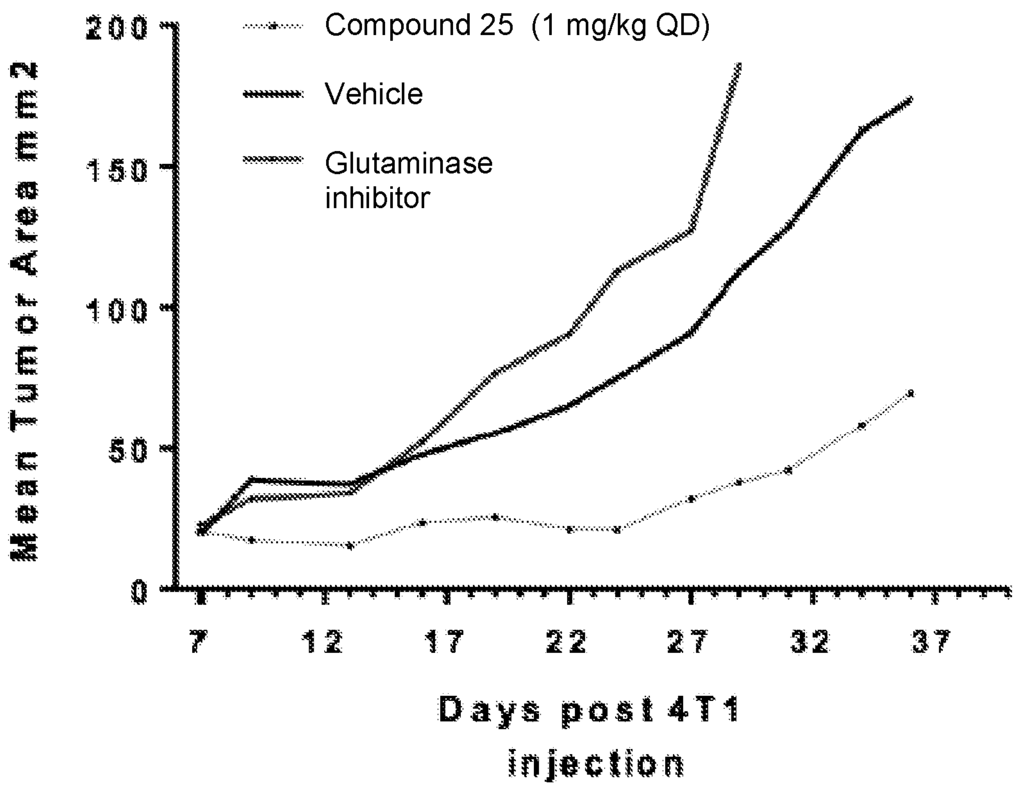
Compound 25 (1 mg/kg QD)
Vehicle
Glutaminase inhibitor
Mean Tumor Area mm2
200
150
100
50
0
7
12
17
22
27
32
37
Days post 4T1 injection

*FIG. 10*

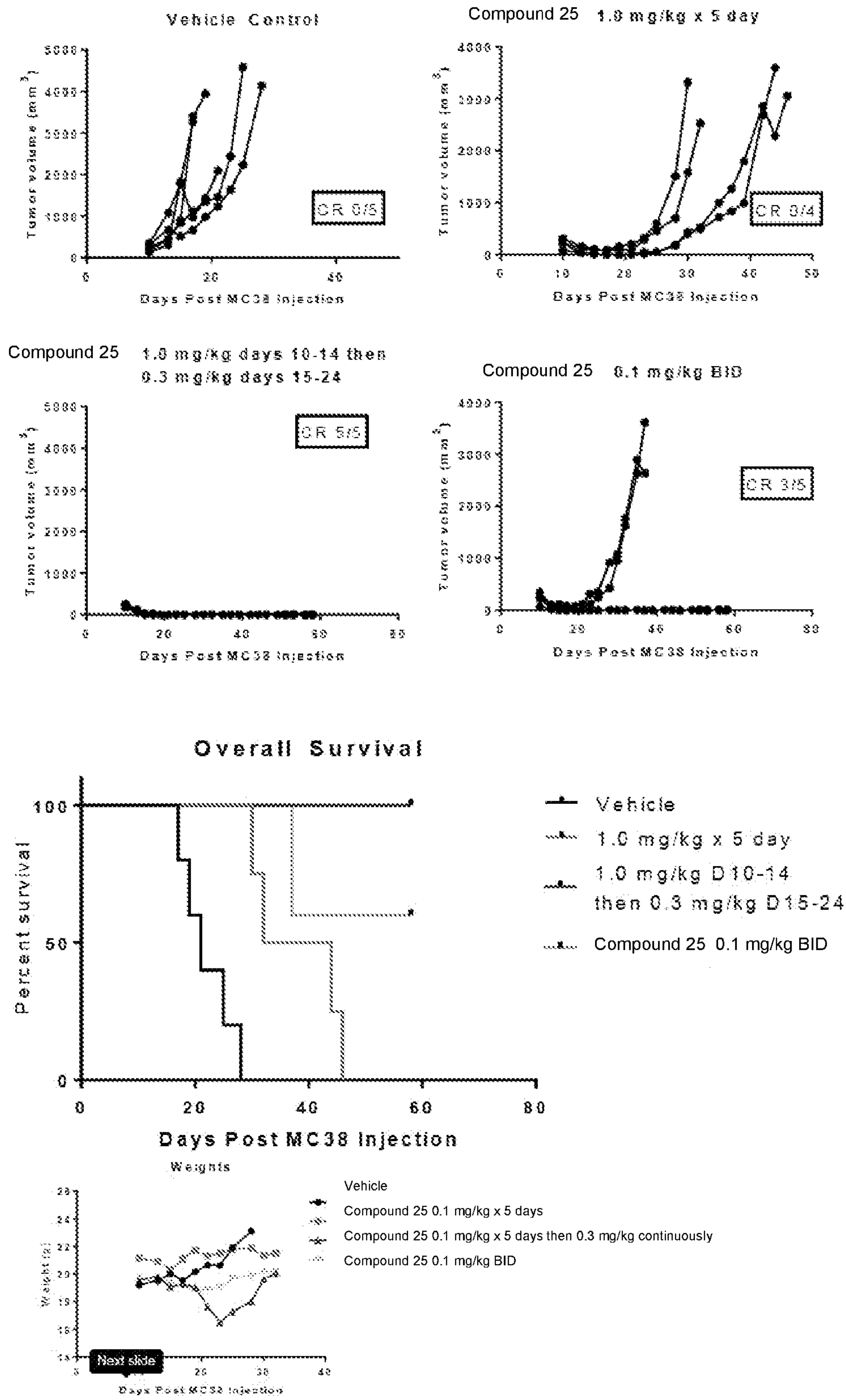
Vehicle Control
CR 0/5
Compound 25 1.0 mg/kg x 5 day
CR 3/4
Compound 25 1.0 mg/kg days 10-14 then 0.3 mg/kg days 15-24
CR 5/5
Compound 25 0.1 mg/kg BID
CR 3/5
Tumor volume (mm3)
Days Post MC38 Injection
Overall Survival
Percent survival
Vehicle
1.0 mg/kg x 5 day
1.0 mg/kg D10-14 then 0.3 mg/kg D15-24
Compound 25 0.1 mg/kg BID
Weights
Vehicle
Compound 25 0.1 mg/kg x 5 days
Compound 25 0.1 mg/kg x 5 days then 0.3 mg/kg continuously
Compound 25 0.1 mg/kg BID
Next slide

*FIG. 11*

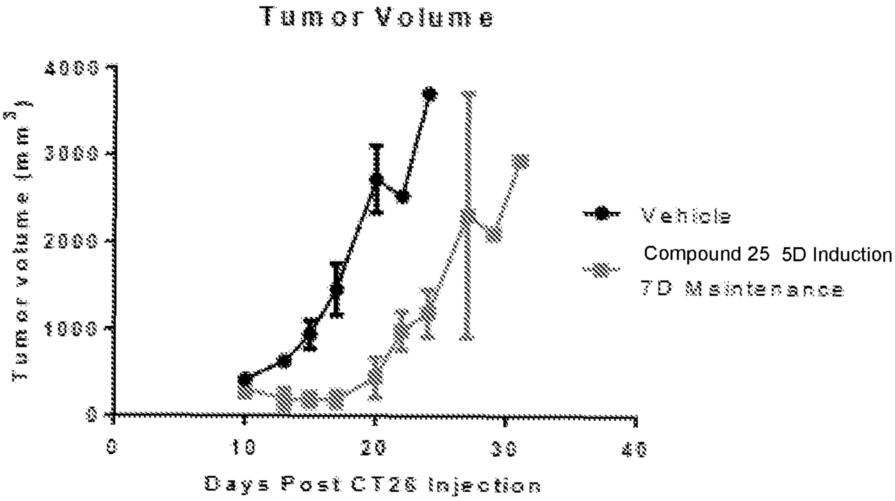
Tumor Volume
Tumor volume (mm³)
0
1000
2000
3000
4000
0
10
20
30
40
Days Post CT26 Injection
Vehicle
Compound 25 5D Induction
7D Maintenance

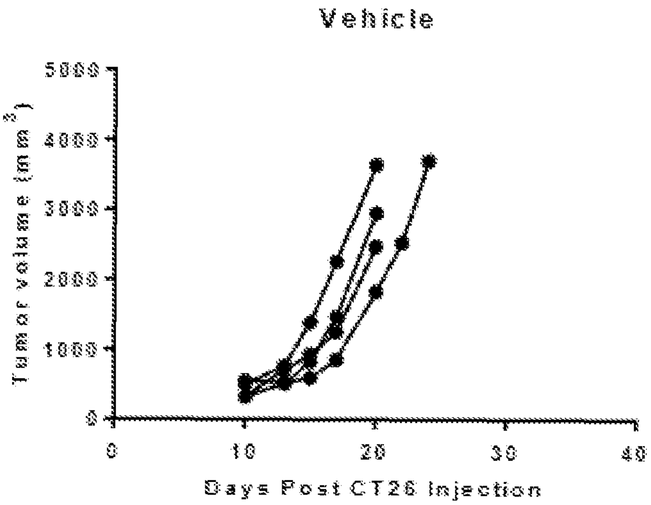
Vehicle
Tumor volume (mm³)
0
1000
2000
3000
4000
5000
0
10
20
30
40
Days Post CT26 Injection

*FIG. 12*

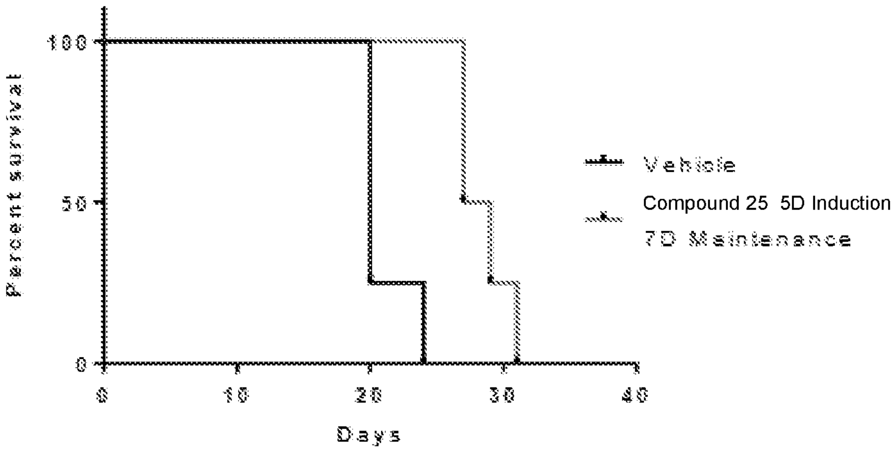
100
50
0
Percent survival
0
10
20
30
40
Days
Vehicle
Compound 25 5D Induction
7D Maintenance

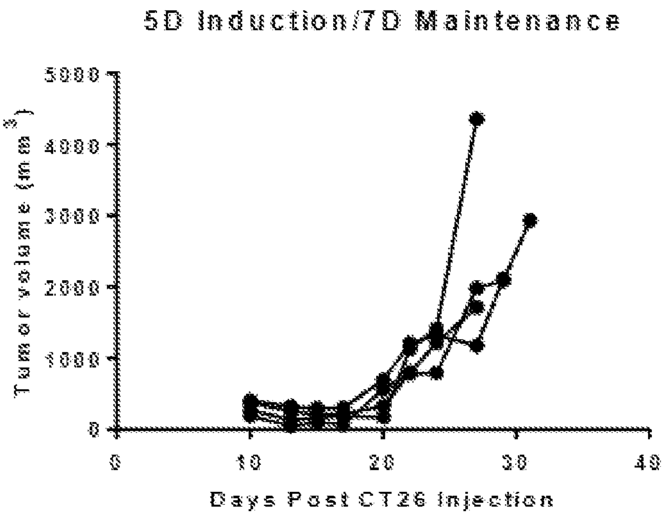
5D Induction/7D Maintenance
5000
4000
3000
2000
1000
0
Tumor volume (mm³)
0
10
20
30
40
Days Post CT26 Injection

*FIG. 12 (Cont.)*

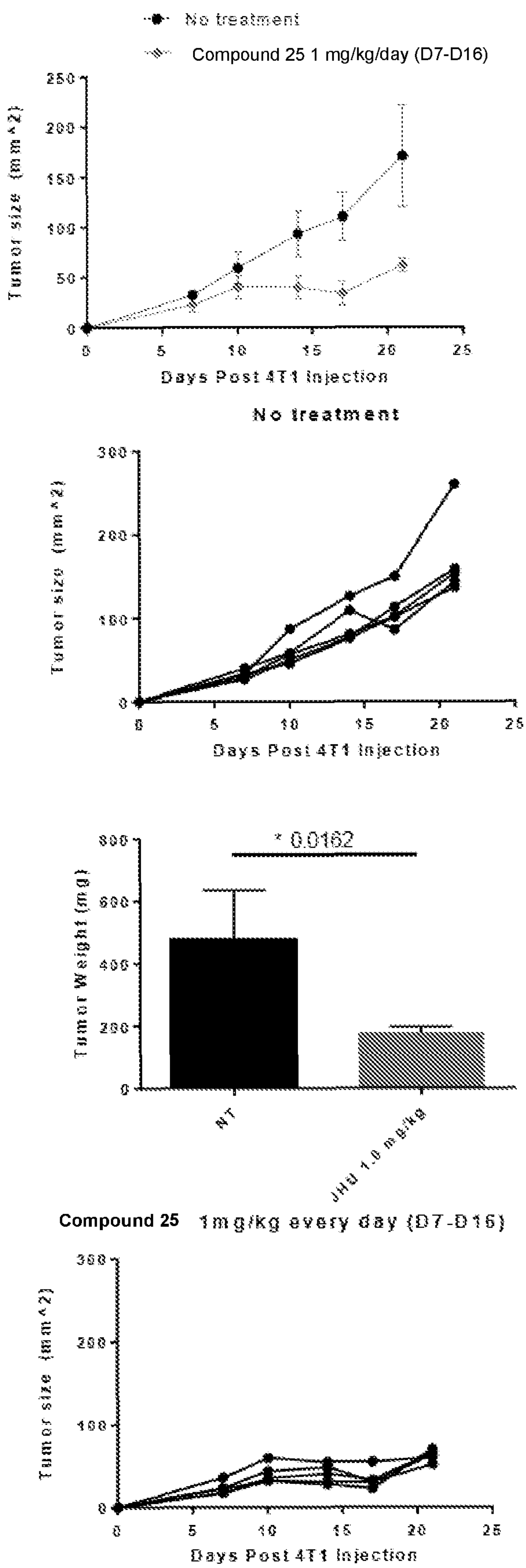
No treatment
Compound 25 1 mg/kg/day (D7-D16)
Tumor size (mm^2)
Days Post 4T1 injection
No treatment
Tumor size (mm^2)
Days Post 4T1 injection
* 0.0162
Tumor Weight (mg)
NT
JHU 1.0 mg/kg
Compound 25 1mg/kg every day (D7-D16)
Tumor size (mm^2)
Days Post 4T1 injection

*FIG. 13*

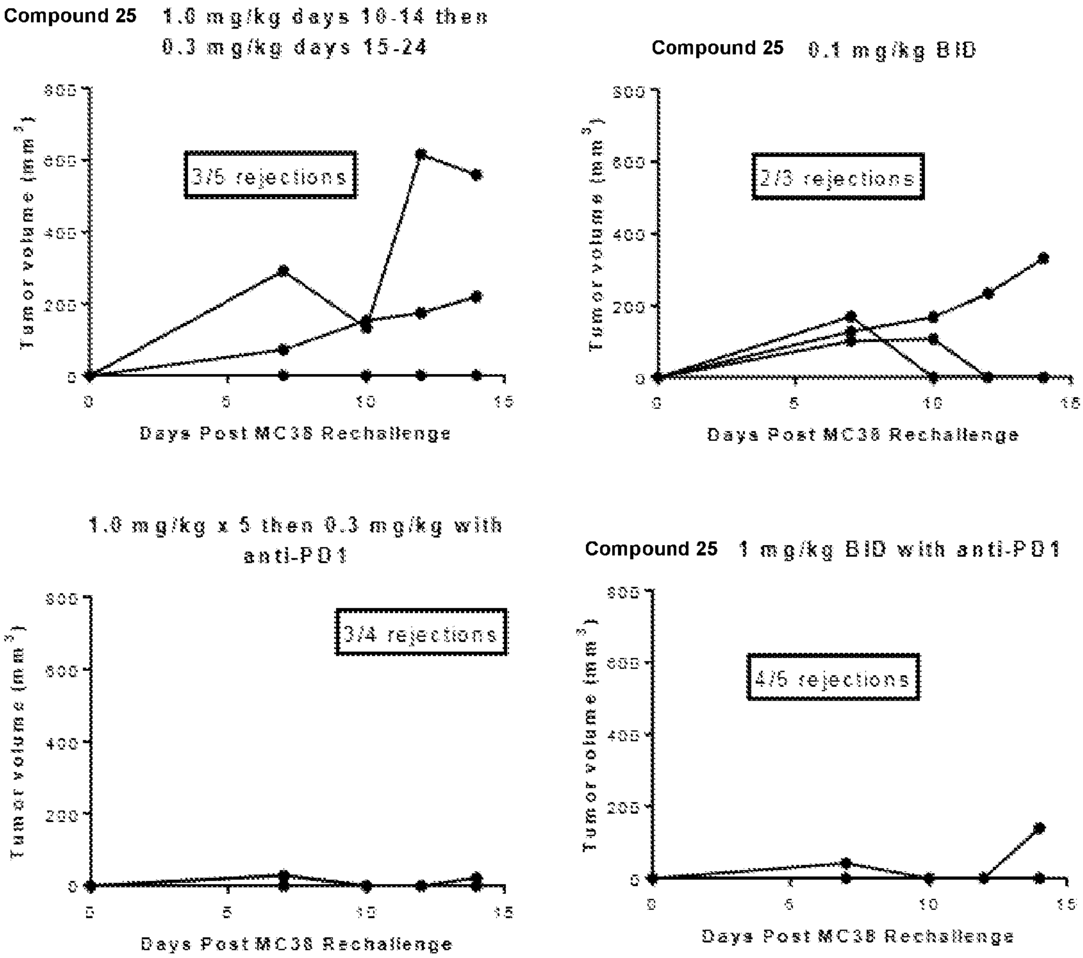
Compound 25 1.0 mg/kg days 10-14 then 0.3 mg/kg days 15-24
3/5 rejections
Tumor volume (mm3)
Days Post MC38 Rechallenge
Compound 25 0.1 mg/kg BID
2/3 rejections
Tumor volume (mm3)
Days Post MC38 Rechallenge
1.0 mg/kg x 5 then 0.3 mg/kg with anti-PD1
3/4 rejections
Tumor volume (mm3)
Days Post MC38 Rechallenge
Compound 25 1 mg/kg BID with anti-PD1
4/5 rejections
Tumor volume (mm3)
Days Post MC38 Rechallenge

*FIG. 14*

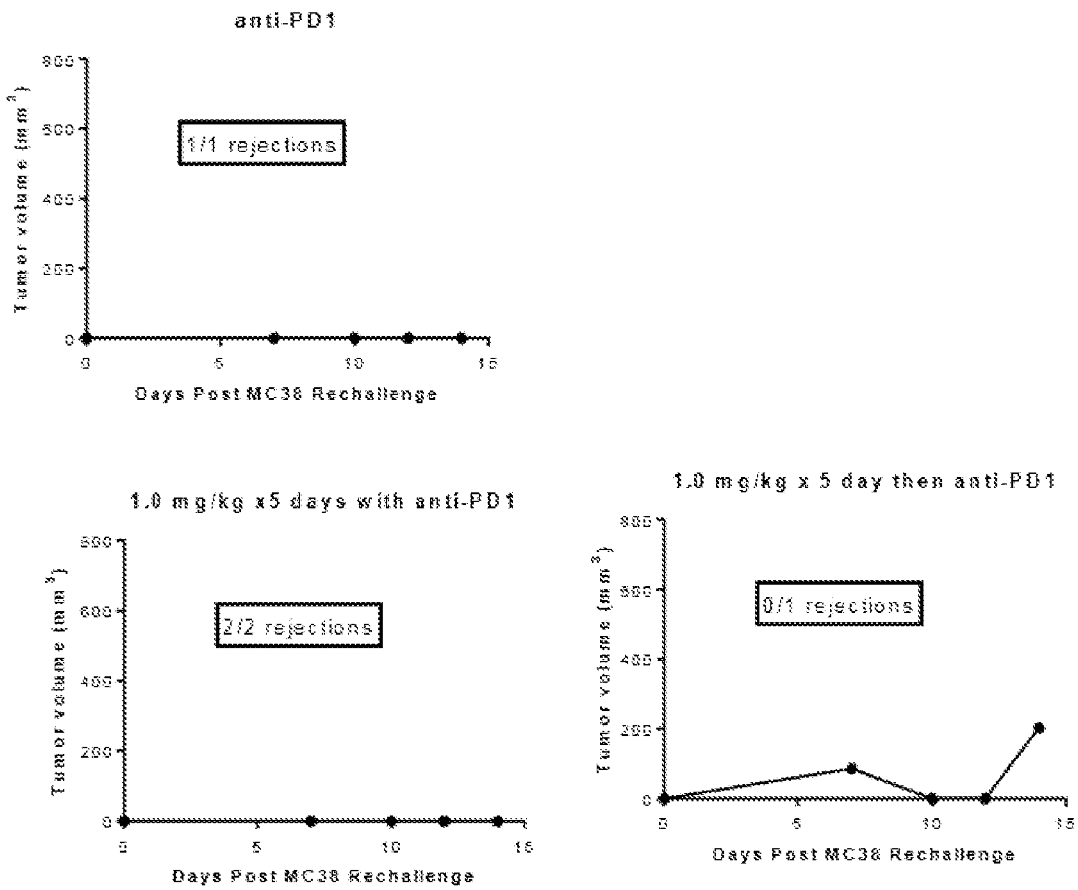
anti-PD1
1/1 rejections
Tumor volume (mm3)
Days Post MC38 Rechallenge
1.0 mg/kg x5 days with anti-PD1
2/2 rejections
Tumor volume (mm3)
Days Post MC38 Rechallenge
1.0 mg/kg x 5 day then anti-PD1
0/1 rejections
Tumor volume (mm3)
Days Post MC38 Rechallenge

FIG. 14 (Cont.)

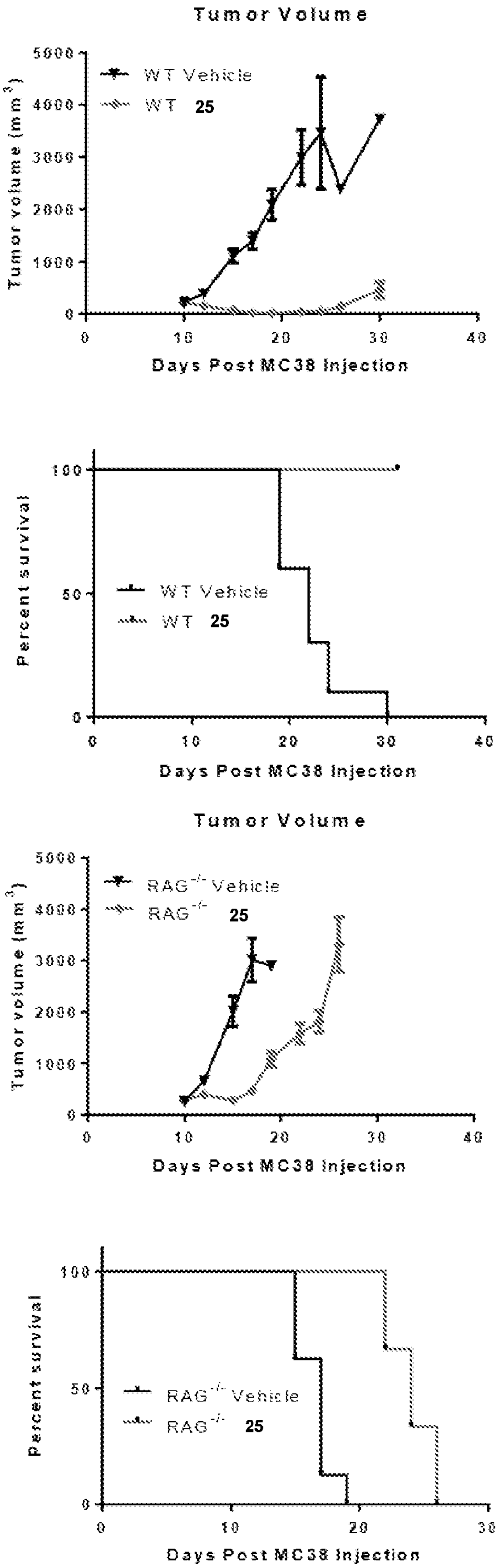
Tumor Volume
Tumor volume (mm3)
WT Vehicle
WT 25
Days Post MC38 Injection
Percent survival
WT Vehicle
WT 25
Days Post MC38 Injection
Tumor Volume
Tumor volume (mm3)
RAG-/- Vehicle
RAG-/- 25
Days Post MC38 Injection
Percent survival
RAG-/- Vehicle
RAG-/- 25
Days Post MC38 Injection

*FIG. 15*

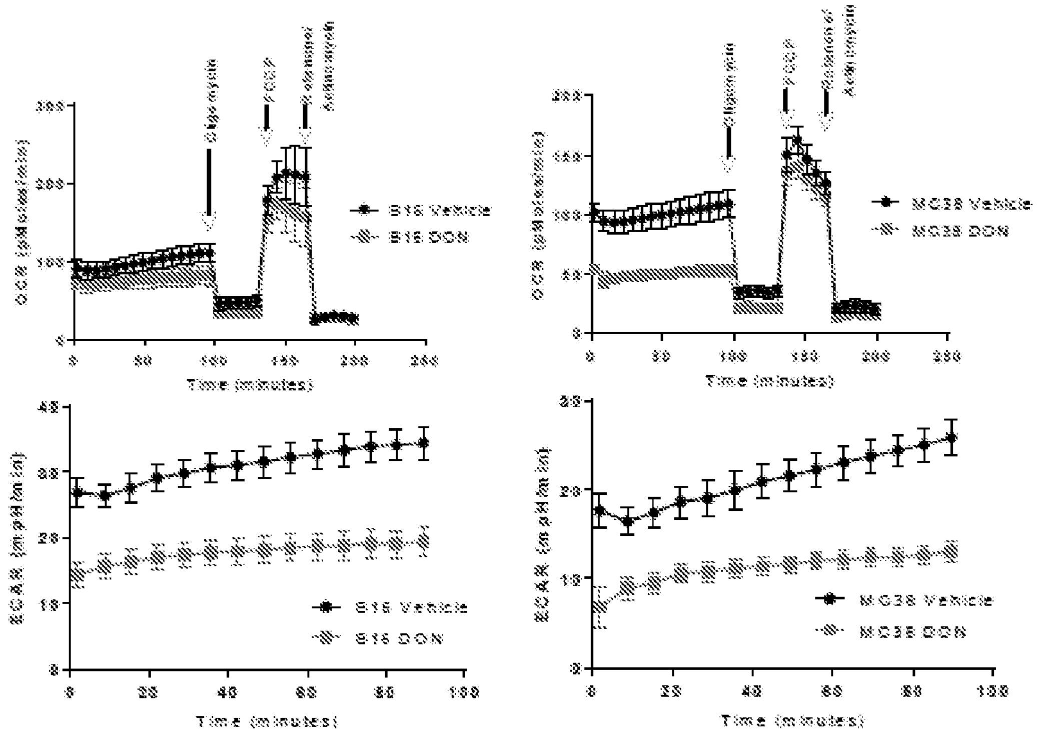
Oligomycin
FCCP
Rotenone/ Antimycin
B16 Vehicle
B16 DON
OCR (pMoles/min)
Time (minutes)
MC38 Vehicle
MC38 DON
ECAR (mpH/min)

*FIG. 16*

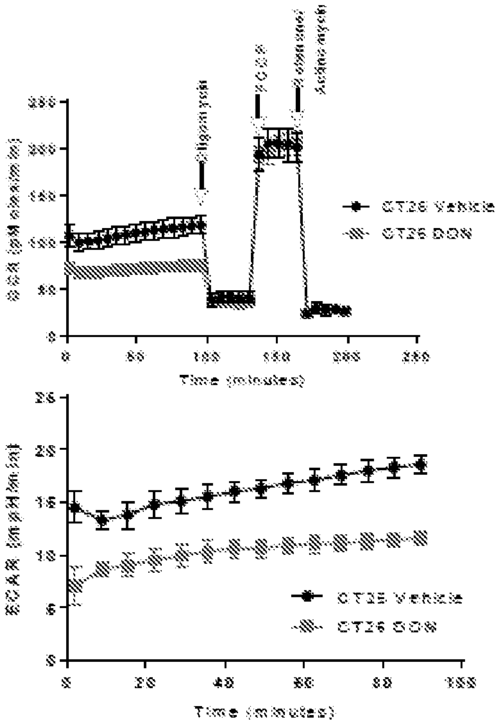
CT26 Vehicle
CT26 DON
Time (minutes)
CT26 Vehicle
CT26 DON
Time (minutes)

# FIG. 16 (Cont.)

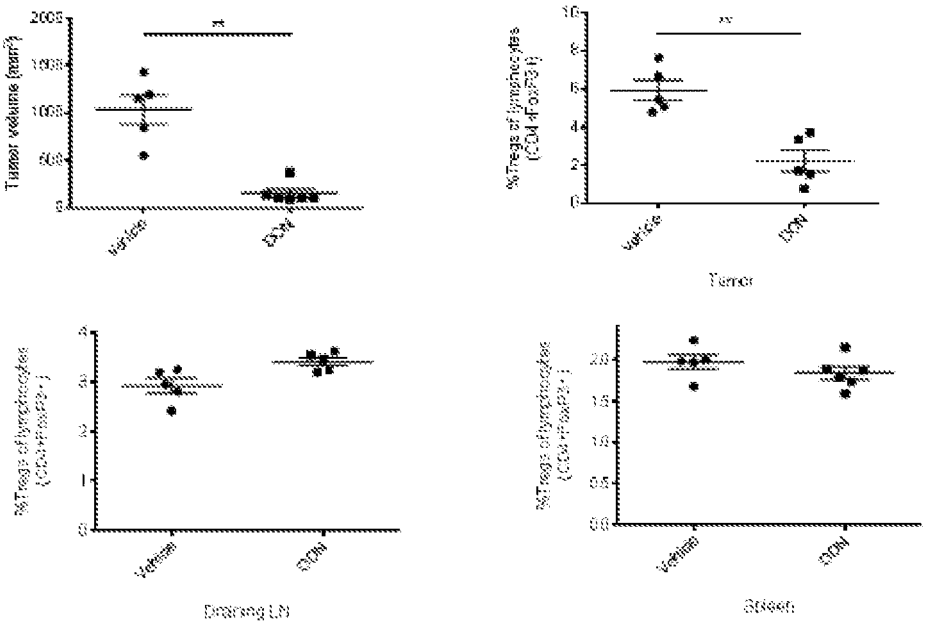

Tumor volume (mm³)
2000
1500
1000
500
0
*
Vehicle
DON
%Tregs of lymphocytes (CD4+FoxP3+)
10
8
6
4
2
0
**
Vehicle
DON
Tumor
4
3
2
1
0
Vehicle
DON
Draining LN
2.0
1.5
1.0
0.5
0.0
Vehicle
DON
Spleen


*FIG. 17*

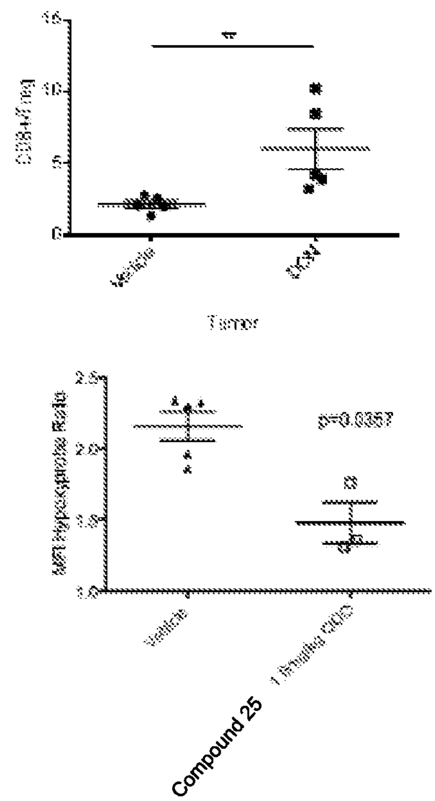


*FIG. 17 (Cont.)*

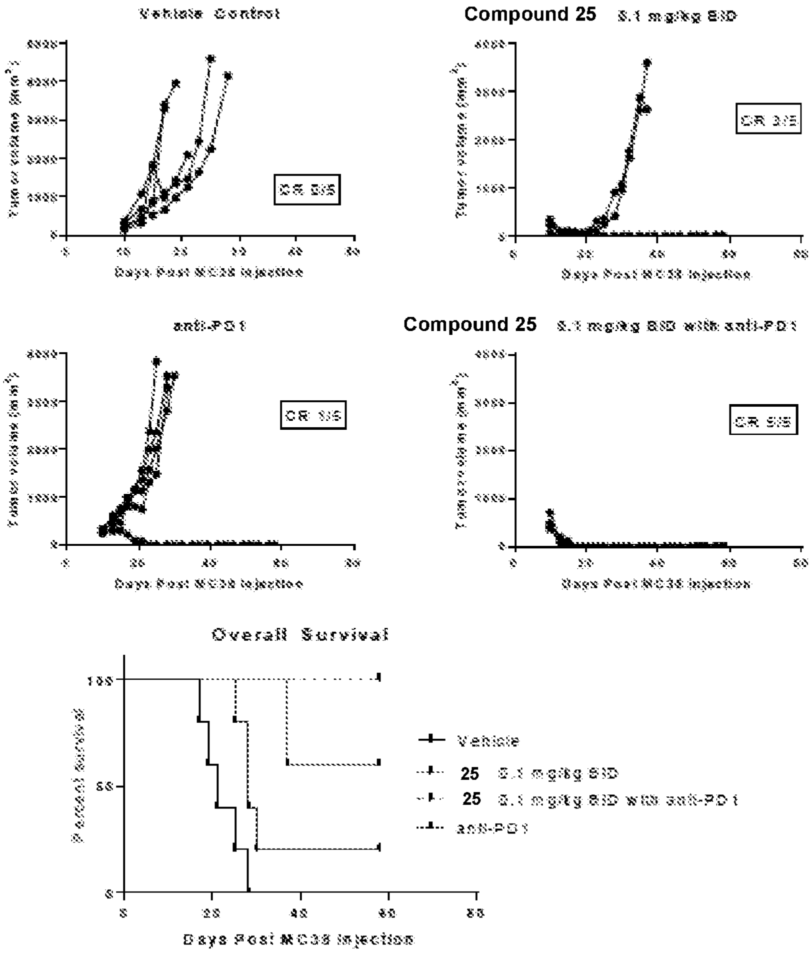
Vehicle Control
Compound 25
anti-PD1
Compound 25
Overall Survival
Percent survival
Days Post MC38 injection
Vehicle
25
25
anti-PD1

*FIG. 18*

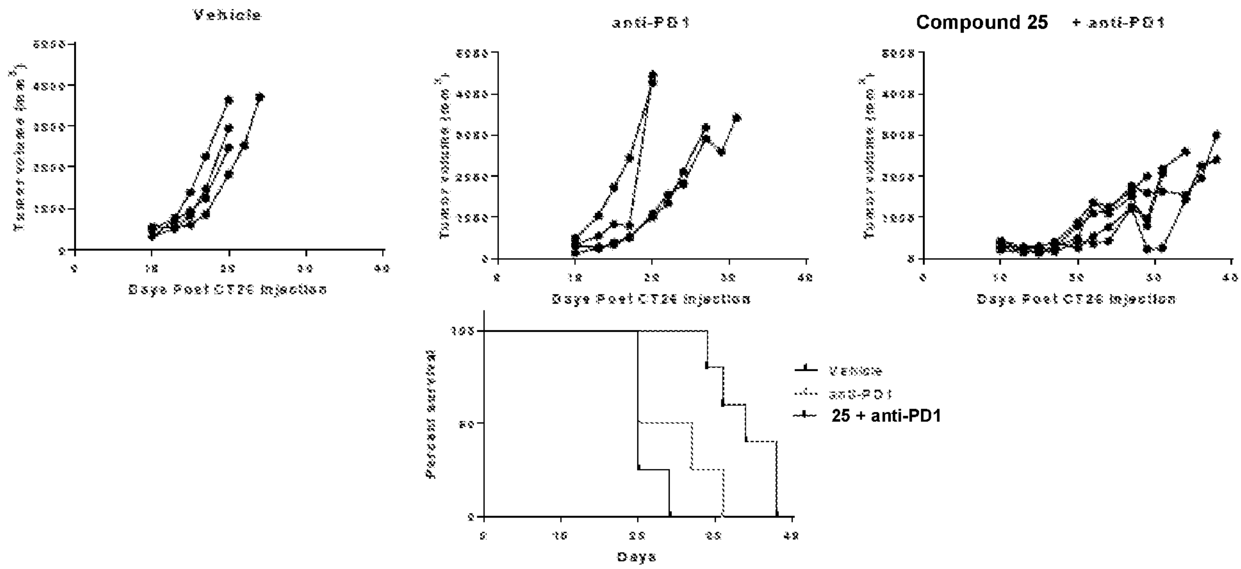
Vehicle
anti-PD1
Compound 25 + anti-PD1
Tumor volume (mm3)
Days Post CT26 injection
Percent survival
Days
Vehicle
anti-PD1
25 + anti-PD1

*FIG. 19*

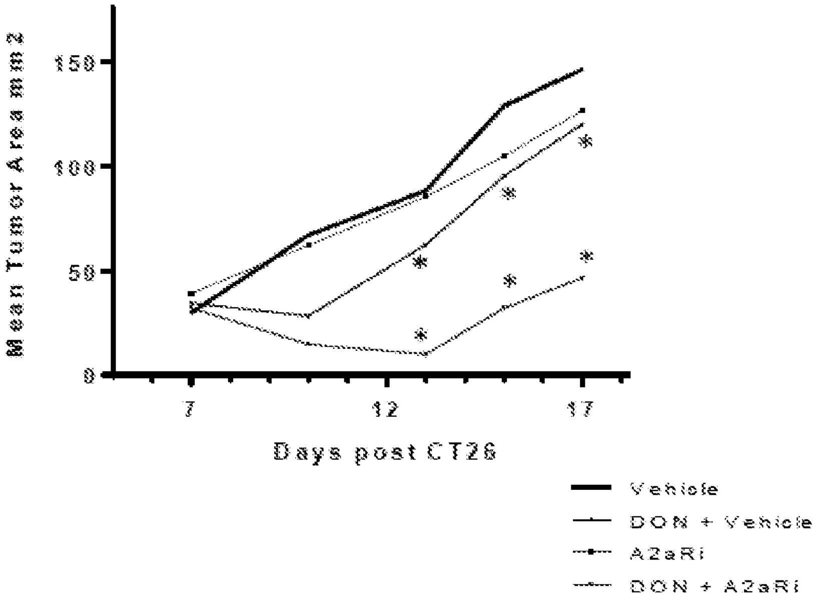

Mean Tumor Area mm2
0
50
100
150
7
12
17
Days post CT26
Vehicle
DON + Vehicle
A2aRi
DON + A2aRi


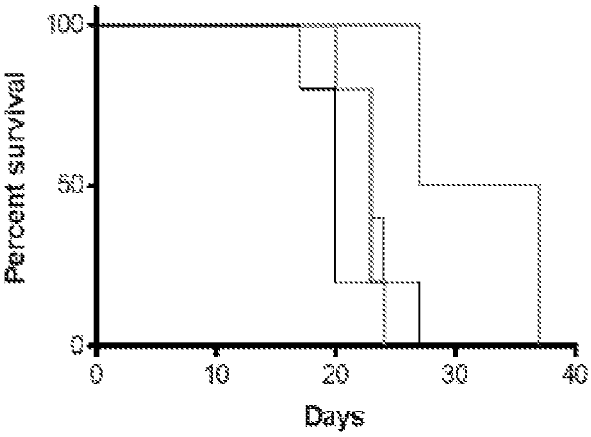

Percent survival
0
50
100
0
10
20
30
40
Days


*FIG. 20*

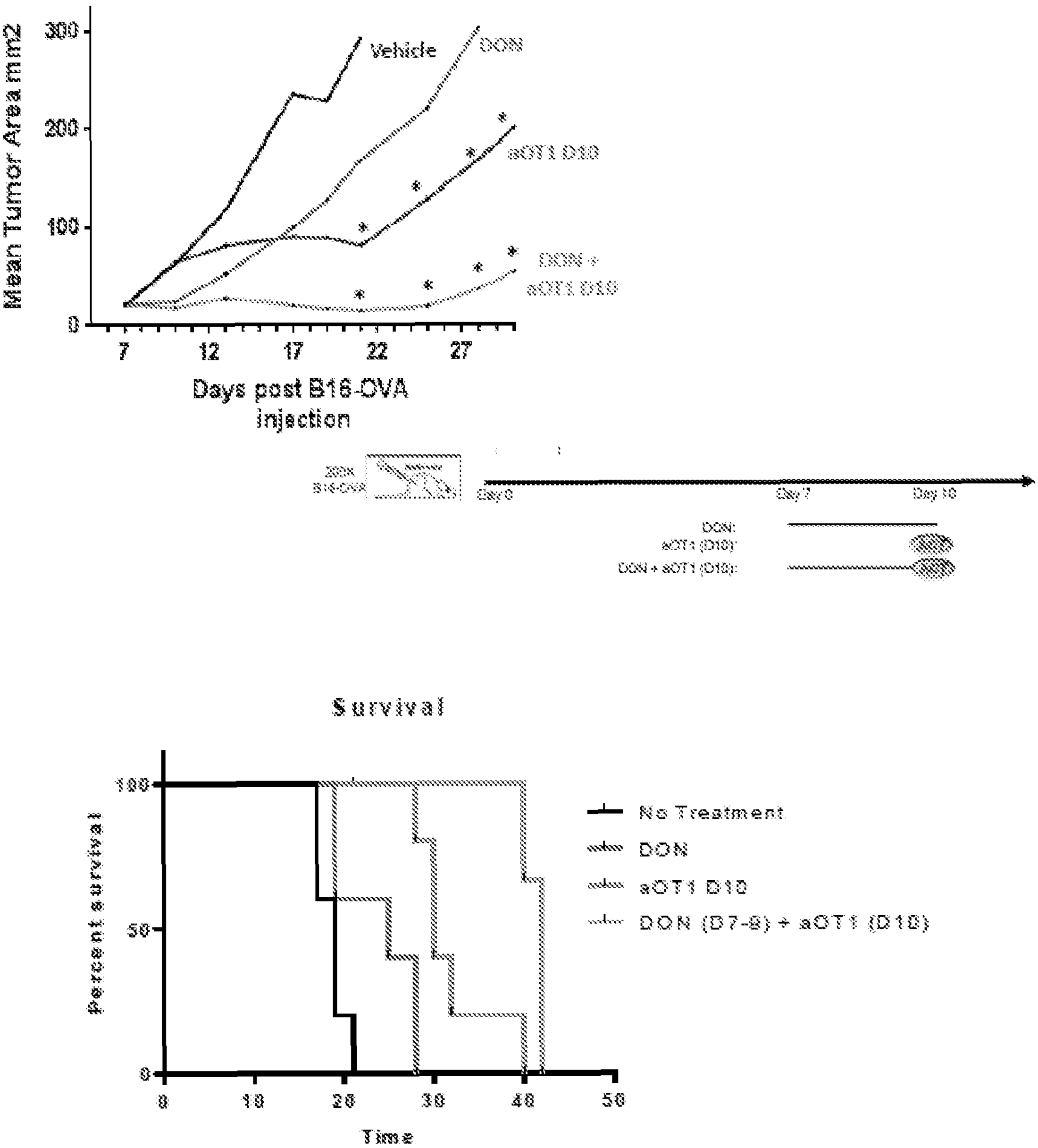
Mean Tumor Area mm2
300
200
100
0
7
12
17
22
27
Days post B16-OVA injection
Vehicle
DON
aOT1 D10
DON + aOT1 D10
Day 0
Day 7
Day 10
DON:
aOT1 (D10):
DON + aOT1 (D10):
Survival
Percent survival
100
50
0
0
10
20
30
40
50
Time
No Treatment
DON
aOT1 D10
DON (D7-9) + aOT1 (D10)

*FIG. 21*

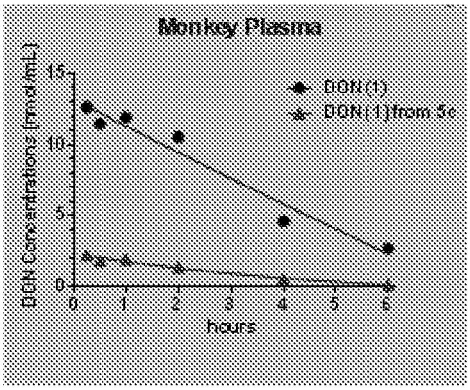


FIG. 22A

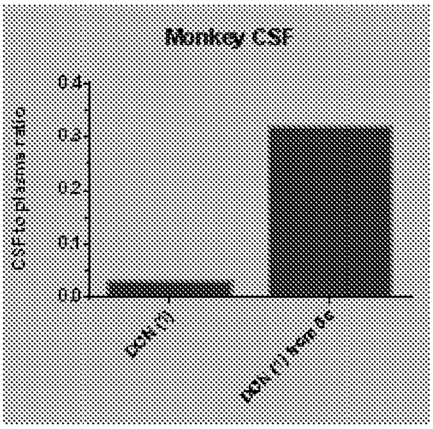


FIG. 22B

| Compound Dosed | Dose (mg/kg equiv) | DON $C_{max}$ (nmol/mL) | DON $T_{max}$ (hr) | DON $AUC_{0-t}$ (hr*nmol/mL) |
|---|---|---|---|---|
| 1 | 1.6 | 12.6 | 0.25 | 42.7 |
| 5c | 1.6 | 2.23 | 0.25 | 5.71 |

FIG. 22C

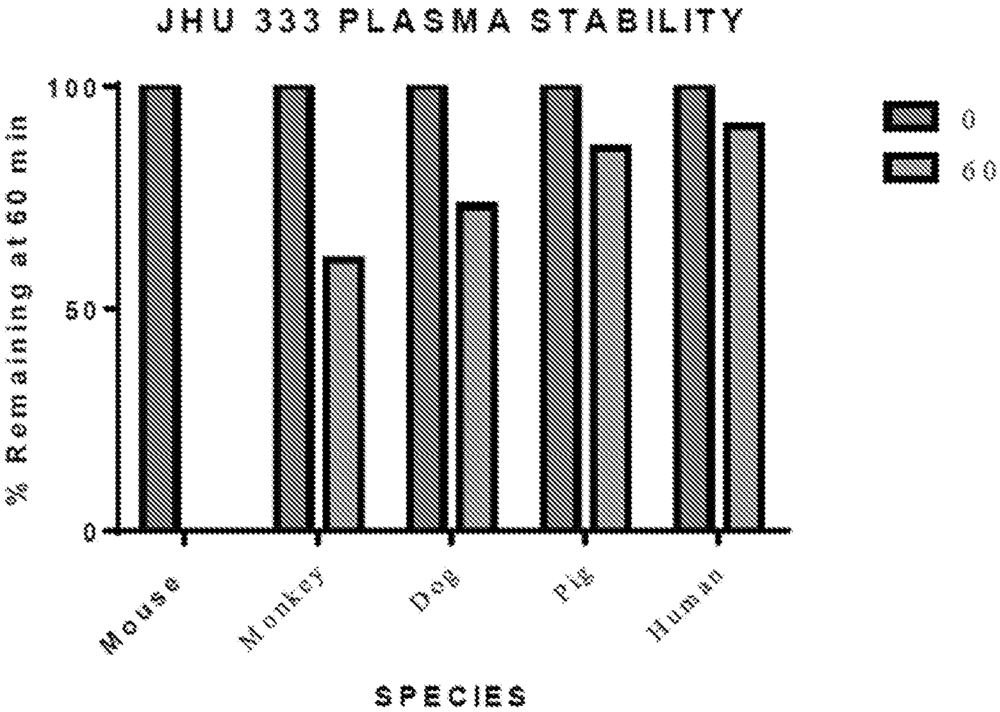
JHU 333 PLASMA STABILITY
% Remaining at 60 min
100
50
0
Mouse
Monkey
Dog
Pig
Human
SPECIES
0
60

*FIG. 23*

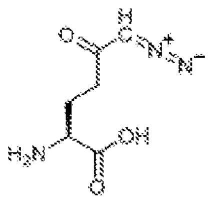

**DON**

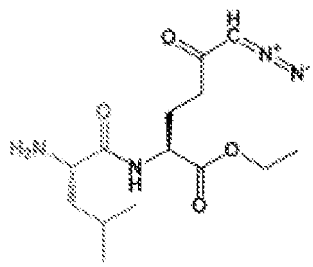

25

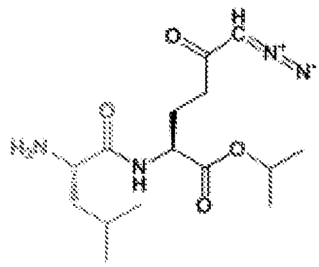

9

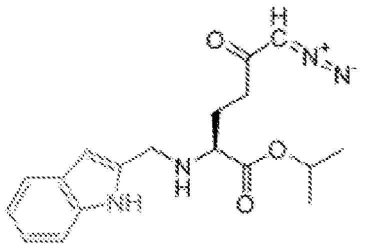

38

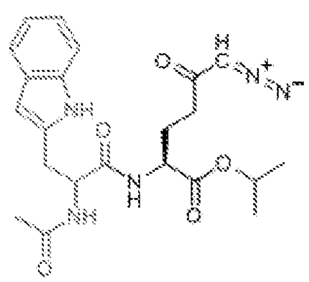

**60**

*FIG. 24*

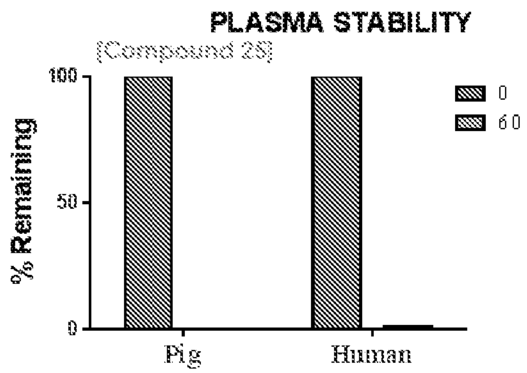


FIG. 25A

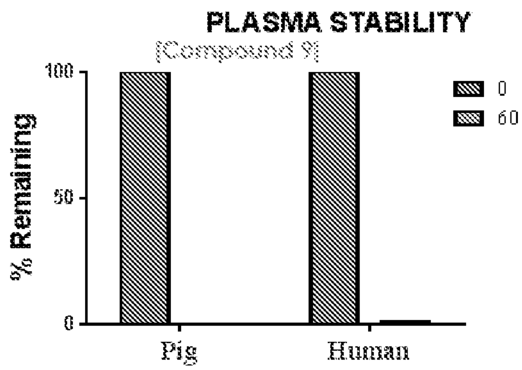


FIG. 25B

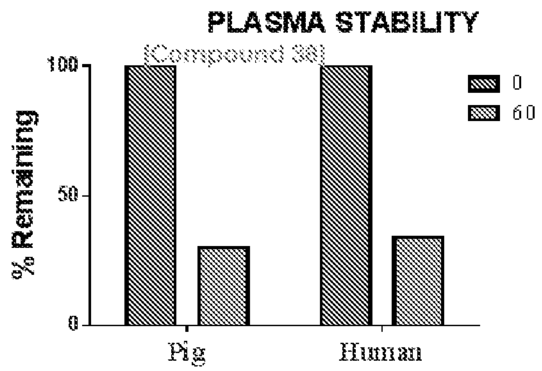


FIG. 25C

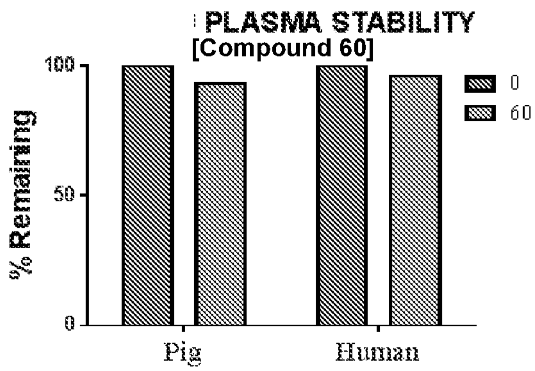


FIG. 25D

[Compound 25]

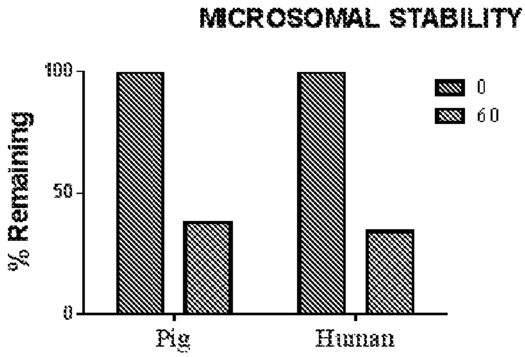


FIG. 26A

[Compound 9]

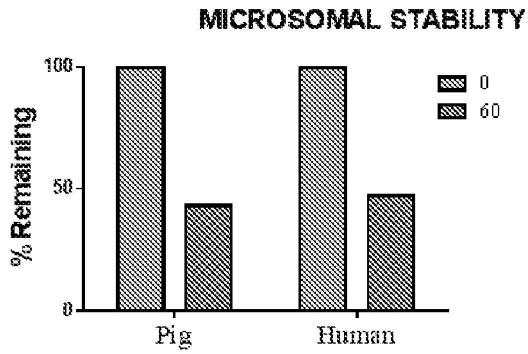


FIG. 26B

[Compound 38]

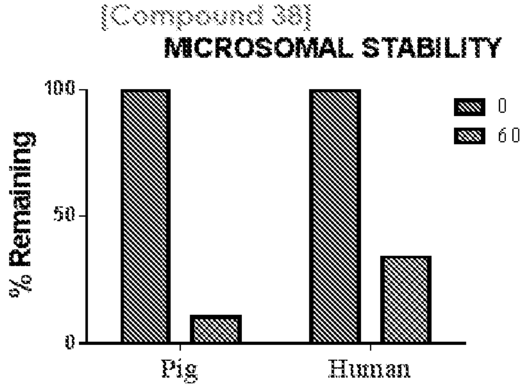


FIG. 26C

[Compound 60]

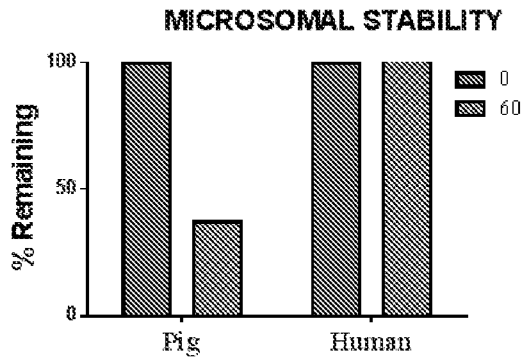


FIG. 26D

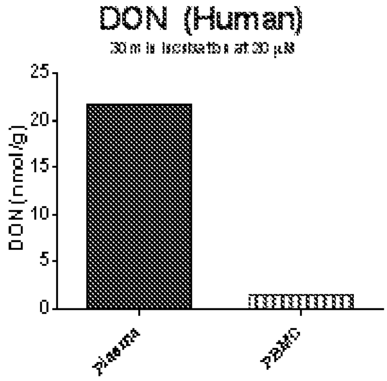


FIG. 27A

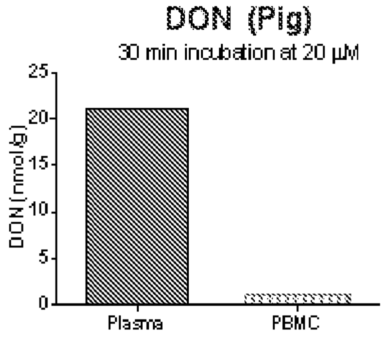


FIG. 27B

[Compound 25]

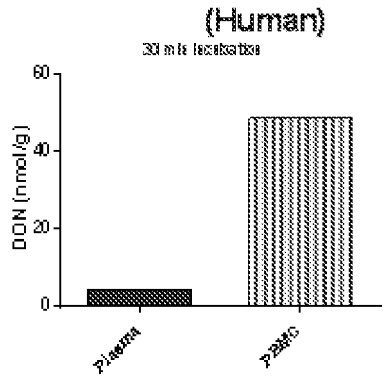


FIG. 27C

[Compound 25]

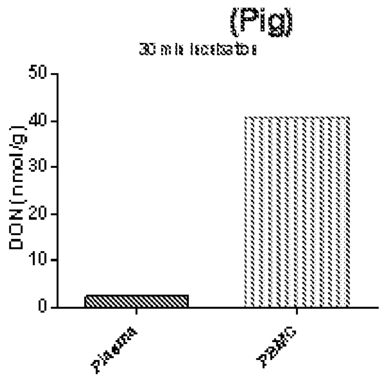


FIG. 27D

[Compound 9]

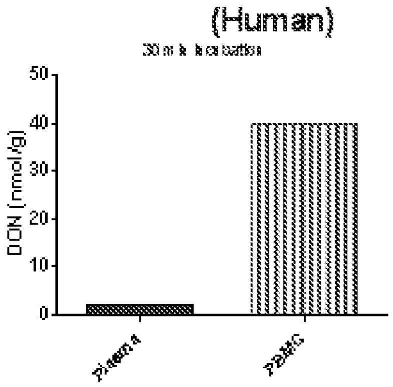


FIG. 27E

[Compound 9]

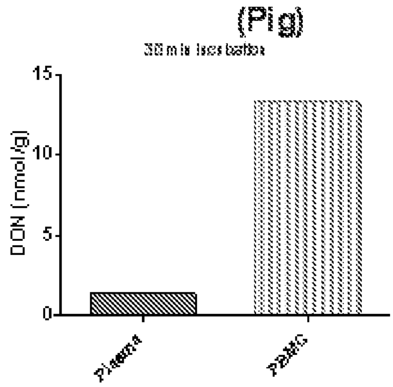


FIG. 27F

[Compound 38]

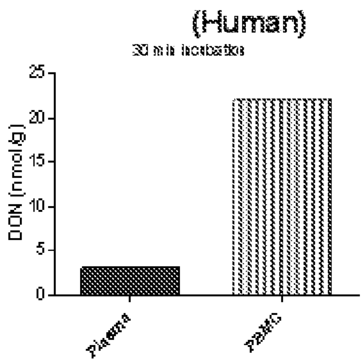


FIG. 27G

[Compound 38]

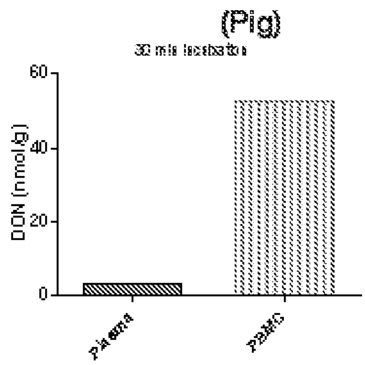


FIG. 27H

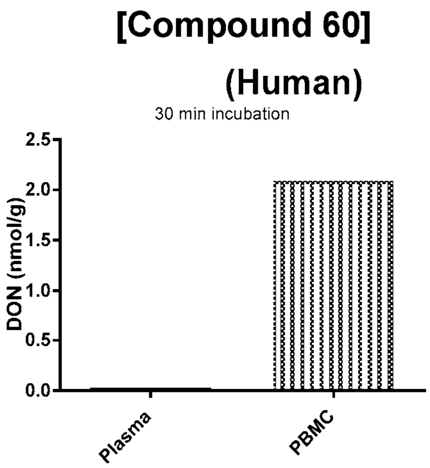


FIG. 27I

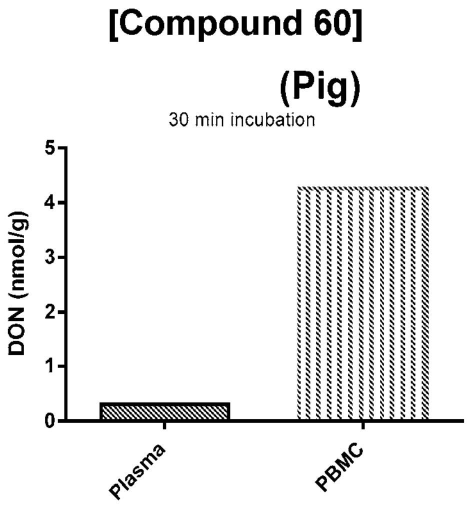


FIG. 27J

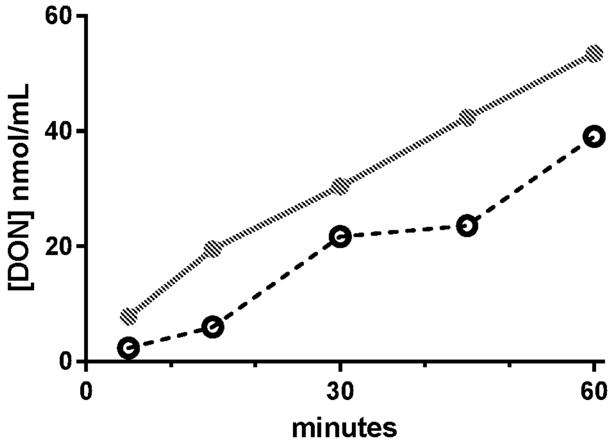


*FIG. 28A*

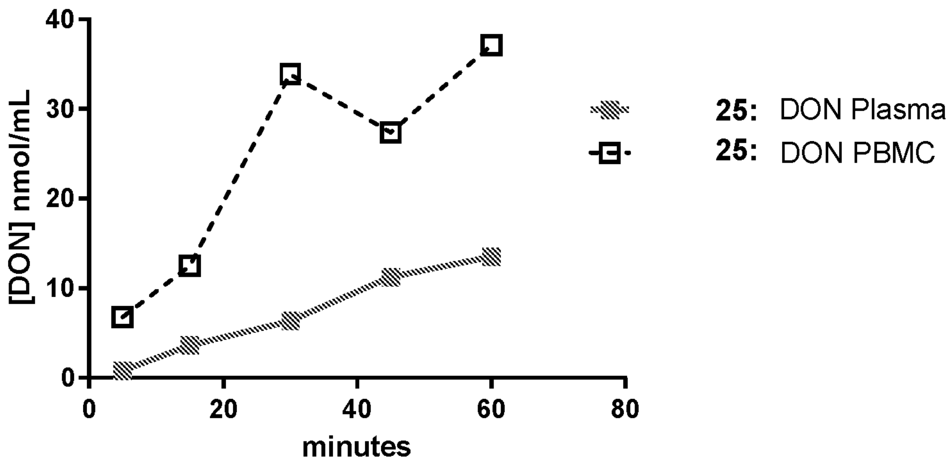


*FIG. 28B*

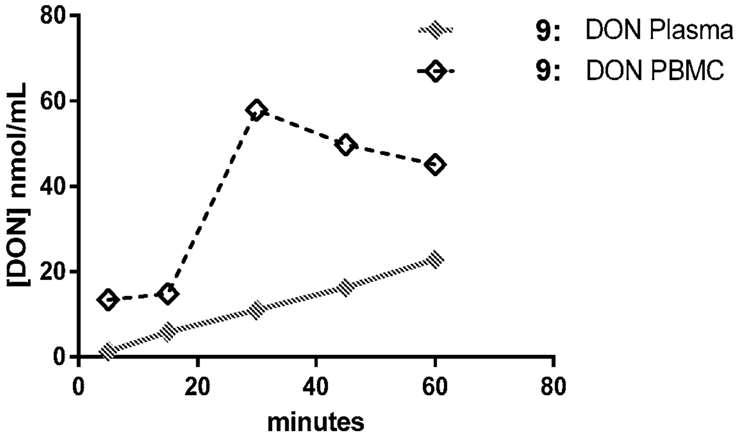


*FIG. 28C*

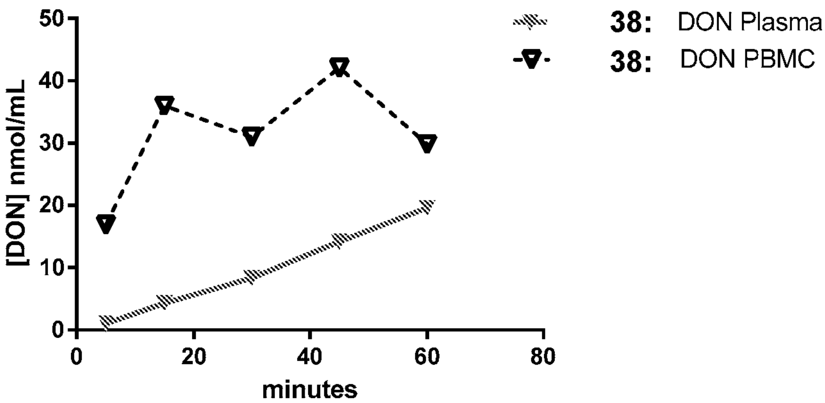


*FIG. 28D*

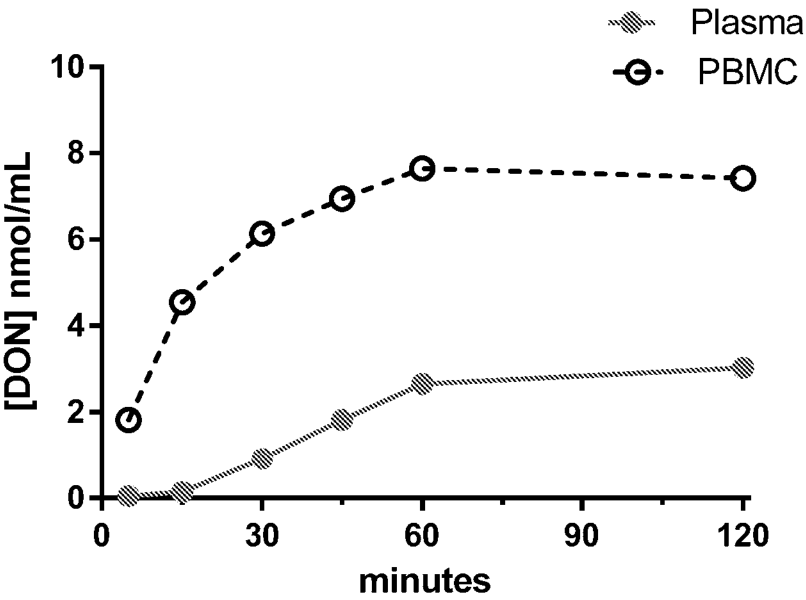


*FIG. 28E*

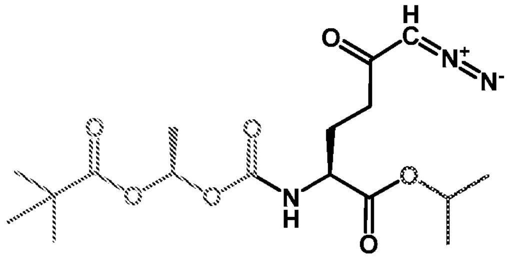

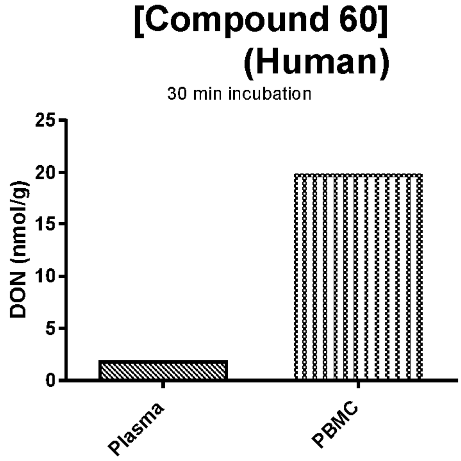


FIG. 29A

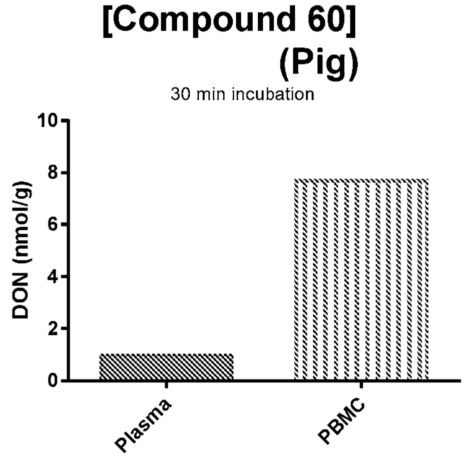


FIG. 29B

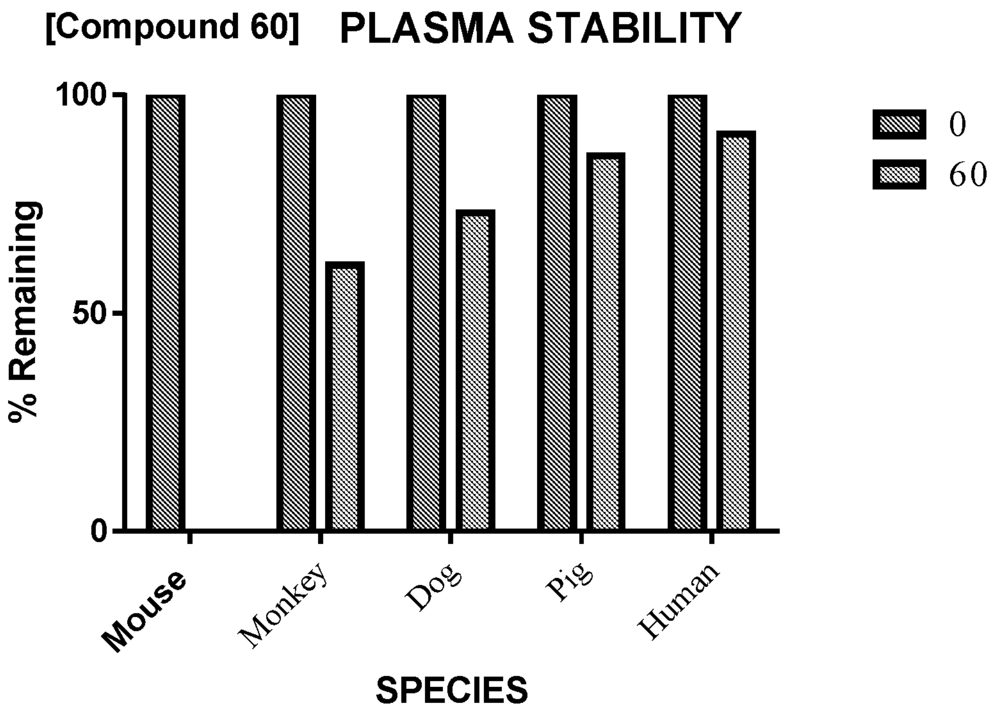


FIG. 29C

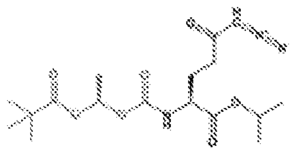

14

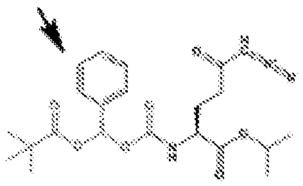

47

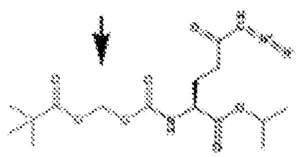

42

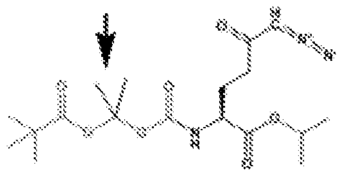

44

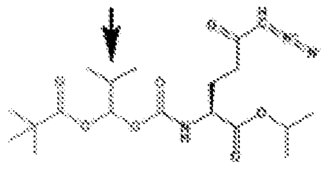

40

*FIG. 30*

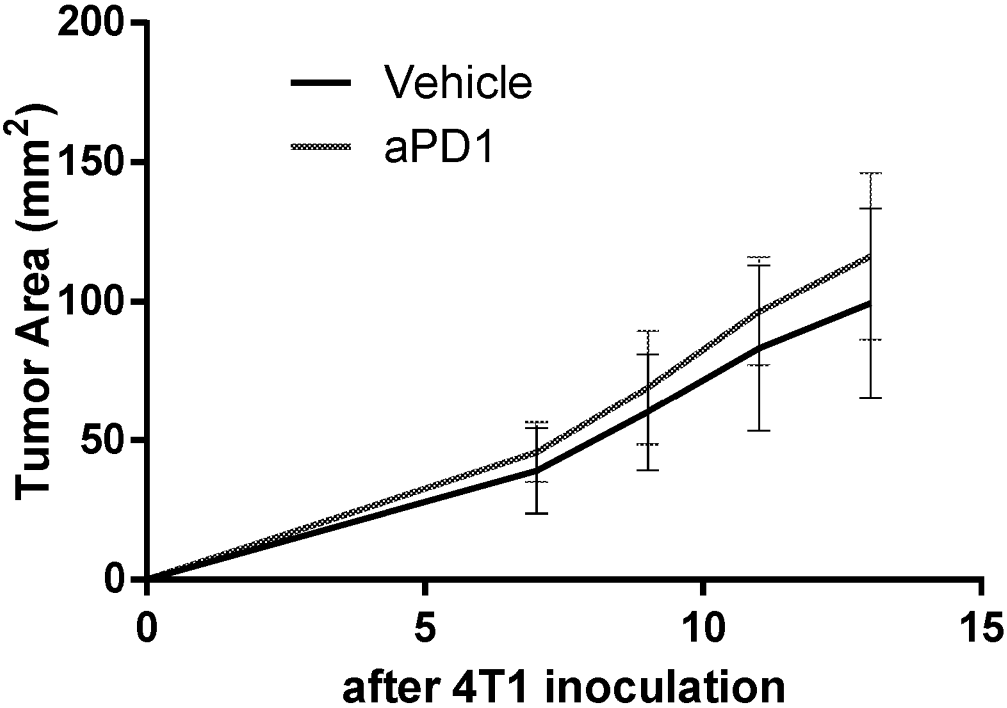
Vehicle
aPD1
Tumor Area (mm2)
200
150
100
50
0
0
5
10
15
after 4T1 inoculation

*FIG. 31*

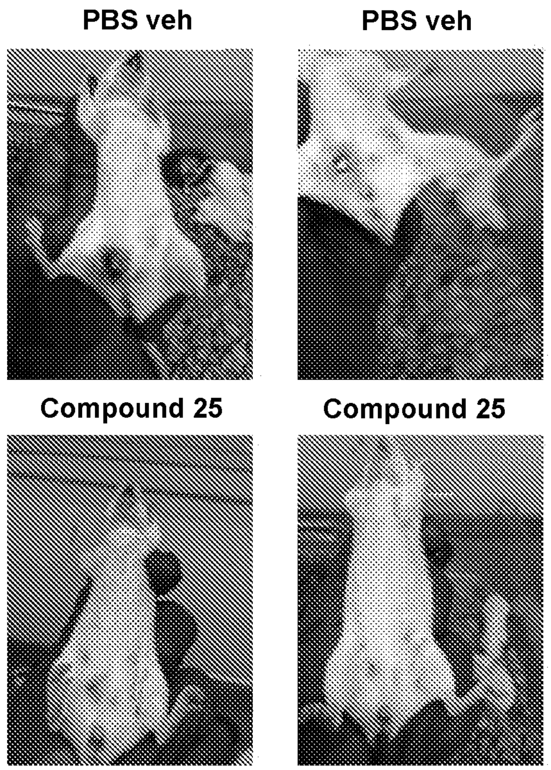


*FIG. 32A*

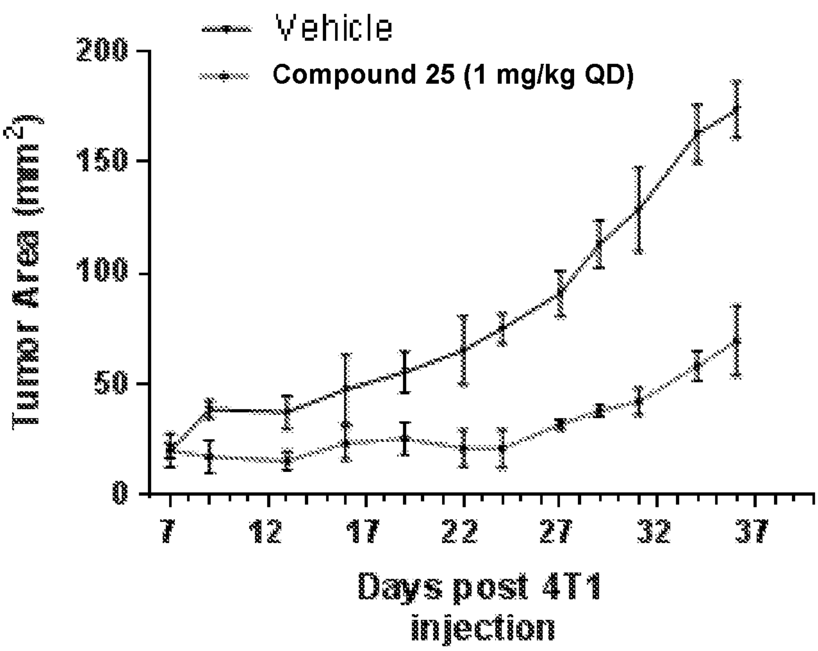


*FIG. 32B*

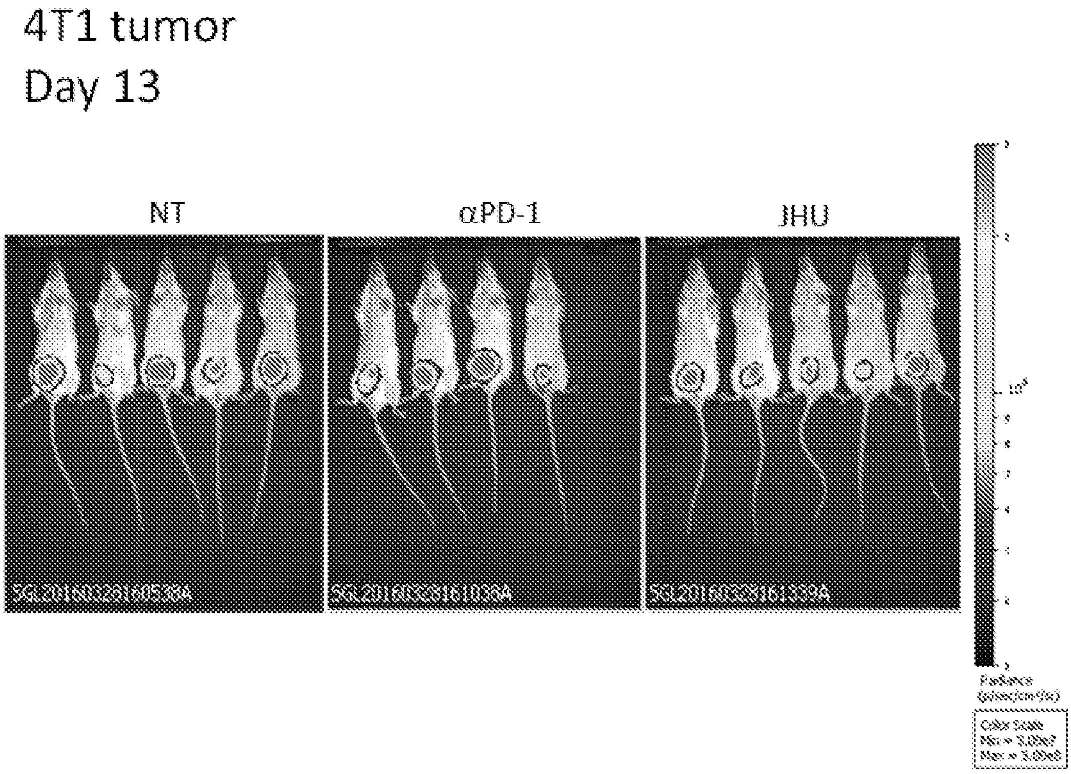


*FIG. 33A*

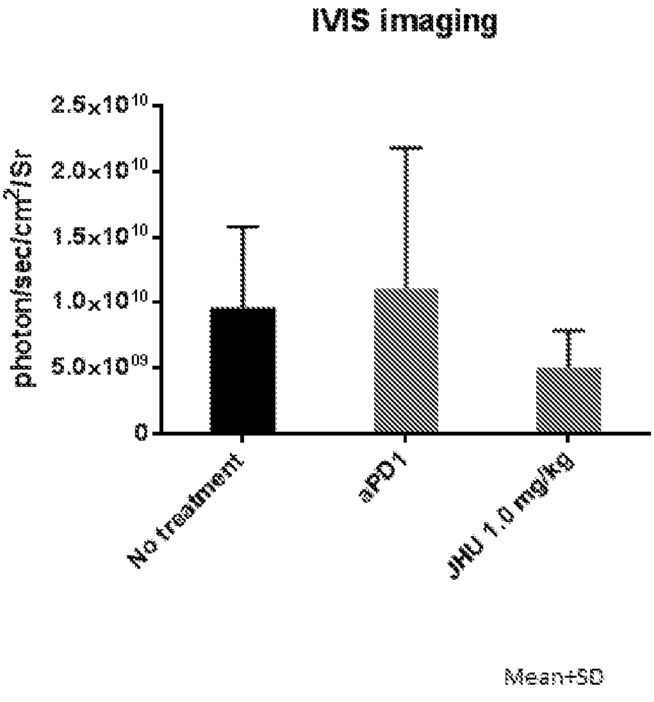


*FIG. 33B*

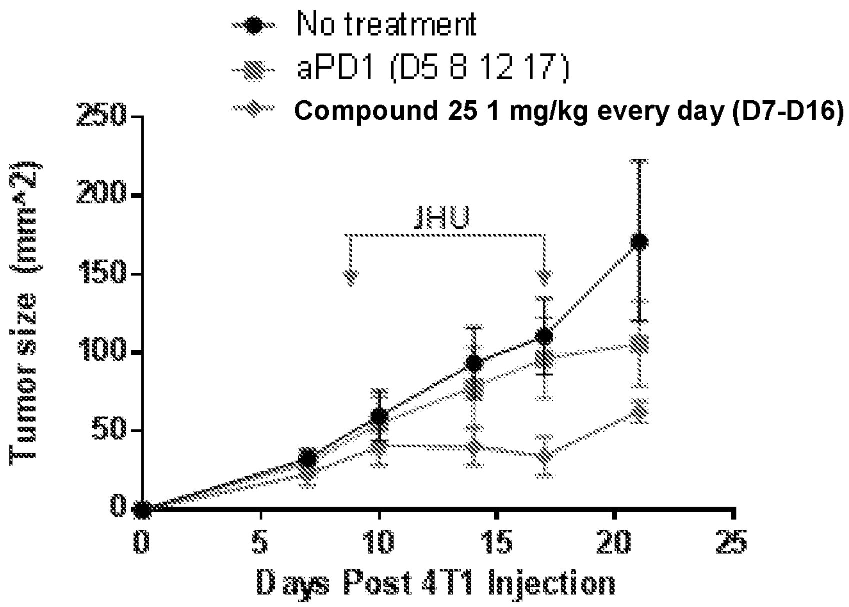


*FIG. 34A*

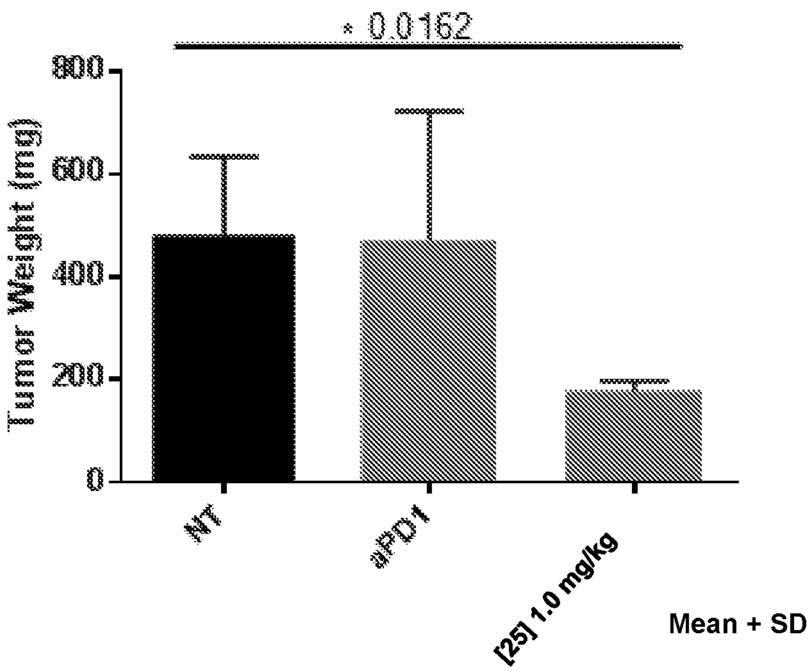


*FIG. 34B*

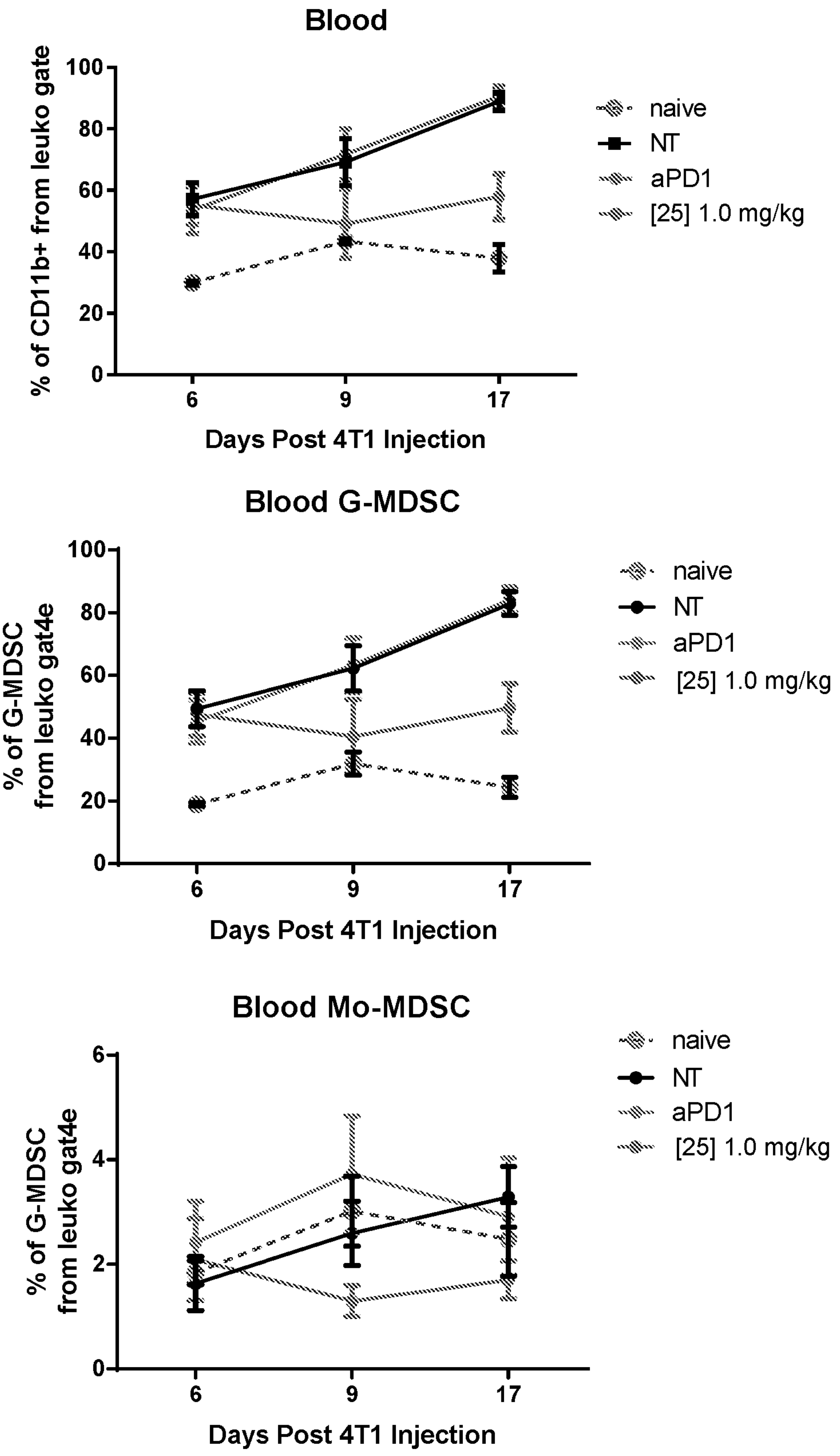
Blood
% of CD11b+ from leuko gate
100
80
60
40
20
0
6
9
17
Days Post 4T1 Injection
naive
NT
aPD1
[25] 1.0 mg/kg
Blood G-MDSC
% of G-MDSC from leuko gat4e
100
80
60
40
20
0
6
9
17
Days Post 4T1 Injection
naive
NT
aPD1
[25] 1.0 mg/kg
Blood Mo-MDSC
% of G-MDSC from leuko gat4e
6
4
2
0
6
9
17
Days Post 4T1 Injection
naive
NT
aPD1
[25] 1.0 mg/kg

*FIG. 35*

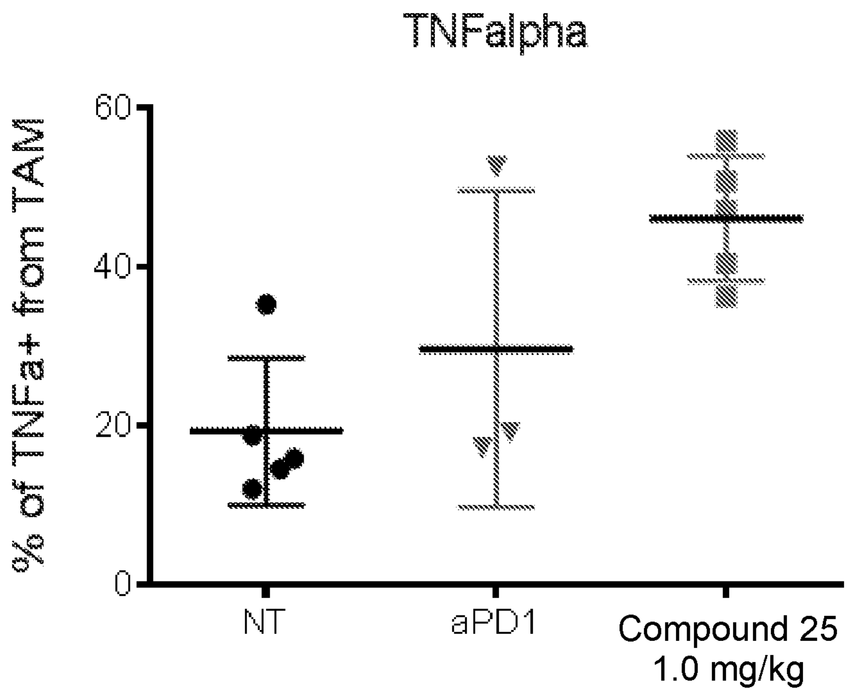
TNFalpha
% of TNFa+ from TAM
60
40
20
0
NT
aPD1
Compound 25
1.0 mg/kg

*FIG. 36*

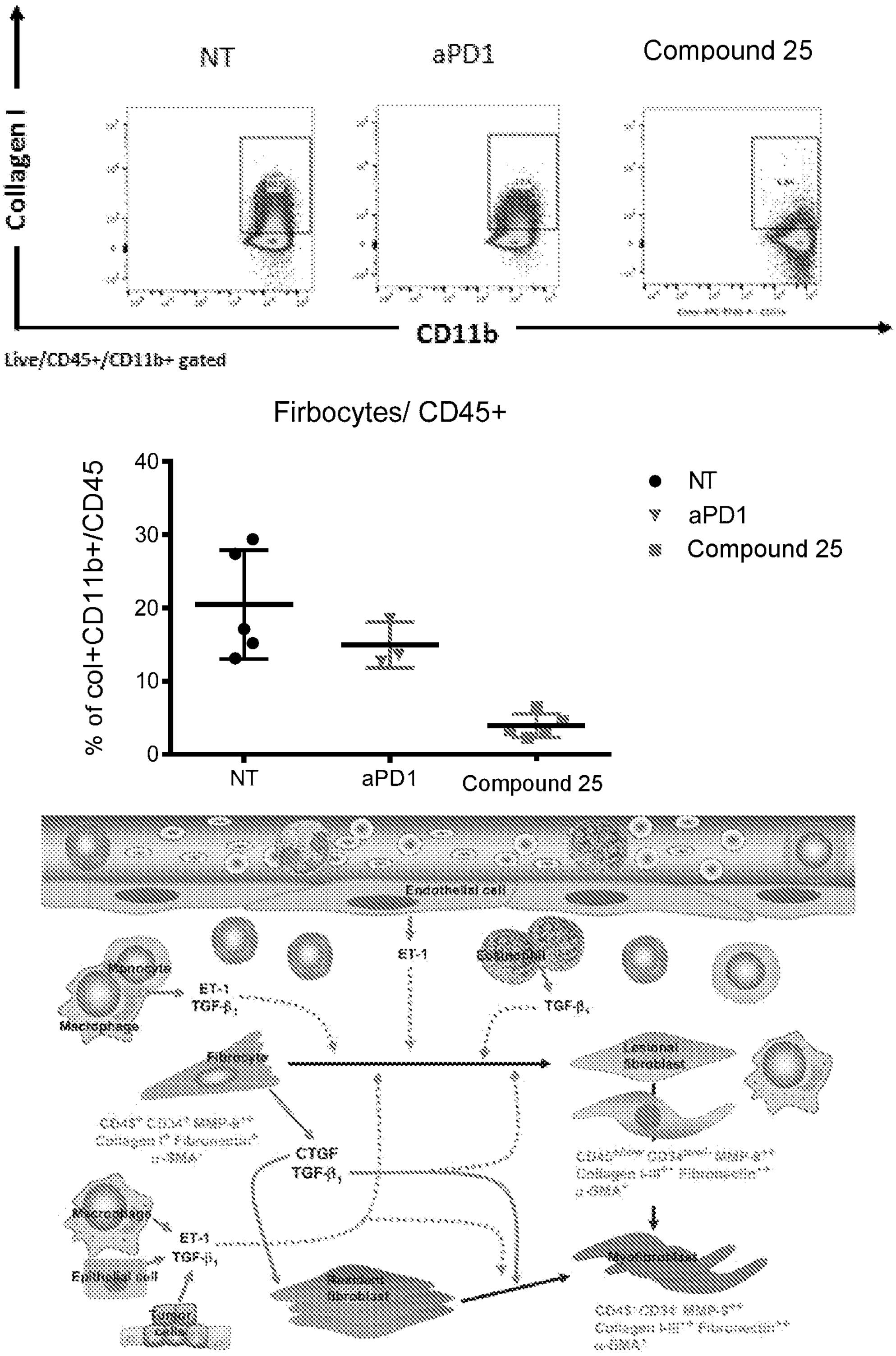
NT
aPD1
Compound 25
Collagen I
CD11b
Live/CD45+/CD11b+ gated
Firbocytes/ CD45+
% of col+CD11b+/CD45
40
30
20
10
0
NT
aPD1
Compound 25
NT
aPD1
Compound 25
Endothelial cell
ET-1
CTGF
TGF-β1

*FIG. 37*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014160071 A
- WO 2014148391 A
- EP 0123170 A
- WO 2004113363 A
- WO 200405513 A2
- US 6362226 B
- WO 2017023774 A1
- US 5328470 A
- WO 2005097108 A
- WO 2005068455 A
- US 20040029801 A
- US 8946168 B
- US 3773919 A
- EP 58481 A
- EP 133988 A
- US 4485045 A
- US 4544545 A
- EP 102324 A


### Non-patent literature cited in the description


- **VANDER HEIDEN et al.** *Science,* 2009, vol. 324 (5930), 1029-1033
- **WARBURG.** *Science,* 1956, vol. 124 (3215), 269-270
- **WARBURG et al.** Zeitschriftfur Naturforschung. Teil B: Chemie, Biochemie, Biophysik, Biologie. *Metabolism of leukocytes,* 1958, vol. 13B (8), 515-516
- **ALAN RAMSAY.** Immune checkpoint blockade immunotherapy to activate anti-tumour T-cell immunity. *British Journal of Haematology,* 21 May 2013, vol. 162 (3), 313-325
- Current Protocols in Molecular Biology, Current Protocols in Immunology, Current Protocols in Protein Science. Current Protocols in Cell Biology. John Wiley & Sons, December 2008
- **SAMBROOK ; RUSSELL ; SAMBROOK.** Molecular Cloning. A Laboratory Manual. Cold Spring Harbor Laboratory Press, 2001
- **HARLOW, E. ; LANE, D.** Antibodies-A Laboratory Manual. Cold Spring Harbor Laboratory Press, 1988
- **FRESHNEY, R. I.** Culture of Animal Cells, A Manual of Basic Technique. John Wiley & Sons, 2005
- **GOODMAN ; GILMAN'S.** The Pharmacological Basis of Therapeutics. McGraw Hill, 2005
- Basic and Clinical Pharmacology. McGraw-Hill/Appleton & Lange, 2006
- Mendelian Inheritance in Man. **MCKUSICK, V. A.** A Catalog of Human Genes and Genetic Disorders. Johns Hopkins University Press, 1998
- **HUBER et al.** Uptake of glutamine antimetabolites 6-diazo-5-oxo-L-norleucine (DON) and acivicin in sensitive and resistant tumor cell lines. *Int. J. Cancer.,* 1988, vol. 41, 752-755
- **JUN et al.** *Generation of antagonistic anti-TIM-3 and anti-LAG-3 monoclonal antibodies for potential novel immunotherapy combinations, http://www.tesarobio.com/documents/2014AACRposterLB266.pdf*
- **LINCH et al.** OX40 Agonists and Combination Immunotherapy: Putting the Pedal to the Metal. *Front Oncol,* 2015, vol. 5, 34
- **DARMAUN et al.** Phenylbutyrate-induce glutamine depletion in humans: effect on leucine metabolism. *Glutamine Depletion and Protein Catabolism, Am. Physiol. Soc.,* 1998, E801-E807
- **VAROQUI et al.** *J. Biol. Chem.,* 2000, vol. 275 (6), 4049-4054
- **PAWLIK et al.** *Am. J. Physiol. Gastrointest. Liver Physiol.,* 2000, vol. 278, G532-G541
- **ZGÒDKA et al.** *Microbiology,* 2001, vol. 147, 1955-1959
- **ACEVEDO et al.** *Tetrahedron,* 2001, vol. 57, 6353-6359
- **SRIKANTH et al.** *Bioorganic and Medicinal Chemistry,* 2002
- **CHEN et al.** *Proc. Natl. Acad. Sci. USA,* 1994, vol. 91, 3054-3057
- **KARLIN ; ALTSCHUL.** *Proc. Natl. Acad. Sci. USA,* 1990, vol. 87, 22264-2268
- **KARLIN ; ALTSCHUL.** *Proc. Natl. Acad. Sci. USA,* 1993, vol. 90, 5873-5877
- **ALTSCHUL et al.** *J. MoT Biol.,* 1990, vol. 215, 403-410
- **ALTSCHUL et al.** *Nucleic Acids Res.,* 1997, vol. 25, 3389-3402
- **SHAH et al.** *Curr. Opin. Drug Discov. Devel.,* 2010, vol. 13, 466-80
- **HART et al.** *Mov. Disord.,* 2009, vol. 24, 647-54

- **SIDMAN et al.** *Biopolymers,* 1983, vol. 22, 547
- **LANGER et al.** *J. Biomed. Mater. Res.,* 1981, vol. 15, 167
- **LANGER.** *Chem. Tech.,* 1982, vol. 12, 98
- **EPSTEIN et al.** *Proc. Natl. Acad. Sci. U.S.A.,* 1985, vol. 82, 3688
- **HWANG et al.** *Proc. Natl. Acad. Sci. U.S.A.,* 1980, vol. 77, 4030
- **CHAPMAN.** *Adv. Drug Deliv. Rev.,* 2002, vol. 54, 531-545
- **CUNNINGHAM-RUNDLES.** *J. Immunol. Meth.,* 1992, vol. 152, 177-190
- **F. C. KULL et al.** *Applied Microbiology,* 1961, vol. 9, 538
- **LIPPINCOTT.** Remington: The Science and Practice of Pharmacy. Williams and Wilkins, 2000
- **DRANOFF et al.** *Cancer Res,* 1985
- **AHLUWALIA, G. S. ; GREM, J. L. ; HAO, Z. ; COONEY, D. A.** Metabolism and action of amino acid analog anti-cancer agents. *Pharmacol Ther,* 1990, vol. 46, 243-271
- **ALT, J. ; POTTER, M. C. ; ROJAS, C. ; SLUSHER, B. S.** Bioanalysis of 6-diazo-5-oxo-l-norleucine in plasma and brain by ultra-performance liquid chromatography mass spectrometry. *Anal Biochem,* 2015, vol. 474, 28-34
- **BARCLAY, R. K. ; PHILLIPPS, M. A.** Effects of 6-diazo-5-oxol-norleucine and other tumor inhibitors on the biosynthesis of nicotinamide adenine dinucleotide in mice. *Cancer Res,* 1966, vol. 26, 282-286
- **CERVANTES-MADRID, D. ; ROMERO, Y. ; DUENAS-GONZALEZ, A.** Reviving Lonidamine and 6-Diazo-5-oxo-L-norleucine to Be Used in Combination for Metabolic Cancer Therapy. *Biomed Res Int,* 2015, vol. 2015, 690492
- **COFFEY, G. L. ; EHRLICH, J. ; FISHER, M. W. ; HILLEGAS, A. B. ; KOHBERGER, D. L. ; MACHAMER, H. E. ; RIGHTSEL, W. A. ; ROEGNER, F. R.** 6-Diazo-5-oxo-L-norleucine, a new tumor-inhibitory substance. I. Biologic studies. *Antibiot Chemother (Northfield),* 1956, vol. 6, 487-497
- **D'ANDREA, S. ; ZHENG, Z. ; SCOLA, P.** Inhibitors of Hepatitis C Virus. *Google Patents,* 2008
- **DION, H. W. ; FUSARI, S. A. ; JAKUBOWSKI, Z. L. ; ZORA, J. G. ; BARTZ, Q. R.** 6-Diazo-5-oxo-L-norleucine, A New Tumor-inhibitory Substance. II.1 Isolation and Characterization. *J. Am. Chem. Soc.,* 1956, vol. 78, 3075-3077
- **DOLAN D.E. ; GUPTA S.** PD-1 pathway inhibitors: changing the landscape of cancer immunotherapy. *Cancer Control,* 2014, vol. 21 (3), 231-7
- **DRANOFF, G. ; ELION, G. B. ; FRIEDMAN, H. S. ; BIGNER, D. D.** Combination chemotherapy in vitro exploiting glutamine metabolism of human glioma and medulloblastoma. *Cancer Res,* 1985, vol. 45, 4082-4086
- **DRANOFF, G. ; ELION, G. B. ; FRIEDMAN, H. S. ; CAMPBELL, G. L. ; BIGNER, D. D.** Influence of glutamine on the growth of human glioma and medulloblastoma in culture. *Cancer Res,* 1985, vol. 45, 4077-4081
- **EAGAN, R. T. ; FRYTAK, S. ; NICHOLS, W. C. ; CREAGAN, E. T. ; INGLE, J. N.** Phase II study on DON in patients with previously treated advanced lung cancer. *Cancer Treat Rep,* 1982, vol. 66, 1665-1666
- **EARHART, R. H. ; AMATO, D. J. ; CHANG, A. Y. ; BORDEN, E. C. ; SHIRAKI, M. ; DOWD, M. E. ; COMIS, R. L. ; DAVIS, T. E. ; SMITH, T. J.** Phase II trial of 6-diazo-5-oxo-L-norleucine versus aclacinomycin-A in advanced sarcomas and mesotheliomas. *Invest New Drugs,* 1990, vol. 8, 113-119
- **EARHART, R. H. ; KOELLER, J. M. ; DAVIS, H. L.** Phase I trial of 6-diazo-5-oxo-L-norleucine (DON) administered by 5-day courses. *Cancer Treat Rep,* 1982, vol. 66, 1215-1217
- **ENGELS, E.A. et al.** Spectrum of Cancer Risk among U.S. Solid Organ Transplant Recipients: The Transplant Cancer Match Study. *JAMA,* 2011
- **ERICKSON, J. W. ; CERIONE, R. A.** Glutaminase: a hot spot for regulation of cancer cell metabolism?. *Oncotarget,* 2010, vol. 1, 734-740
- **ESHLEMAN, J. S. ; CARLSON, B. L. ; MLADEK, A. C. ; KASTNER, B. D. ; SHIDE, K. L. ; SARKARIA, J. N.** Inhibition of the mammalian target of rapamycin sensitizes U87 xenografts to fractionated radiation therapy. *Cancer Res,* 2002, vol. 62, 7291-7297
- **FOGAL, V. ; BABIC, I. ; CHAO, Y. ; PASTORINO, S. ; MUKTHAVARAM, R. ; JIANG, P. ; CHO, Y. J. ; PINGLE, S. C. ; CRAWFORD, J. R. ; PICCIONI, D. E.** Mitochondrial p32 is upregulated in Myc expressing brain cancers and mediates glutamine addiction. *Oncotarget,* 2015, vol. 6, 1157-1170
- **GALLOP, M. A. ; XU, F. ; PHAN, T. ; DILIP, U. ; PENG, G.** Acyloxyalkyl carbamate prodrugs, methods of synthesis and use. *In Google Patents,* 2008
- **GRAYZEL, A. I. ; SEEGMILLER, J. E. ; LOVE, E.** Suppression of uric acid synthesis in the gouty human by the use of 6-diazo-5-oxo-L-norleucine. *J Clin Invest,* 1960, vol. 39, 447-454
- **GROSS, M. I. ; DEMO, S. D. ; DENNISON, J. B. ; CHEN, L. ; CHERNOV-ROGAN, T. ; GOYAL, B. ; JANES, J. R. ; LAIDIG, G. J. ; LEWIS, E. R. ; LI, J.** Antitumor Activity of the Glutaminase Inhibitor CB-839 in Triple-Negative Breast Cancer. *Mol Cancer Ther,* 2014

- **HARDING, J. J. T., M.L. ; MUNSTER, P.N. ; LE, M.H. ; MOLINEAUX, C. ; BENNETT, M.K. ; MITTRA, E. ; BURRIS,H.A. ; CLARK, A.S. ; DUNPHY, M. ; MERIC-BERNSTAM, F.** Safety and tolerability of increasing doses of CB-839, a first-in-class, orally administered small molecule inhibitor of glutaminase, in solid tumors. *J Clin Oncol,* 2015
- **HENSLEY, C. T. ; WASTI, A. T. ; DEBERARDINIS, R. J.** Glutamine and cancer: cell biology, physiology, and clinical opportunities. *J Clin Invest,* 2013, vol. 123, 3678-3684
- **HOFER, A. ; STEVERDING, D. ; CHABES, A. ; BRUN, R. ; THELANDER, L.** Trypanosoma brucei CTP synthetase: a target for the treatment of African sleeping sickness. *Proc Natl Acad Sci USA,* 2001, vol. 98, 6412-6416
- **HU, X. ; STERN, H. M. ; GE, L. ; O'BRIEN, C. ; HAYDU, L. ; HONCHELL, C. D. ; HAVERTY, P. M. ; PETERS, B. A. ; WU, T. D. ; AMLER, L. C.** Genetic alterations and oncogenic pathways associated with breast cancer subtypes. *Mol Cancer Res,* 2009, vol. 7, 511-522
- **KEICHER, J. D. ; ROBERTS, C. D. ; RAJWANSHI, V. K. ; GRIFFITH, R. C. ; ZHENG, X. ; LIEHR, S. J. R. ; PRHAVC, M. ; KIM, C. U. ; RAY, A. S.** Amino tricyclic-nucleoside compounds, compositions, and methods of use. *In Google Patents,* 2009
- In Phase 1 study: Safety and tolerability of increasing doses of cb-839, an orally-administered small molecule inhibitor of glutaminase, in acute leukemia. **KONOPLEVA, M. Y. ; FLINN, I. W. ; WANG, E. ; DINARDO, C. D. ; BENNETT, M. ; MOLINEAUX, C. ; LE, M. ; MARIS, M. ; FRANKFURT, O.** Haematologica. Ferrata Storti Foundation Via Giuseppe Belli, 2015, 378-379
- **LE, A. ; LANE, A. N. ; HAMAKER, M. ; BOSE, S. ; GOUW, A. ; BARBI, J. ; TSUKAMOTO, T. ; ROJAS, C. J. ; SLUSHER, B. S. ; ZHANG, H.** Glucose-independent glutamine metabolism via TCA cycling for proliferation and survival in B cells. *Cell Metab,* 2012, vol. 15, 110-121
- **LEE, Y. Z. ; YANG, C. W. ; CHANG, H. Y. ; HSU, H. Y. ; CHEN, I. S. ; CHANG, H. S. ; LEE, C. H. ; LEE, J. C. ; KUMAR, C. R. ; QIU, Y. Q.** Discovery of selective inhibitors of Glutaminase-2, which inhibit mTORC1, activate autophagy and inhibit proliferation in cancer cells. *Oncotarget,* 2014, vol. 5, 6087-6101
- **LIDDY et al.** *Nature Med.,* 2012, vol. 18, 980-7
- **GRUPP et al.** *New England J. Med.,* 2013, vol. 368, 1509-18
- **LYNCH, G. ; KEMENY, N. ; CASPER, E.** Phase II evaluation of DON (6-diazo-5-oxo-L-norleucine) in patients with advanced colorectal carcinoma. *Am J Clin Oncol,* 1982, vol. 5, 541-543
- **MAGILL, G. B. ; MYERS, W. P.** Alterations in calcium metabolism in cancer patients treated with 6-diazo-5-oxo-L-norleucine. *Proc Soc Exp Biol Med,* 1956, vol. 93, 314-318
- **MAGILL, G. B. ; MYERS, W. P. ; REILLY, H. C. ; PUTNAM, R. C. ; MAGILL, J. W. ; SYKES, M. P. ; ESCHER, G. C. ; KARNOFSKY, D. A. ; BURCHENAL, J. H.** Pharmacological and initial therapeutic observations on 6-diazo-5-oxo-1-norleucine (DON) in human neoplastic disease. *Cancer,* 1957, vol. 10, 1138-1150
- **MCDERMOTT, L. A. ; IYER, P. ; VERNETTI, L. ; RIMER, S. ; SUN, J. ; BOBY, M. ; YANG, T. ; FIORAVANTI, M. ; O'NEILL, J. ; WANG, L.** Design and evaluation of novel glutaminase inhibitors. *Bioorg Med Chem,* 2016, vol. 24, 1819-1839
- **NGIOW S.F. ; TENG M.W. ; SMYTH M.J.** Prospects for TIM3-Targeted Antitumor Immunotherapy. *Cancer Res,* 2011, vol. 71 (21), 6567-71
- **OSTROM, Q. T. ; GITTLEMAN, H. ; FULOP, J. ; LIU, M. ; BLANDA, R. ; KROMER, C. ; WOLINSKY, Y. ; KRUCHKO, C. ; BARNHOLTZ-SLOAN, J. S.** CBTRUS Statistical Report: Primary Brain and Central Nervous System Tumors Diagnosed in the United States in 2008-2012. *Neuro Oncol,* 2015, vol. 17 (4), iv1-iv62
- **POTTER, M. C. ; BAXTER, V. K. ; MATHEY, R. W. ; ALT, J. ; ROJAS, C. ; GRIFFIN, D. E. ; SLUSHER, B. S.** Neurological sequelae induced by alphavirus infection of the CNS are attenuated by treatment with the glutamine antagonist 6-diazo-5-oxo-l-norleucine. *J Neurovirol,* 2015, vol. 21, 159-173
- **POWELL et al.** A2aR antagonists: Next generation checkpoint blockade for cancer immunotherapy. *Comput StructBiotechnol J.,* 2015, vol. 13, 265-272
- **RAHMAN, A. ; SMITH, F. P. ; LUC, P. T. ; WOOLLEY, P. V.** Phase I study and clinical pharmacology of 6-diazo-5-oxo-L-norleucine (DON). *Invest New Drugs,* 1985, vol. 3, 369-374
- **RAUTIO, J. ; KUMPULAINEN, H. ; HEIMBACH, T. ; OLIYAI, R. ; OH, D. ; JARVINEN, T. ; SAVOLAINEN, J.** Prodrugs: design and clinical applications. *Nat Rev Drug Discov,* 2008, vol. 7, 255-270
- **RU, P. ; WILLIAMS, T. M. ; CHAKRAVARTI, A. ; GUO, D.** Tumor metabolism of malignant gliomas. *Cancers (Basel),* 2013, vol. 5, 1469-1484
- **SCHULZE, A. ; HARRIS, A. L.** How cancer metabolism is tuned for proliferation and vulnerable to disruption. *Nature,* 2012, vol. 491, 364-373
- **SHUKLA, K. ; FERRARIS, D. V. ; THOMAS, A. G. ; STATHIS, M. ; DUVALL, B. ; DELAHANTY, G. ; ALT, J. ; RAIS, R. ; ROJAS, C. ; GAO, P.** Design, synthesis, and pharmacological evaluation of bis-2-(5-phenylacetamido-1,2,4-thiadiazol-2-yl)ethyl sulfide 3 (BPTES) analogs as glutaminase inhibitors. *J Med Chem,* 2012, vol. 55, 10551-10563

- **SKLAROFF, R. B. ; CASPER, E. S. ; MAGILL, G. B. ; YOUNG, C. W.** Phase I study of 6-diazo-5-oxo-L-norleucine (DON. *Cancer Treat Rep,* 1980, vol. 64, 1247-1251
- **STUPP, R. ; HEGI, M. E. ; MASON, W. P. ; VAN DEN BENT, M. J. ; TAPHOORN, M. J. ; JANZER, R. C. ; LUDWIN, S. K. ; ALLGEIER, A. ; FISHER, B. ; BELANGER, K.** Effects of radiotherapy with concomitant and adjuvant temozolomide versus radiotherapy alone on survival in glioblastoma in a randomised phase III study: 5-year analysis of the EORTC-NCIC trial. *Lancet Oncol,* 2009, vol. 10, 459-466
- **STUPP, R. ; MASON, W. P. ; VAN DEN BENT, M. J. ; WELLER, M. ; FISHER, B. ; TAPHOORN, M. J. ; BELANGER, K. ; BRANDES, A. A. ; MAROSI, C. ; BOGDAHN, U.** Radiotherapy plus concomitant and adjuvant temozolomide for glioblastoma. *N Engl J Med,* 2005, vol. 352, 987-996
- **SULLIVAN, M. P. ; BEATTY, E. C., JR. ; HYMAN, C. B. ; MURPHY, M. L. ; PIERCE, M. I. ; SEVERO, N. C.** A comparison of the effectiveness of standard dose 6-mercaptopurine, combination 6-mercaptopurine and DON, and high-loading 6-mercaptopurine therapies in treatment of the acute leukemias of childhood: results of a coperative study. *Cancer Chemother Rep,* 1962, vol. 18, 83-95
- **SULLIVAN, M. P. ; NELSON, J. A. ; FELDMAN, S. ; VAN NGUYEN, B.** Pharmacokinetic and phase I study of intravenous DON (6-diazo-5-oxo-L-norleucine) in children. *Cancer Chemother Pharmacol,* 1988, vol. 21, 78-84
- **TANAKA, K. ; SASAYAMA, T. ; IRINO, Y. ; TAKATA, K. ; NAGASHIMA, H. ; SATOH, N. ; KYOTANI, K. ; MIZOWAKI, T. ; IMAHORI, T. ; EJIMA, Y.** Compensatory glutamine metabolism promotes glioblastoma resistance to mTOR inhibitor treatment. *J Clin Invest,* 2015, vol. 125, 1591-1602
- **THANGAVELU, K. ; CHONG, Q. Y. ; LOW, B. C. ; SIVARAMAN, J.** Structural basis for the active site inhibition mechanism of human kidney-type glutaminase (KGA). *Sci Rep,* 2014, vol. 4, 3827
- **UPADHYAY, R. K.** Drug delivery systems, CNS protection, and the blood brain barrier. *Biomed Res Int,* 2014, vol. 2014, 869269
- **WELLER, M. ; VAN DEN BENT, M. ; HOPKINS, K. ; TONN, J. C. ; STUPP, R. ; FALINI, A. ; COHEN-JONATHAN-MOYAL, E. ; FRAPPAZ, D. ; HENRIKSSON, R. ; BALANA, C.** EANO guideline for the diagnosis and treatment of anaplastic gliomas and glioblastoma. *Lancet Oncol,* 2014, vol. 15, e395-403
- **WILLIS, R. C. ; SEEGMILLER, J. E.** The inhibition by 6-diazo-5-oxo-1-norleucine of glutamine catabolism of the cultured human lymphoblast. *J Cell Physiol,* 1977, vol. 93, 375-382
- **WINDMUELLER, H. G. ; SPAETH, A. E.** Uptake and metabolism of plasma glutamine by the small intestine. *J Biol Chem,* 1974, vol. 249, 5070-5079
- **WISE, D. R. ; THOMPSON, C. B.** Glutamine addiction: a new therapeutic target in cancer. *Trends Biochem Sci,* 2010, vol. 35, 427-433